# EUROPEAN PATENT APPLICATION

(11) **EP 2 141 214 A2**
(43) Date of publication of application: **06.01.2010**
(21) Application number: 09155969.0
(22) Date of filing: 24.03.2009
(51) Int. Cl.: C09K 11/06, H01L 51/50, H05B 33/14

(54) **Novel organic electroluminescent compounds and organic electroluminescent device using the same**

(30) Priority: 25.06.2008 KR 20080060376
(71) Applicant: Gracel Display Inc., Seoul 133-833 (KR)
(72) Inventor: Kim, Chi-Sik, 156-825, Seoul (KR); Cho, Young Jun, 136-060, Seoul (KR); Kwon, Hyuck Joo, 130-100, Seoul (KR); Kim, Bong Ok, 135-090, Seoul (KR); Kim, Sung Min, 157-886, Seoul (KR); Yoon, Seung Soo, 135-884, Seoul (KR)
(74) Representative: Kent, Venetia Katherine

(57) **Abstract**

Provided are novel organic electroluminescent compounds **characterized in that** they are represented by Chemical Formula (1): 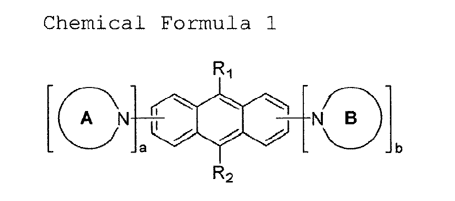 wherein, ring A and ring B independently represent a saturated 5- or 6-membered heterocyclic amino group, or a substituent selected from the following structures: 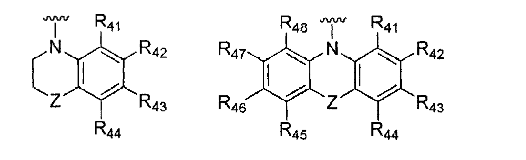 wherein the saturated 5- or 6-membered heterocyclic amino group may further comprise one or more heteroatom(s) selected from NR₃₆, O and S; and
a and b independently represent an integer from 0 to 4, provided that (a + b) is an integer from 1 to 4.

## Description

### FIELD OF THE INVENTION

The present invention relates to novel organic electroluminescent compounds, and organic electroluminescent devices employing the same in an electroluminescent layer. More specifically, the invention relates to novel organic electroluminescent compounds employed as green electroluminescent material, and organic electroluminescent devices employing the compounds as dopant.

### BACKGROUND OF THE INVENTION

The most important factor in developing organic electroluminescent devices of high efficiency and long life is development of electroluminescent material of high performance. In view of current development of electroluminescent material, green electroluminescent materials show superior electroluminescent property to red or blue electroluminescent materials. However, conventional green electroluminescent materials still have many problems to achieve manufacturing panels of large size with low power consumption. In fact, in view of efficiency and life, various kinds of electroluminescent materials for green have been reported up to now. Though they exhibit from 2 to 5 times of electroluminescent property as compared to red or blue electroluminescent materials, development of green electroluminescent material is getting challenged by the improvement of properties of red or blue electroluminescent material. In the meanwhile, enhancement of lifetime of green material is still insufficient, so that a green electroluminescent material providing long life is seriously required.

As green fluorescent material, a coumarin derivative (Compound D), quinacrydone derivatives (Compound E), DPT (Compound F) and the like have been known. Compound D is the structure of C545T that is the most widely used coumarin derivative up to the present. In general, those materials are doped, by using Alq as the host, at a concentration of several % to about several ten %, to form an electroluminescent device.

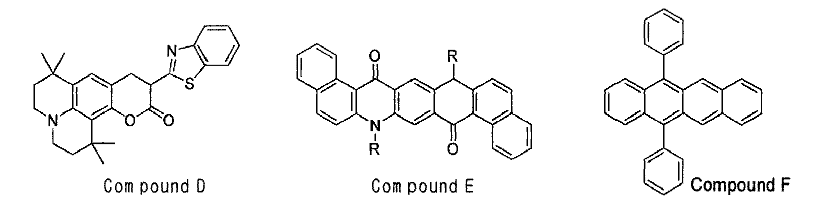

Japanese Patent Laid-Open No. 2001-131541 discloses bis(2,6-diarylamino)-9,10-diphenylanthracene derivatives represented by Compound G shown below, wherein diarylamino groups are directly substituted at 2- and 6-position of anthracene, respectively.

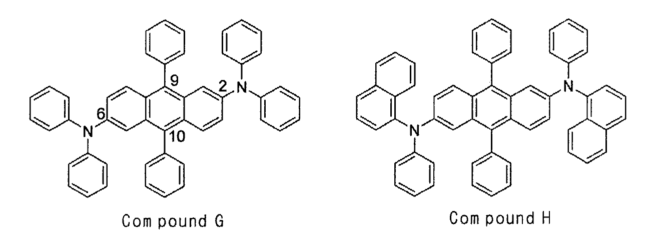

Japanese Patent Laid-Open No. 2003-146951 (which discloses compounds for a hole transport layer) does not mention the compounds wherein diarylamino groups are directly substituted at 2- and 6-position of anthracene, respectively, but simply describing the compounds having phenyl substituents at 9- and 10-position of anthracene. As considering that Japanese Patent Laid-Open No. 2003-146951 indicated the problem of Compound (H) (wherein diarylamino groups are directly substituted at 2- and 6-position of the anthracene ring, respectively) having poor luminous efficiency, it is found that the invention of Japanese Patent Laid-Open No. 2003-146951 did not recognize the compounds other than those having phenyl substituents at 9- and 10-position of anthracene.

In the meanwhile, Japanese Patent Laid-Open No. 2004-91334 suggested the organic electroluminescent compounds represented by Compound (J), which overcomes poor luminous efficiency of conventional compounds but exhibits low ionization potential and excellent hole transportation, by further substituting the aryl group of the diarylamino group with diarylamino groups, even though diarylamino groups are directly substituted on the anthracene group.

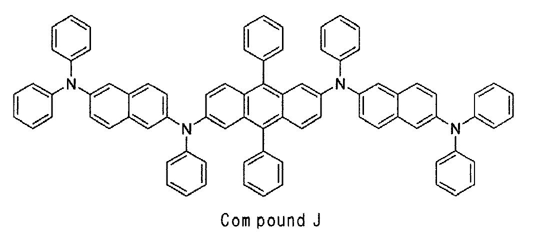

The compounds suggested by Japanese Patent Laid-Open No. 2004-91334 (applied as a hole transport layer), however, show the problem of shortened operation life as a hole transport layer because of too many amine functional groups, even though they showed lowered ionization potential due to many amine functional groups and overcame the problem of increase in hole transporting property.

### SUMMARY OF THE INVENTION

Thus, the inventors have intensively endeavored to overcome the problems of prior arts as described above and to develop novel electroluminescent compounds which can realize an organic electroluminescent device having excellent luminous efficiency with noticeably prolonged life.

The object of the present invention is to provide organic electroluminescent compounds having the backbone to provide excellent luminous efficiency with very good device life as compared to conventional dopant material, and to provide organic electroluminescent devices with high efficiency and long life which employ said organic electroluminescent compounds.

The present invention relates to organic electroluminescent compounds represented by Chemical Formula (1), and organic electroluminescent devices comprising the same. Since organic electroluminescent compounds according to the invention exhibit high luminous efficiency and excellent color purity and life property of material, an OLED device having very good operation life can be advantageously prepared therefrom. 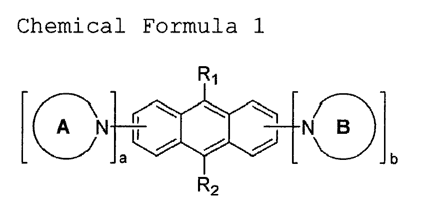 wherein, R₁ and R₂ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or a substituent selected from the following structures: 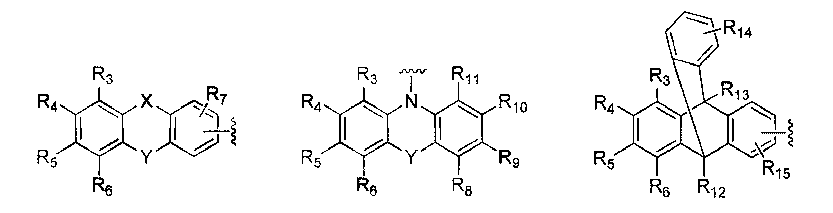 wherein, R₃ through R₁₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₃ through R₁₅ may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, or - (CR₂₁R₂₂)ₙ-, -N(R₂₃)-, -S-, -O-, -Si(R₂₄)(R₂₅)-, -P(R₂₆)-, -C(=O)-, -B(R₂₇)-, -In(R₂₈)-, -Se-, -Ge(R₂₉)(R₃₀)-, -Sn(R₃₁)(R₃₂)-, Ga(R₃₃)- or -(R₃₄)C=C(R₃₅)-;
R₂₁ through R₃₅ independently represent hydrogen, halogen, (C1-C60)alkyl, (C6-C60)aryl, (C3-C60)heteroaryl, 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₂₁ and R₂₂, R₂₄ and R₂₅, R₂₉ and R₃₀, R₃₁ and R₃₂, or R₃₄ and R₃₅ may be linked via (C3-C60)alkylene or (C3-C60)alkenylene with or without a fused ring to form an alicyclic ring, or a monocyclic or polycyclic aromatic ring;
ring A and ring B independently represent a saturated 5-or 6-membered heterocyclic amino group, which may further contain one or more heteroatom(s) selected from NR₃₆, O and S, or a substituent selected from the following structures: 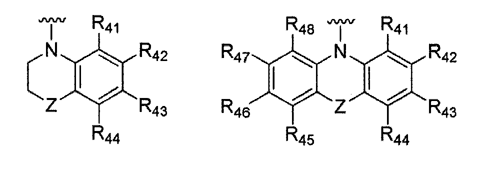 R₃₆ represents hydrogen, halogen, (C1-C60)alkyl, (C6-C60)aryl, (C3-C60)heteroaryl, (C3-C60)cycloalkyl, adamantyl, (C7-C60)bicycloalkyl or (C6-C60)ar(C1-C60)alkyl;
R₄₁ through R₄₈ independently represent hydrogen, halogen, (C1-C60)alkyl, (C6-C60)aryl, (C3-C60)heteroaryl, 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₄₁ through R₄₈ may be linked to an adjacent substituent via (C3-C60)alkylene or (C3-C60)alkenylene with or without a fused ring to form an alicyclic ring, or a monocyclic or polycyclic aromatic ring;
Z represents a chemical bond, or -(CR₅₁R₅₂)ₘ-, -N(R₅₃)-, - S-, -O-, -Si(R₅₄)(R₅₅)-, -P(R₅₆)-, -C(=O)-, -B(R₅₇)-, -In(R₅₈)-, - Se-, -Ge(R₅₉)(R₆₀)-, -Sn(R₆₁)(R₆₂)-, -Ga(R₆₃)- or -(R₆₄)C=C(R₆₅)-;
wherein, R₅₁ through R₆₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₅₁ and R₅₂, R₅₄ and R₅₅, R₅₉ and R₆₀, R₆₁ and R₆₂, or R₆₄ and R₆₅ may be linked via (C3-C60)alkylene or (C3-C60)alkenylene with or without a fused ring to form an alicyclic ring, or a monocyclic or polycyclic aromatic ring;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₁, R₂, R₃ through R₁₅, R₂₁ through R₃₆, R₄₁ through R₄₈, and R₅₁ through R₆₅ may be further substituted by one or more substituent(s) selected from halogen, (C1-C60)alkyl with or without halogen substituent(s), (C6-C60)aryl, (C3-C60)heteroaryl with or without (C6-C60)aryl substituent(s), 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
m and n independently represent an integer from 1 to 4; and
a and b independently represent an integer from 0 to 4, provided that (a + b) is an integer from 1 to 4.

### BRIEF DESCRIPTION OF THE DRAWINGS

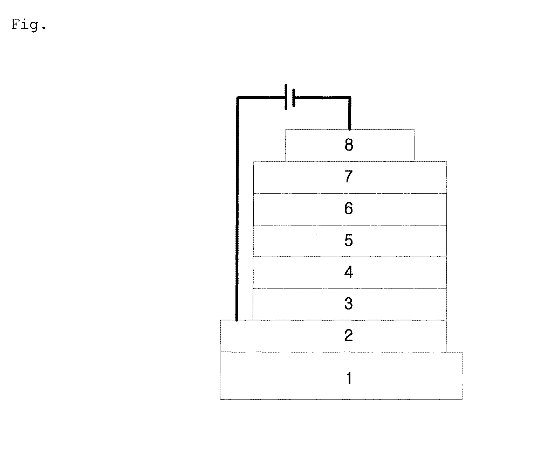

Fig. 1 is a cross-sectional view of an organic light emitting diode (OLED).

### DETAILED DESCRIPTION OF THE INVENTION

Referring now to the Drawings, Fig. 1 illustrates a cross-sectional view of an OLED of the present invention comprising a Glass 1, Transparent electrode 2, Hole injecting layer 3, Hole transport layer 4, Electroluminescent layer 5, Electron transport layer 6, Electron injecting layer 7 and Al cathode 8.

The term "alkyl", "alkoxy" and other subsituents containing "alkyl" moiety include both linear and branched species.

The term "aryl" described herein means an organic radical derived from aromatic hydrocarbon via elimination of one hydrogen atom. Each ring suitably comprises a monocyclic or fused ring system containing from 4 to 7, preferably from 5 to 6 cyclic atoms. Specific examples include phenyl, naphthyl, biphenyl, anthryl, indenyl, fluorenyl, phenanthryl, triphenylenyl, pyrenyl, perylenyl, chrysenyl, naphthacenyl and fluoranthenyl, but they are not restricted thereto.

The term "heteroaryl" described herein means an aryl group containing from 1 to 4 heteroatom(s) selected from N, O and S for the aromatic cyclic backbone atoms, and carbon atom(s) for remaining aromatic cyclic backbone atoms. The heteroaryl may be 5- or 6-membered monocyclic heteroaryl or a polycyclic heteroaryl which is fused with one or more benzene ring(s), and may be partially saturated. The heteroaryl groups may include divalent aryl groups of which the heteroatoms are oxidized or quarternized to form N-oxides, quaternary salts, or the like. Specific examples include monocyclic heteroaryl groups such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; and polycyclic heteroaryl groups such as benzofuranyl, benzothiophenyl, isobenzofuranyl, benzimidazolyl, benzothiazolyl, benzisothiazolyl, benzisoxazolyl, benzoxazolyl, isoindolyl, indolyl, indazolyl, benzothiadiazolyl, quinolyl, isoquinolyl, cinnolinyl, quinazolinyl, quinolizinyl, quinoxalinyl, carbazolyl, phenanthridinyl and benzodioxolyl, and corresponding N-oxides (e.g., pyridyl N-oxides, quinolyl N-oxides) and quaternary salts thereof; but they are not restricted thereto.

The expression "saturated 5- or 6-membered heterocyclic amino" described herein denotes the compounds containing nitrogen in the 5- or 6-membered ring comprised of saturated bonds, and the compound may further contain one or more heteroatom(s) selected from N, O and S.

The substituents comprising "(C1-C60)alkyl" moiety described herein may contain 1 to 60 carbon atoms, 1 to 20 carbon atoms, or 1 to 10 carbon atoms. The substituents comprising "(C6-C60)aryl" moiety may contain 6 to 60 carbon atoms, 6 to 20 carbon atoms, or 6 to 12 carbon atoms. The substituents comprising "(C3-C60)heteroaryl" moiety may contain 3 to 60 carbon atoms, 4 to 20 carbon atoms, or 4 to 12 carbon atoms. The substituents comprising "(C3-C60)cycloalkyl" moiety may contain 3 to 60 carbon atoms, 3 to 20 carbon atoms, or 3 to 7 carbon atoms. The substituents comprising "(C2-C60)alkenyl or alkynyl" moiety may contain 2 to 60 carbon atoms, 2 to 20 carbon atoms, or 2 to 10 carbon atoms.

The organic electroluminescent compounds according to the present invention may be selected from the compounds represented by one of Chemical Formulas (2) to (5): 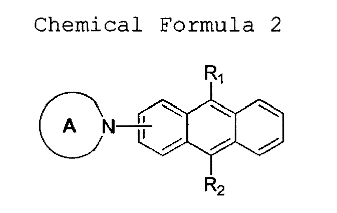 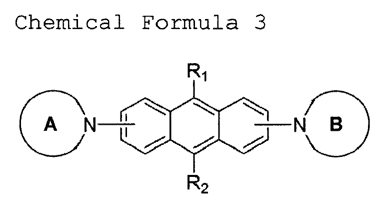 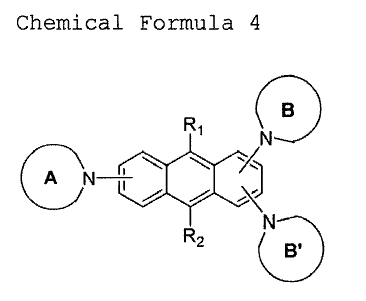 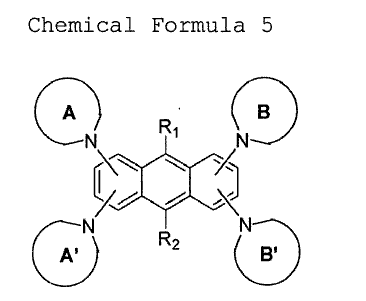 wherein, R₁ and R₂ are defined as in Chemical Formula (1);
ring A, ring A', ring B and ring B' independently represent a saturated 5- or 6-membered heterocyclic amino group, which may further contain one or more heteroatom(s) selected from NR₃₆, O and S, or a substituent selected from the following structures:

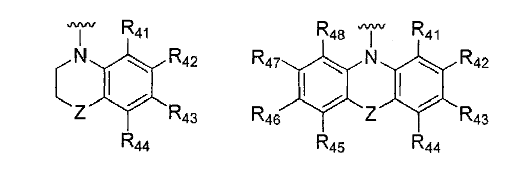

R₃₆ represents hydrogen, halogen, (C1-C60)alkyl, (C6-C60)aryl, (C3-C60)heteroaryl, (C3-C60)cycloalkyl, adamantyl, (C7-C60)bicycloalkyl or (C6-C60)ar(C1-C60)alkyl;
R₄₁ through R₄₈ independently represent hydrogen, halogen, (C1-C60)alkyl, (C6-C60) aryl, (C3-C60)heteroaryl, 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₄₁ through R₄₈ may be linked to an adjacent substituent via (C3-C60)alkylene or (C3-C60)alkenylene with or without a fused ring to form an alicyclic ring, or a monocyclic or polycyclic aromatic ring;
Z represents a chemical bond, or -(CR₅₁R₅₂)ₘ-, -N(R₅₃)-, - S-, -O-, -Si(R₅₄)(R₅₅)-, -P(R₅₆)-, -C(=O)-, -B(R₅₇)-, -In(R₅₈)-, - Se-, -Ge(R₅₉)(R₆₀)-, -Sn(R₆₁)(R₆₂)-, -Ga(R₆₃)- or (R₆₄)C=C(R₆₅)-;
R₅₁ through R₆₅ independently represent hydrogen, halogen, (C1-C60)alkyl, (C6-C60)aryl, (C3-C60)heteroaryl, 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₅₁ and R₅₂, R₅₄ and R₅₅, R₅₉ and R₆₀, R₆₁ and R₆₂, or R₆₄ and R₆₅ may be linked via (C3-C60)alkylene or (C3-C60)alkenylene with or without a fused ring to form an alicyclic ring, or a monocyclic or polycyclic aromatic ring;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₃₆, R₄₁ through R₄₈, and R₅₁ through R₆₅ may be further substituted by one or more substituent(s) selected from halogen, (C1-C60)alkyl with or without halogen substituent(s), (C6-C60)aryl, (C3-C60)heteroaryl with or without (C6-C60)aryl substituent(s), 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
m is an integer from 1 to 4.

In the chemical formulas, ring A, ring A', ring B and ring B' are independently selected from the following structures, but not restricted thereto.

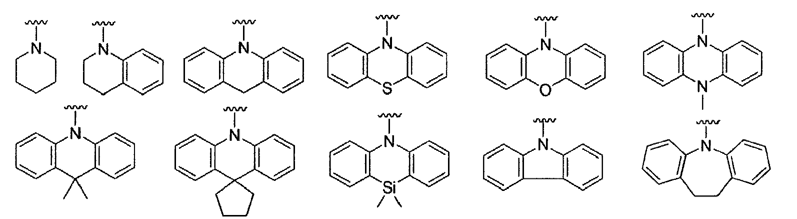

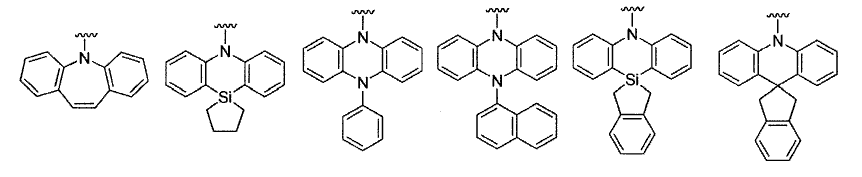

In the chemical formulas, R₁ and R₂ independently represent methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, benzyl, trifluoromethyl, perfluoroethyl, trifluoroethyl, perfluoropropyl, perfluorobutyl, methoxy, ethoxy, n-propoxy, i-propoxy, n-butoxy, i-butoxy, t-butoxy, n-pentoxy, i-pentoxy, n-hexyloxy, n-heptoxy, trimethylsilyl, triethylsilyl, tripropylsilyl, tri(t-butyl)silyl, t-butyldimethylsilyl, dimethylphenylsilyl, triphenylsilyl, cyclopropyl, cyclobutyl, cyclopentyl, cyclohexyl, cycloheptyl, cyclooctyl, cyclononyl, cyclodecyl, morpholino, thiomorpholino, adamantyl, bicyclo[2.2.1]heptyl, bicyclo[2.2.2]octyl, bicyclo[3.2.1]octyl, bicyclo[5.2.0]nonyl, bicyclo[4.2.2]decyl, bicyclo[2.2.2]octyl, 4-pentylbicyclo[2.2.2]octyl, ethenyl, phenylethenyl, ethynyl, phenylethynyl, cyano, dimethylamino, diphenylamino, monomethylamino, monophenylamino, phenyloxy, phenylthio, methoxycarbonyl, ethoxycarbonyl, t-butoxycarbonyl, carboxyl, nitro, chloro, fluoro or hydroxyl, or they are selected from the following structures, but not restricted thereto: 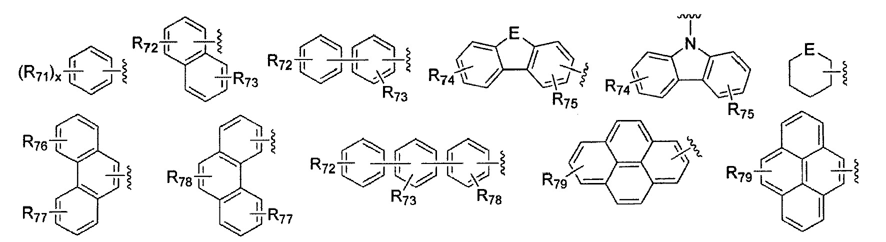 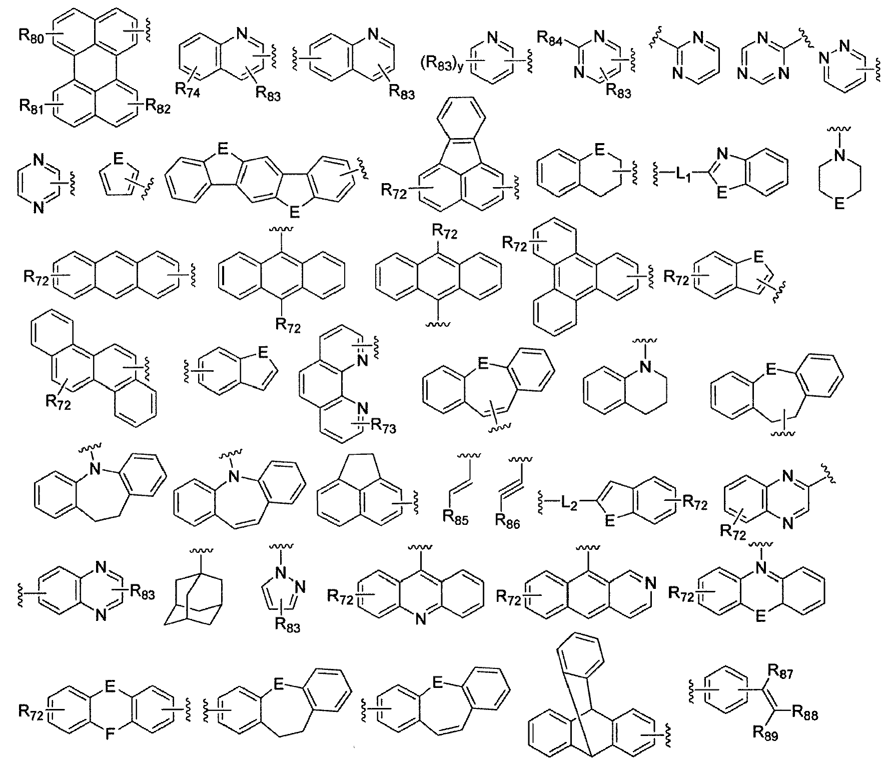 wherein, R₇₁ through R₈₆ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl; the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₇₁ through R₈₆ may be further substituted by halogen, (C1-C60)alkyl, (C6-C60)aryl, (C3-C60)heteroaryl, 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
R₈₇ through R₈₉ independently represent hydrogen, halogen, (C1-C60)alkyl with or without halogen substituent(s), (C6-C60)aryl, (C3-C60)heteroaryl with or without (C6-C60)aryl substituent(s), 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
L₁ and L₂ independently represent a chemical bond, (C6-C60)arylene or (C3-C60)heteroarylene, wherein the arylene or heteroarylene of L₁ and L₂ may be substituted by one or more substituent(s) selected from (C1-C60)alkyl, halogen, cyano, (C1-C60)alkoxy, (C1-C60)alkylthio, (C3-C60)cycloalkyl, (C6-C60)aryl, (C3-C60)heteroaryl, adamantyl, (C7-C60)bicycloalkyl, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, tri(C1-C30)alkylsilyl, di(C1-C30)alkyl(C6-C30)arylsilyl and tri(C6-C30)arylsilyl;
E and F independently represent -C(R₉₁)(R₉₂)-, -N(R₉₃)-, - S-, -O-, -Si(R₉₄)(R₉₅)-, -P(R₉₆)-, -C(=O)-, -B(R₉₇)-, -In(R₉₈)-, - Se-, -Ge(R₉₉)(R₁₀₀)-, -Sn(R₁₀₁)(R₁₀₂)- or -Ga(R₁₀₃)-;
R₉₁ through R₁₀₃ independently represent hydrogen, halogen, (C1-C60)alkyl, (C6-C60)aryl, (C3-C60)heteroaryl, 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₉₁ and R₉₂, R₉₄ and R₉₅, R₉₉ and R₁₀₀, and R₁₀₁ and R₁₀₂ may be linked via (C3-C60)alkylene or (C3-C60)alkenylene with or without a fused ring to form an alicyclic ring, or a monocyclic or polycyclic aromatic ring;
x is an integer from 1 to 5; and
y is an integer from 1 to 4.

More specifically, R₁ and R₂ are independently selected from the following structures, but not restricted thereto.

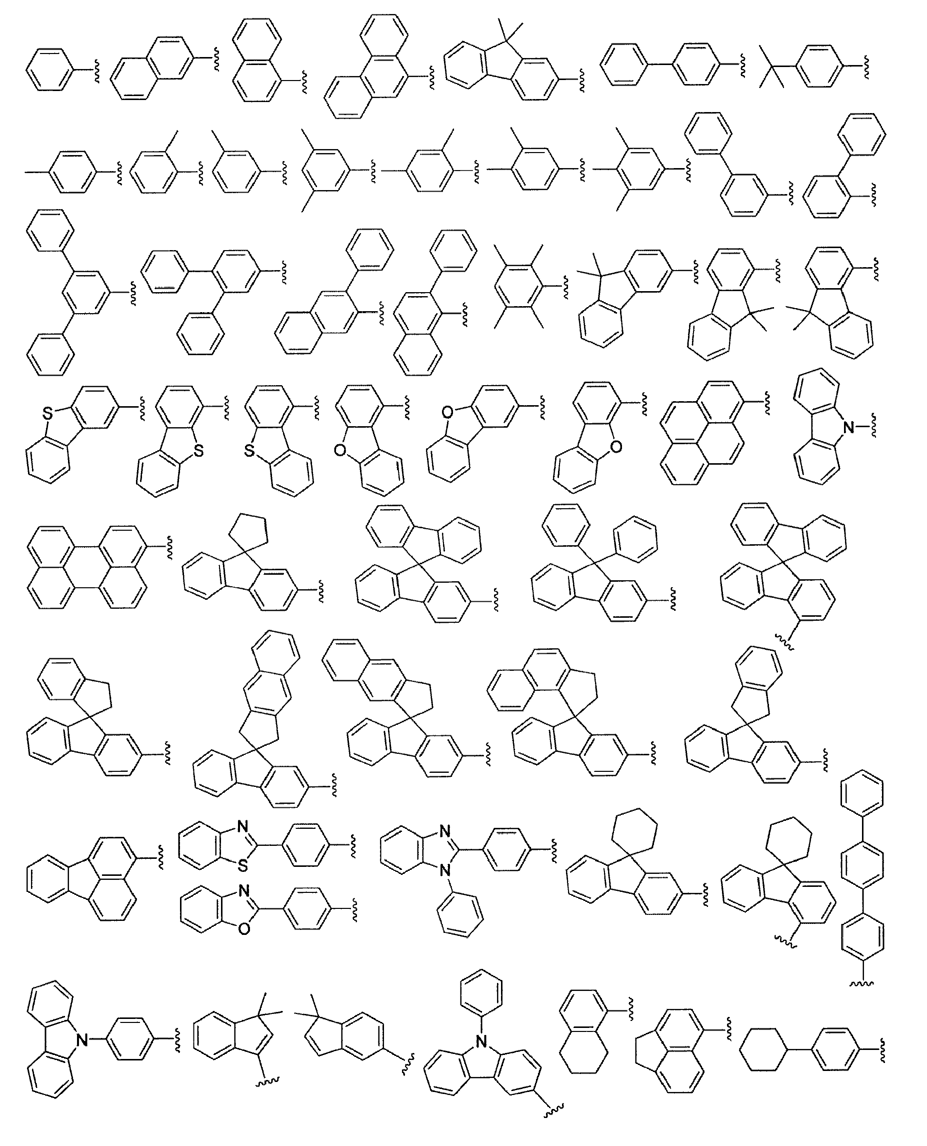

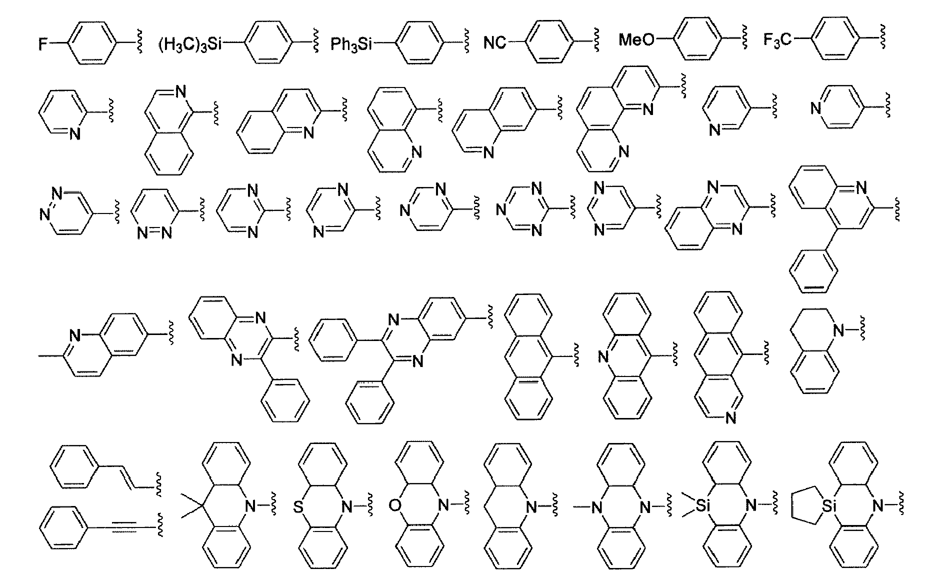

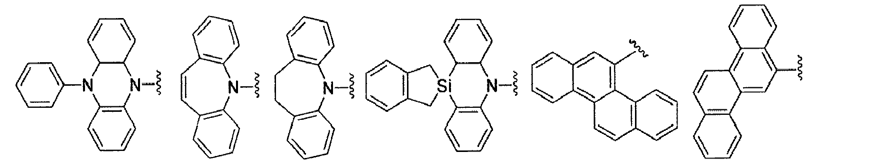

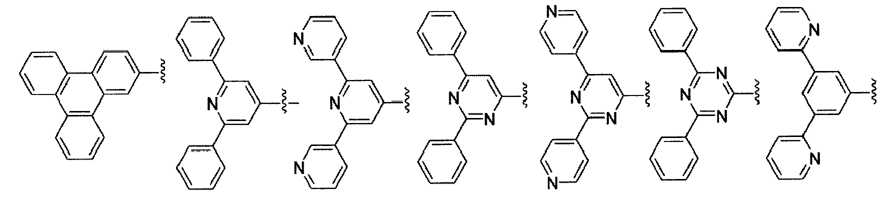

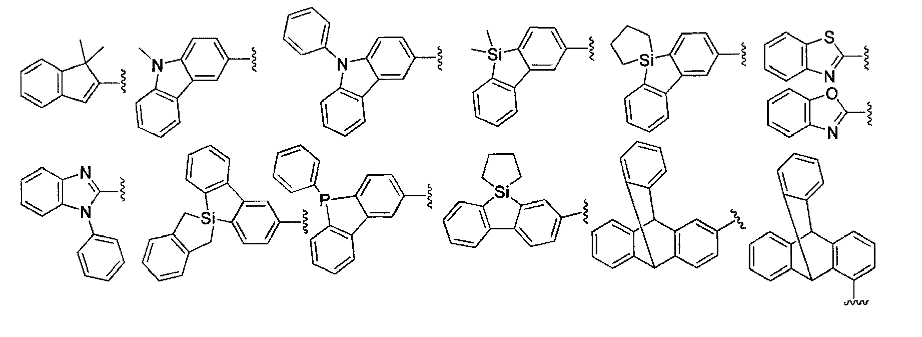

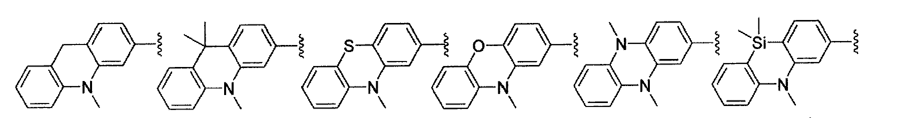

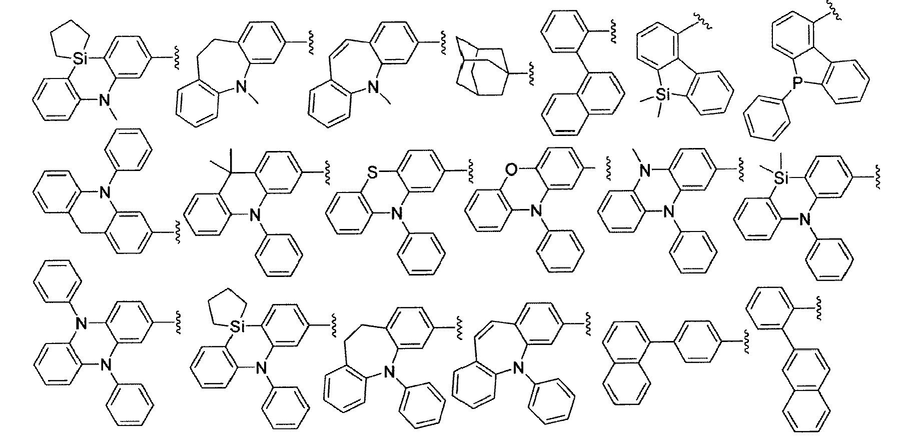

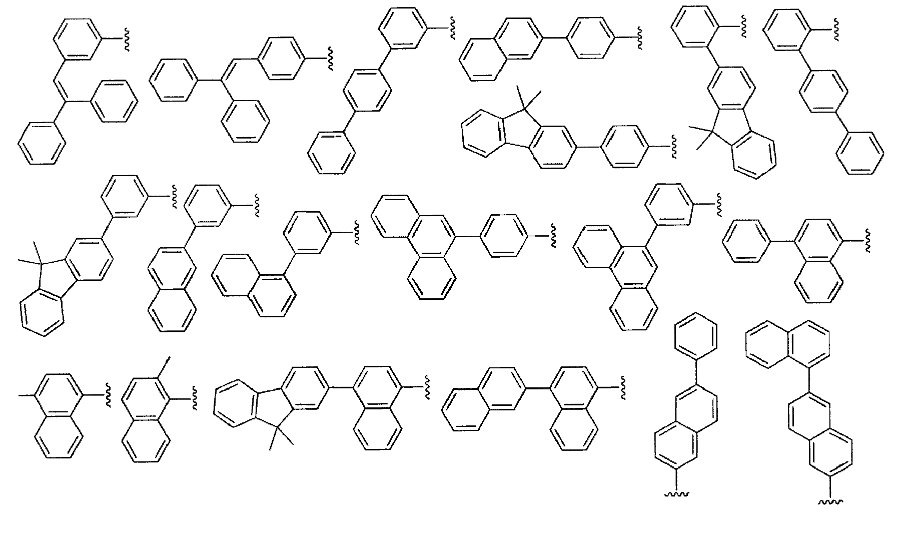

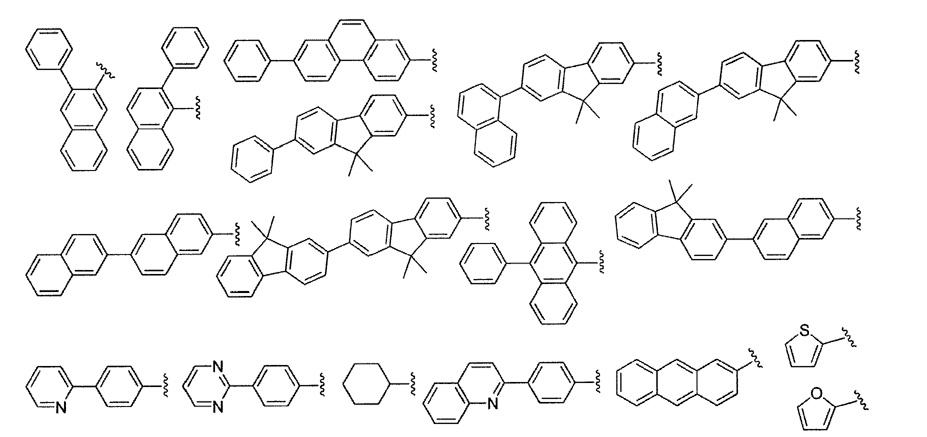

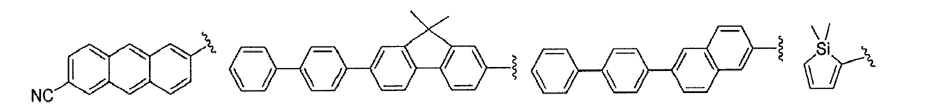

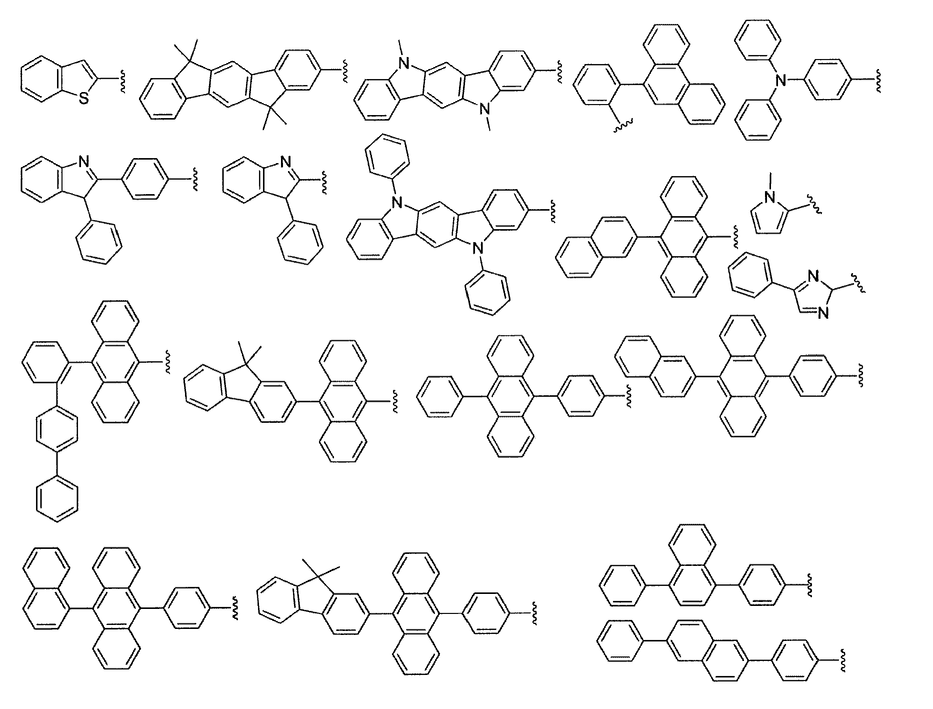

The organic electroluminescent compounds according to the present invention can be more specifically exemplified by the following compounds, but they are not restrictive.

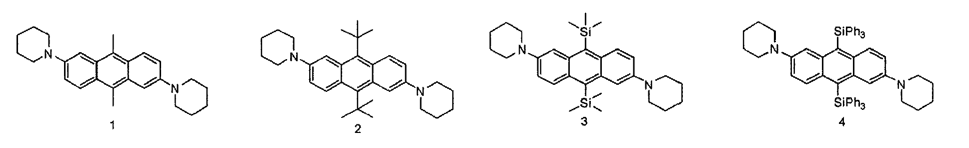

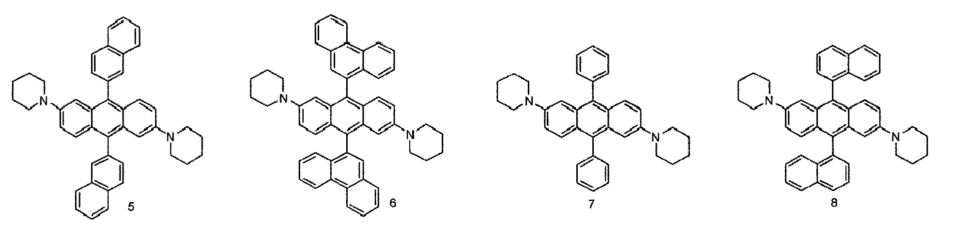

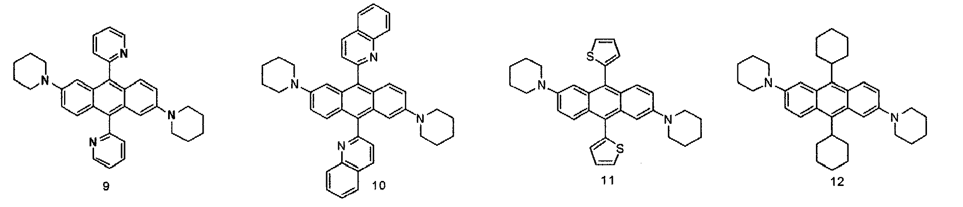

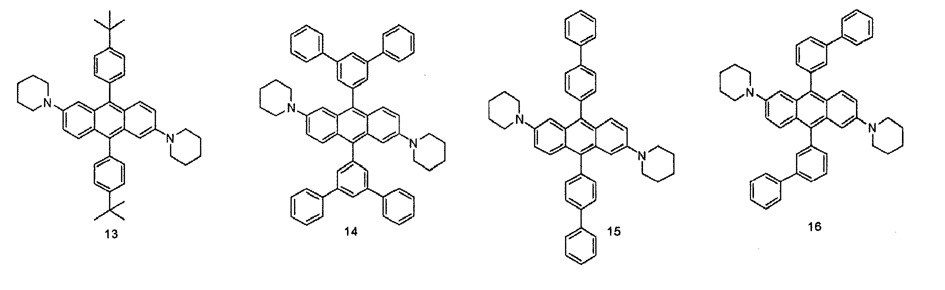

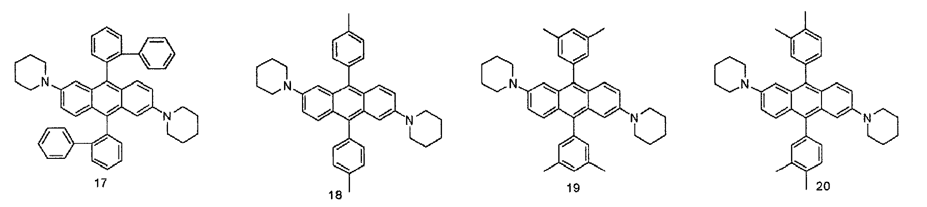

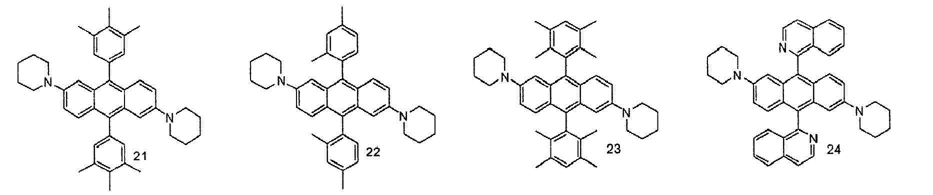

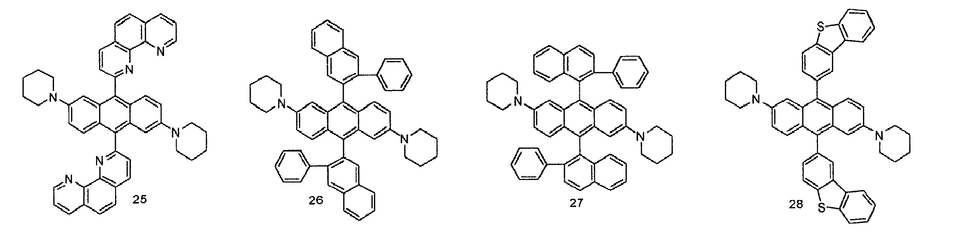

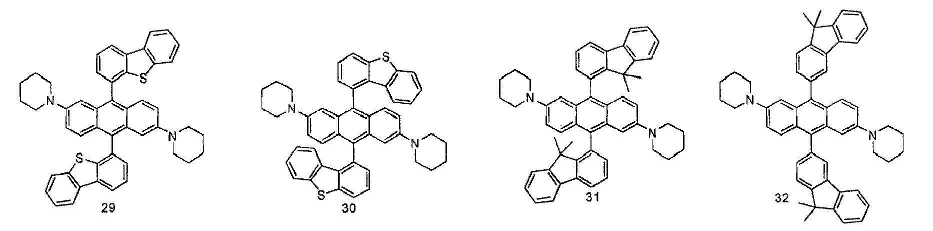

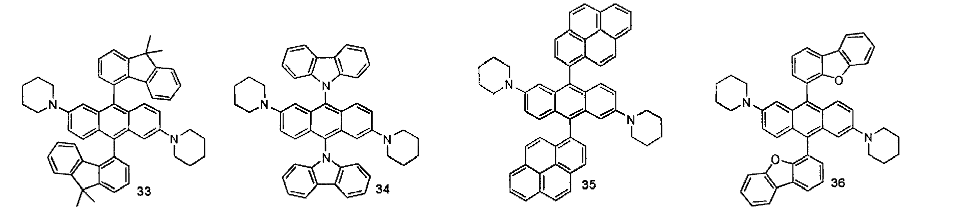

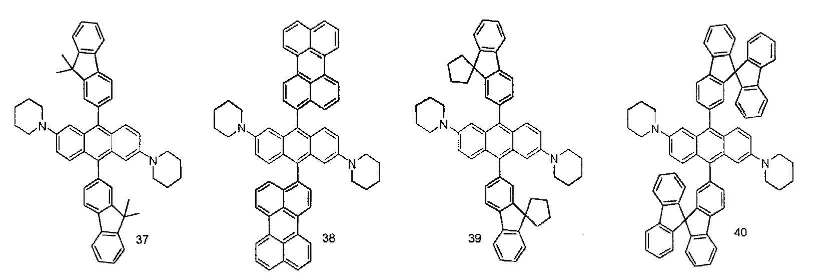

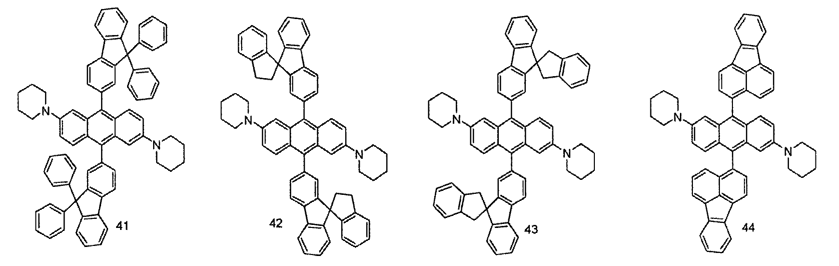

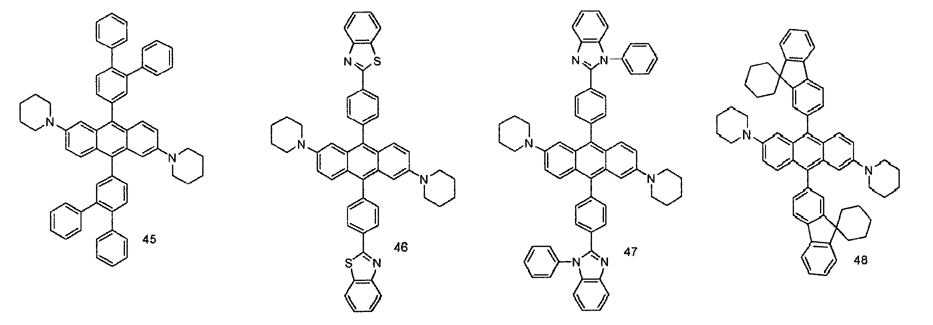

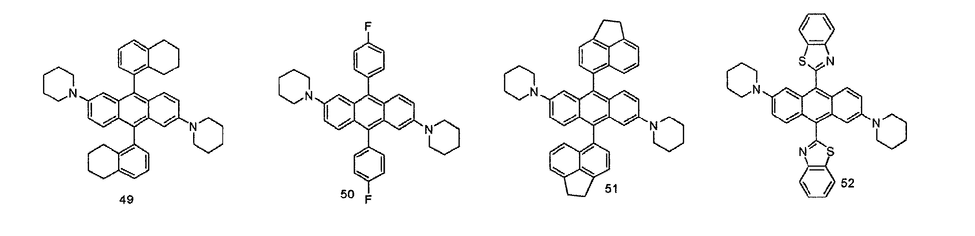

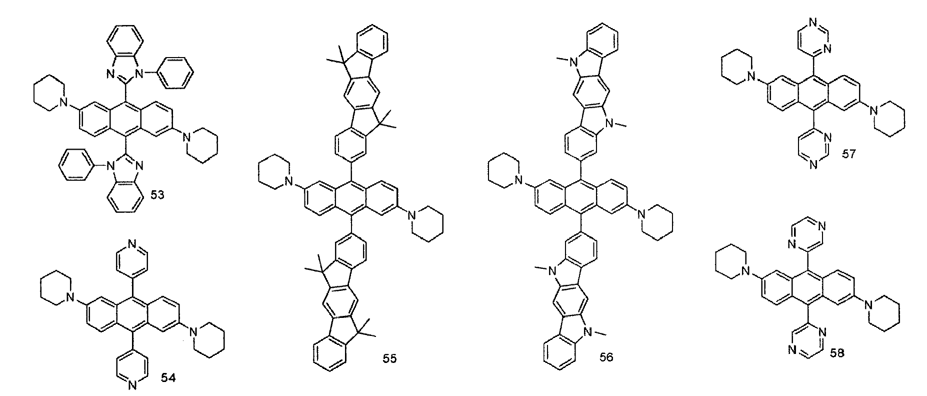

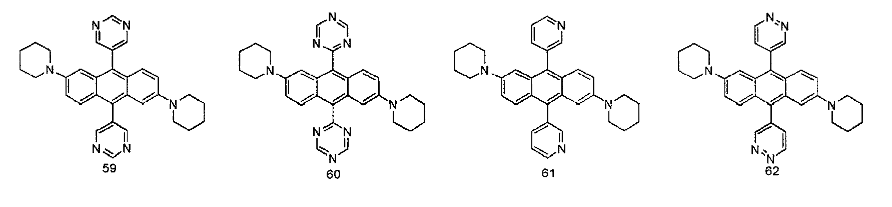

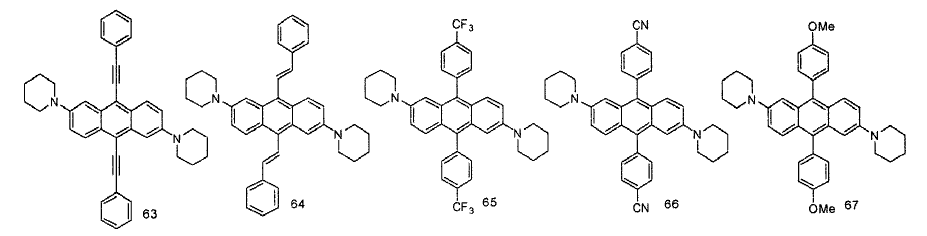

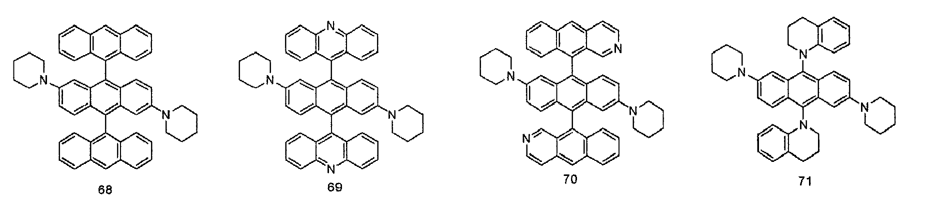

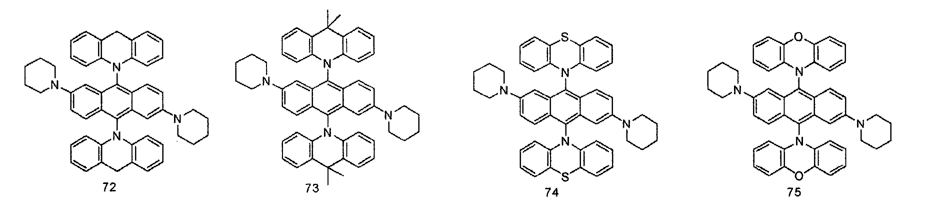

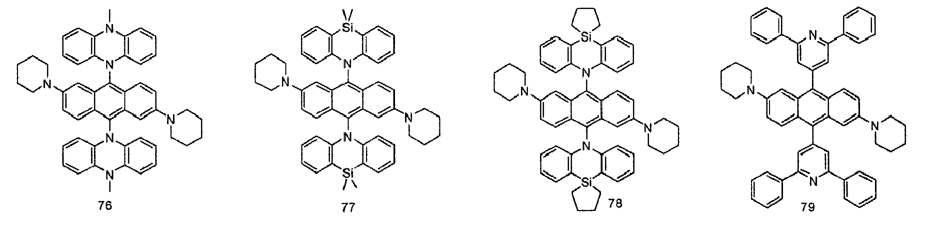

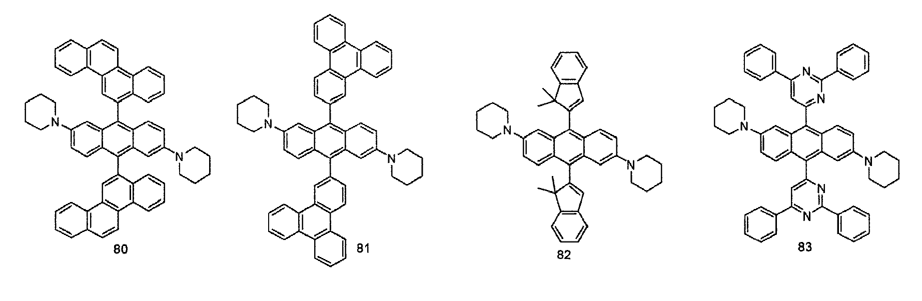

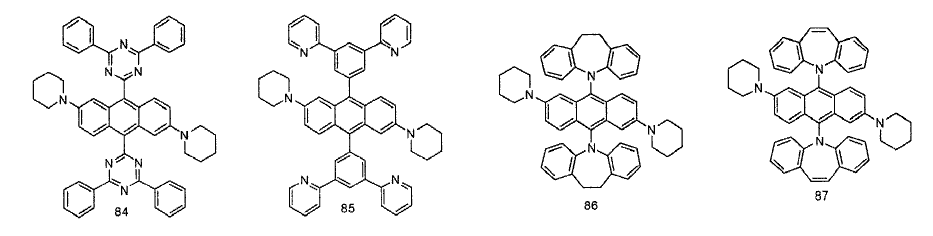

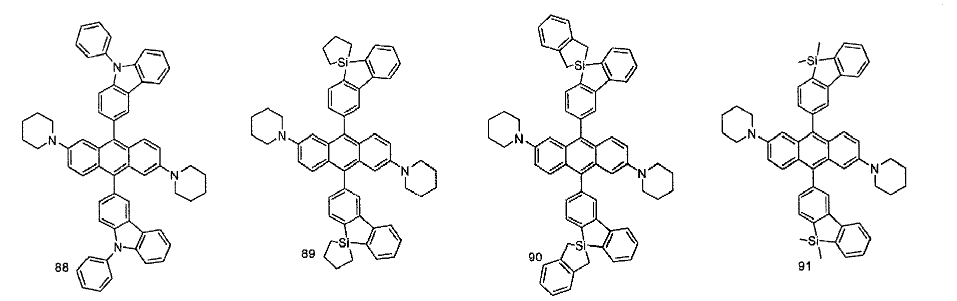

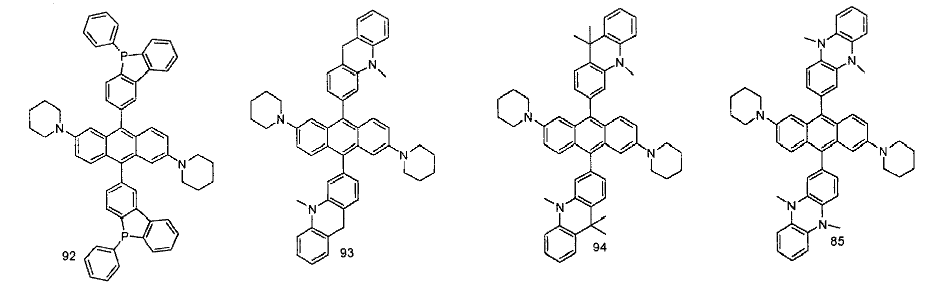

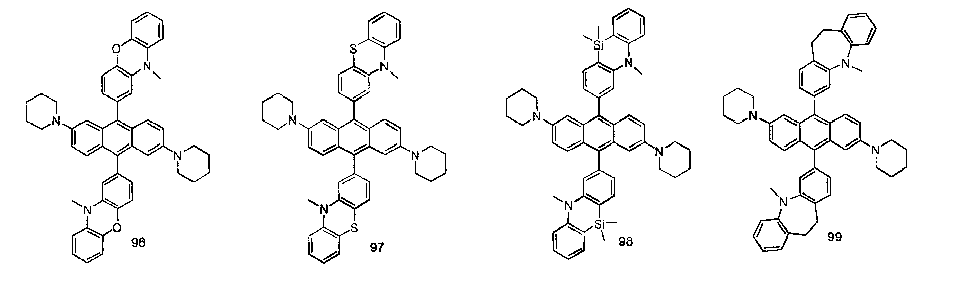

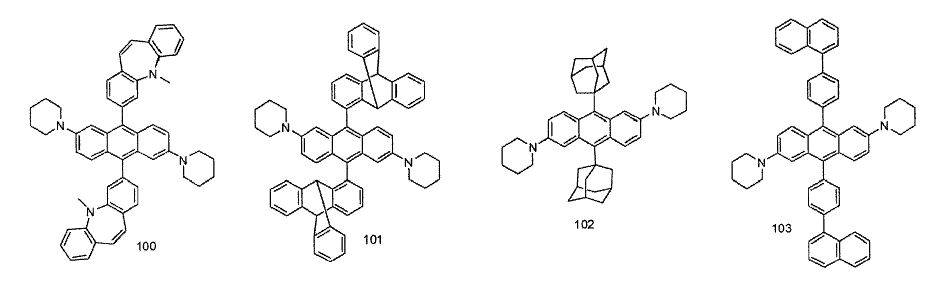

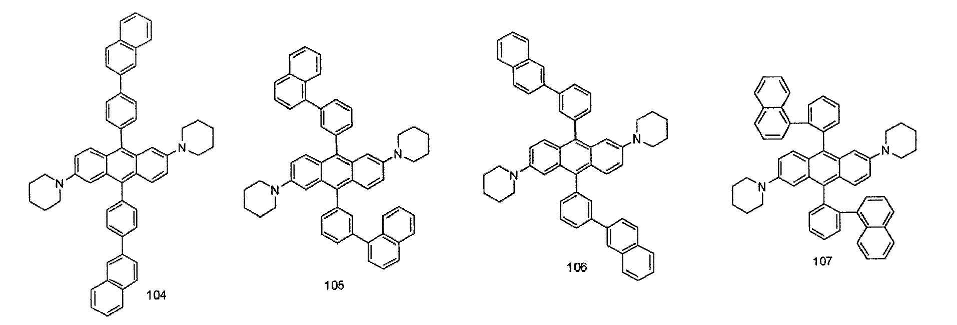

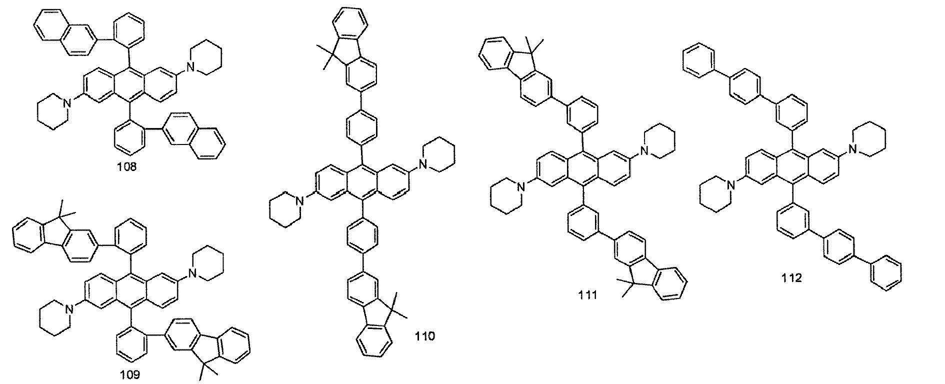

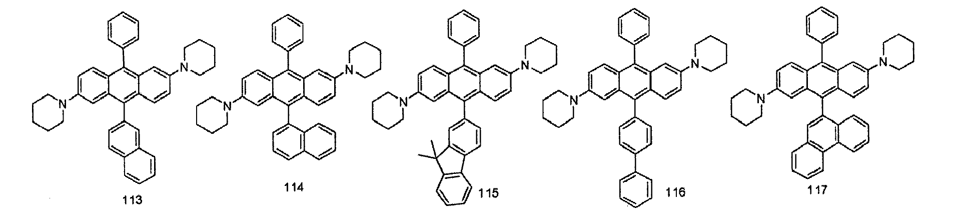

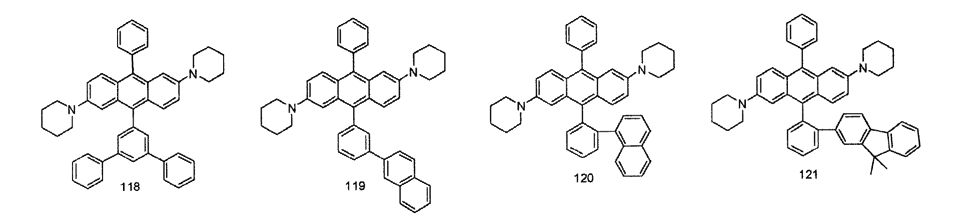

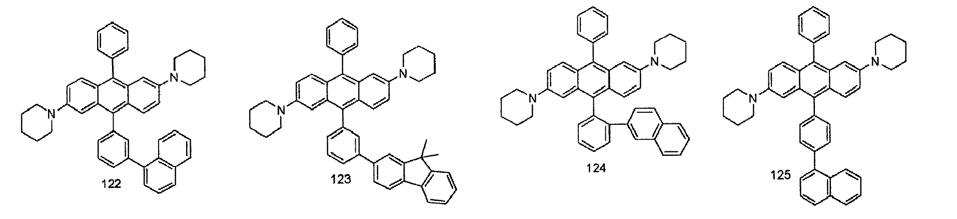

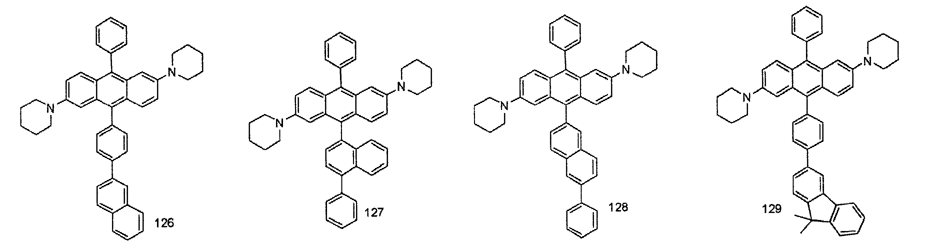

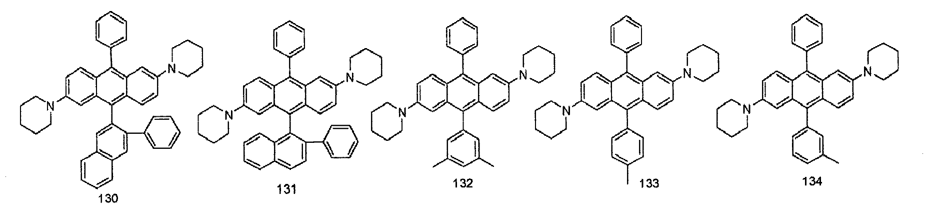

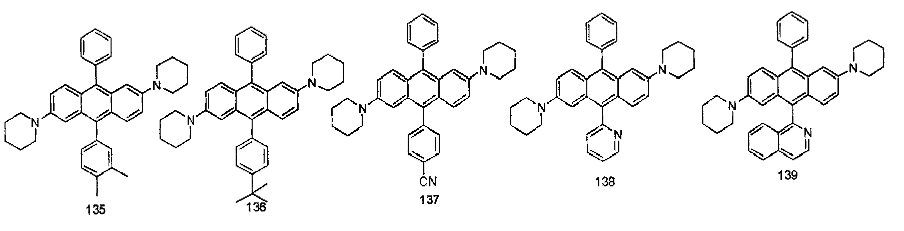

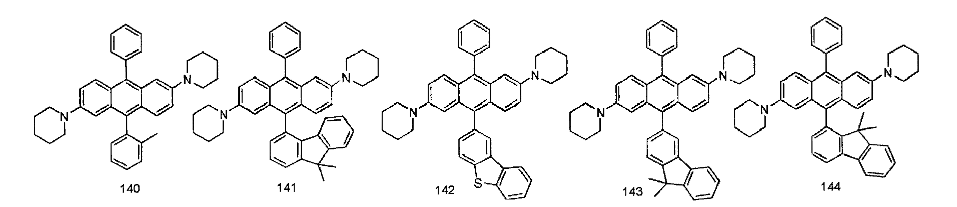

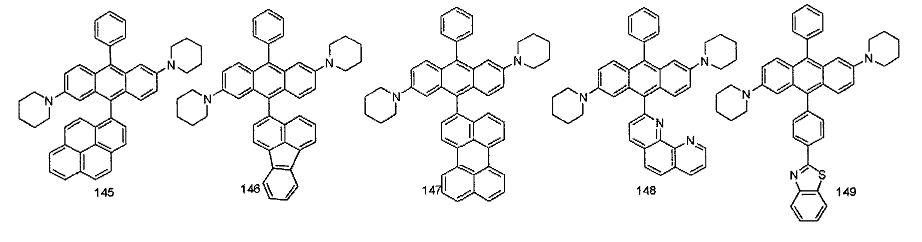

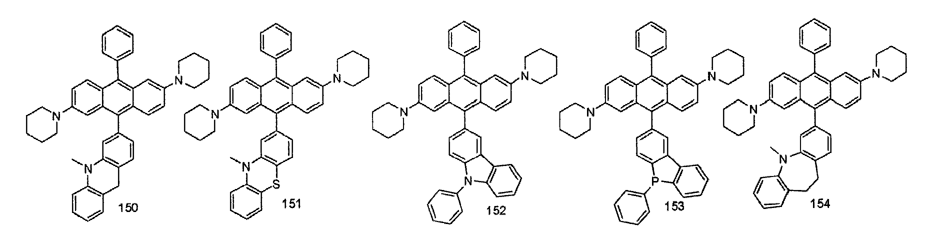

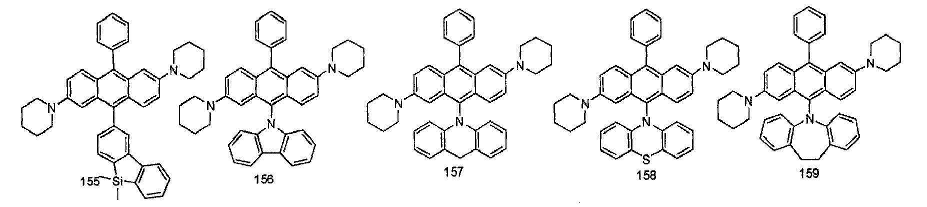

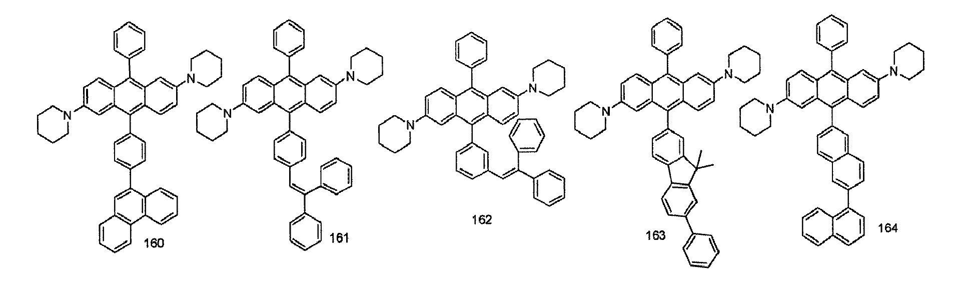

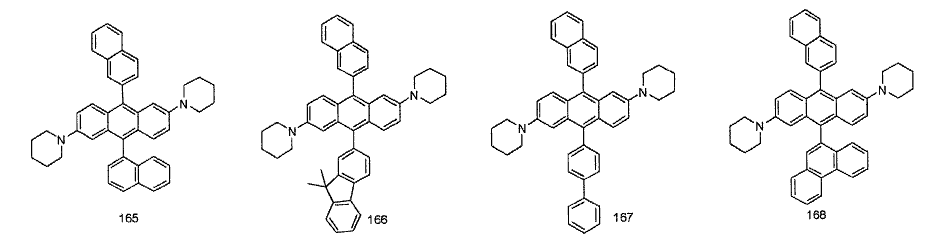

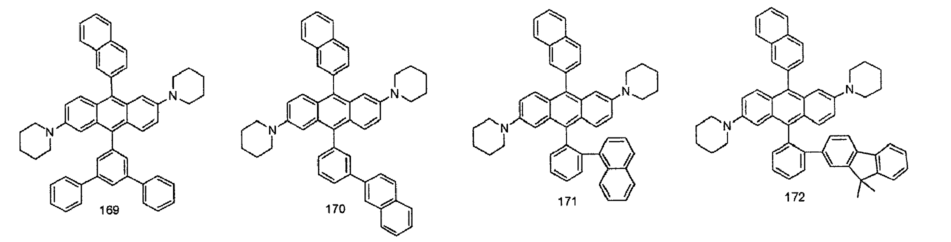

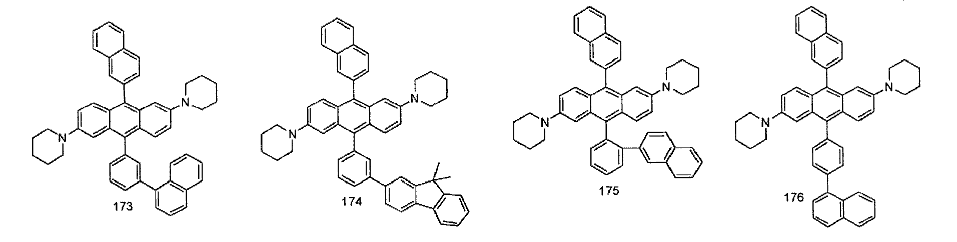

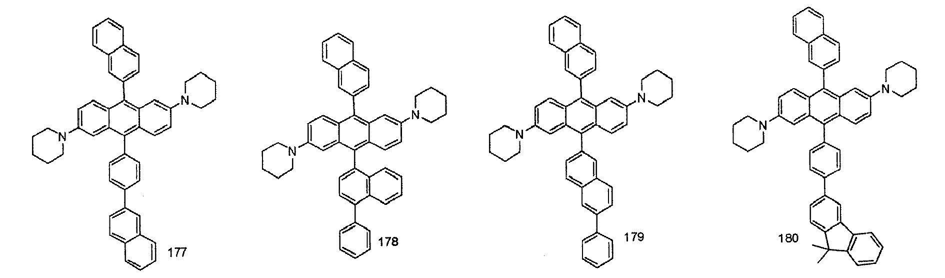

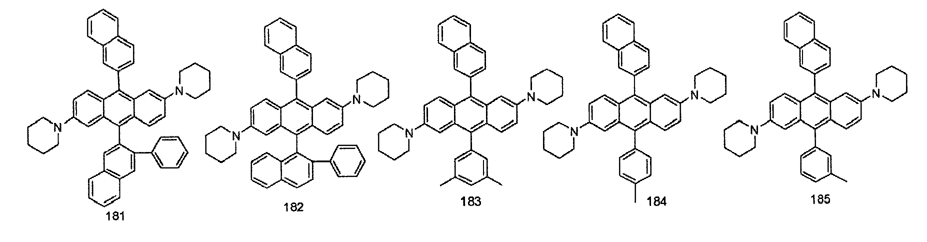

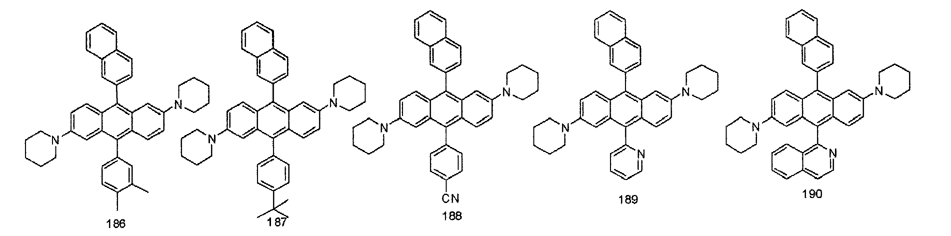

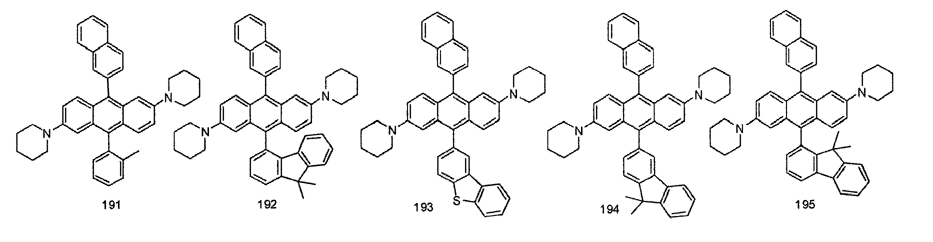

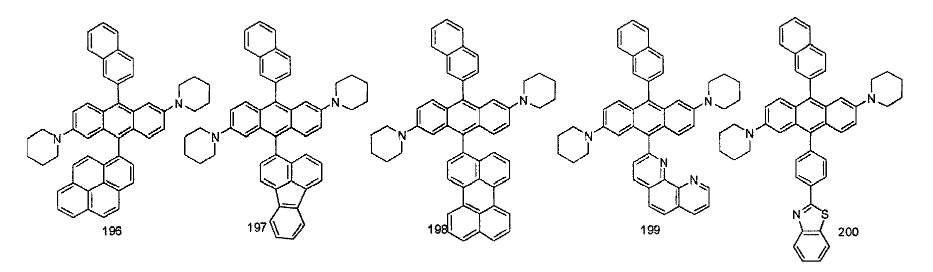

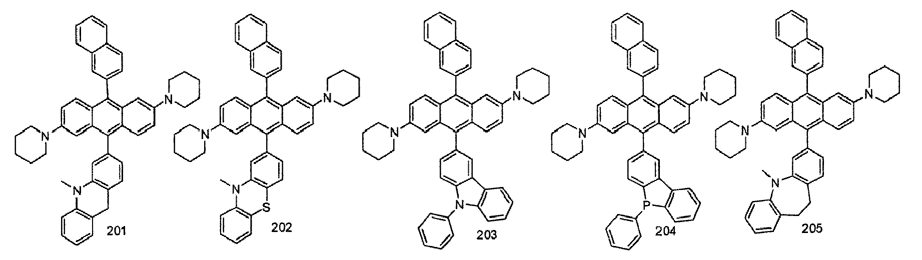

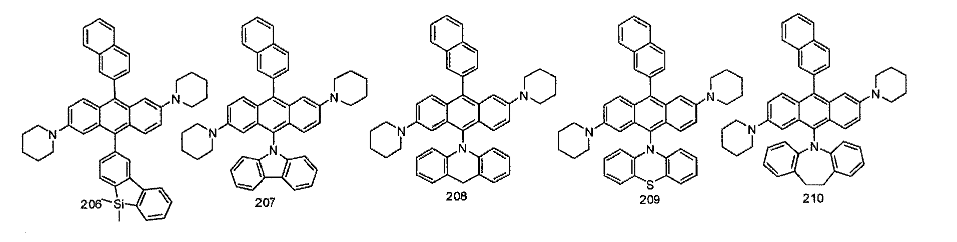

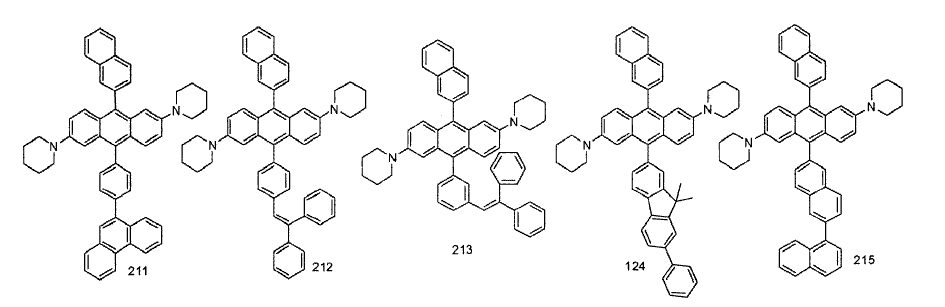

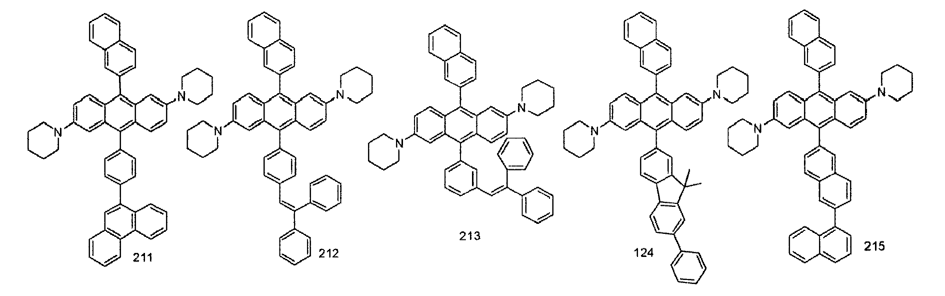

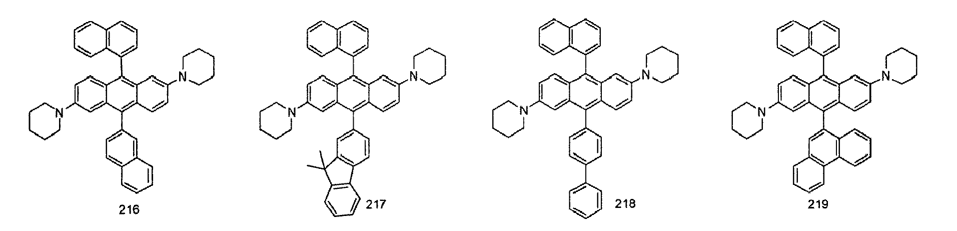

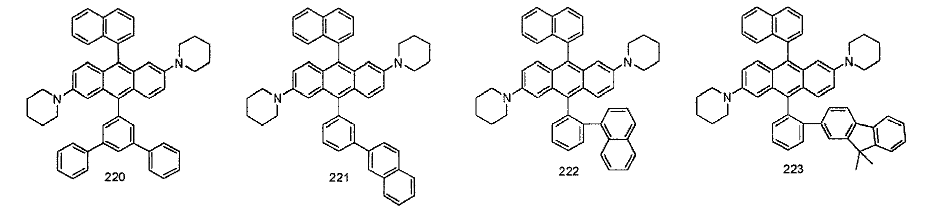

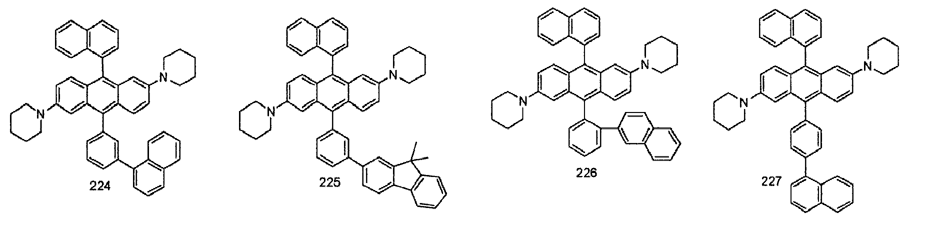

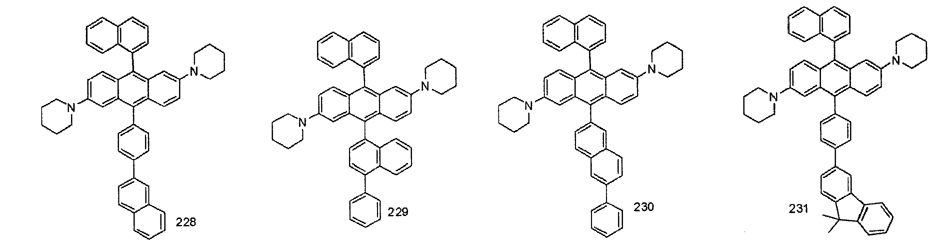

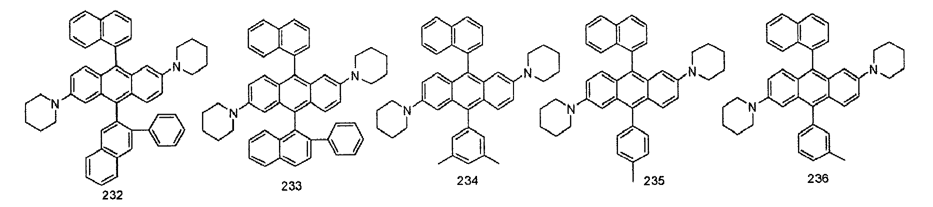

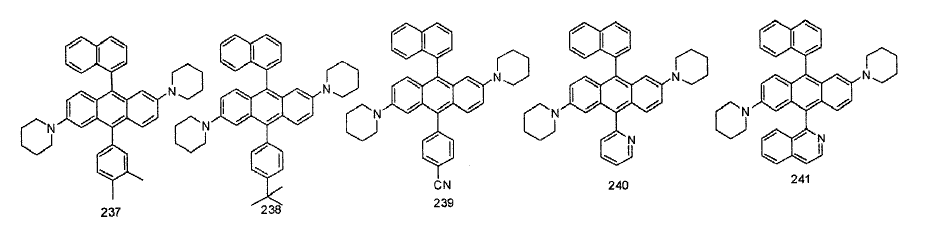

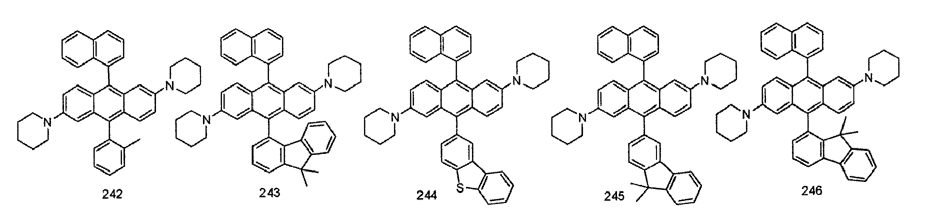

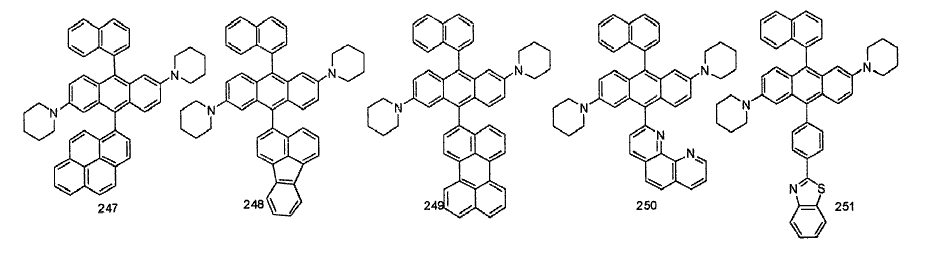

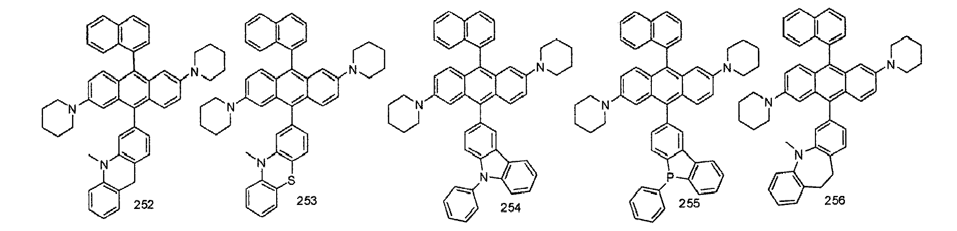

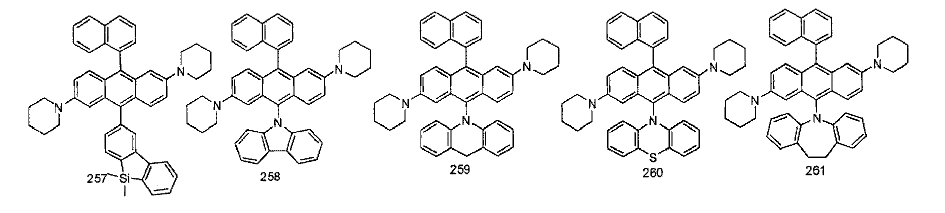

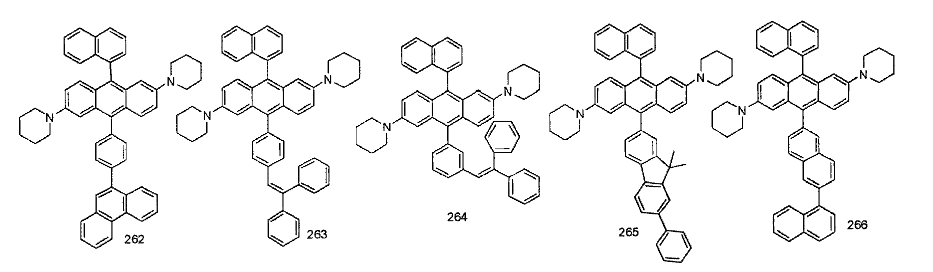

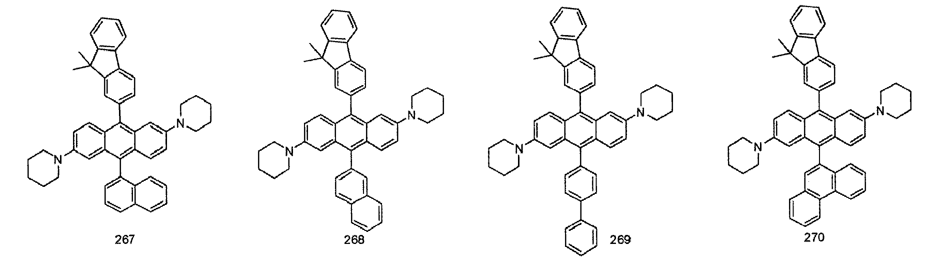

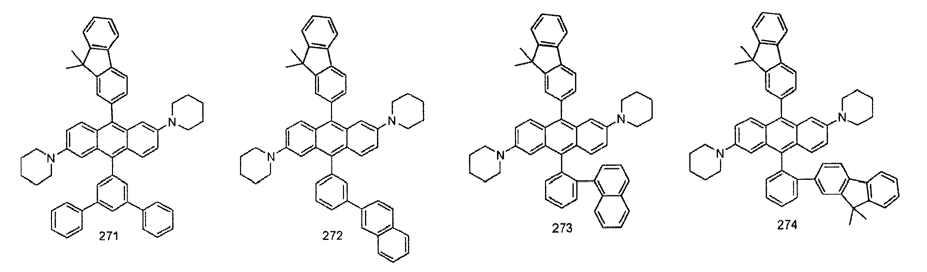

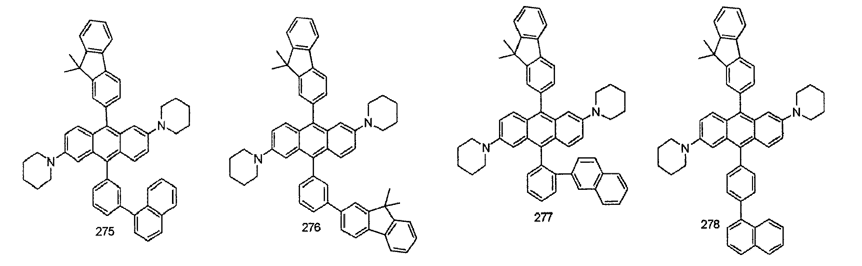

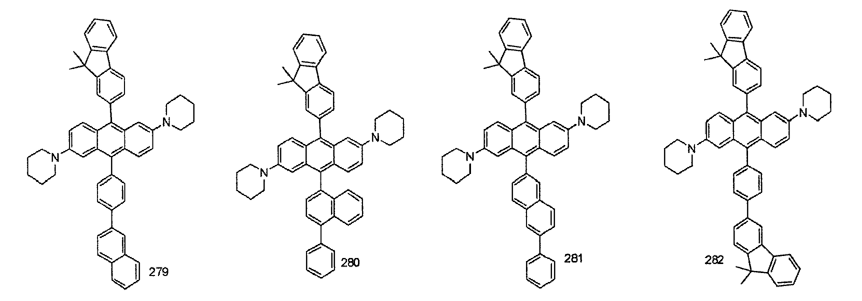

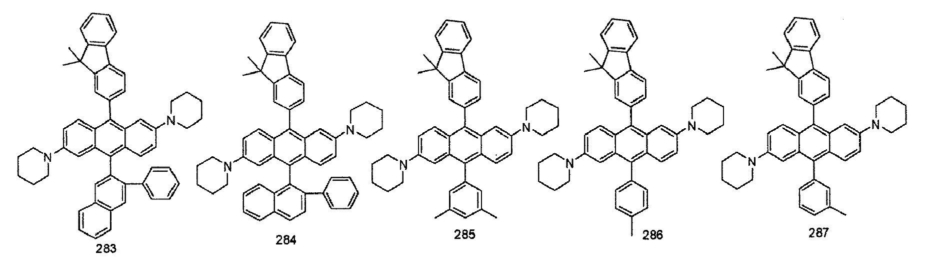

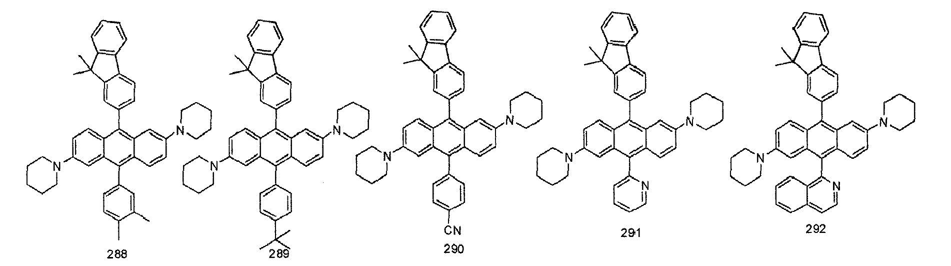

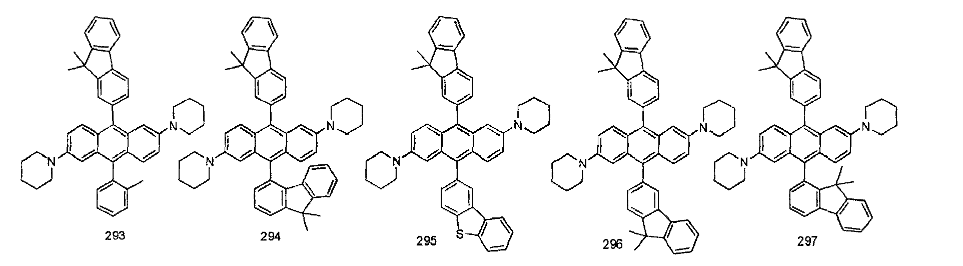

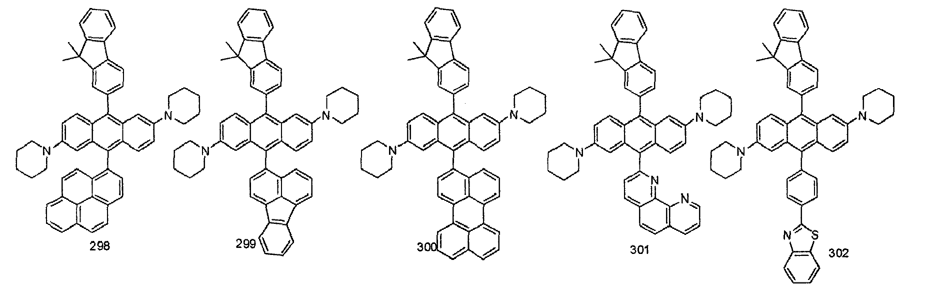

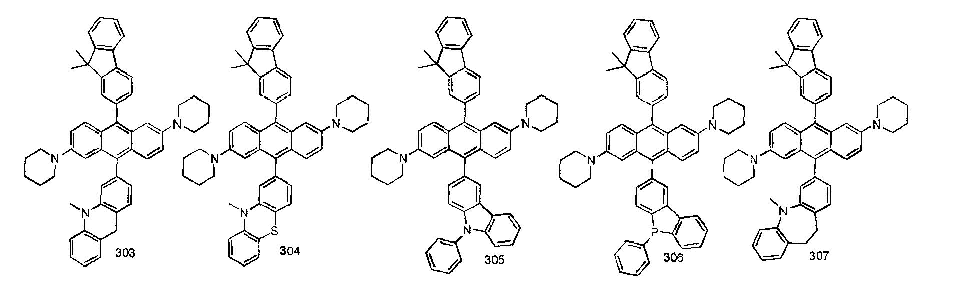

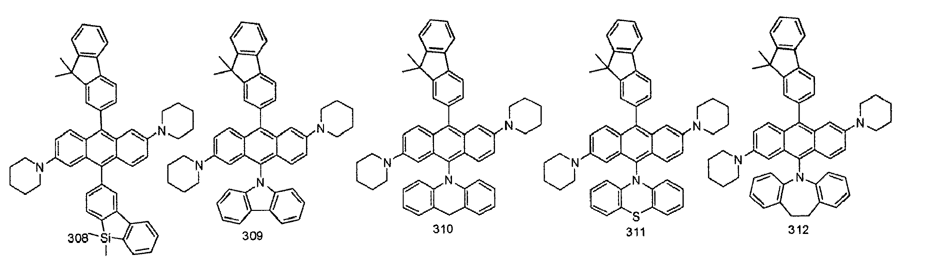

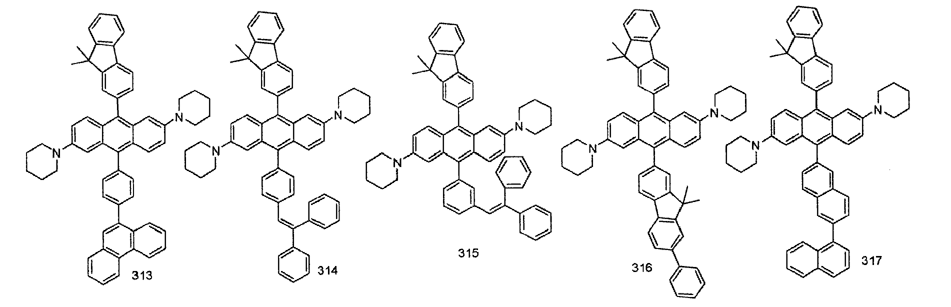

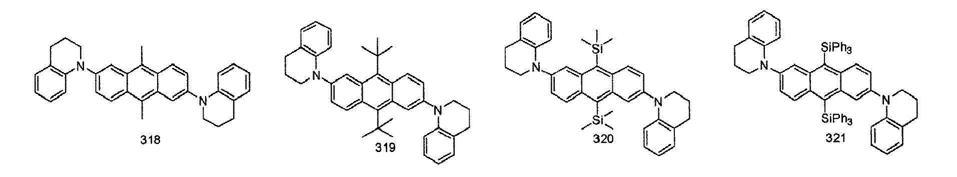

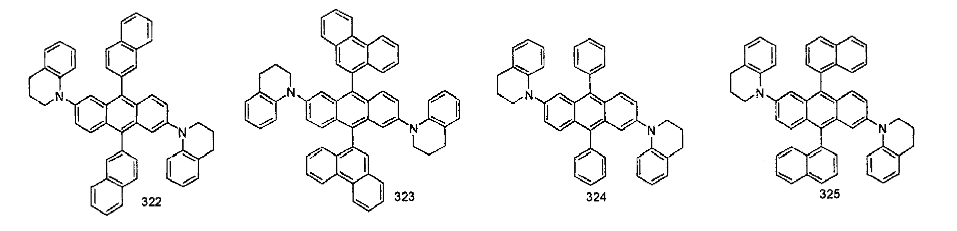

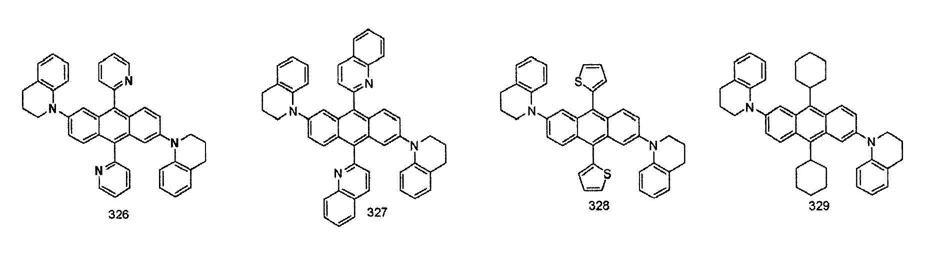

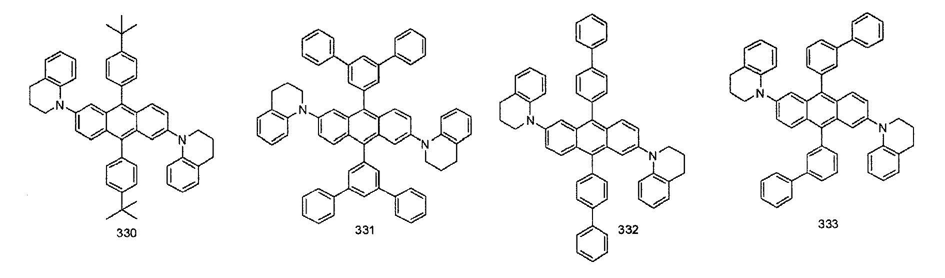

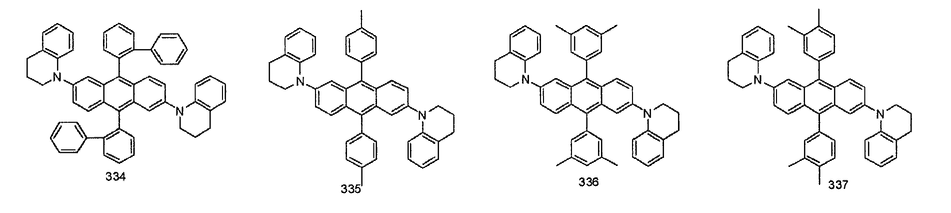

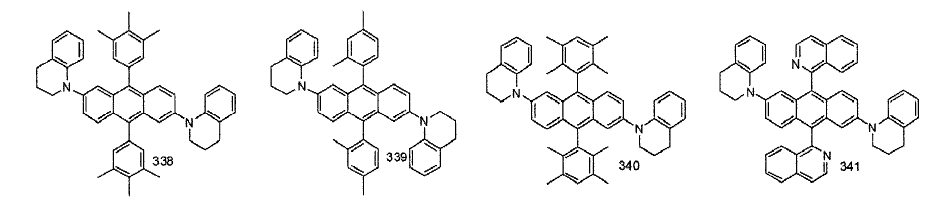

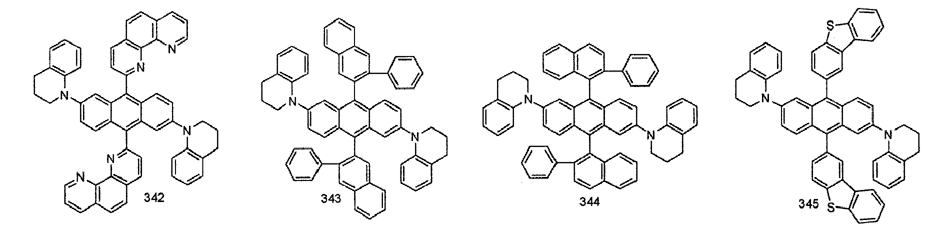

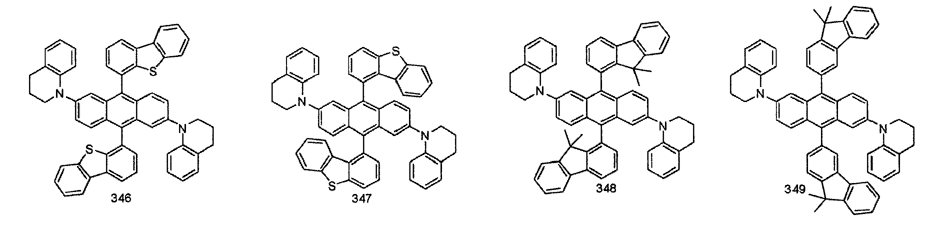

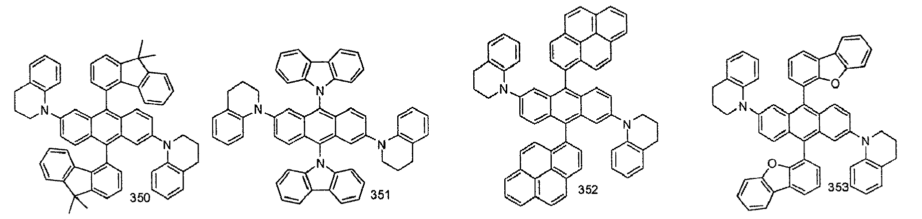

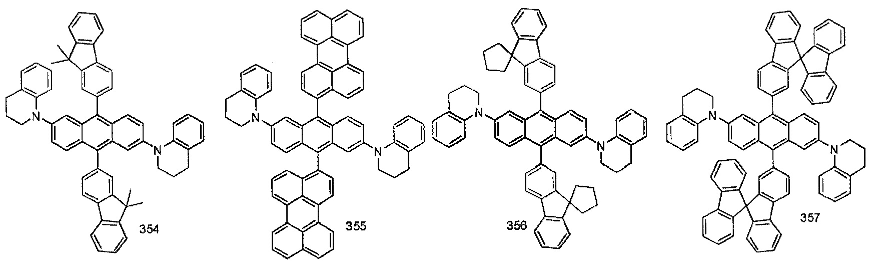

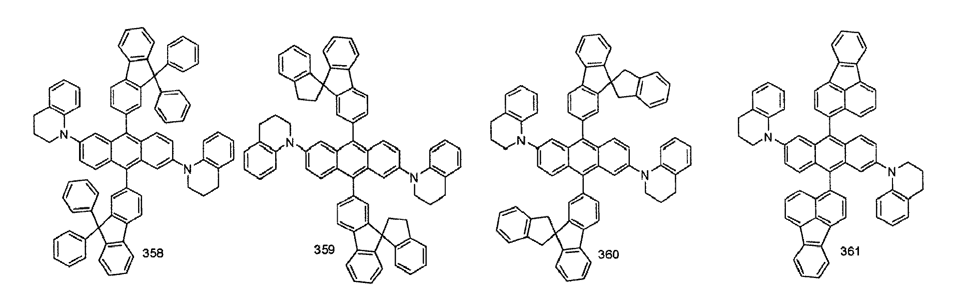

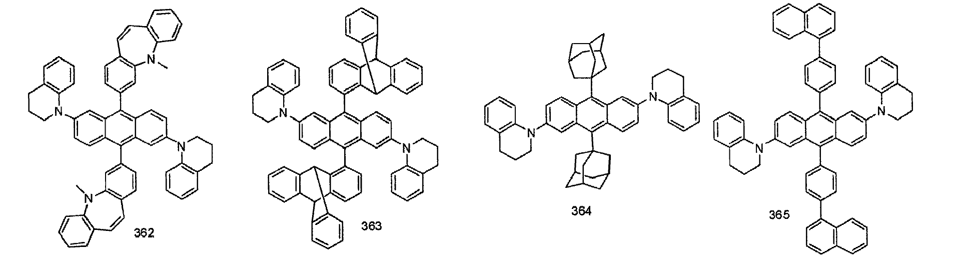

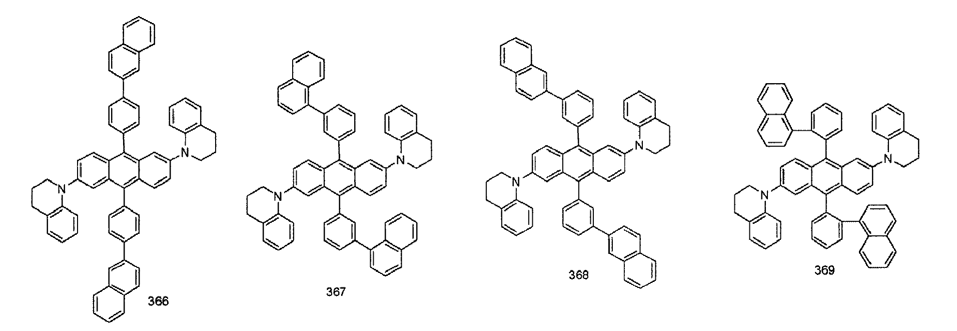

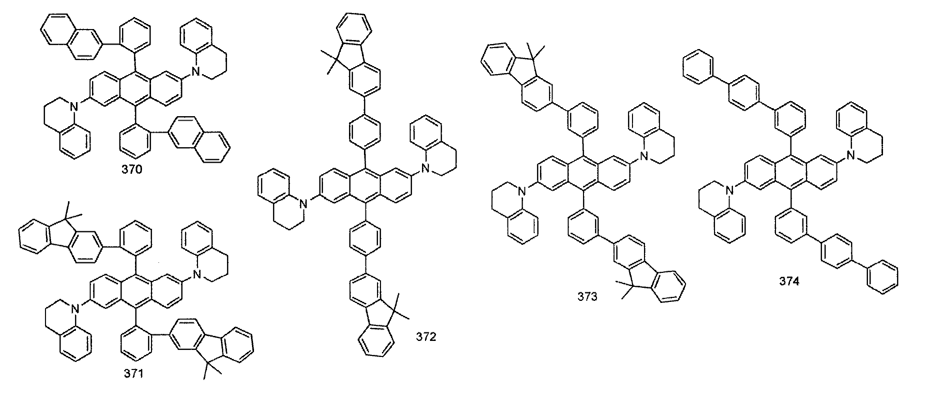

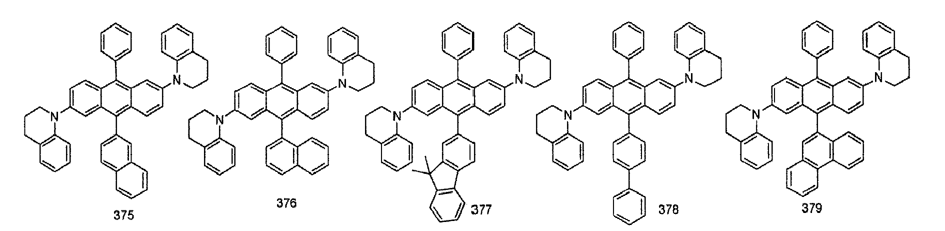

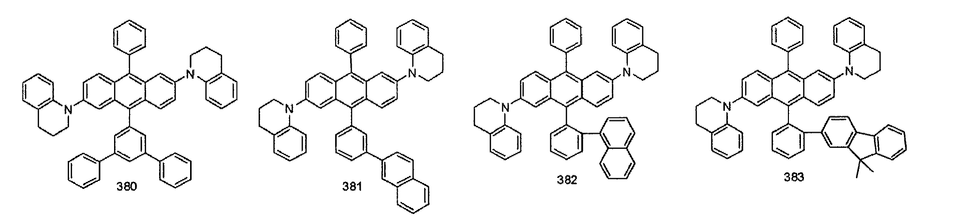

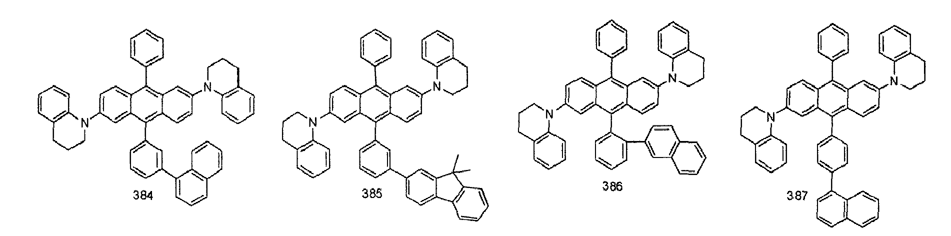

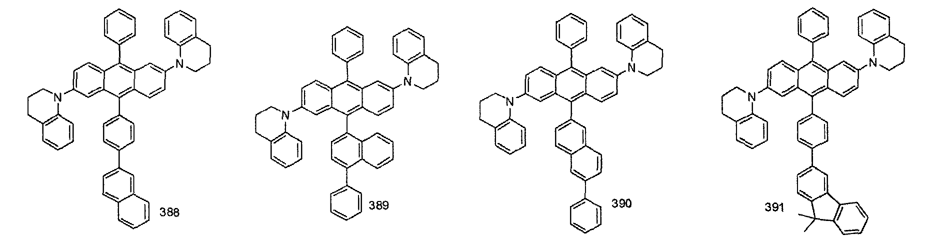

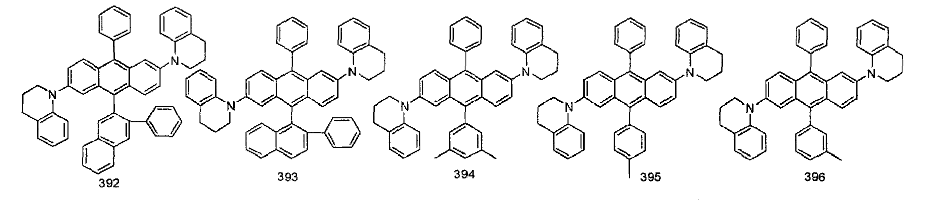

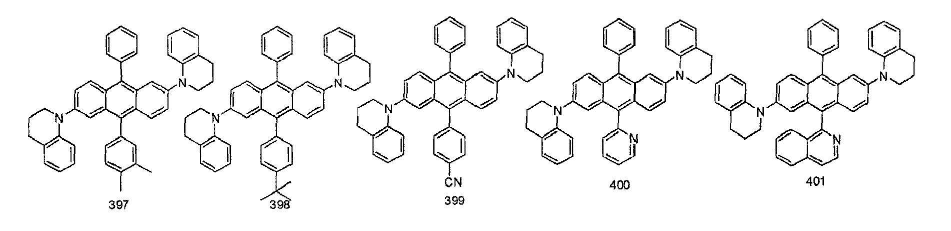

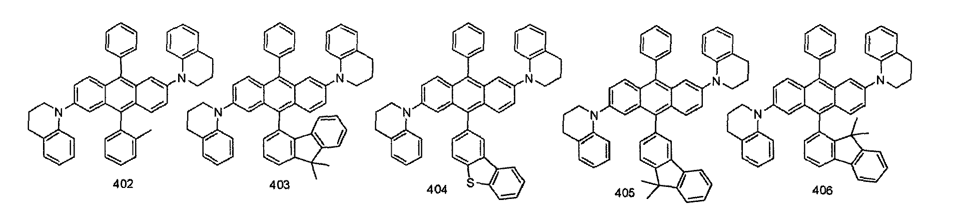

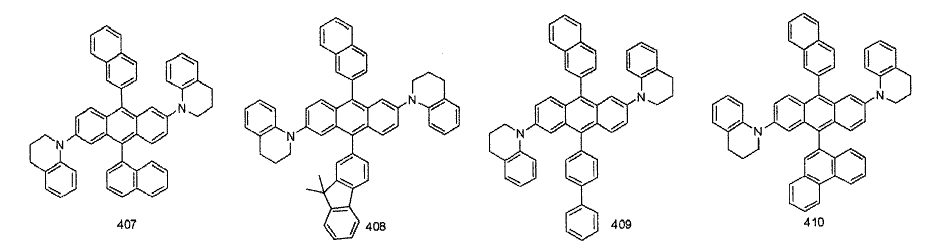

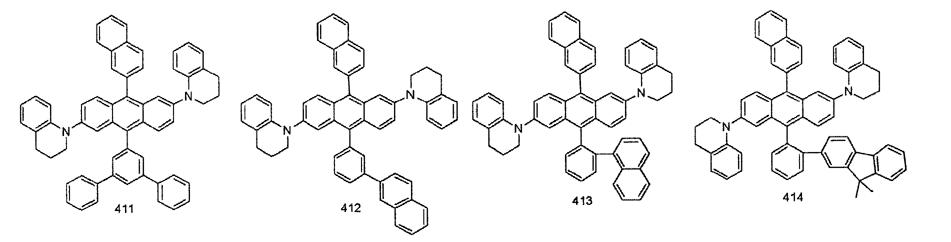

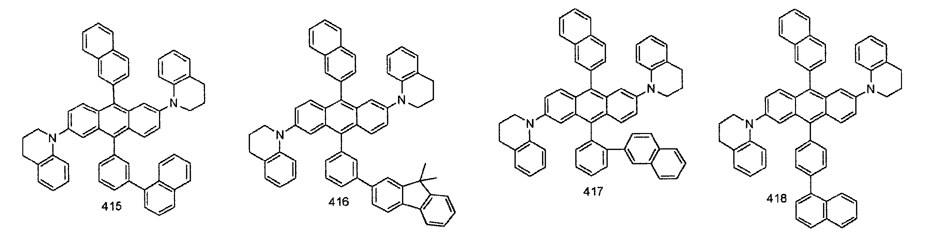

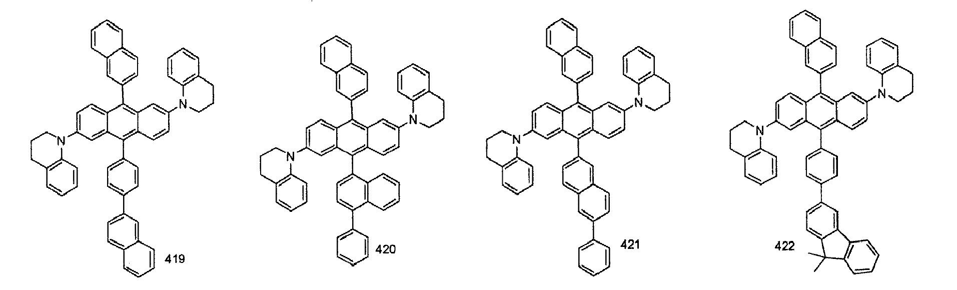

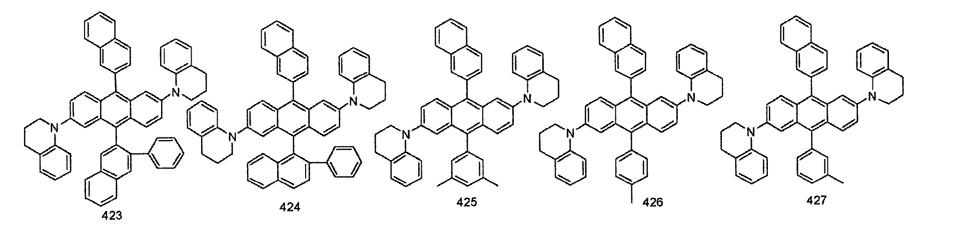

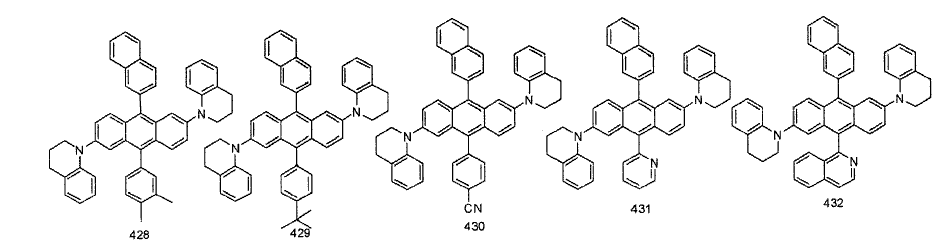

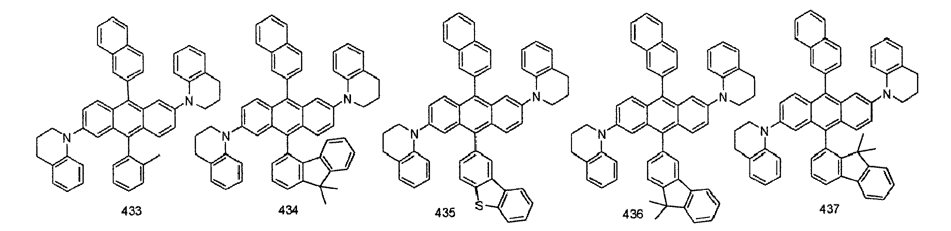

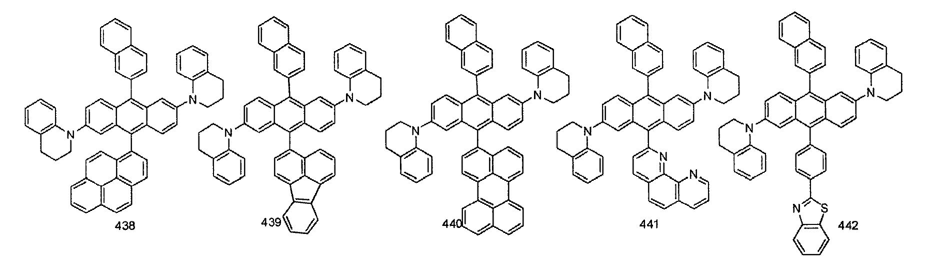

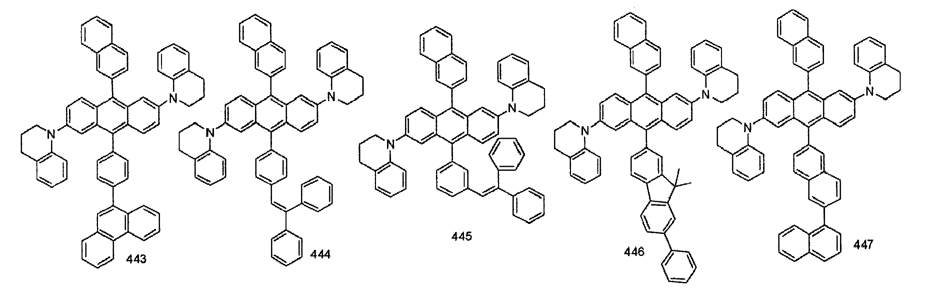

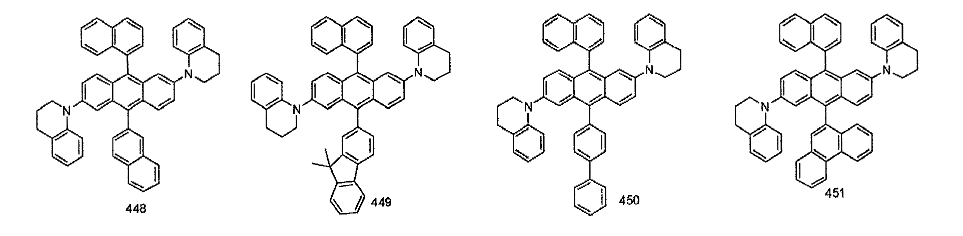

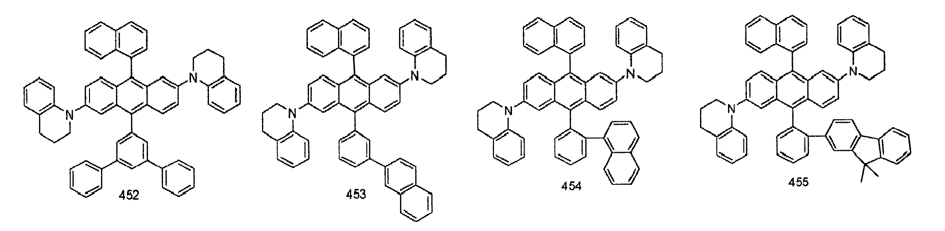

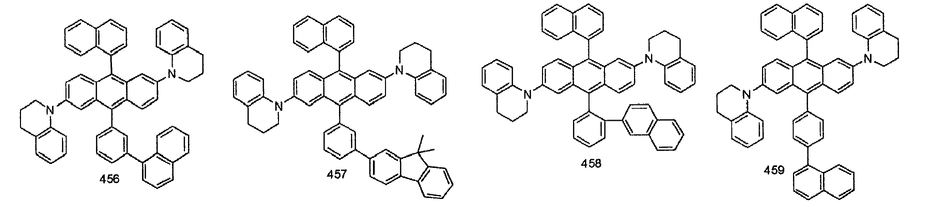

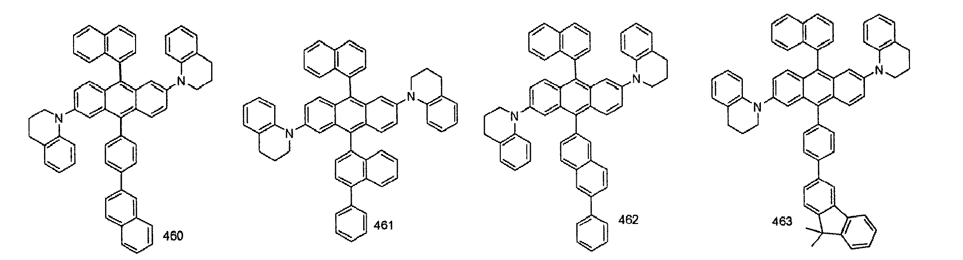

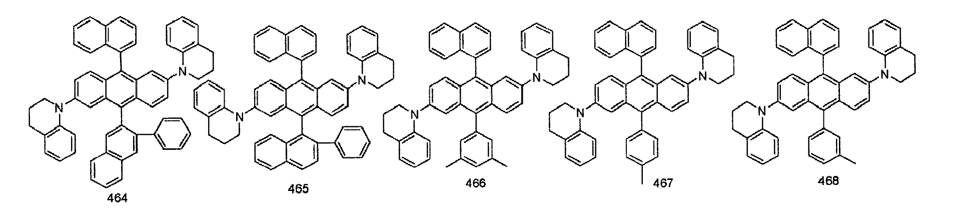

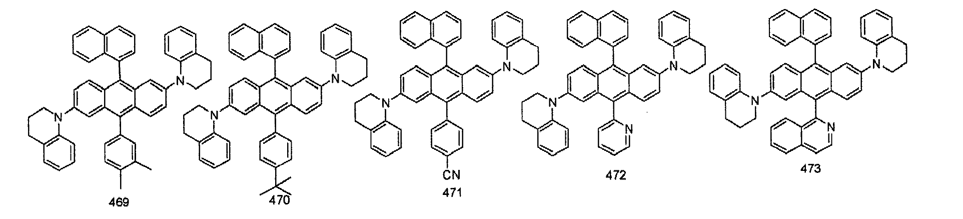

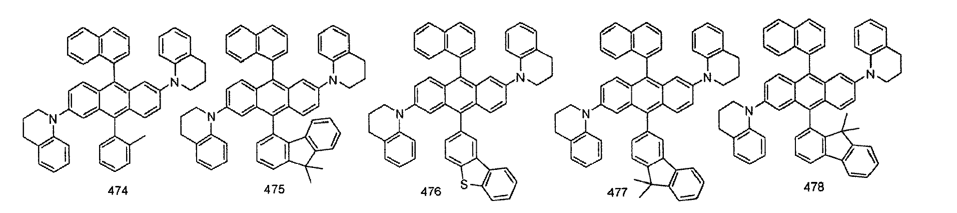

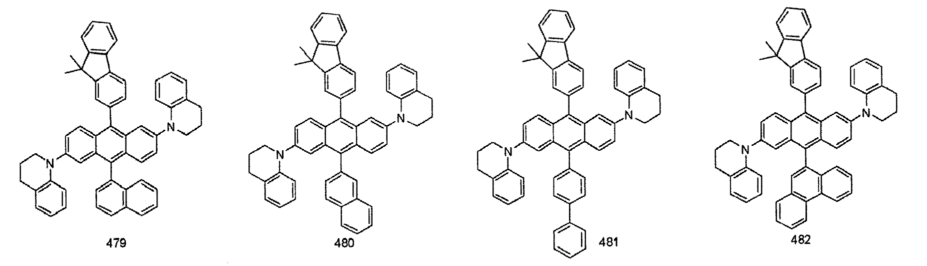

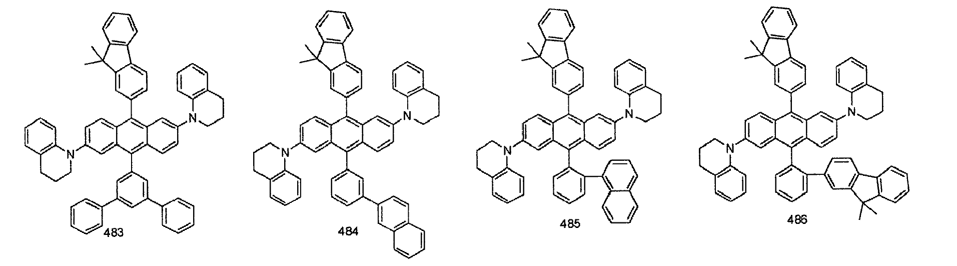

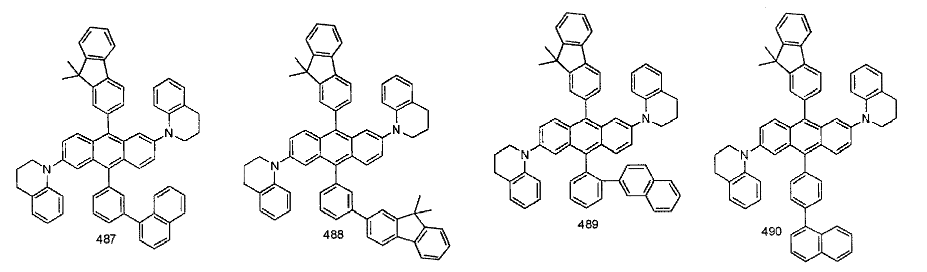

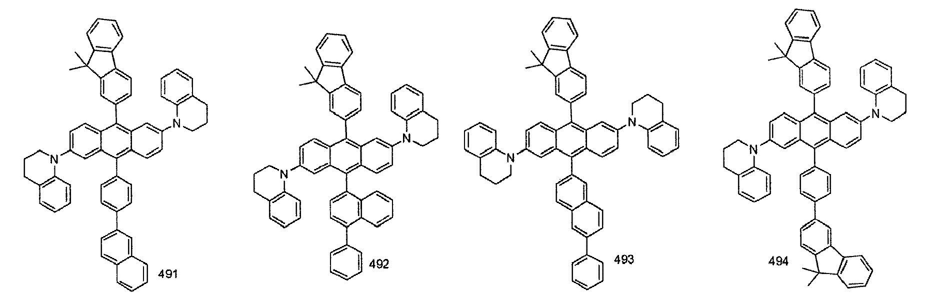

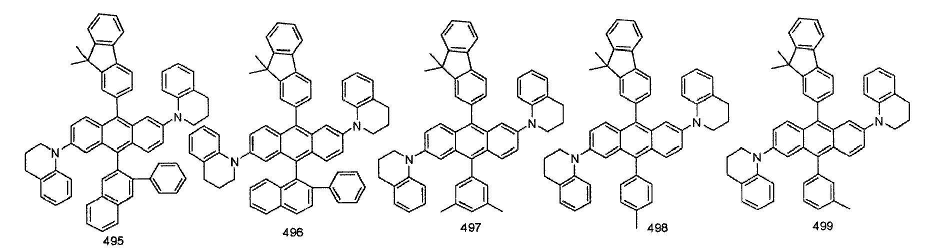

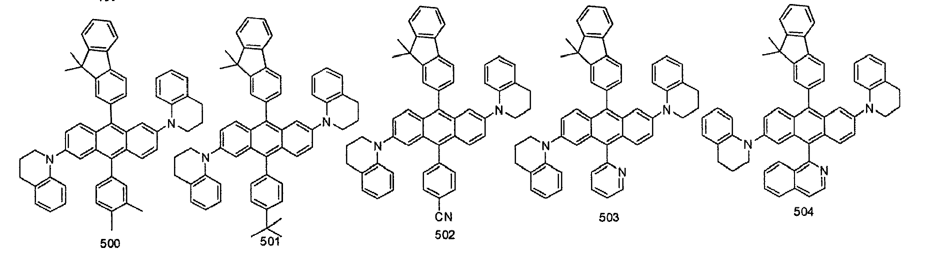

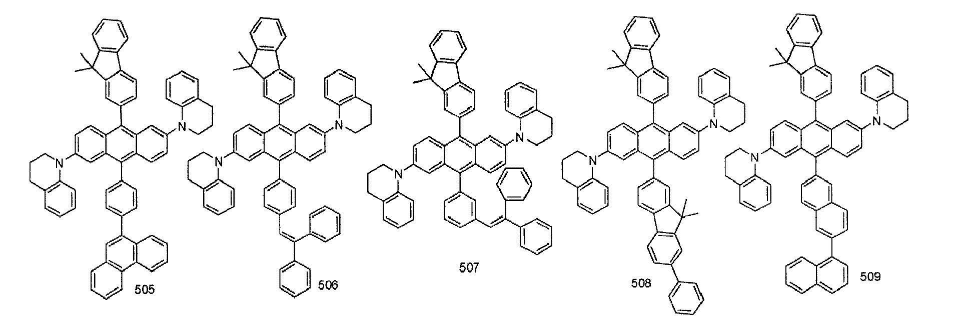

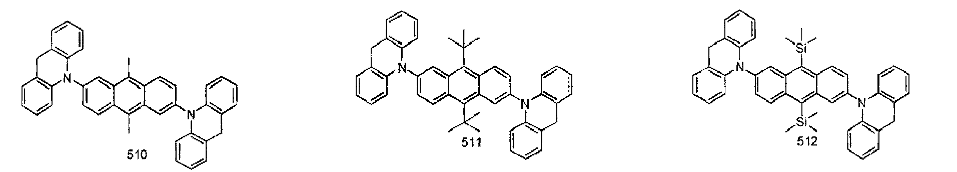

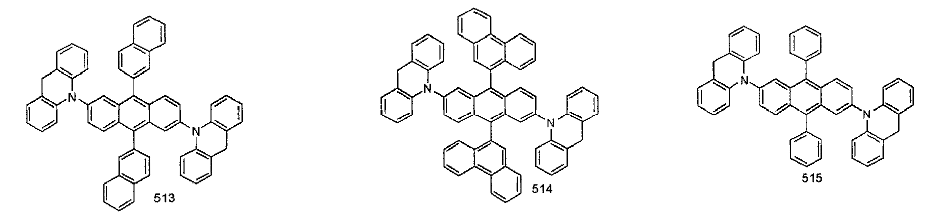

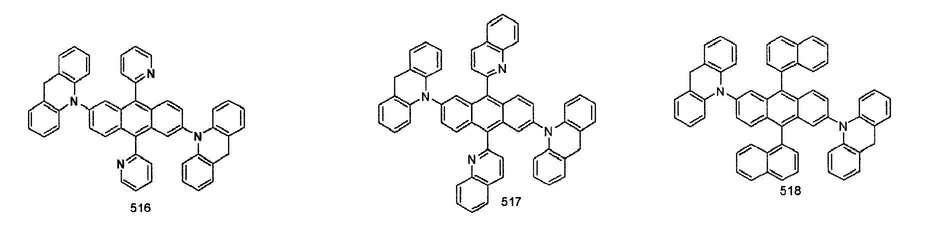

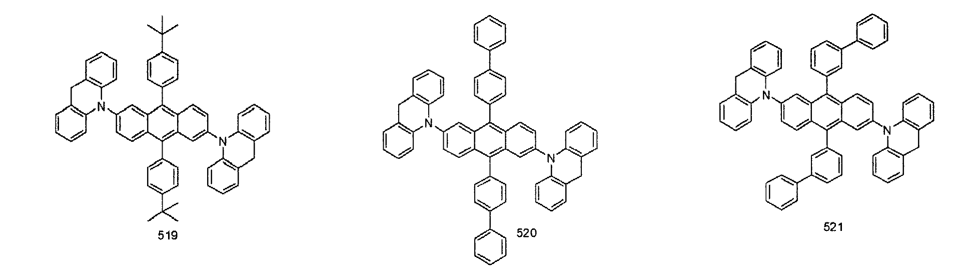

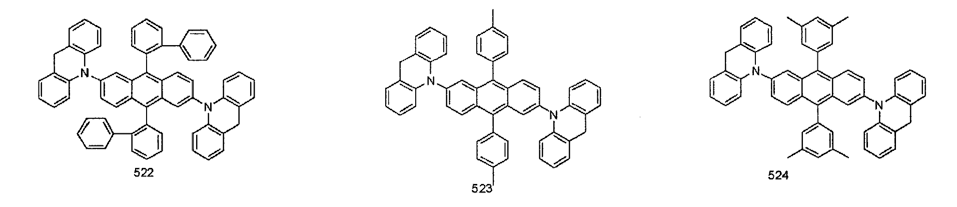

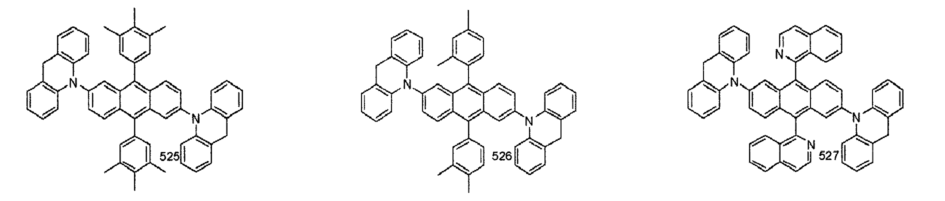

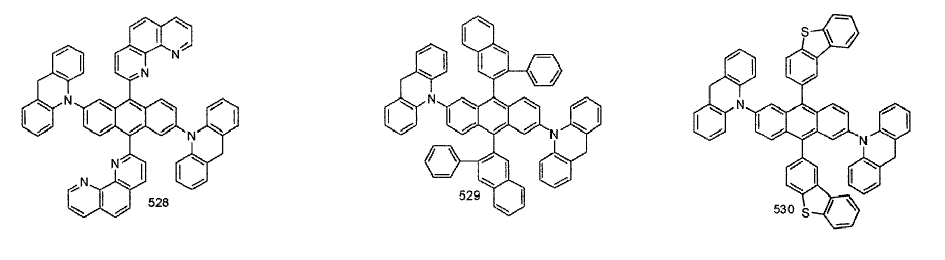

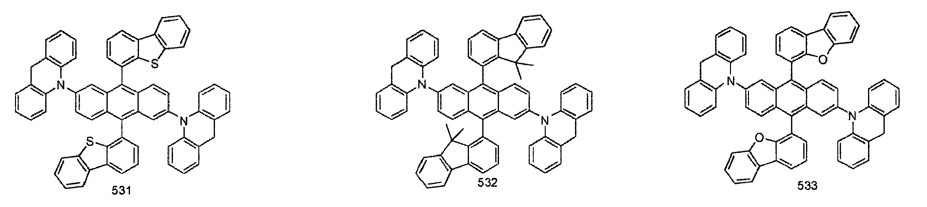

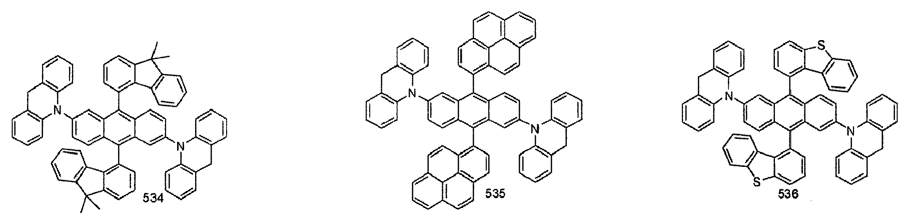

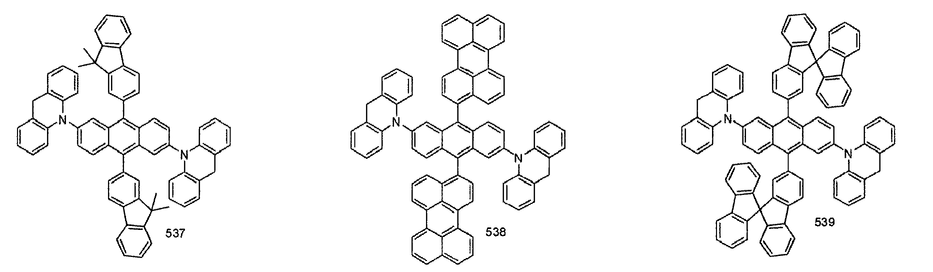

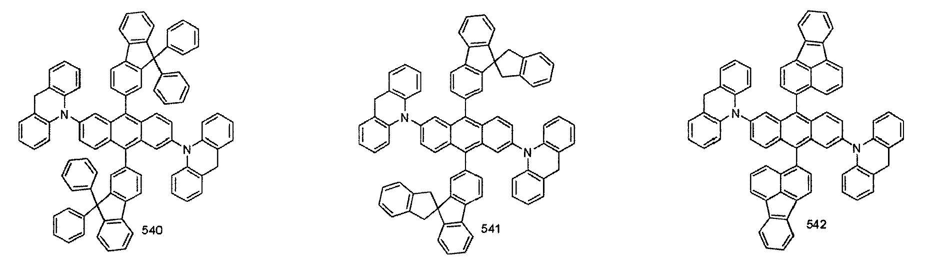

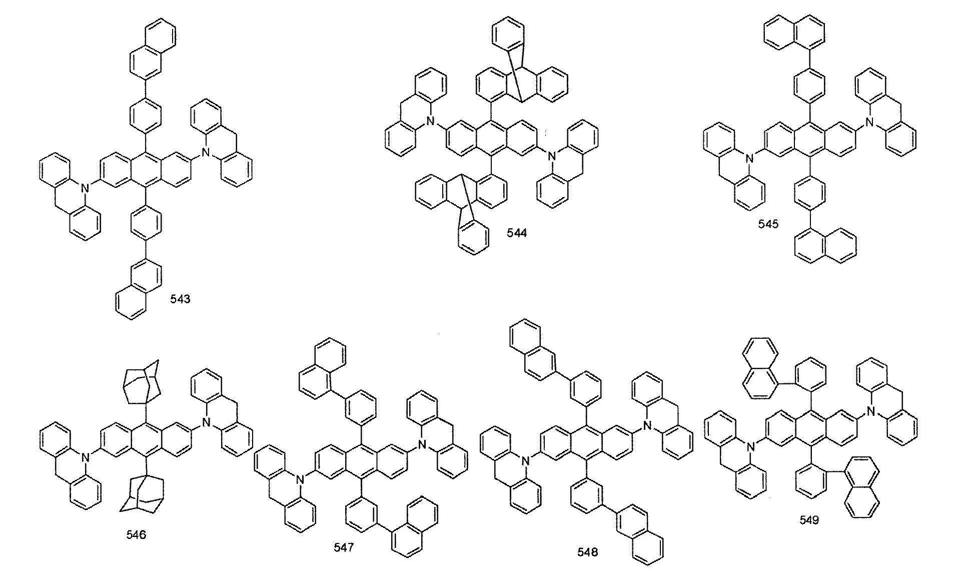

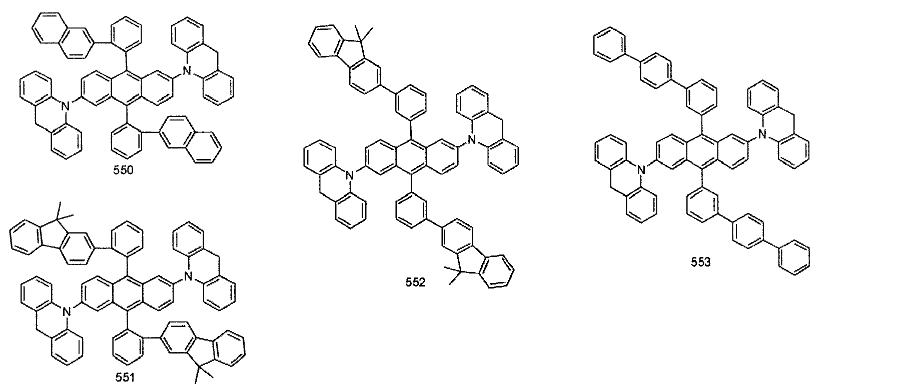

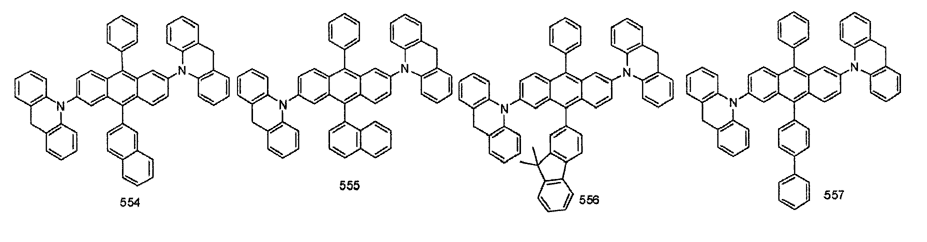

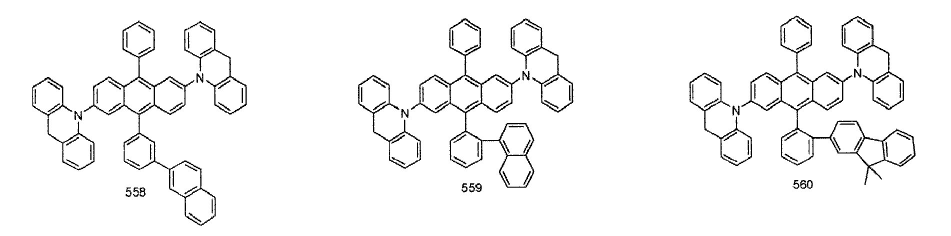

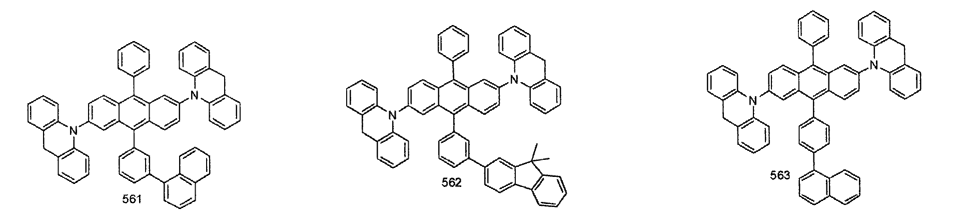

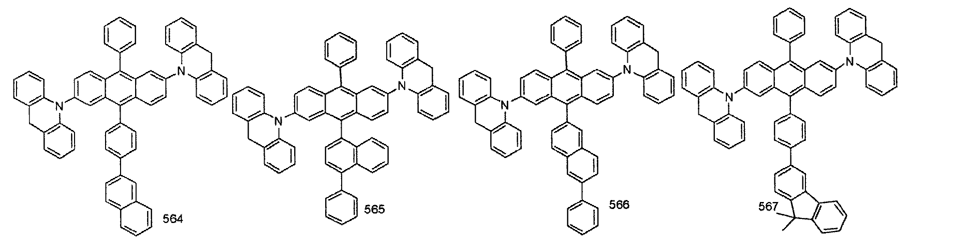

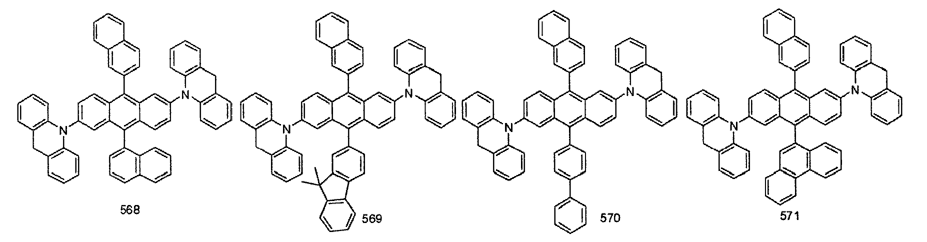

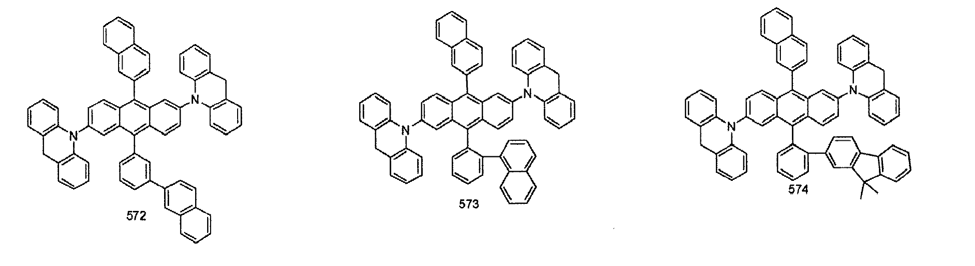

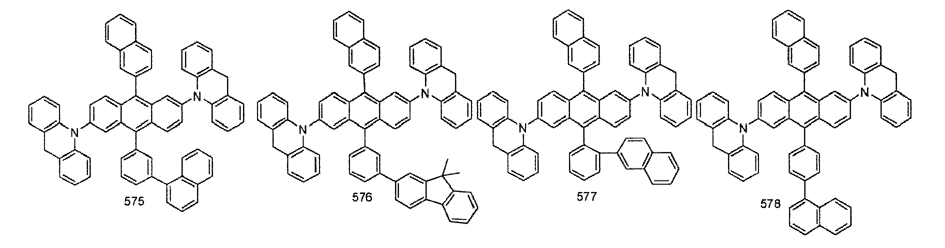

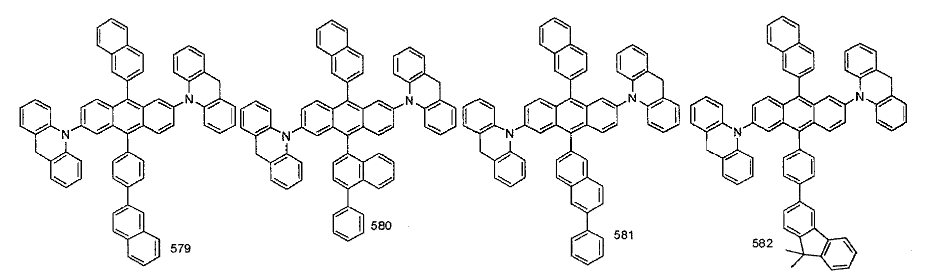

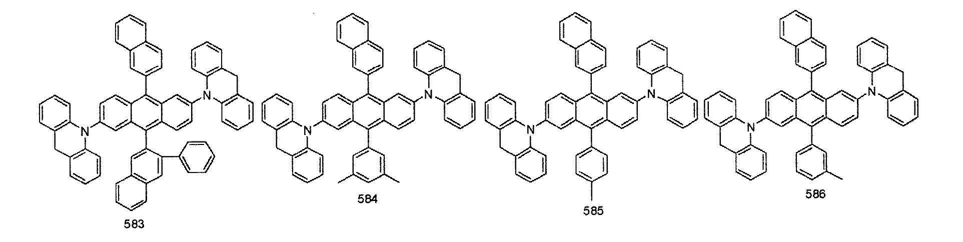

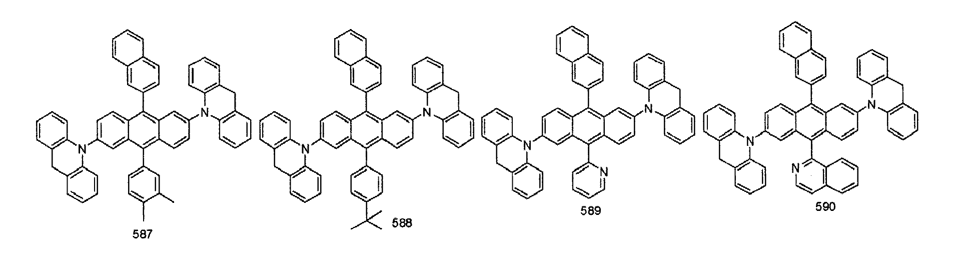

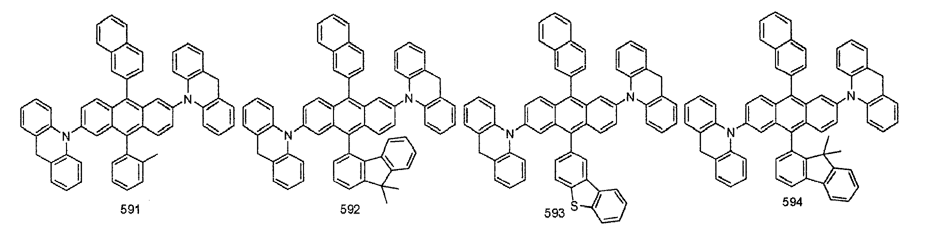

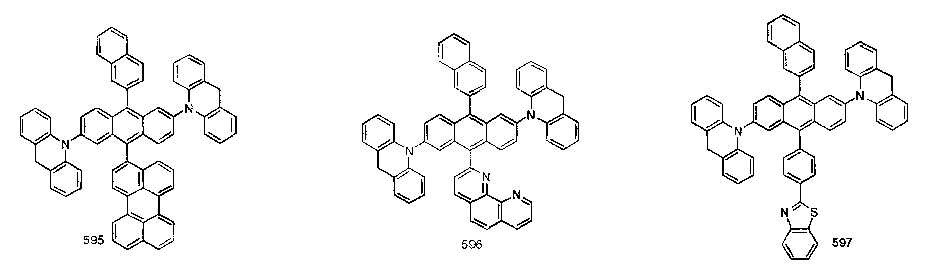

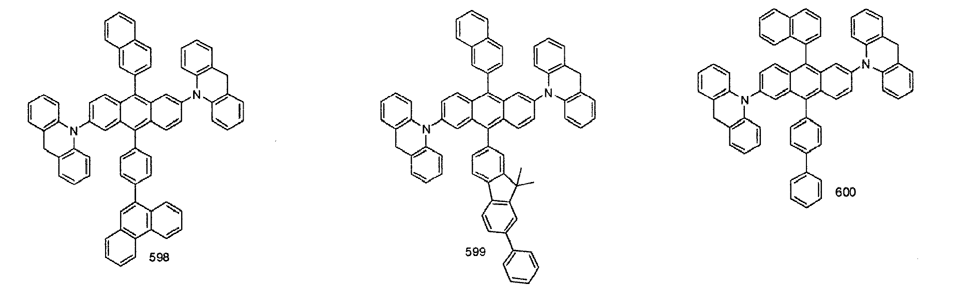

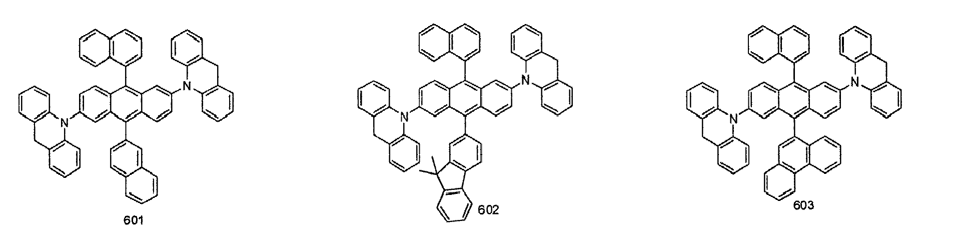

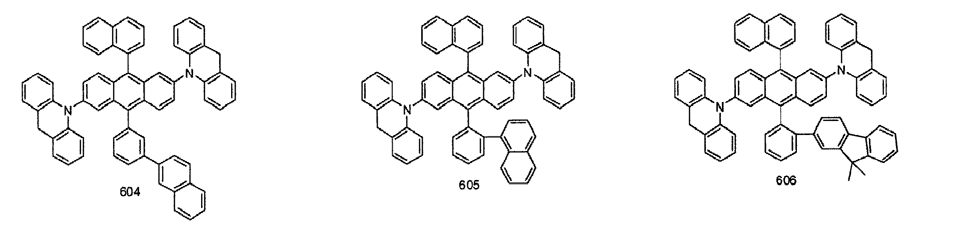

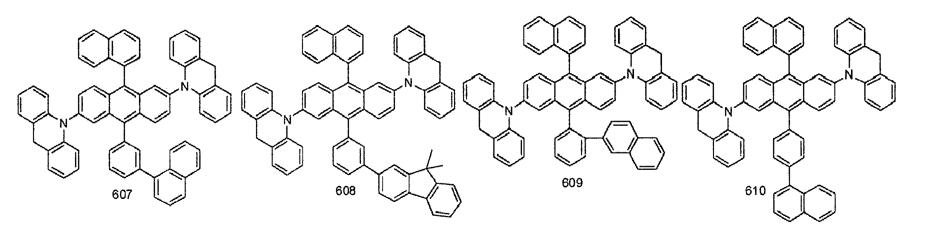

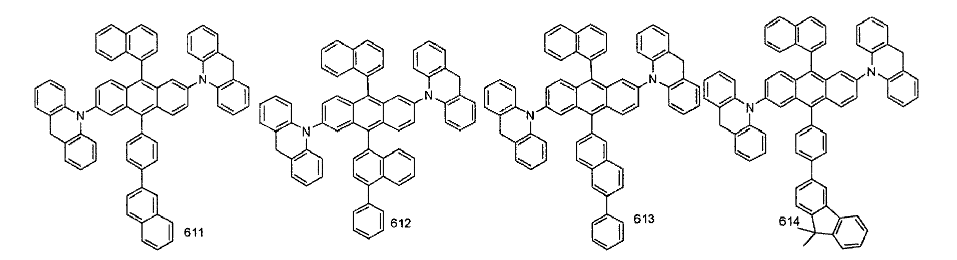

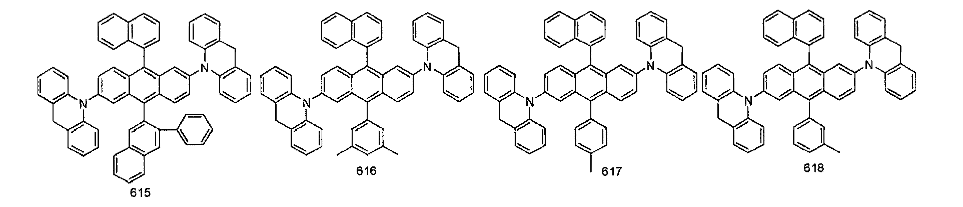

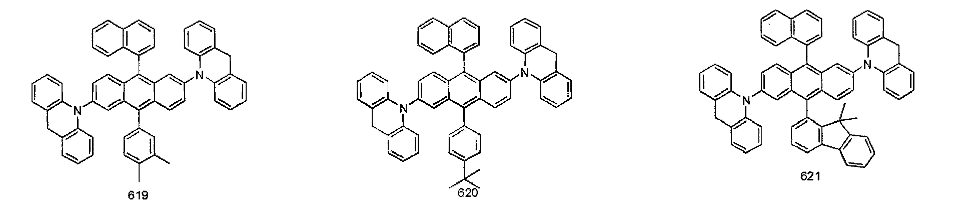

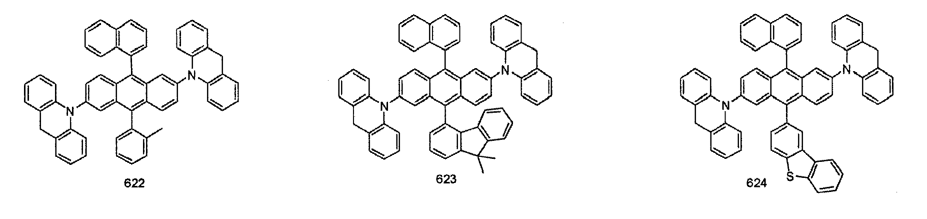

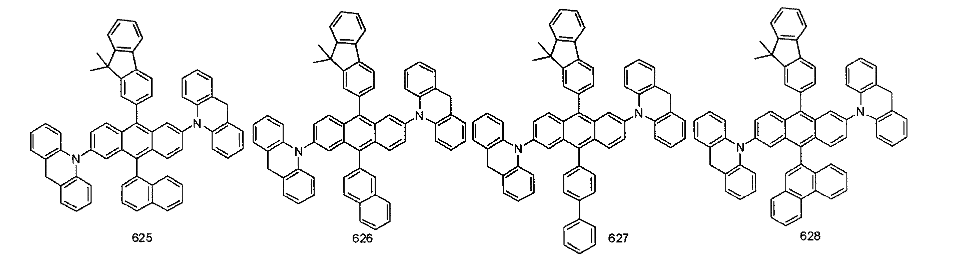

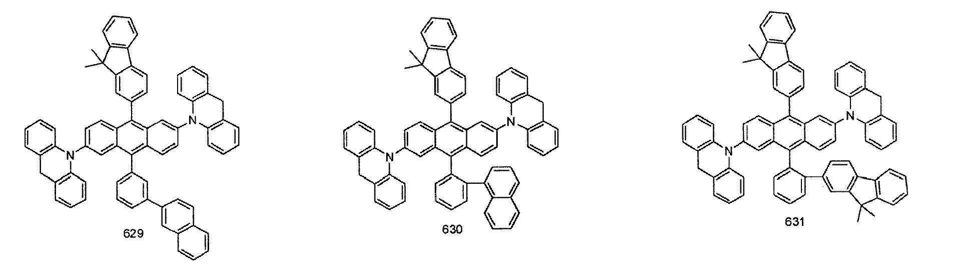

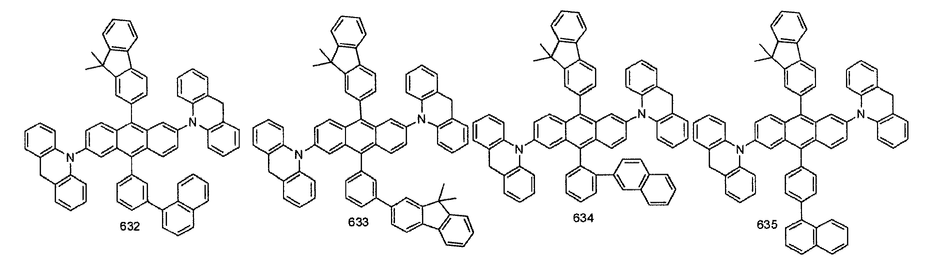

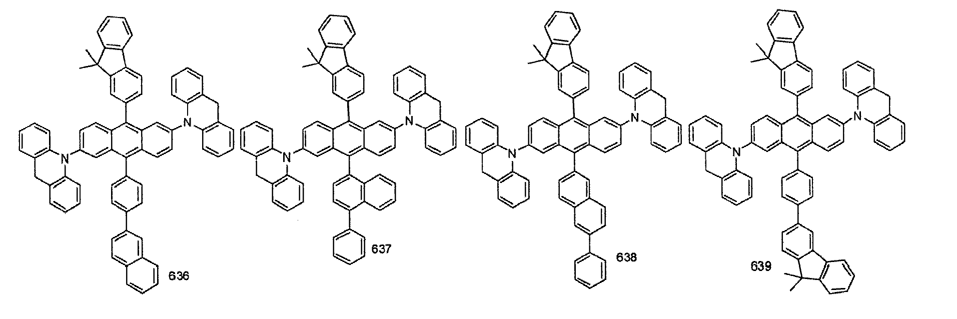

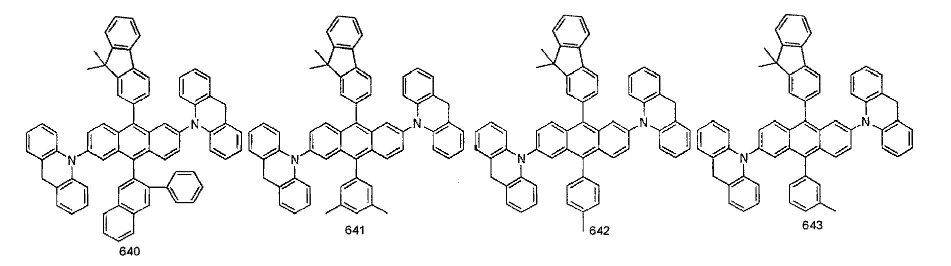

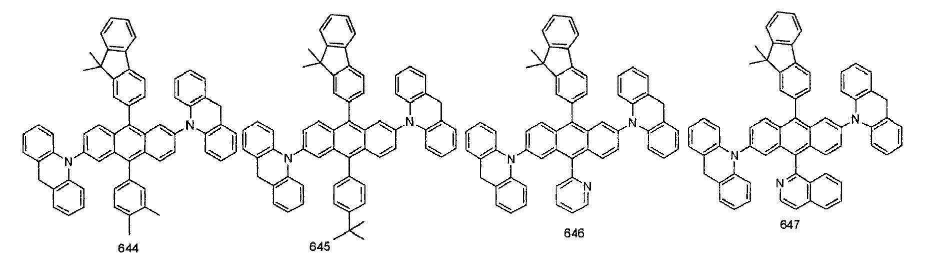

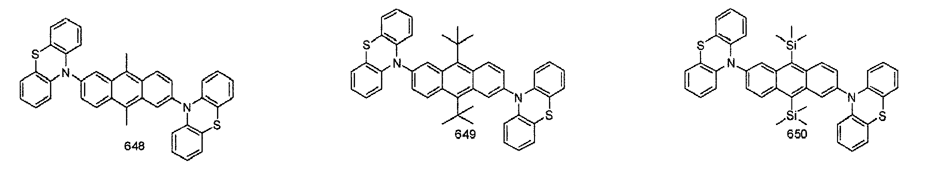

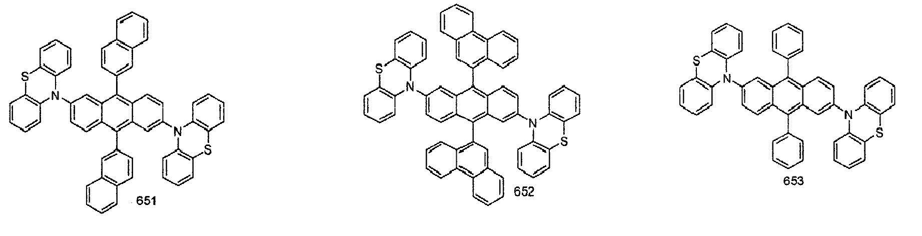

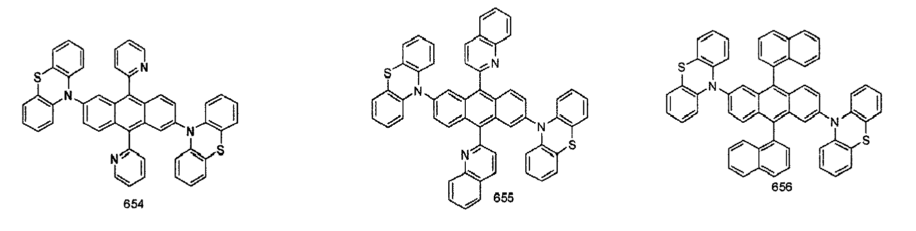

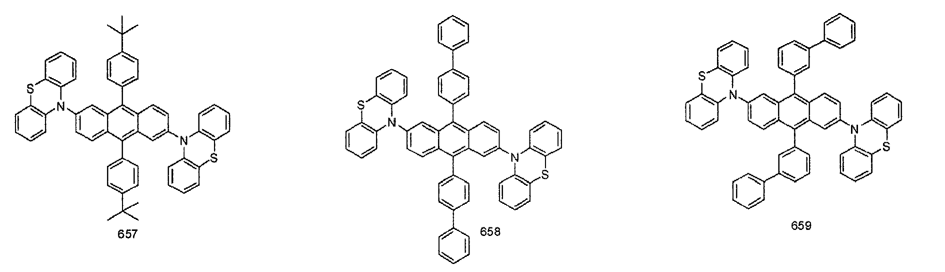

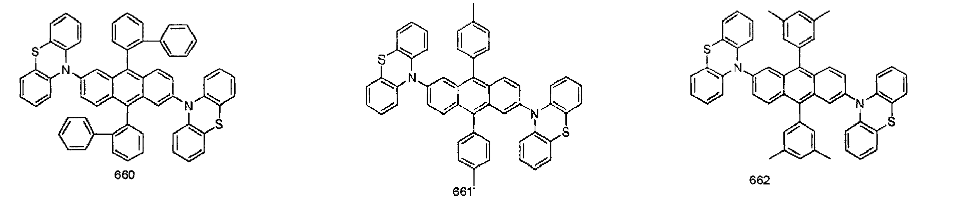

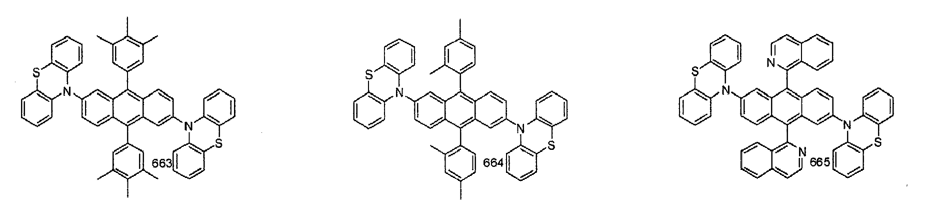

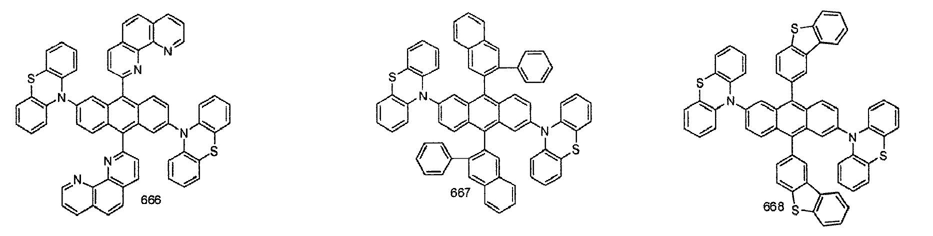

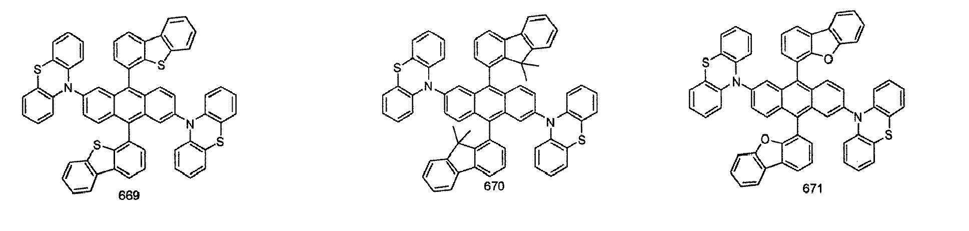

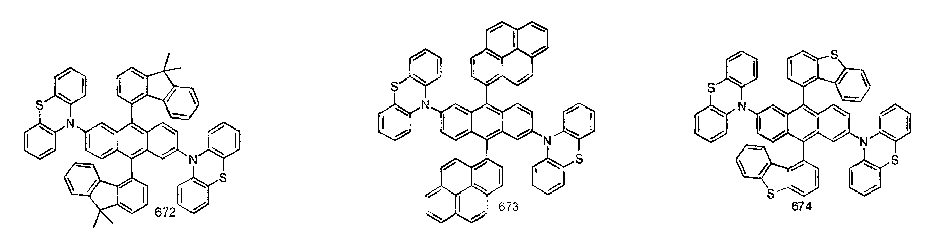

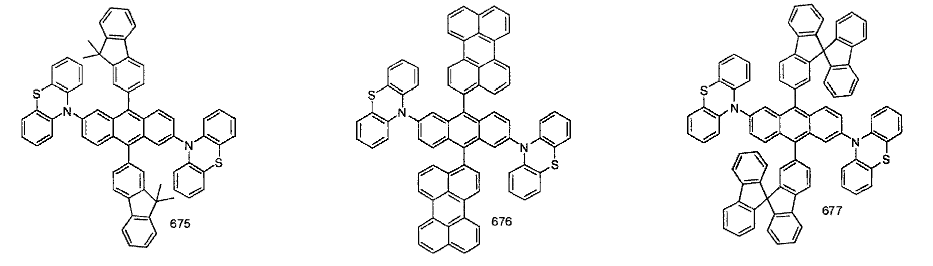

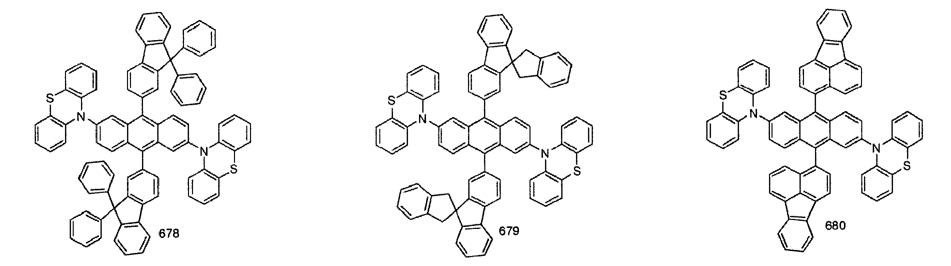

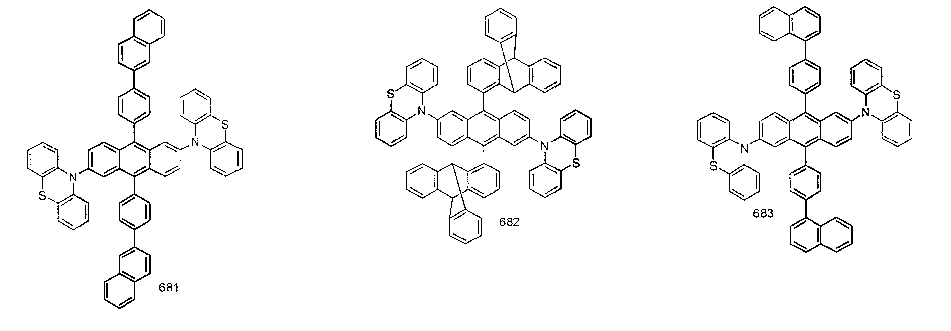

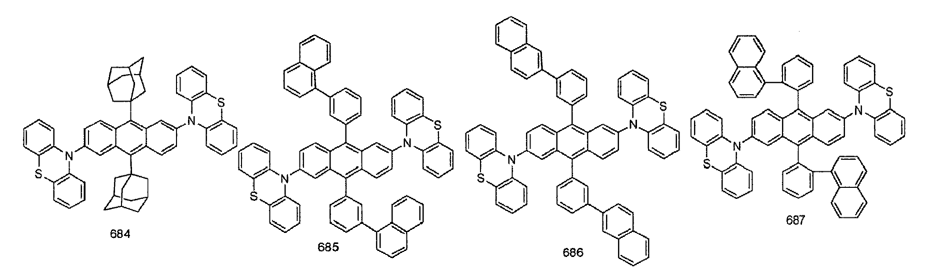

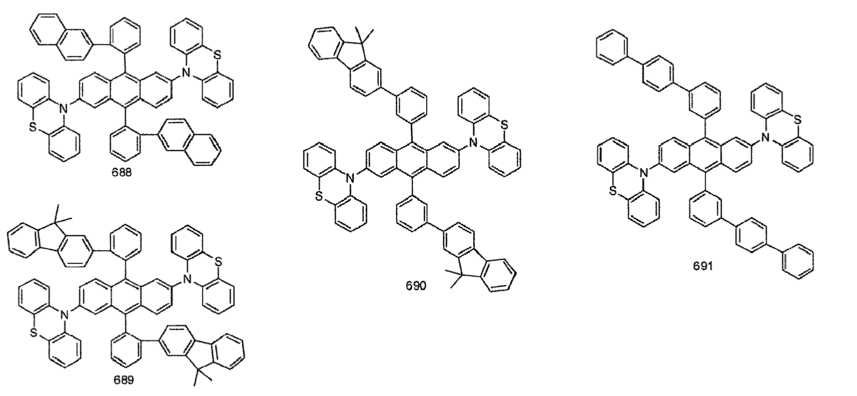

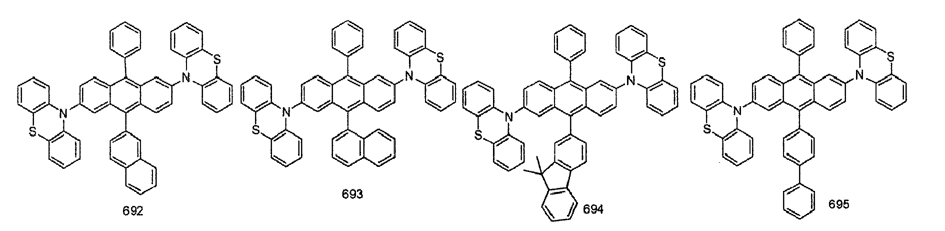

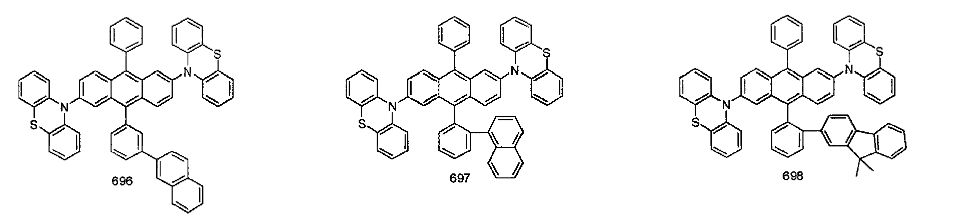

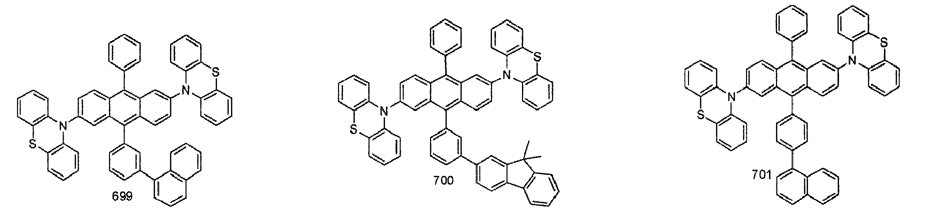

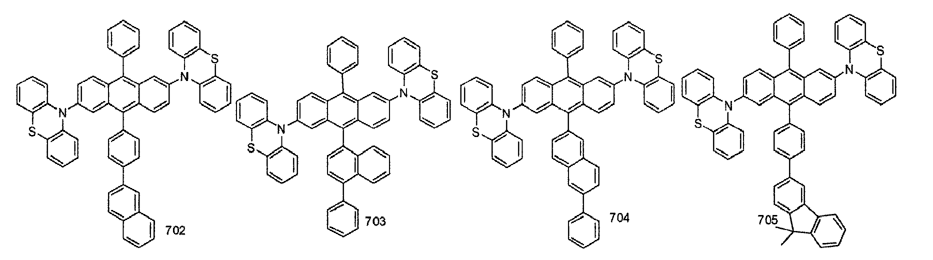

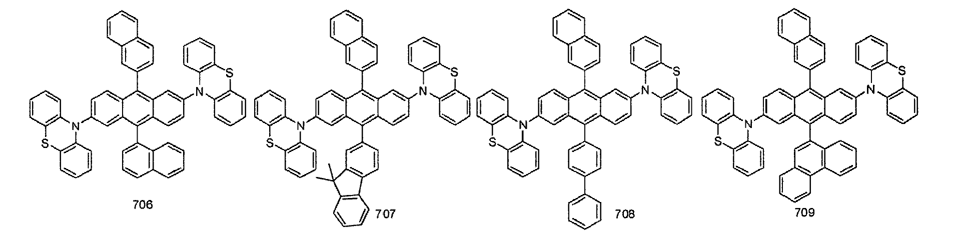

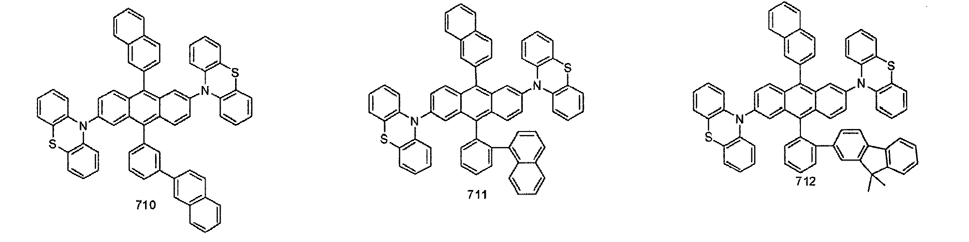

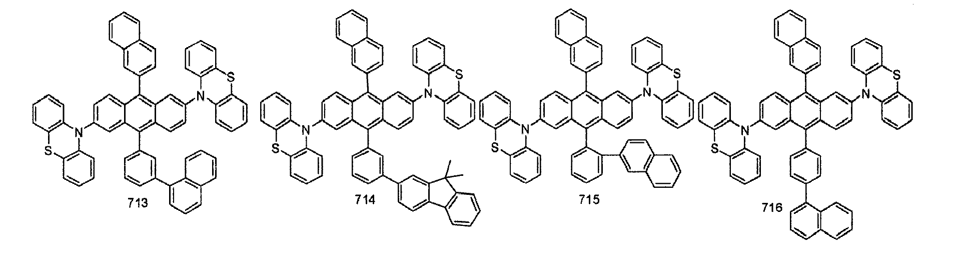

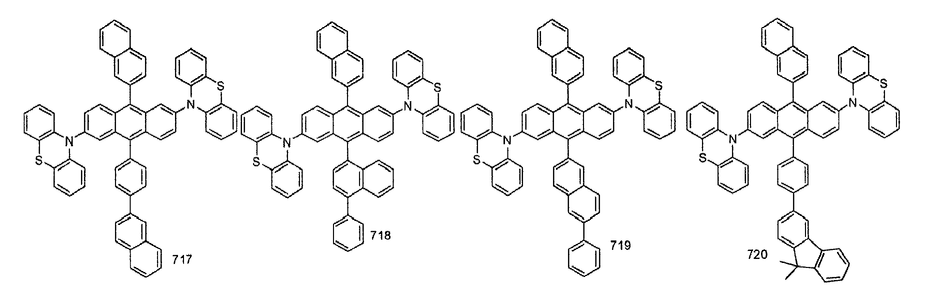

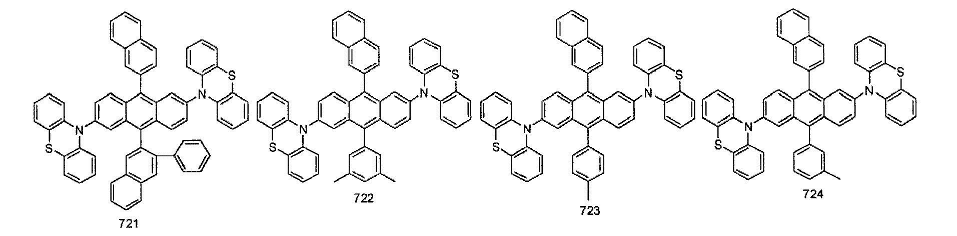

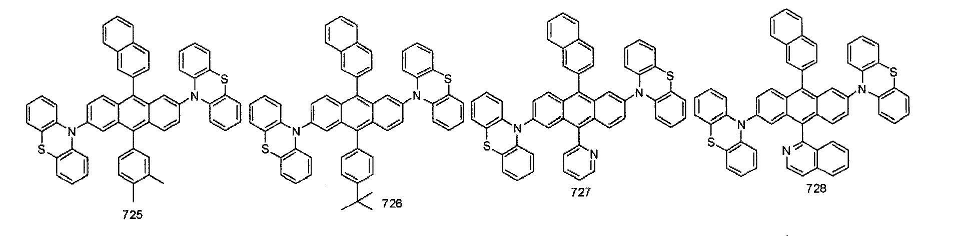

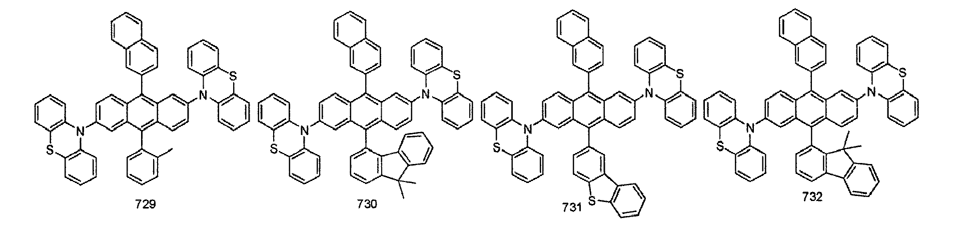

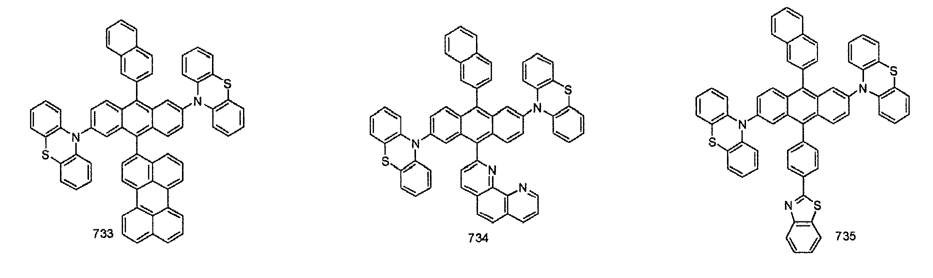

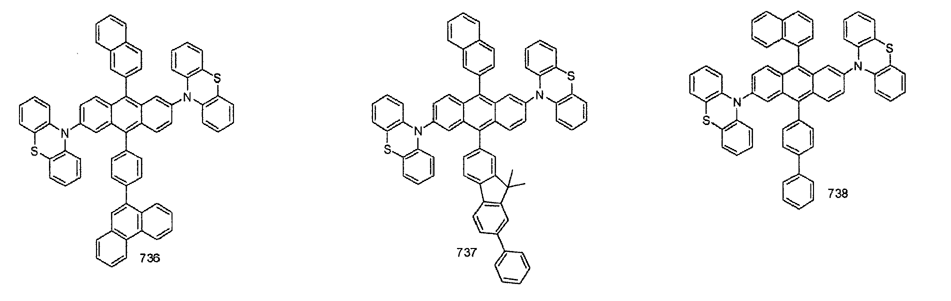

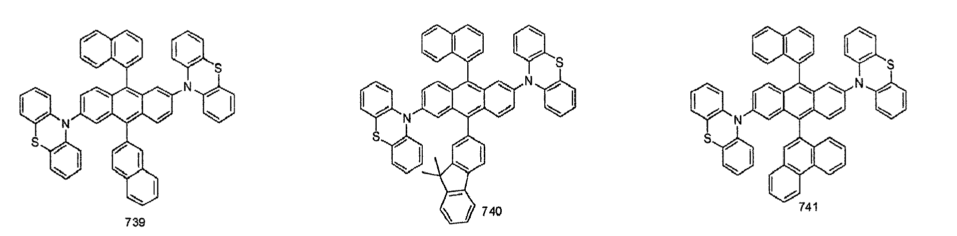

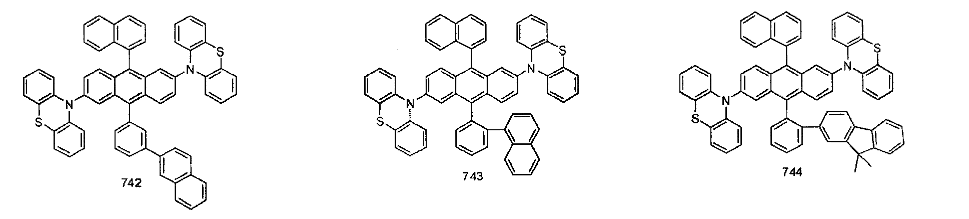

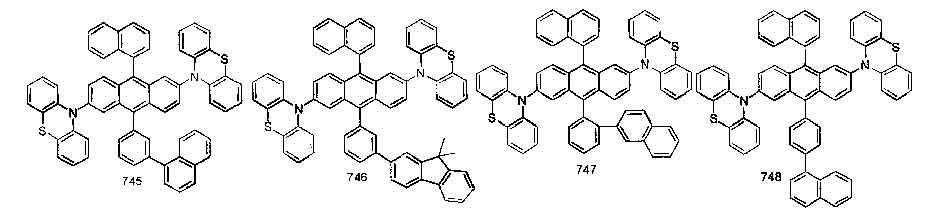

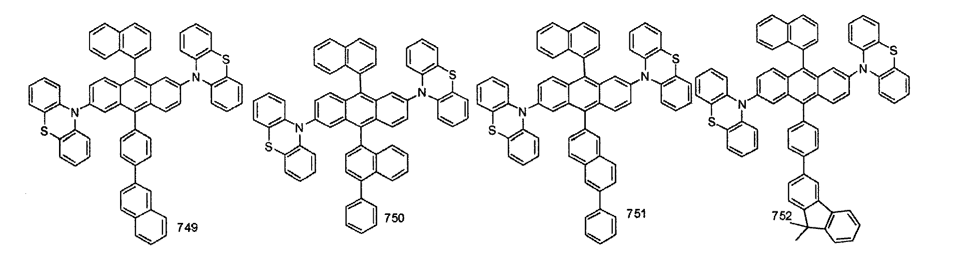

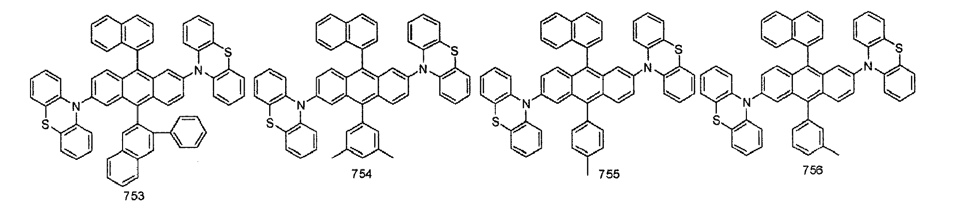

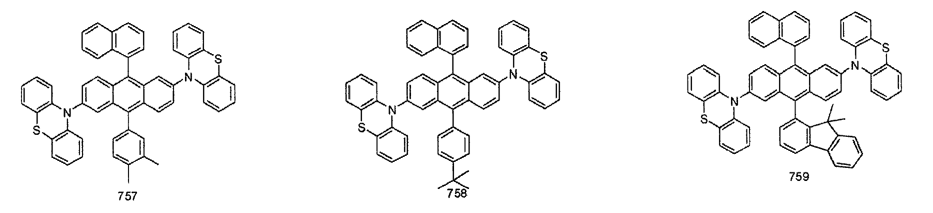

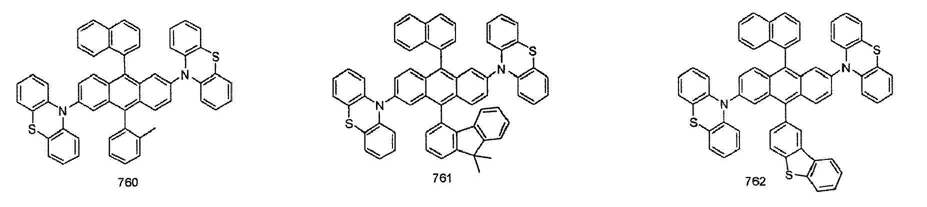

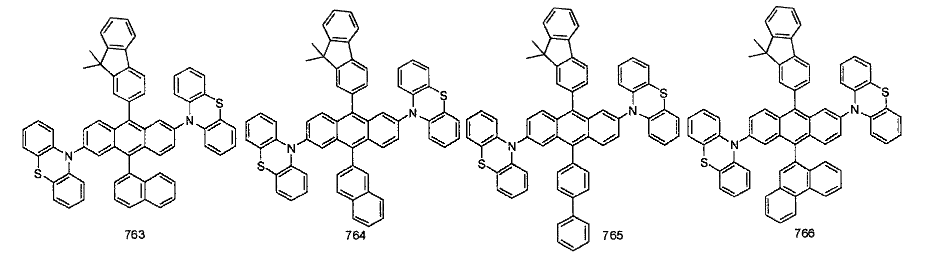

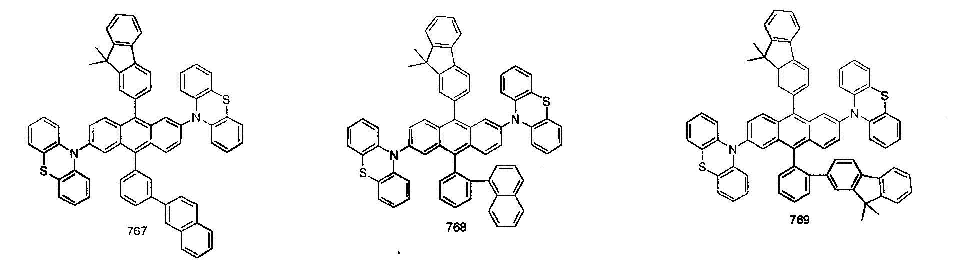

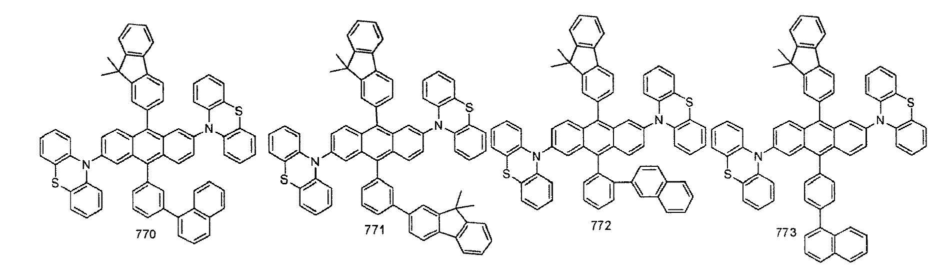

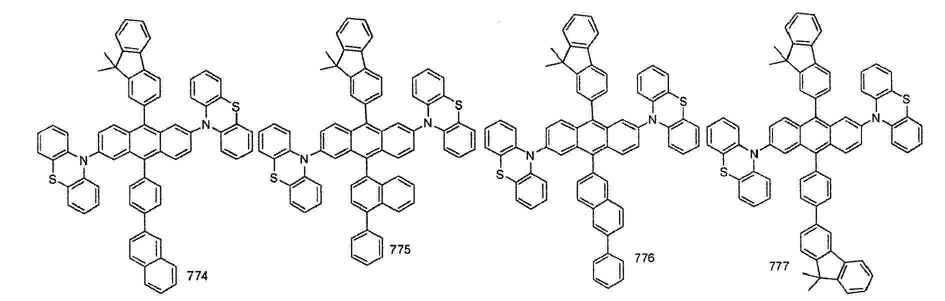

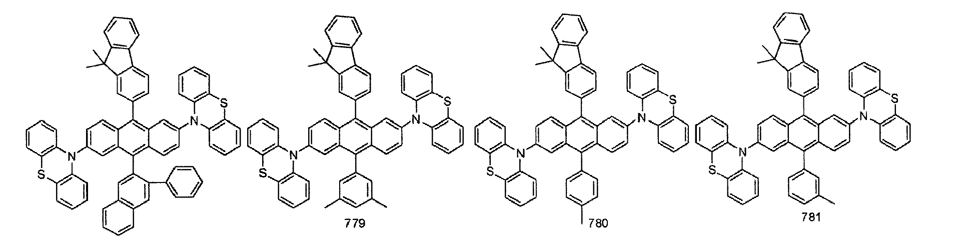

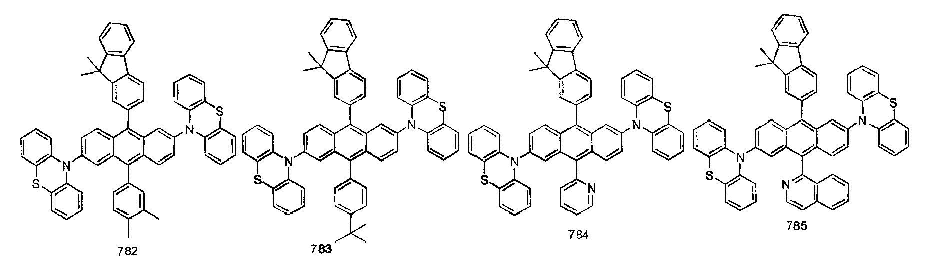

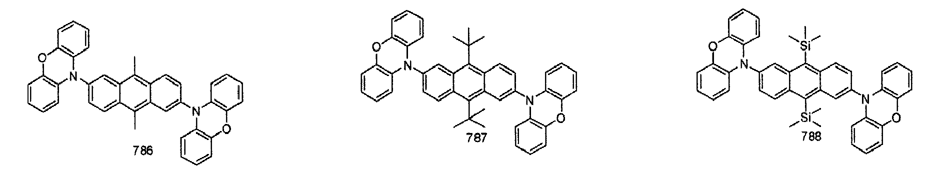

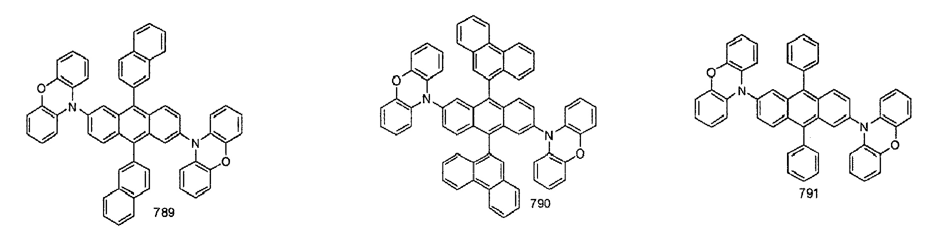

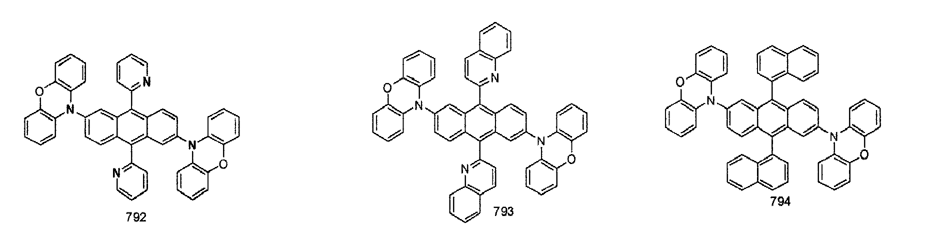

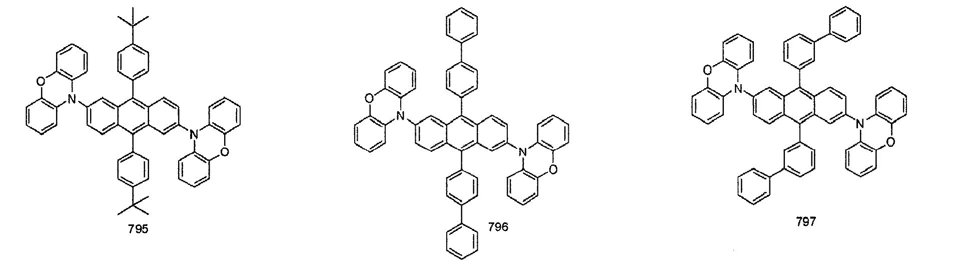

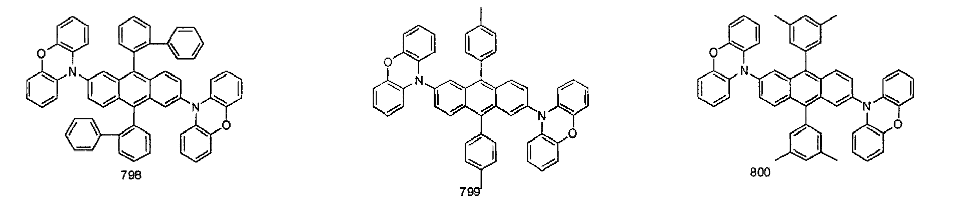

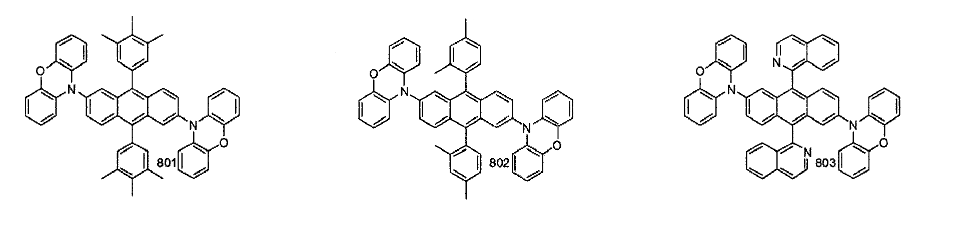

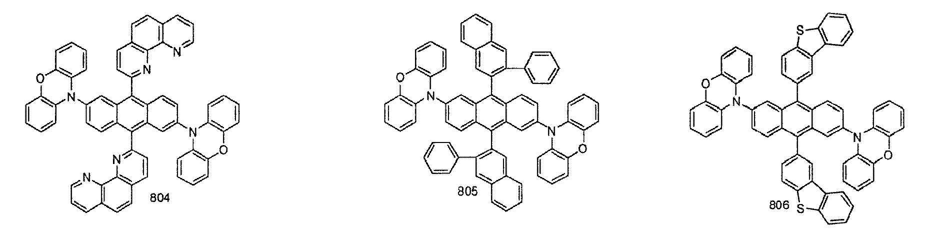

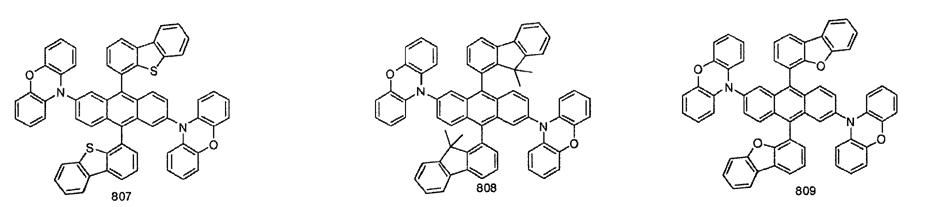

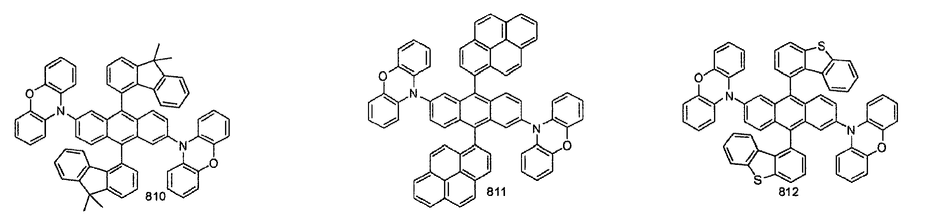

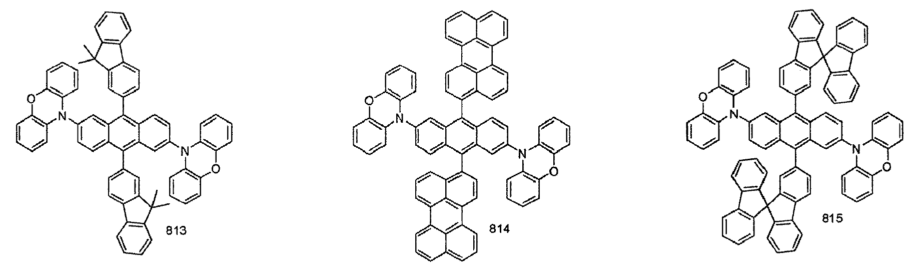

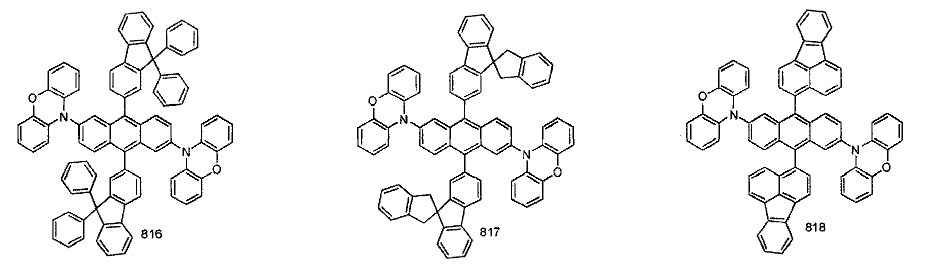

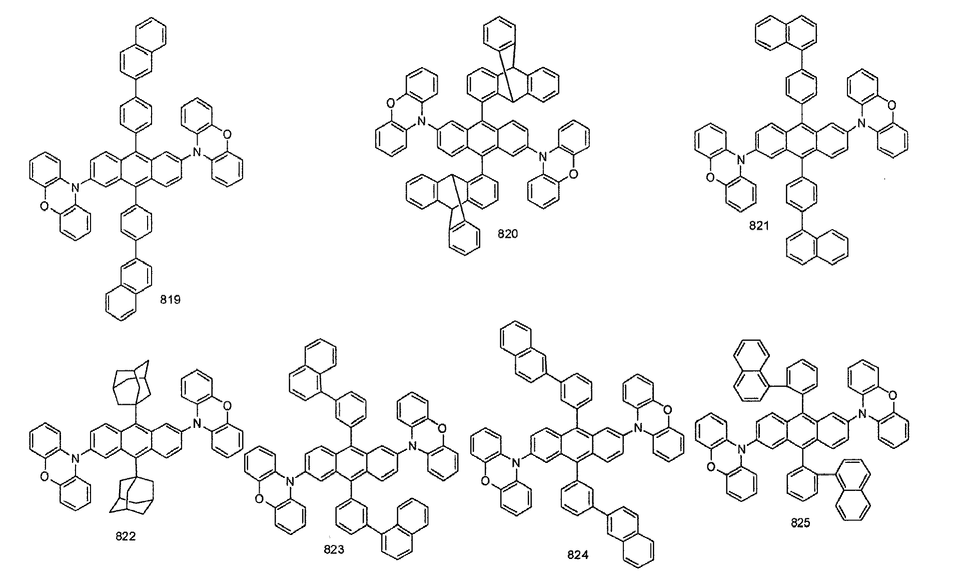

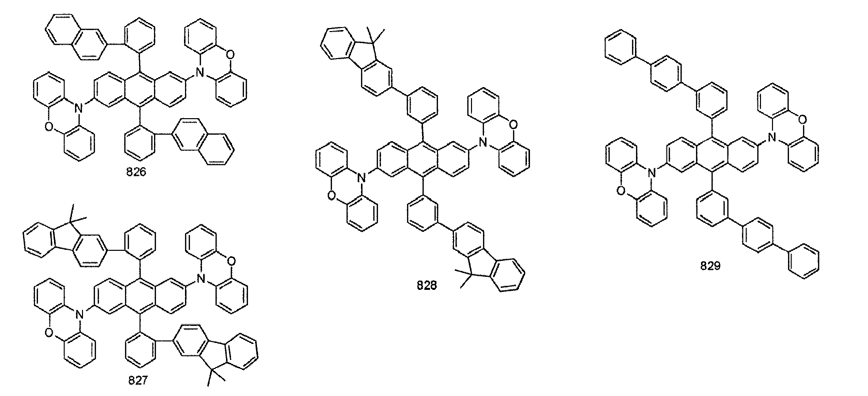

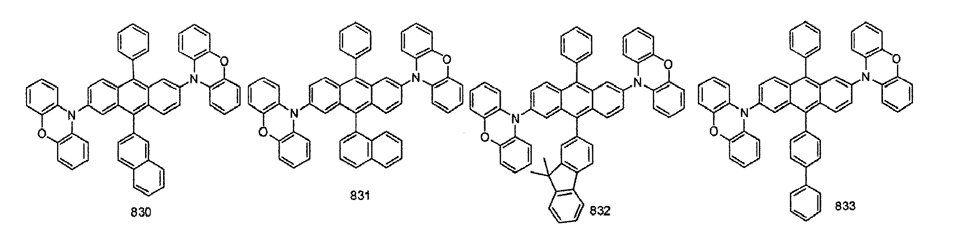

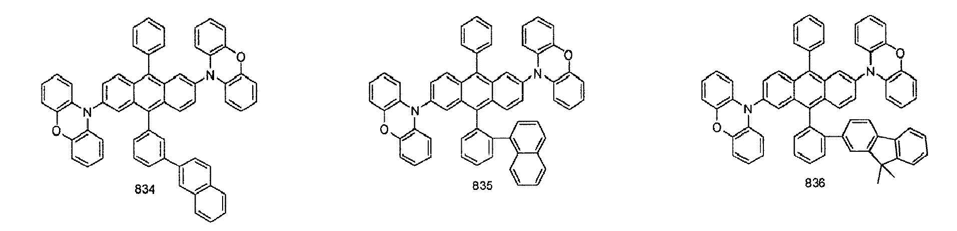

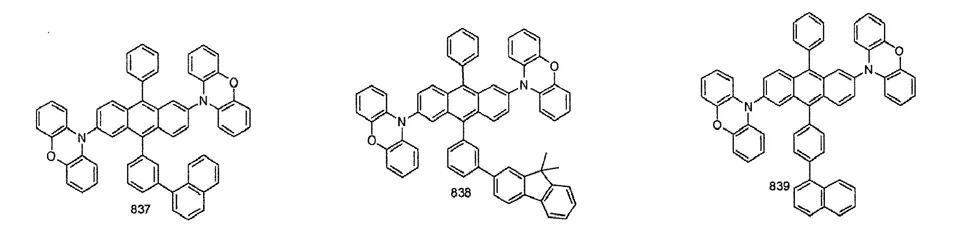

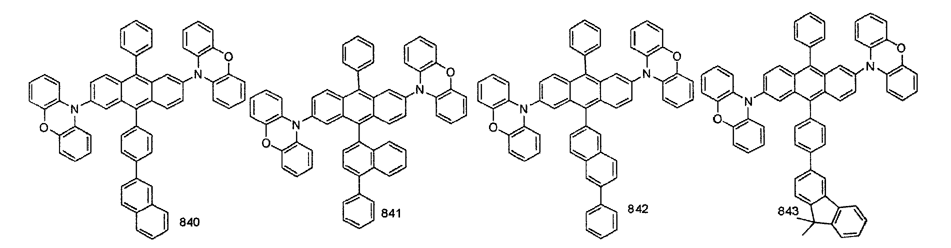

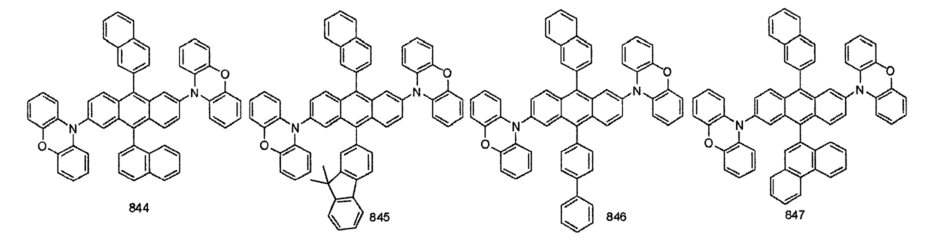

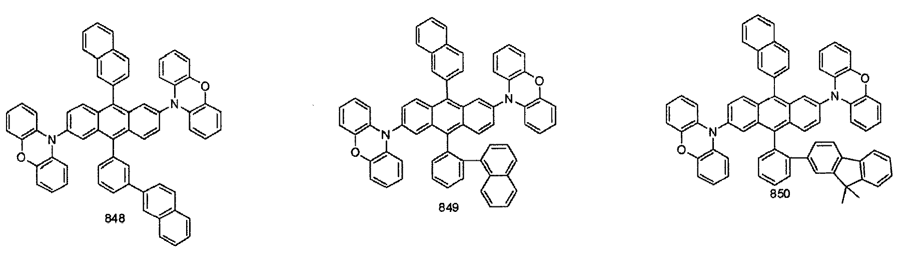

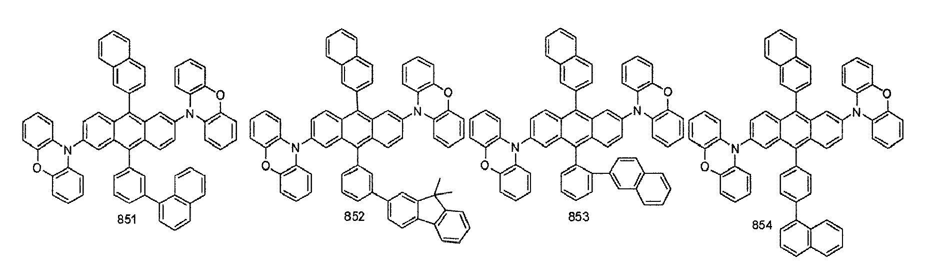

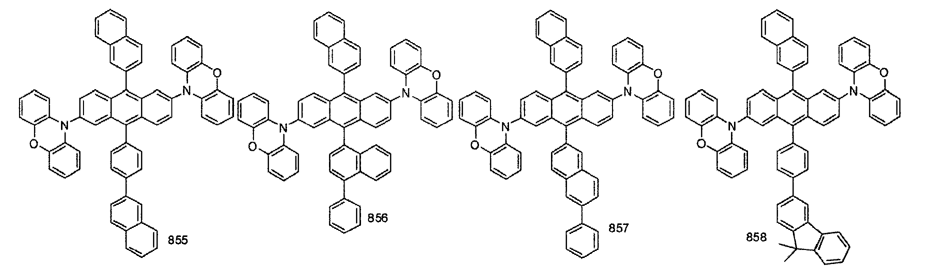

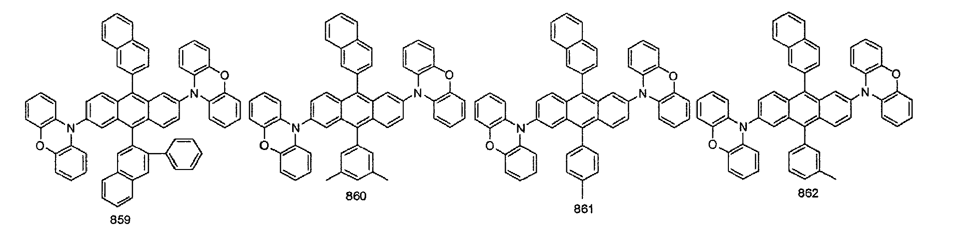

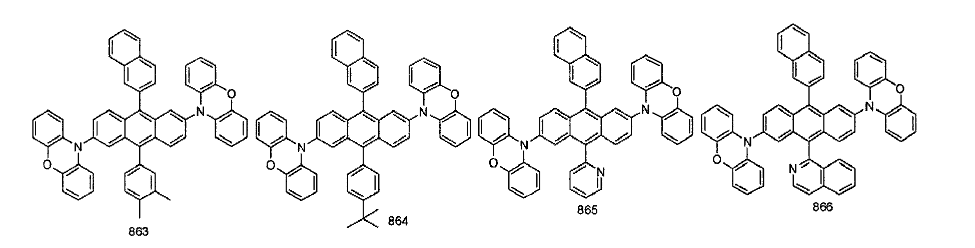

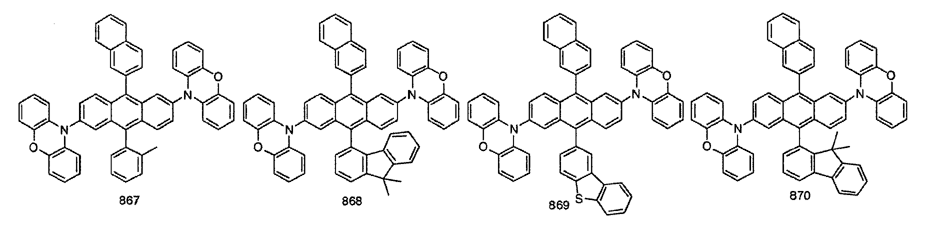

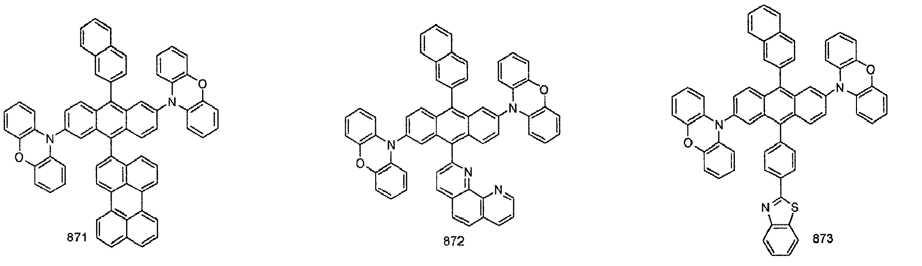

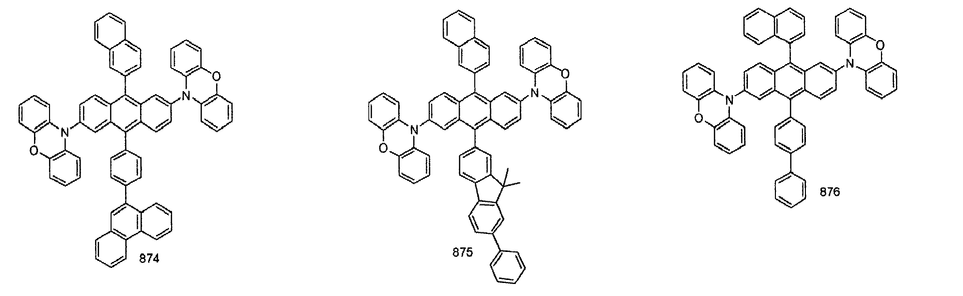

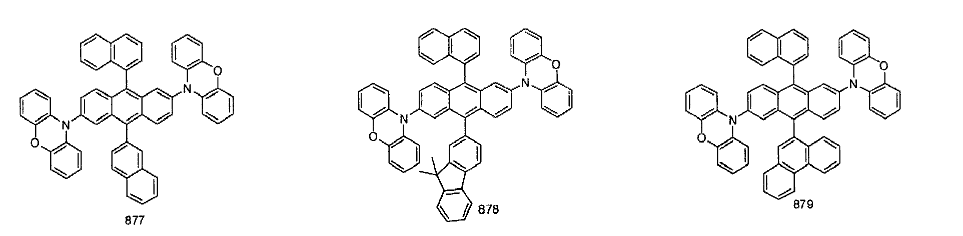

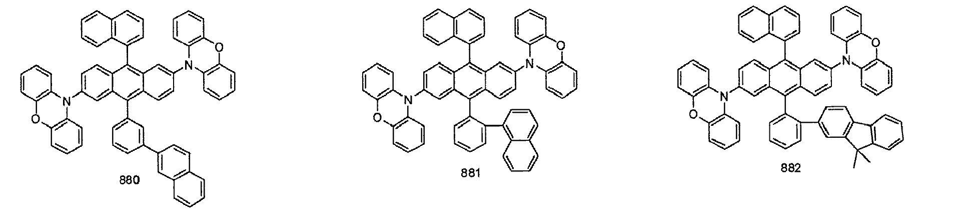

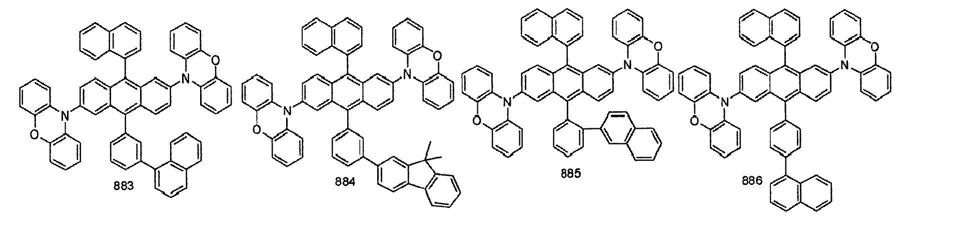

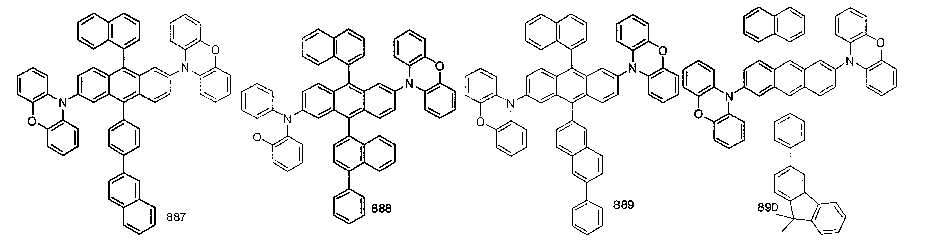

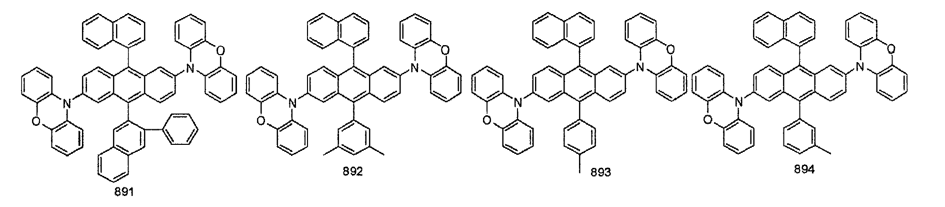

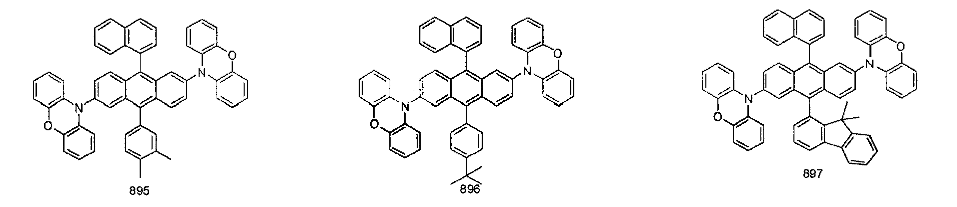

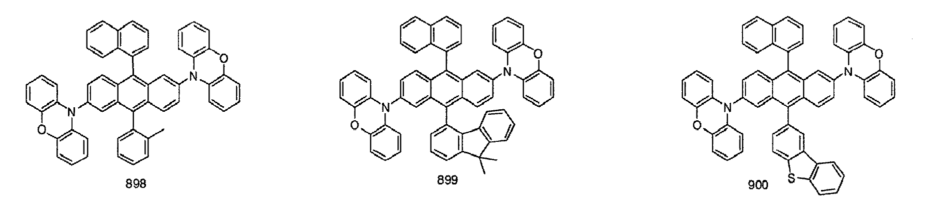

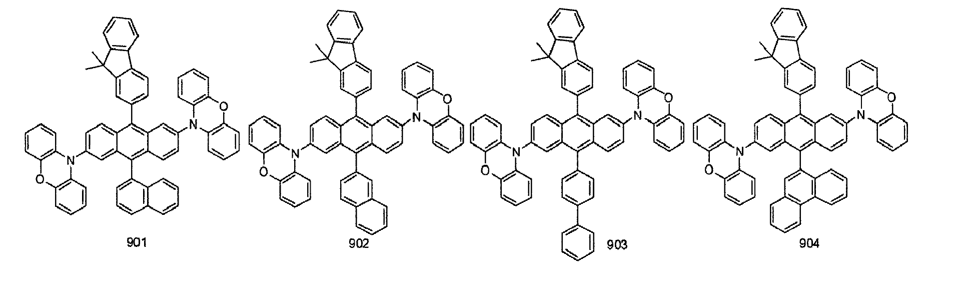

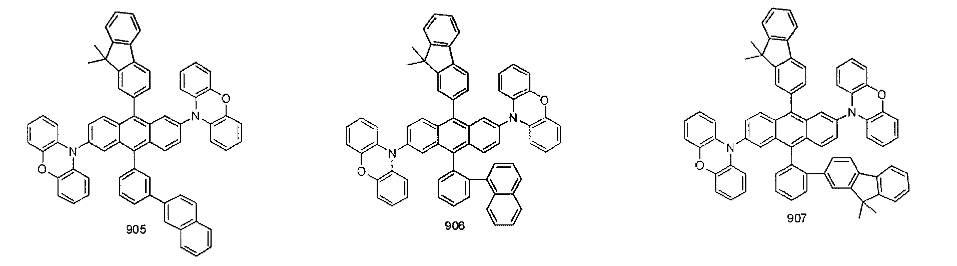

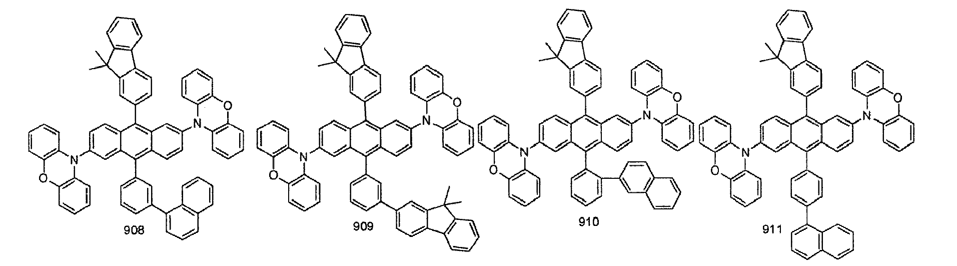

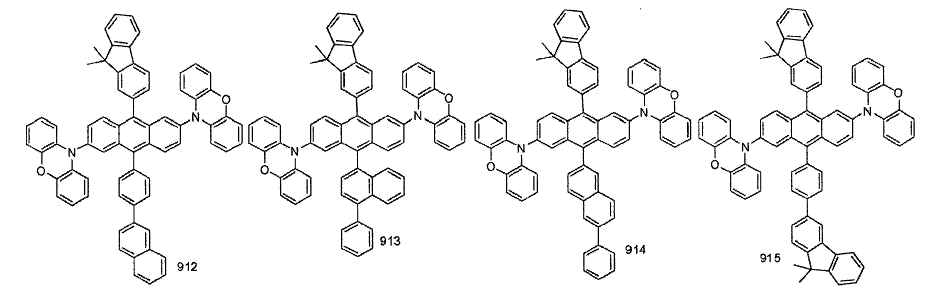

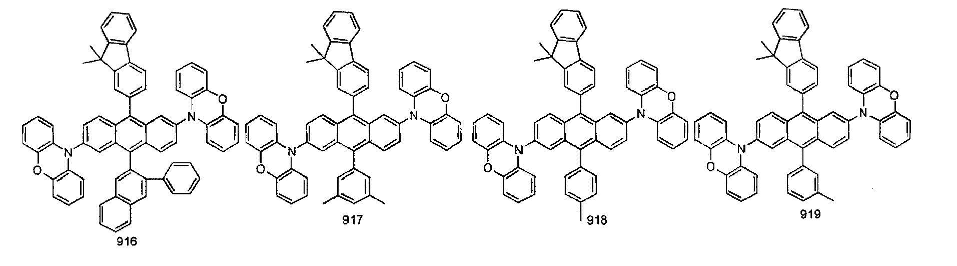

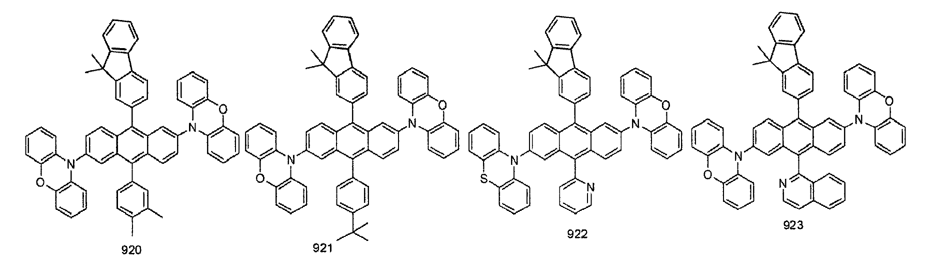

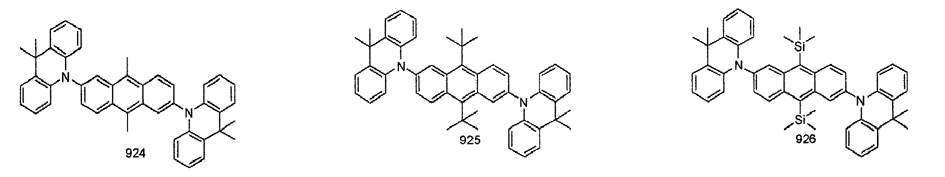

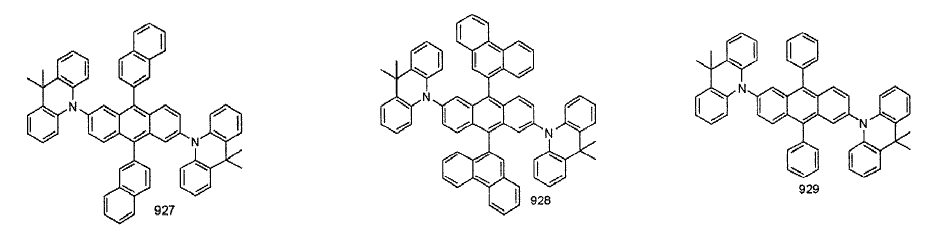

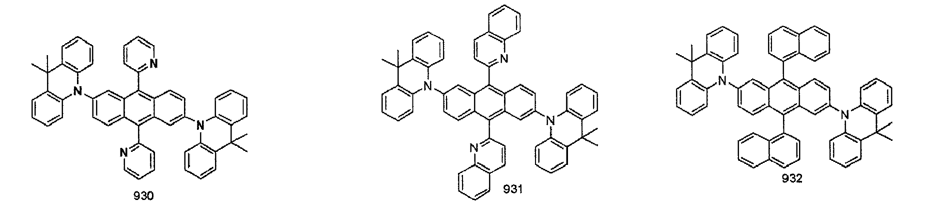

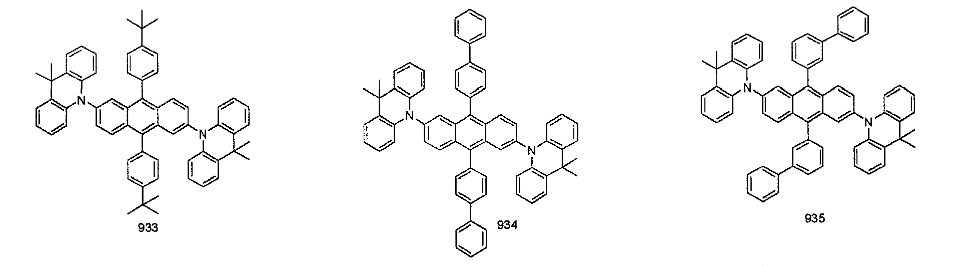

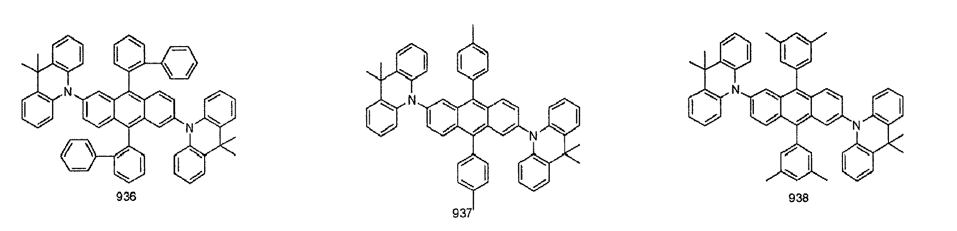

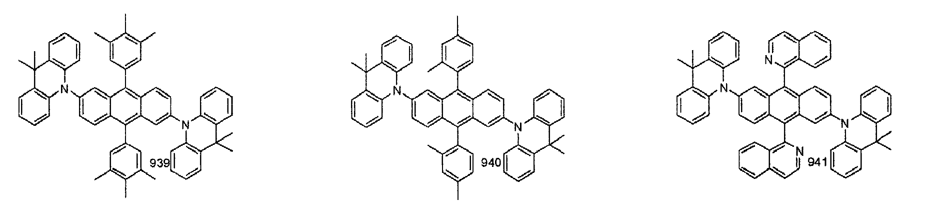

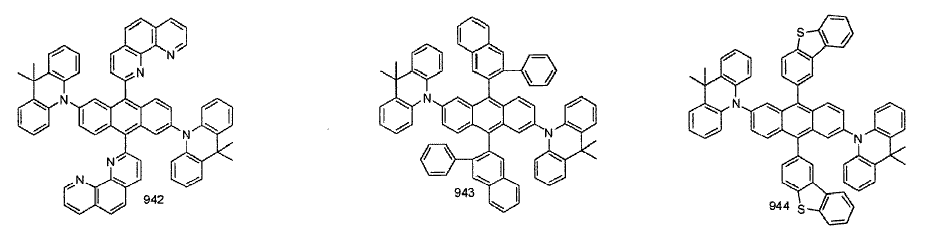

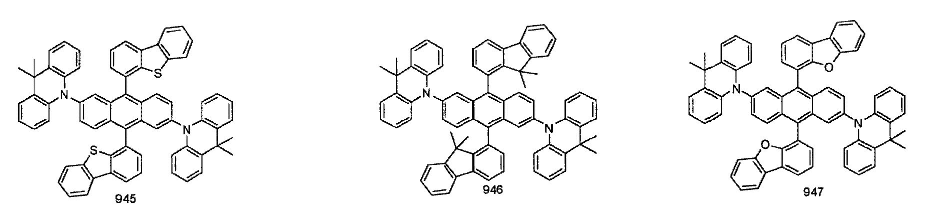

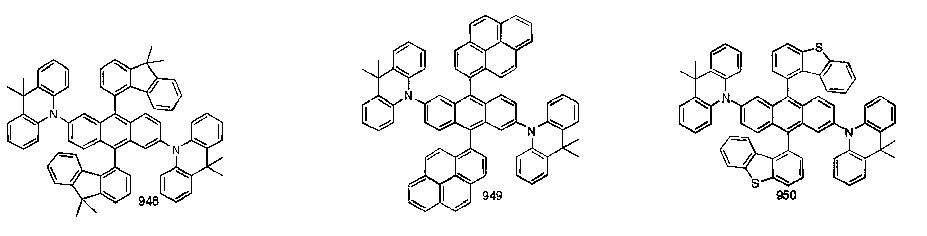

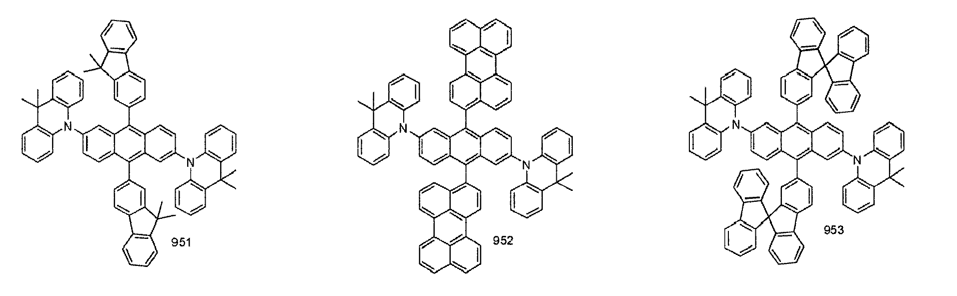

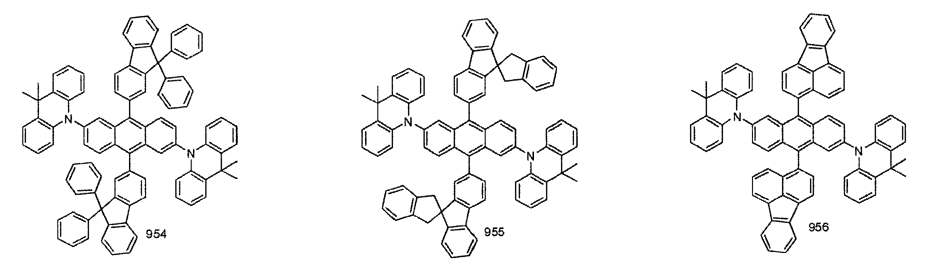

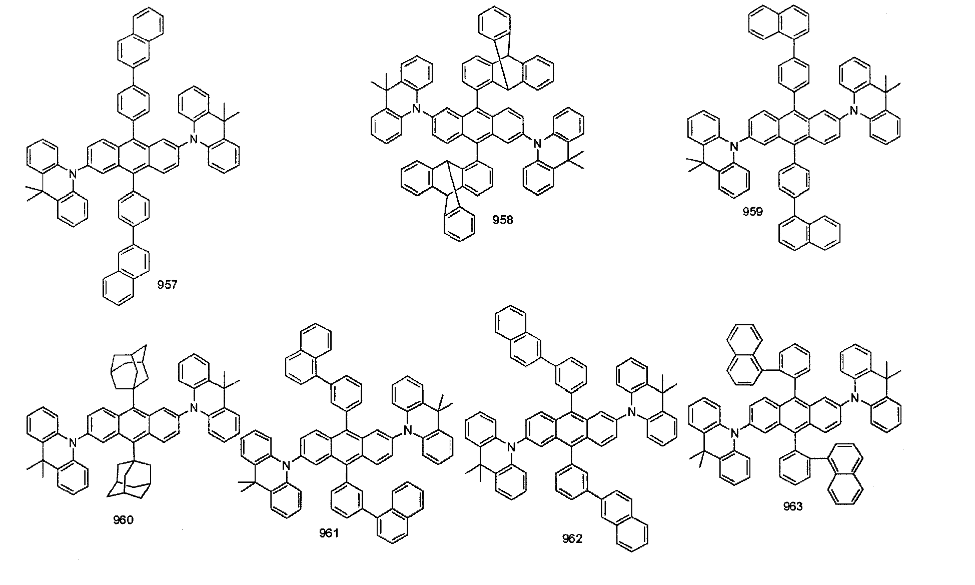

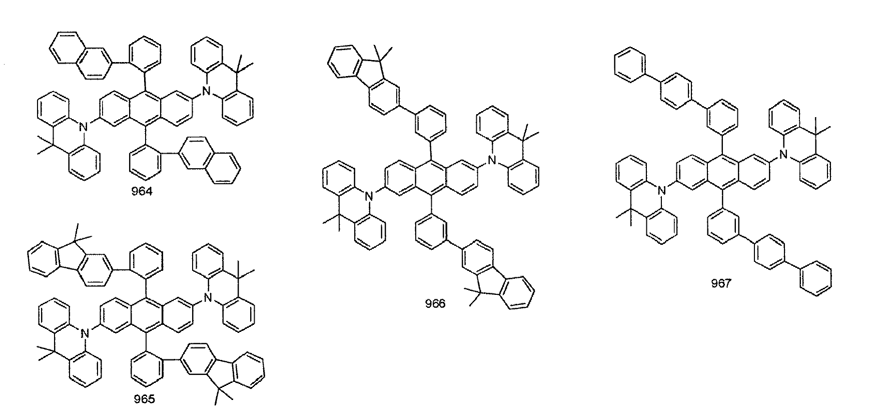

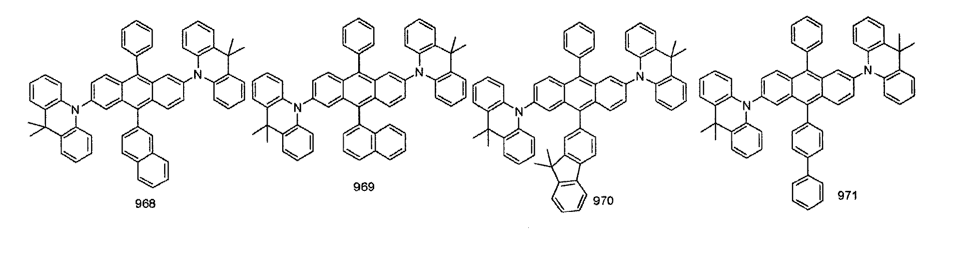

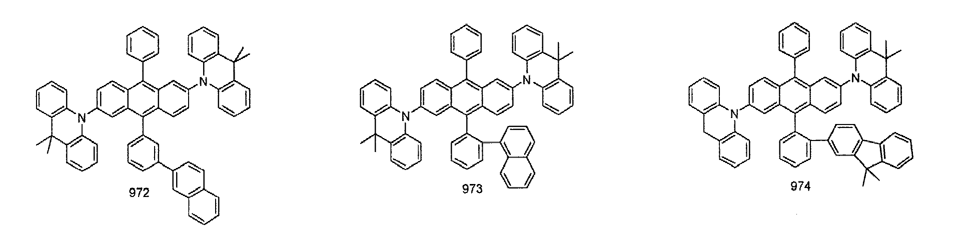

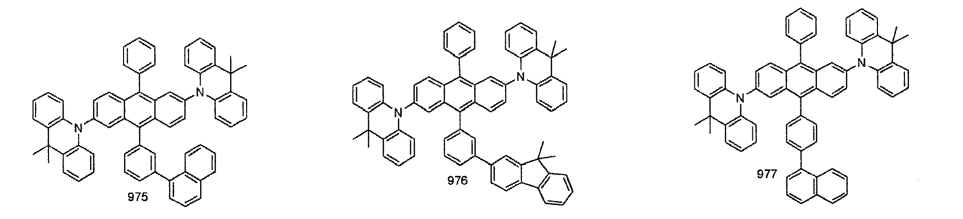

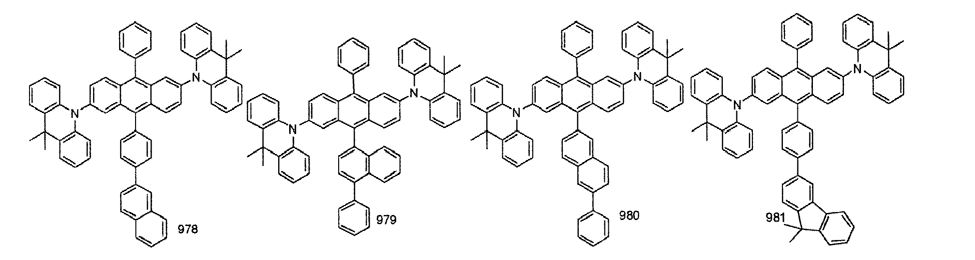

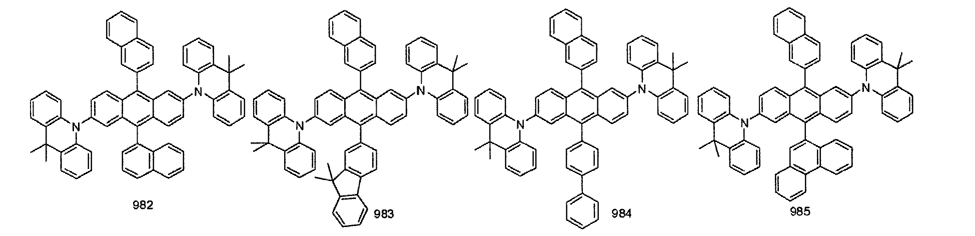

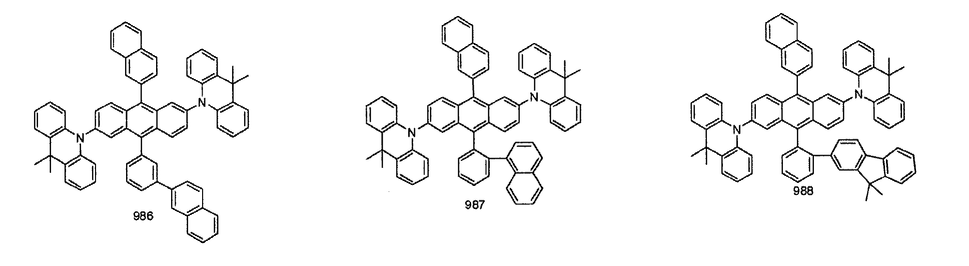

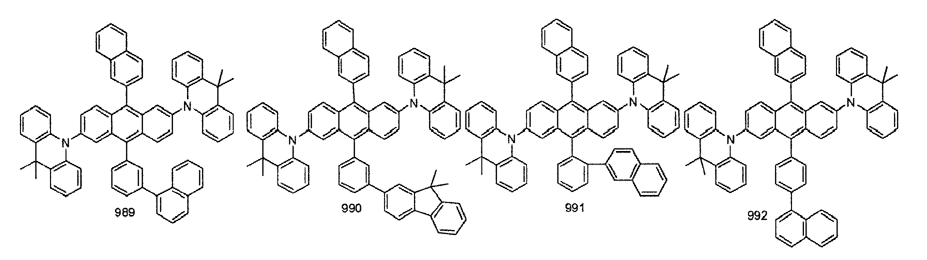

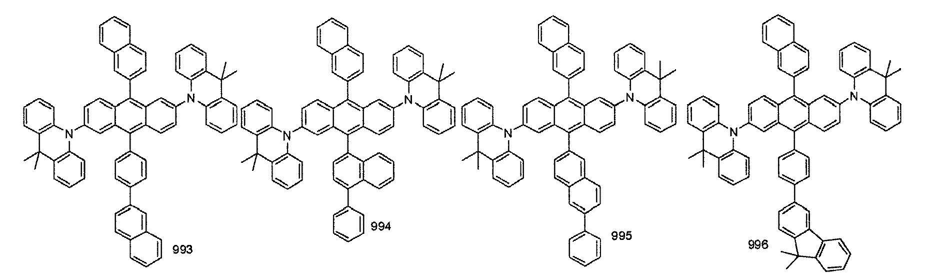

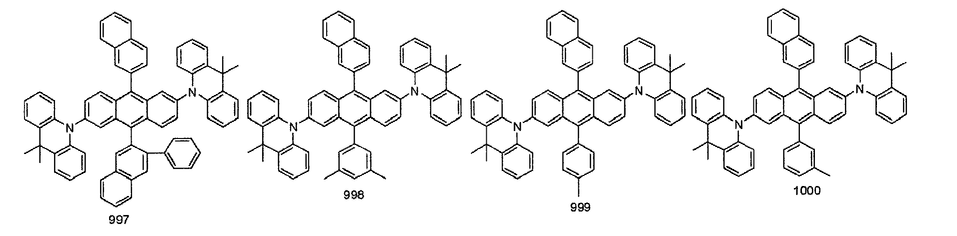

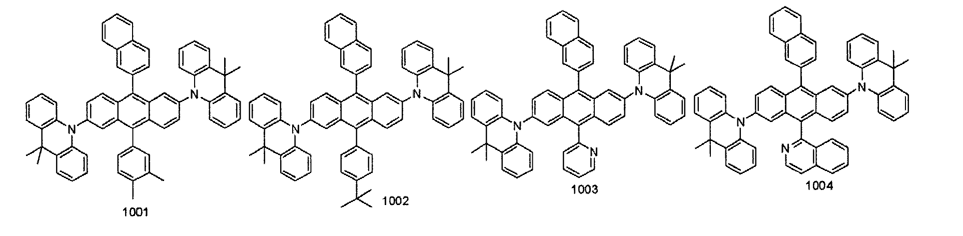

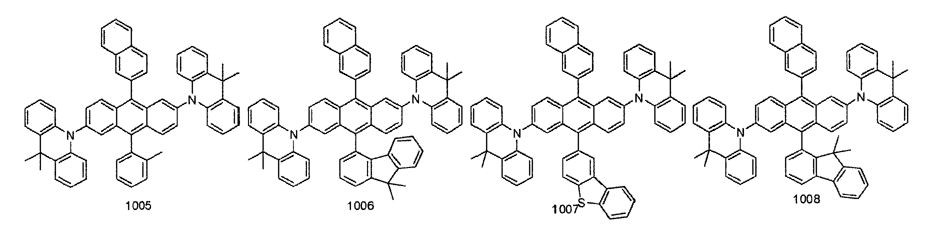

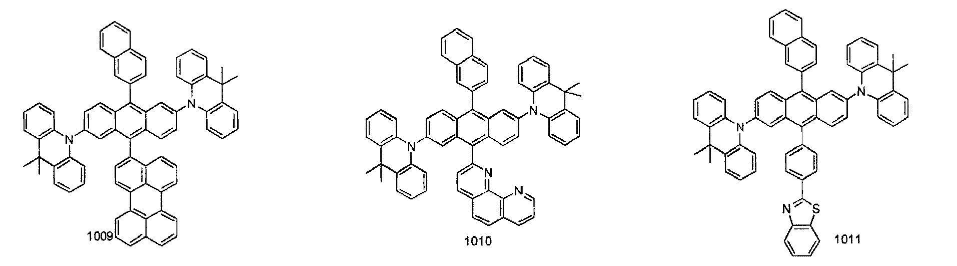

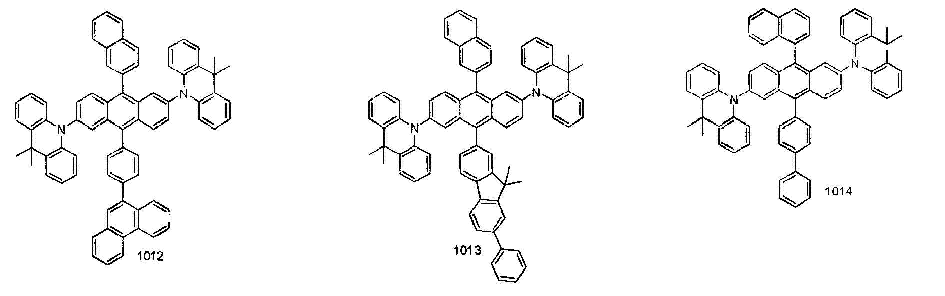

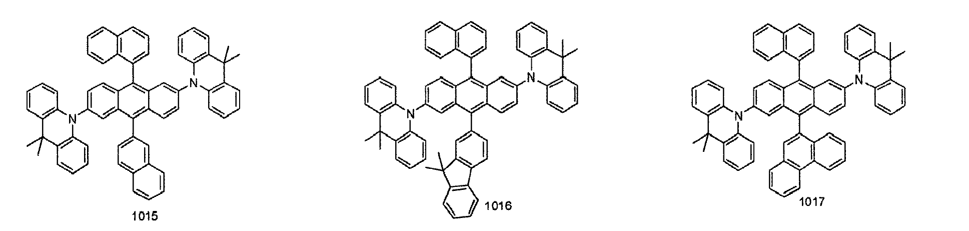

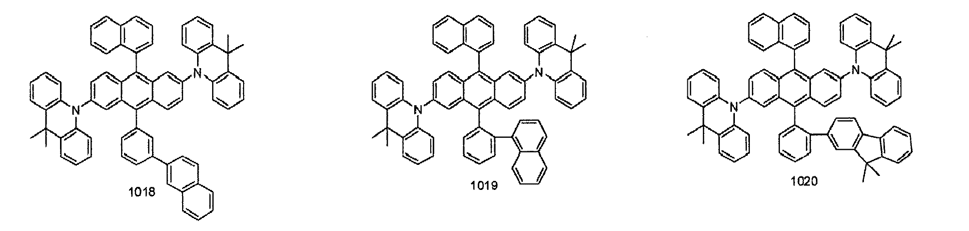

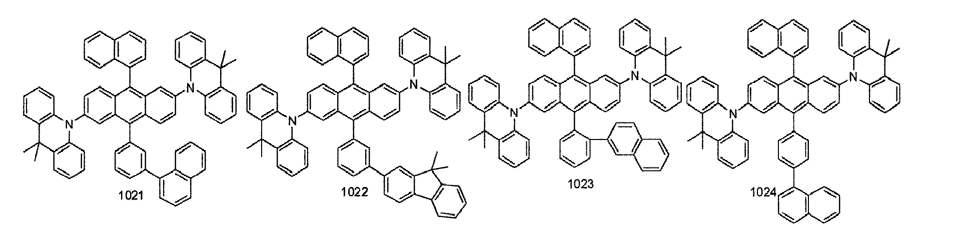

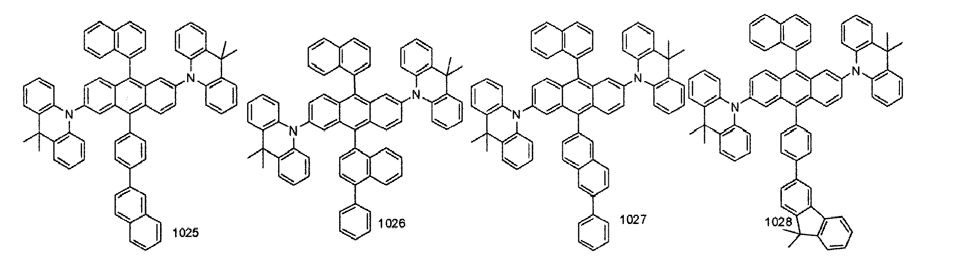

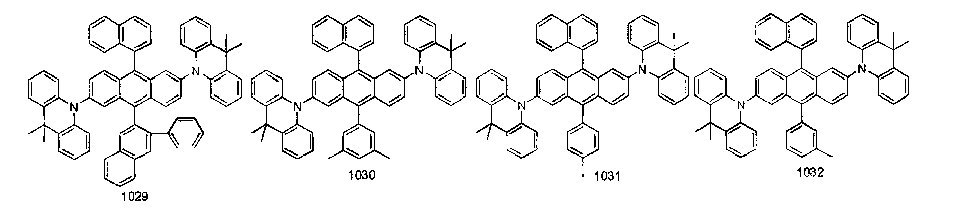

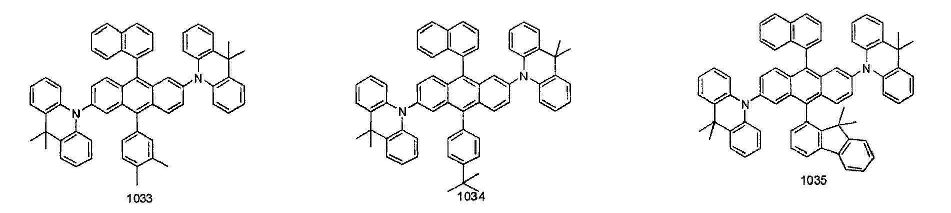

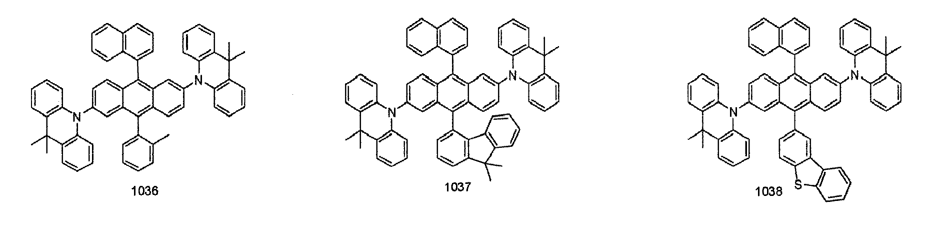

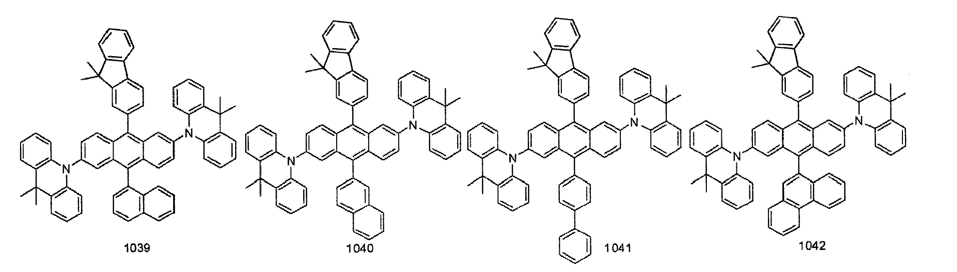

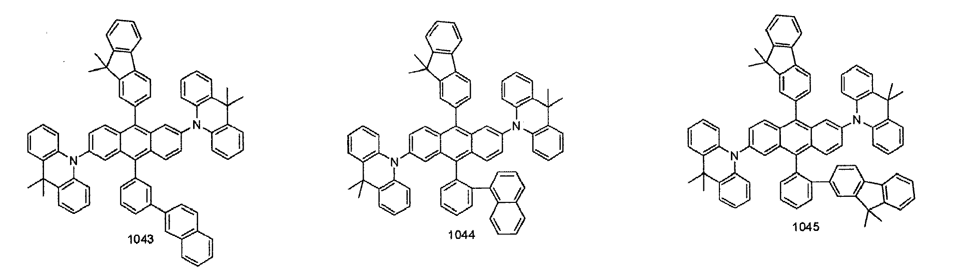

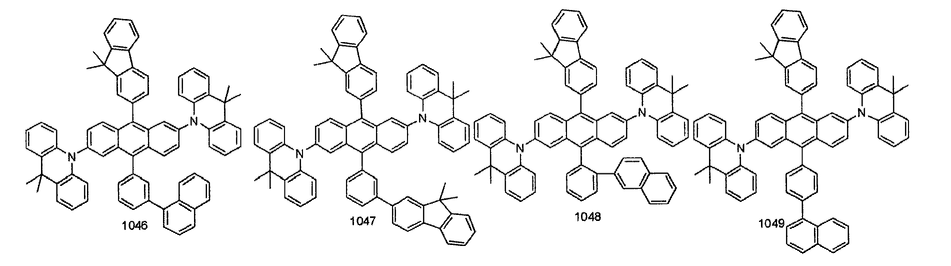

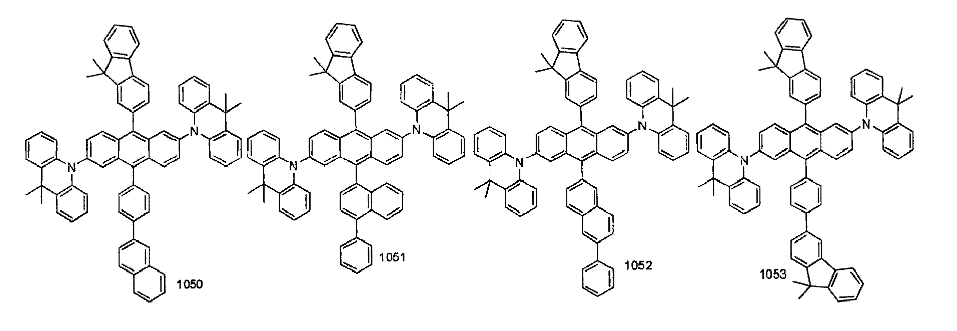

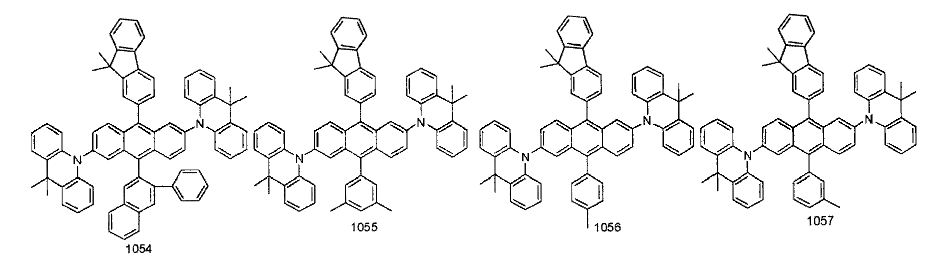

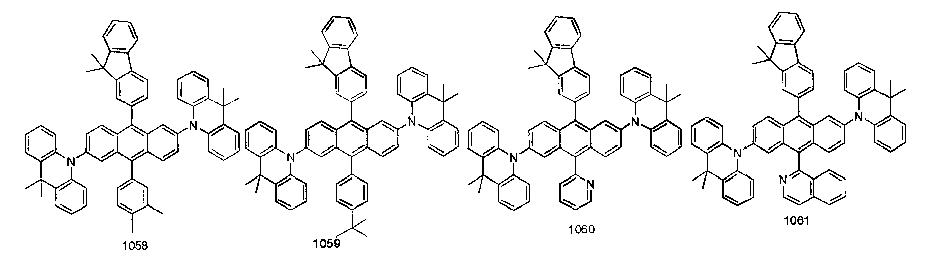

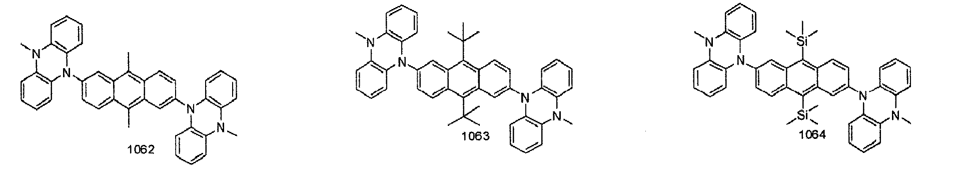

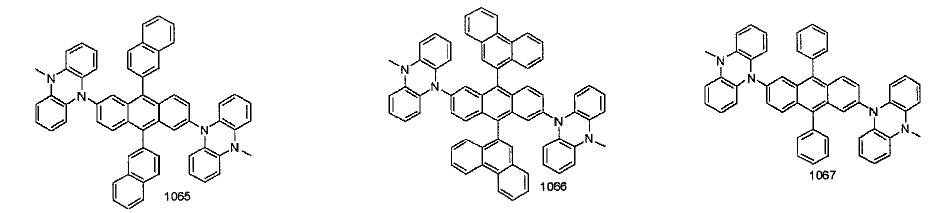

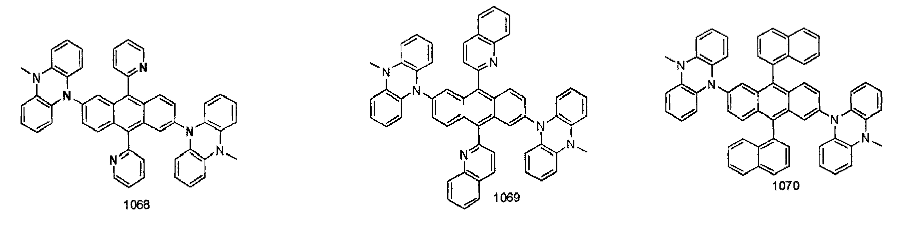

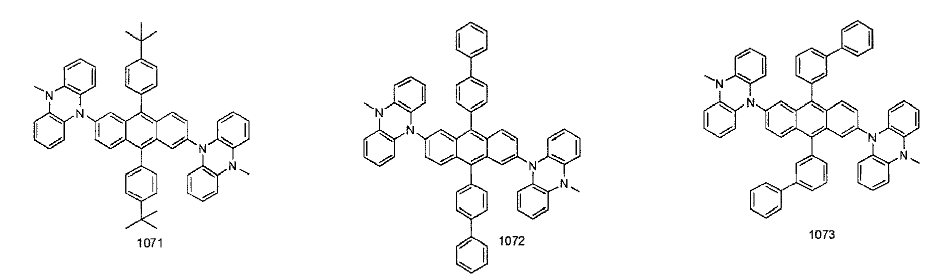

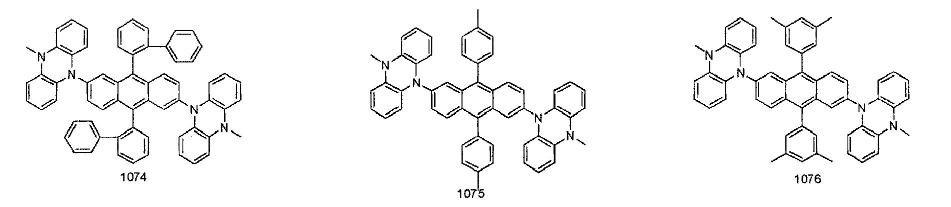

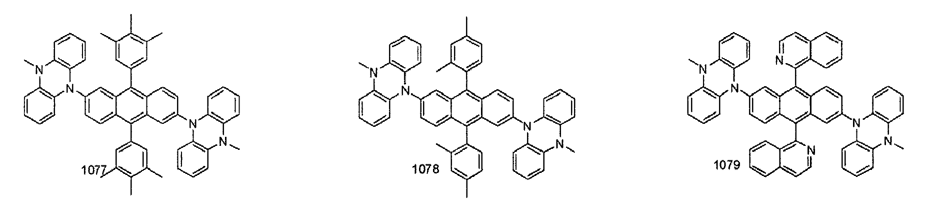

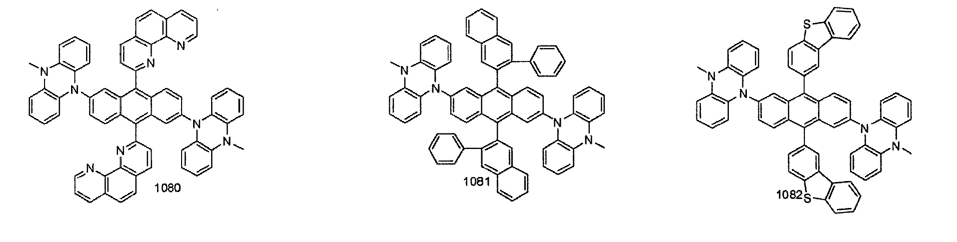

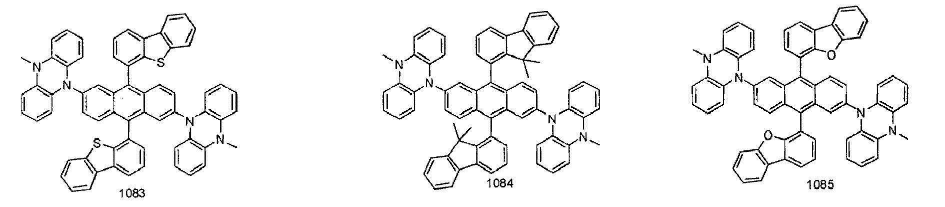

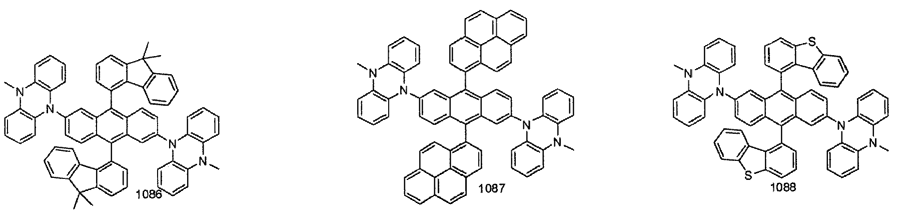

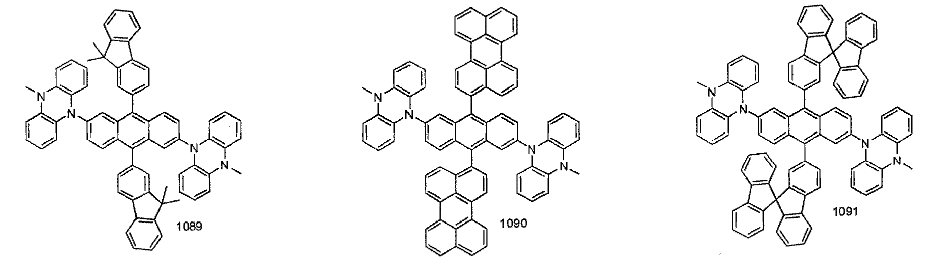

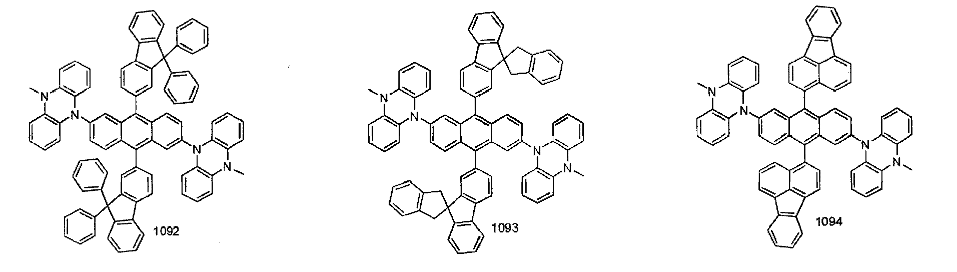

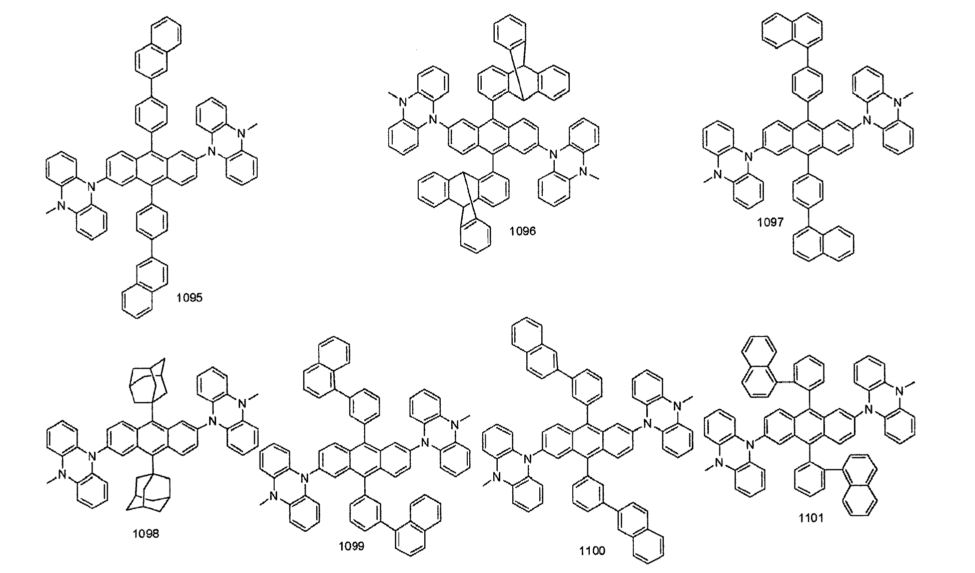

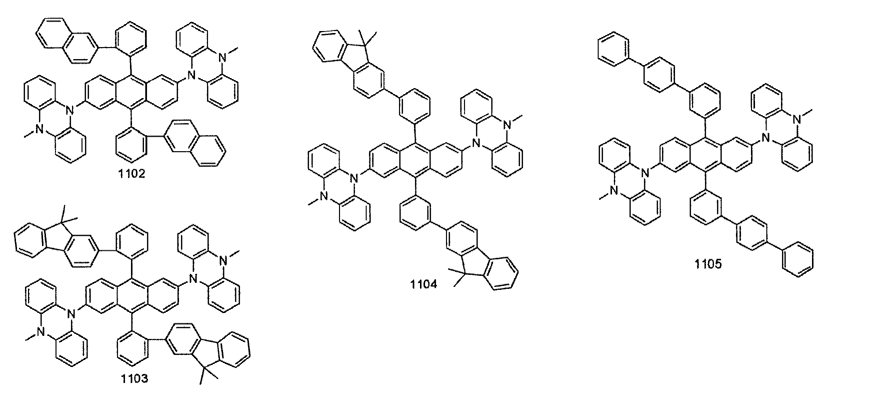

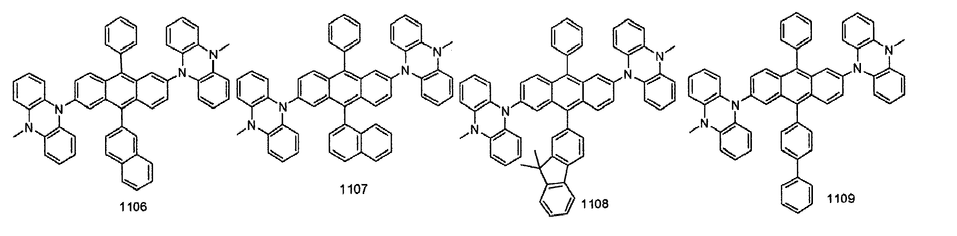

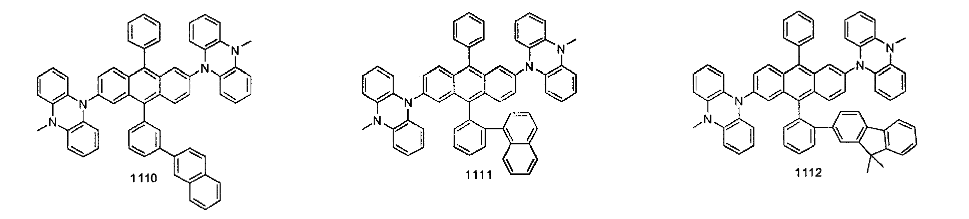

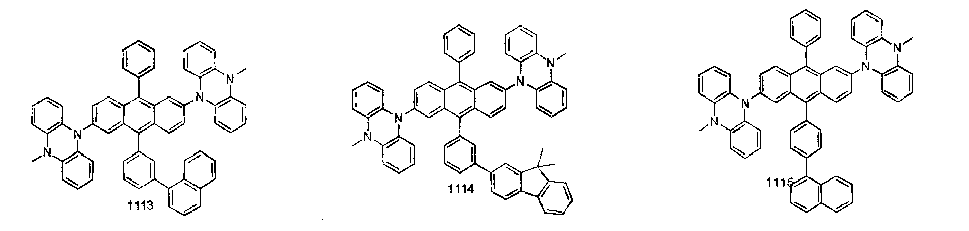

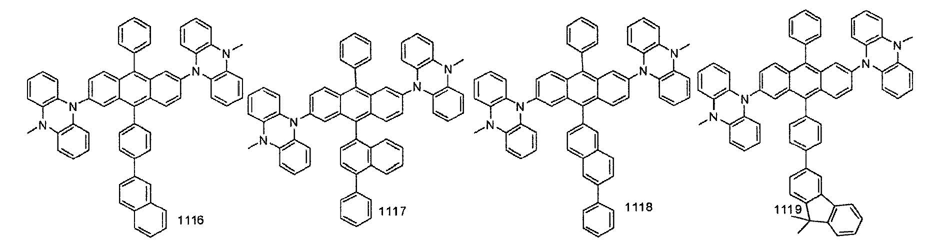

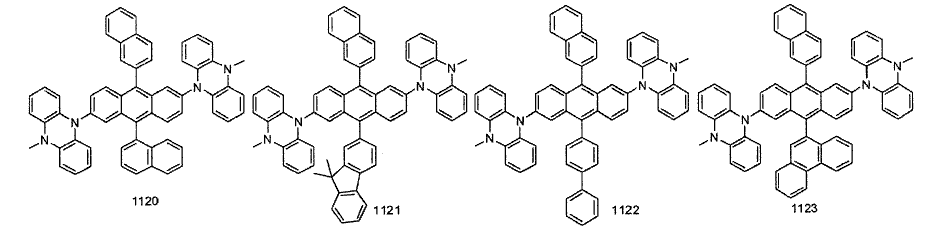

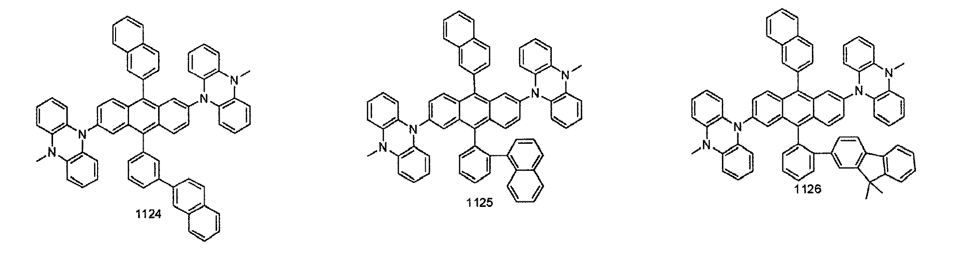

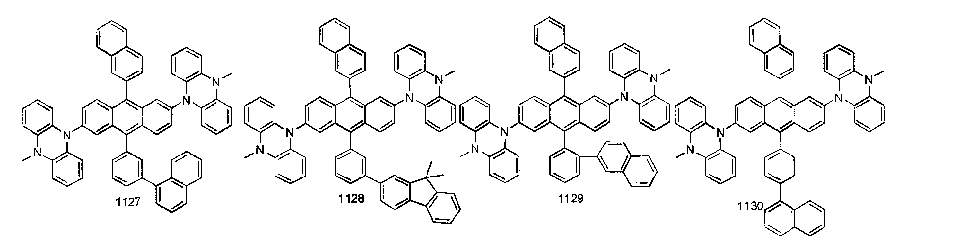

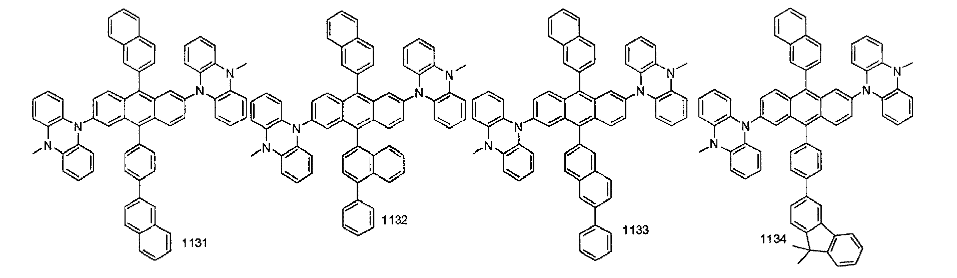

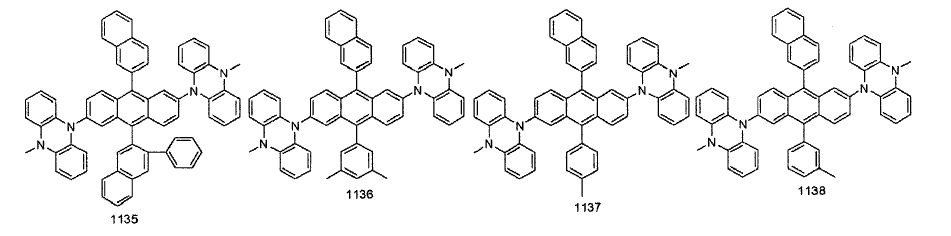

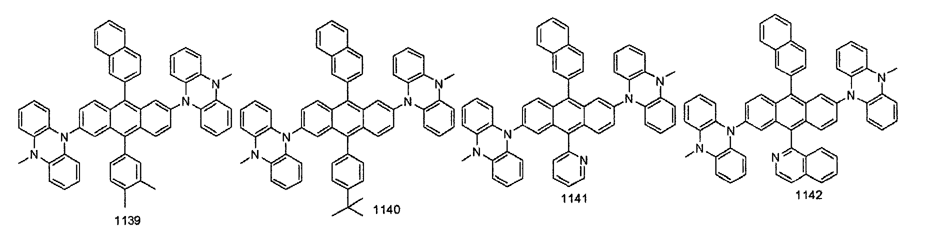

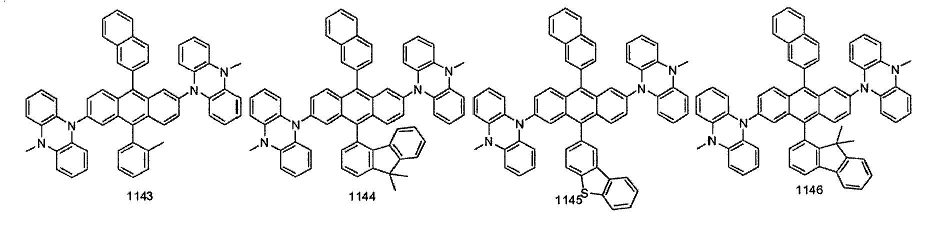

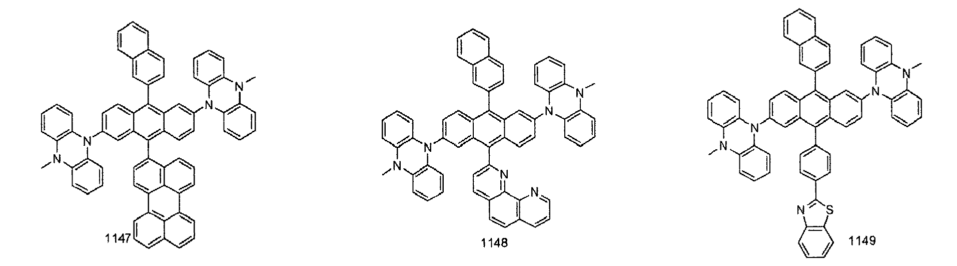

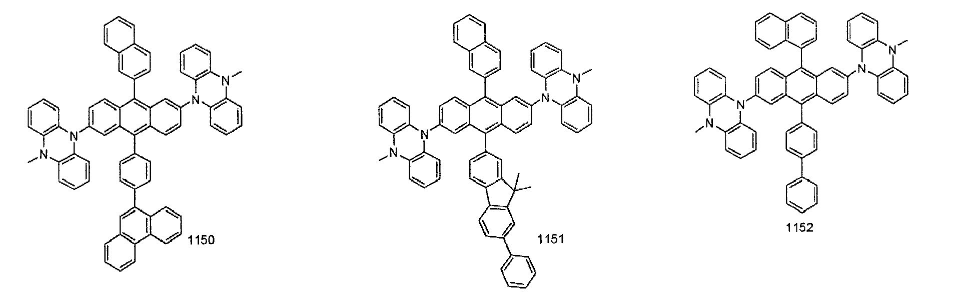

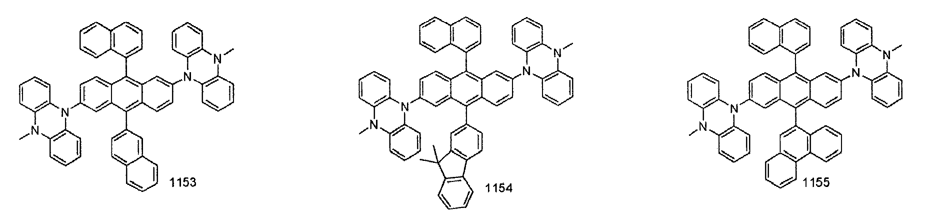

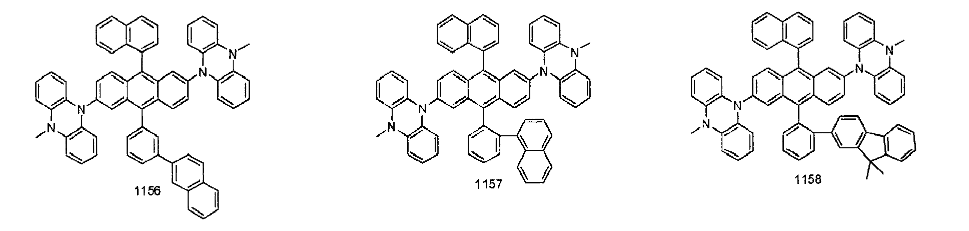

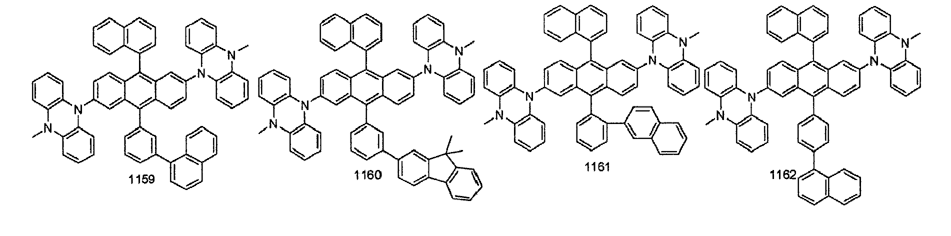

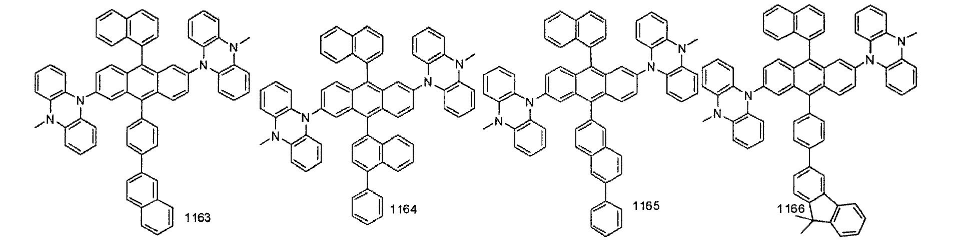

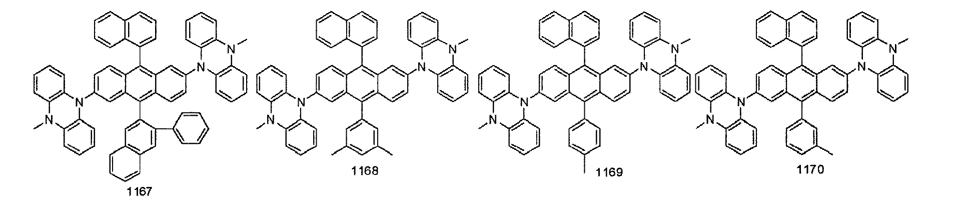

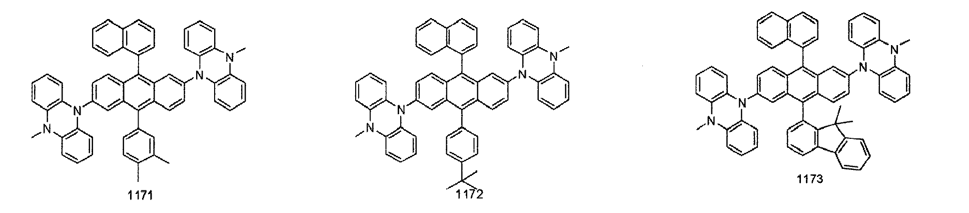

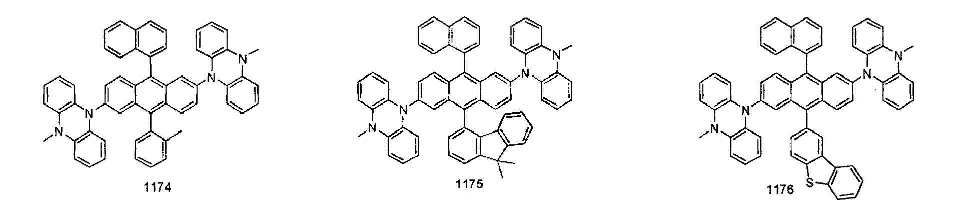

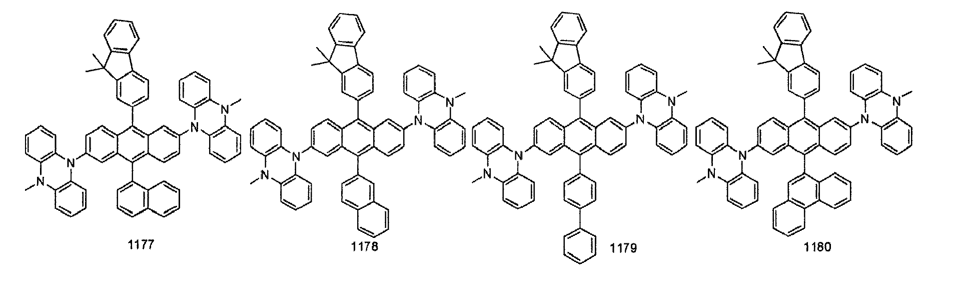

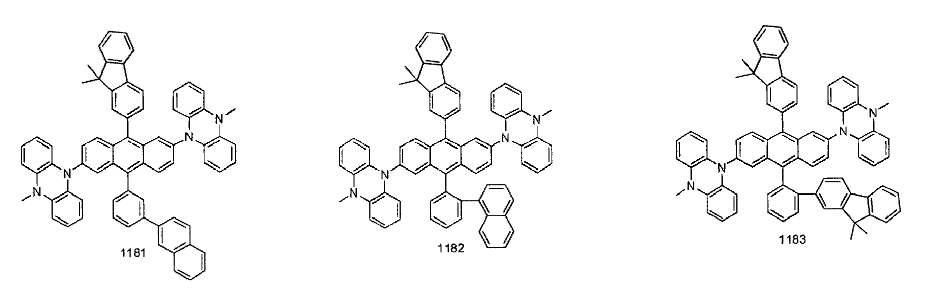

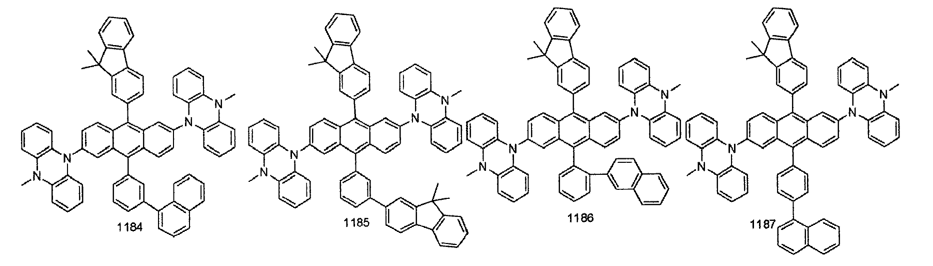

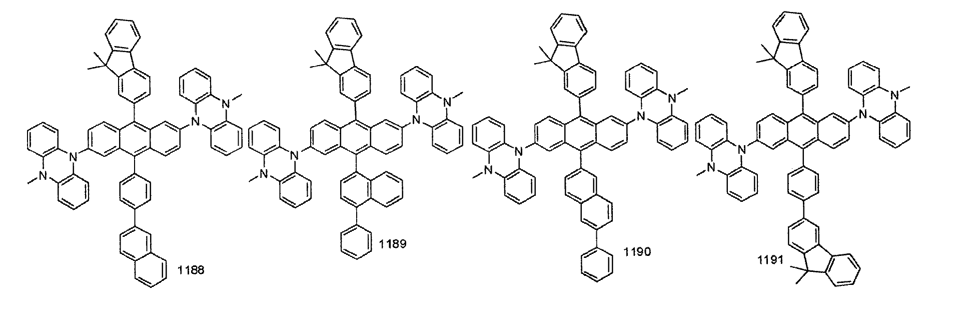

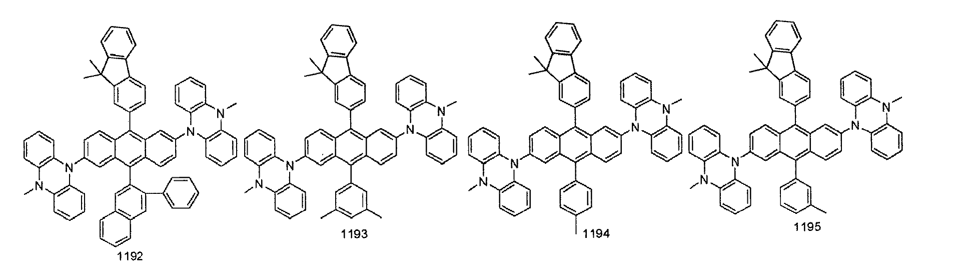

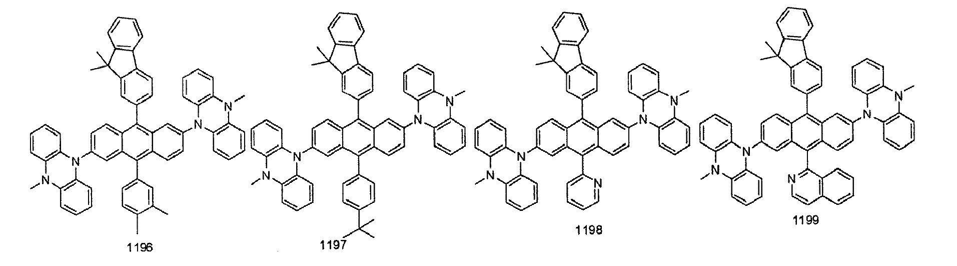

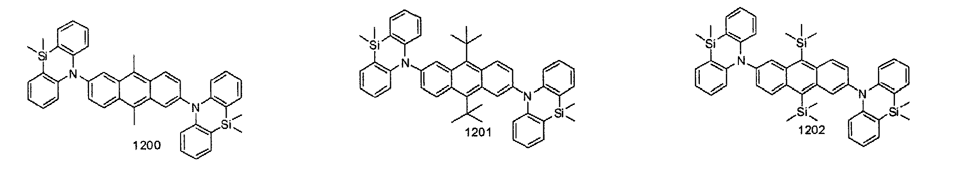

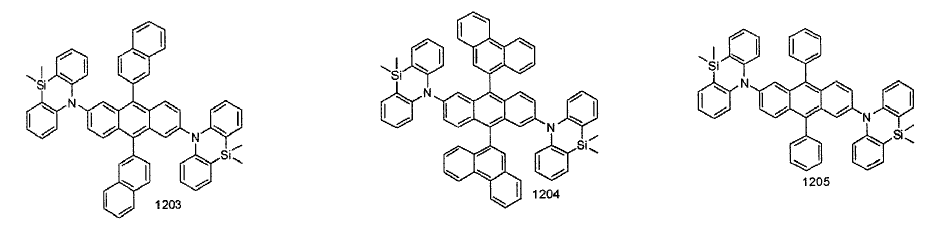

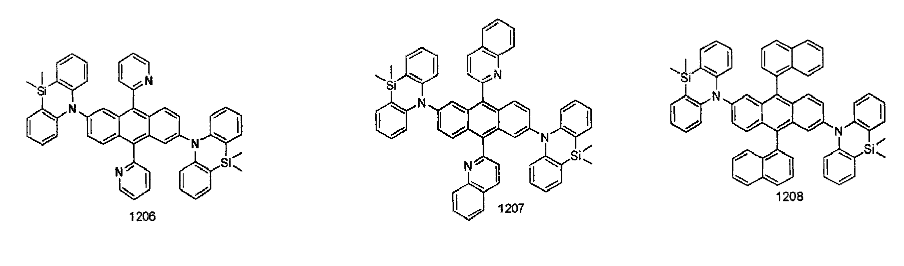

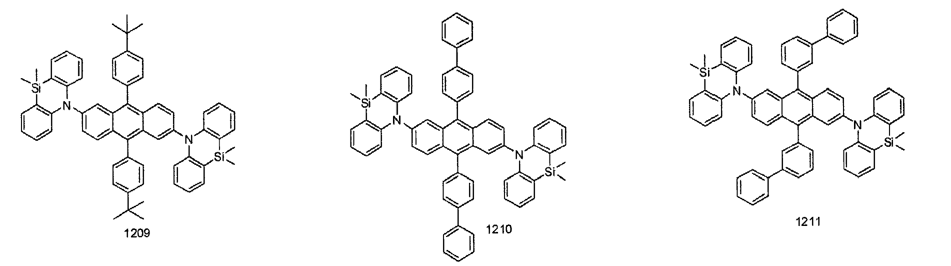

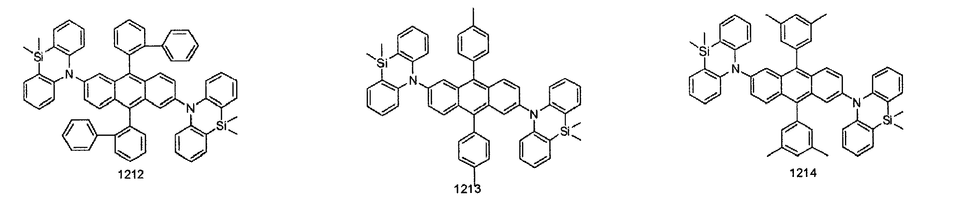

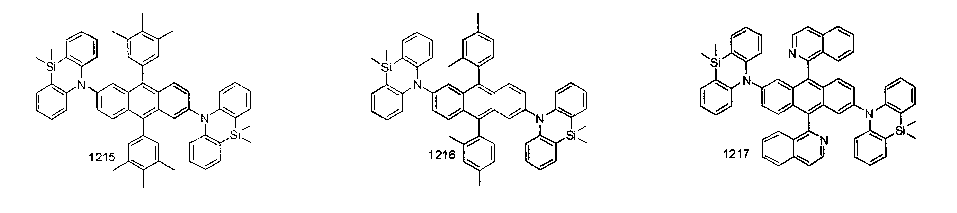

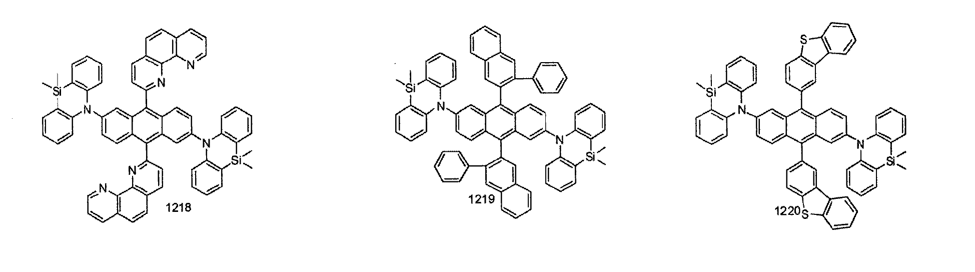

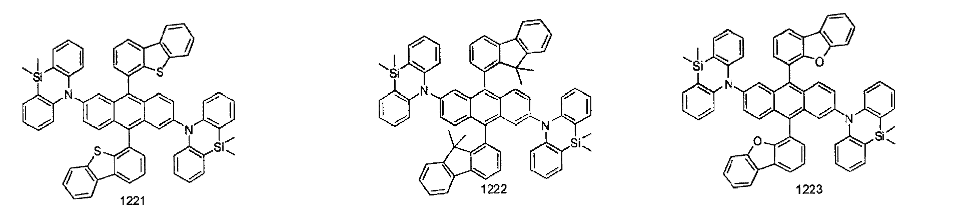

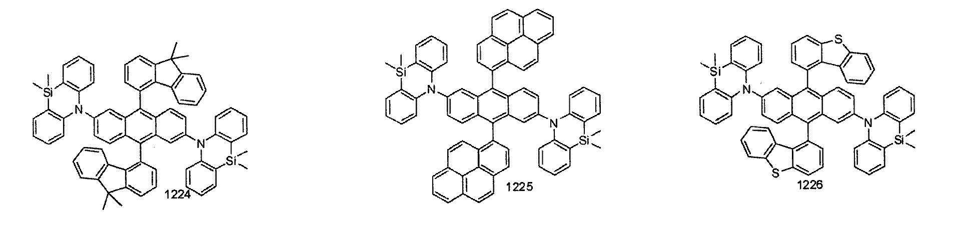

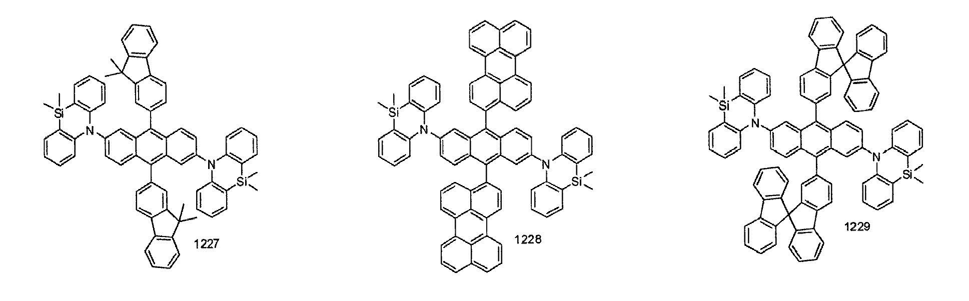

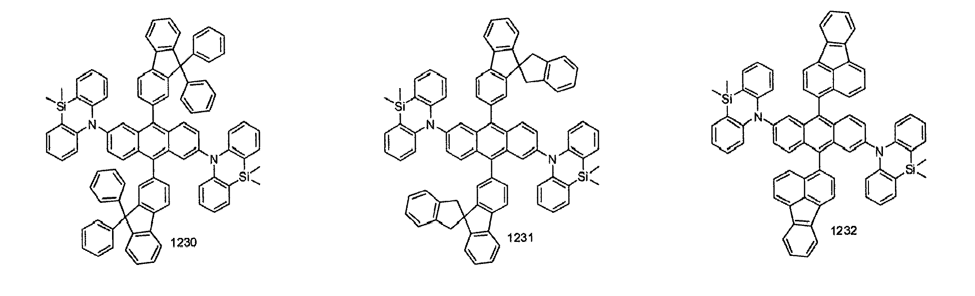

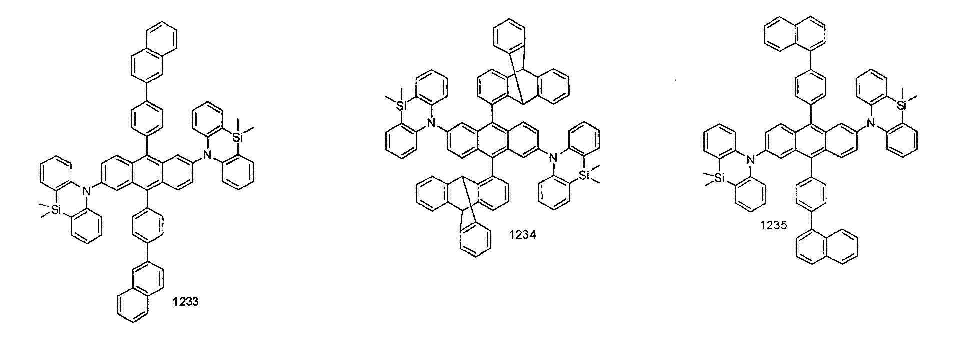

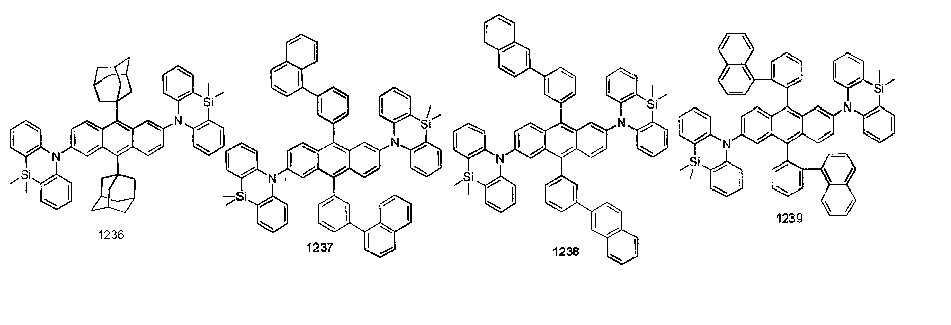

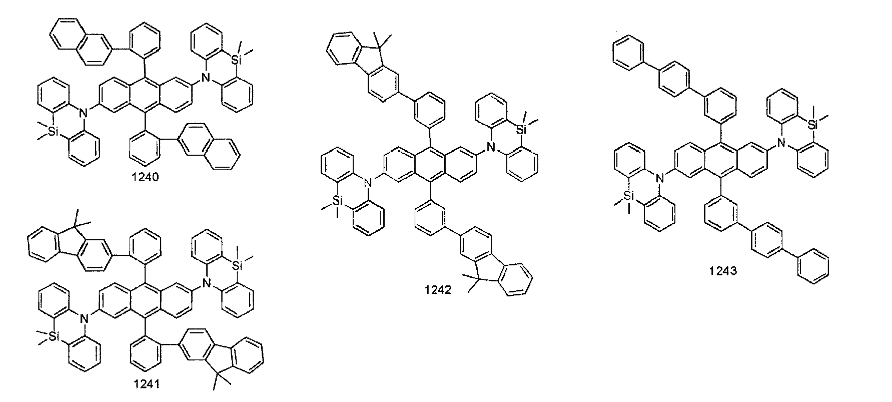

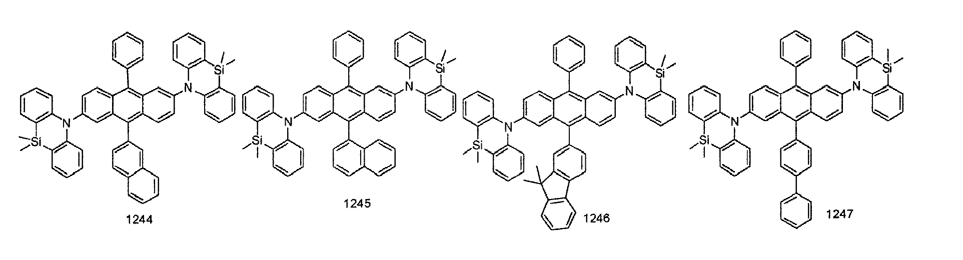

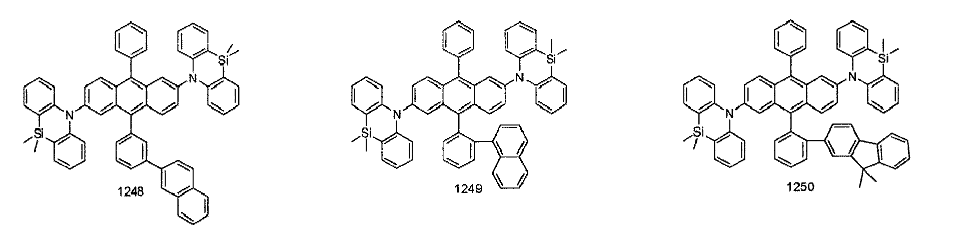

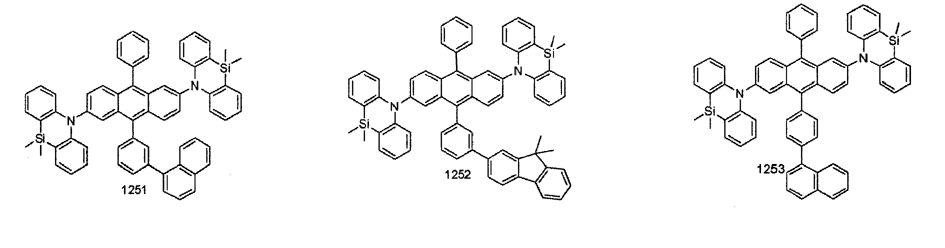

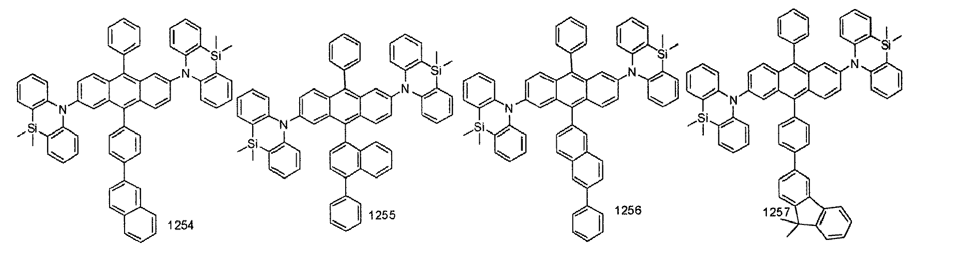

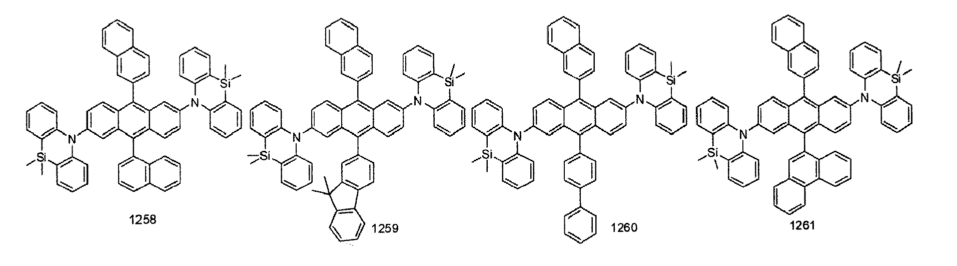

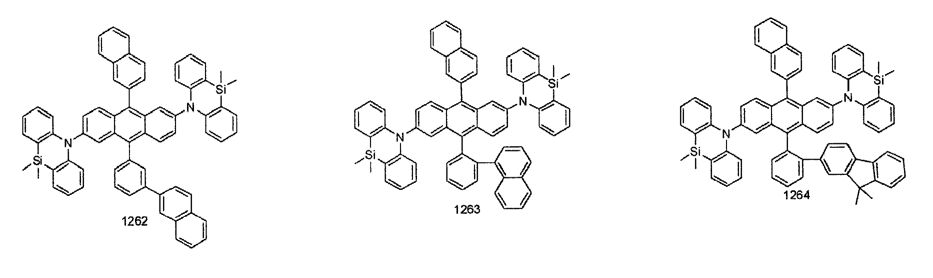

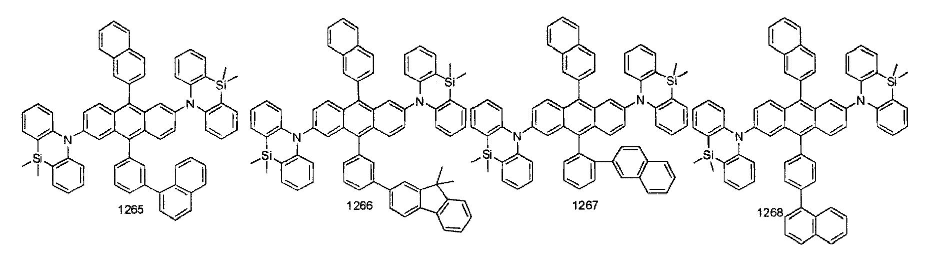

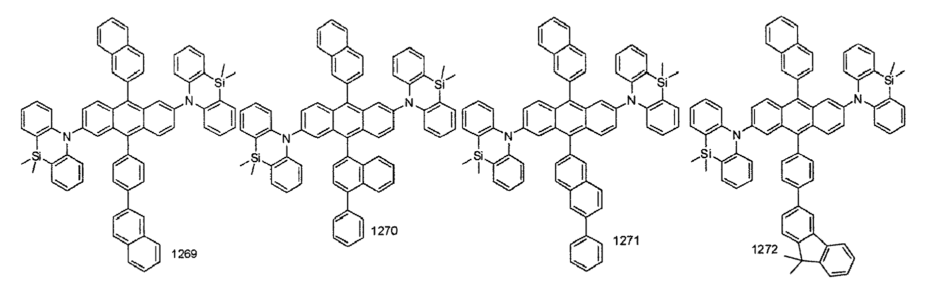

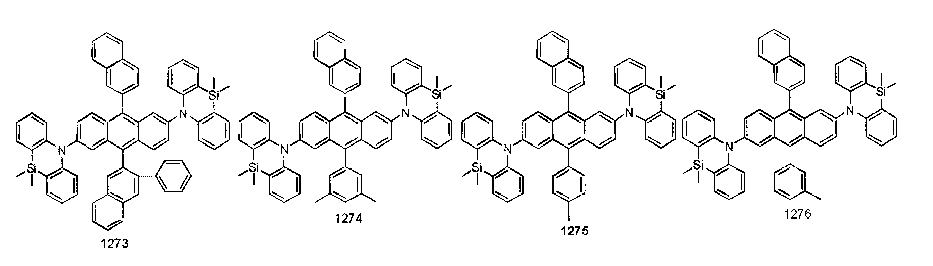

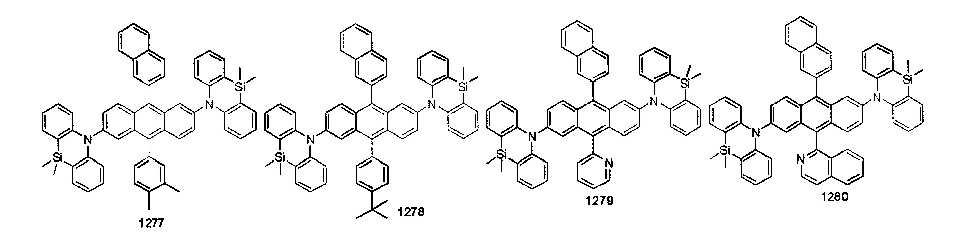

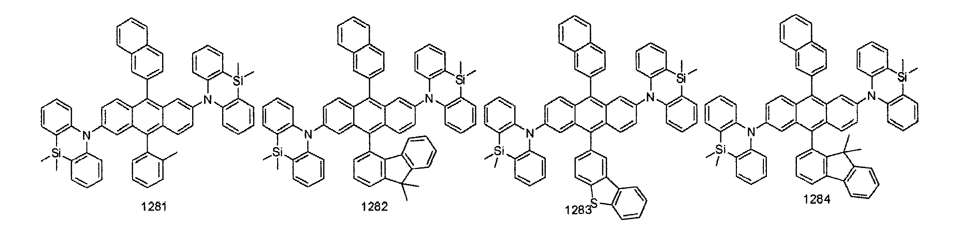

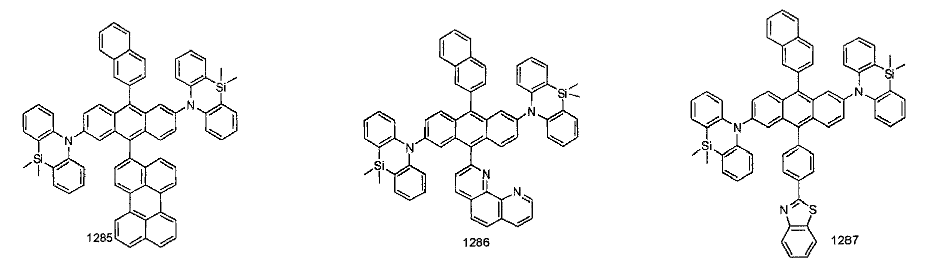

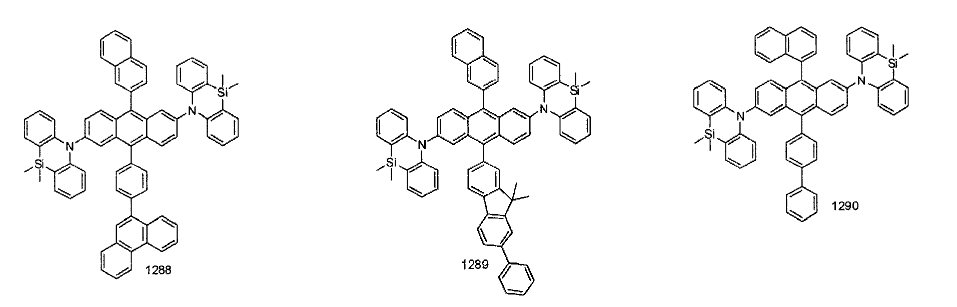

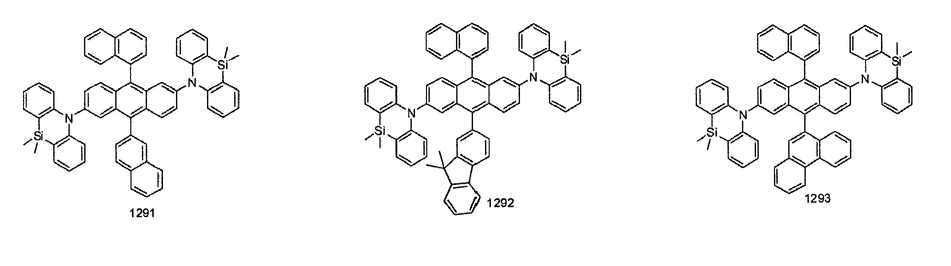

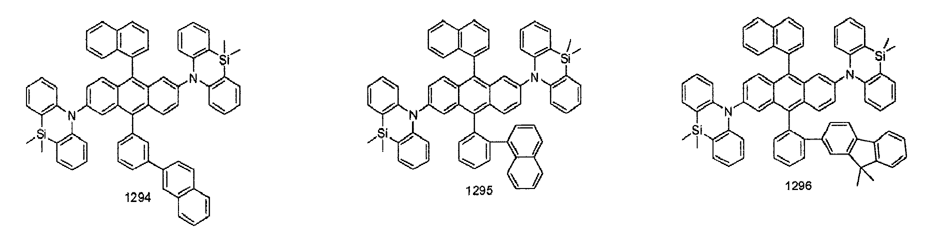

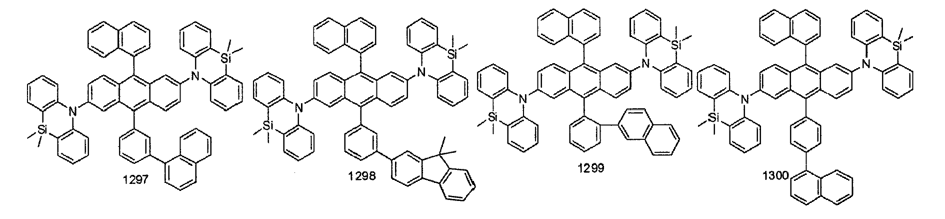

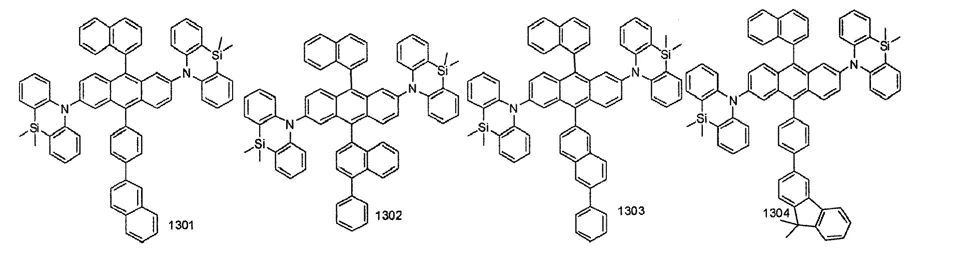

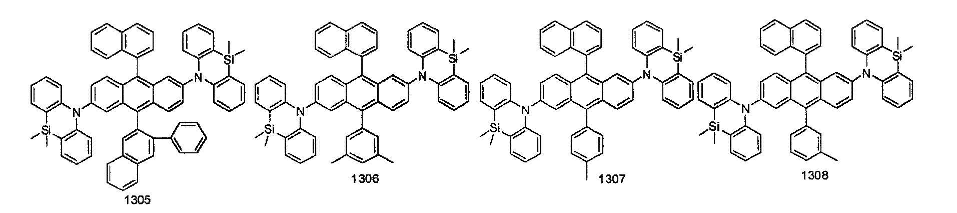

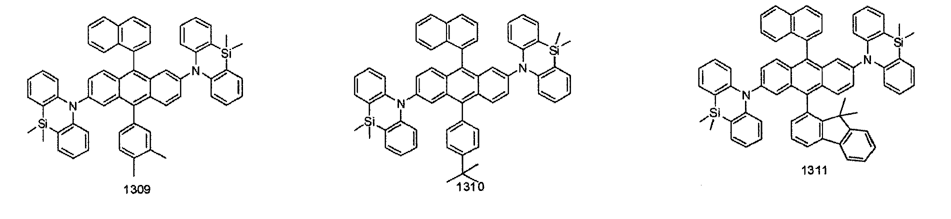

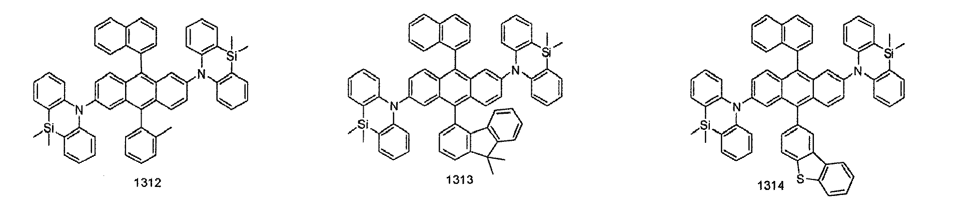

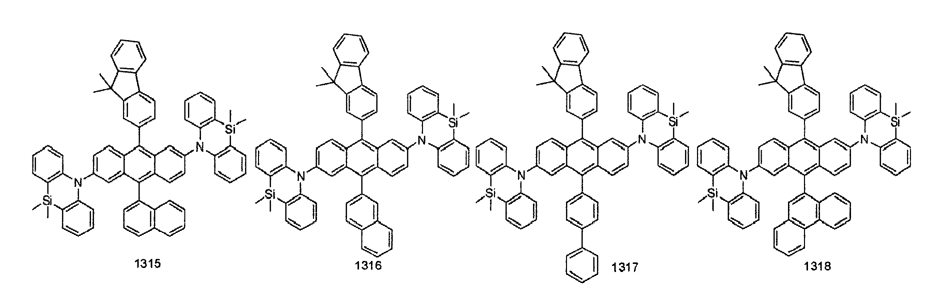

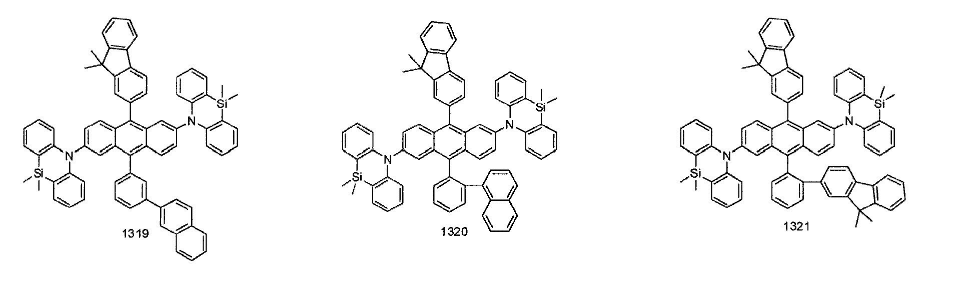

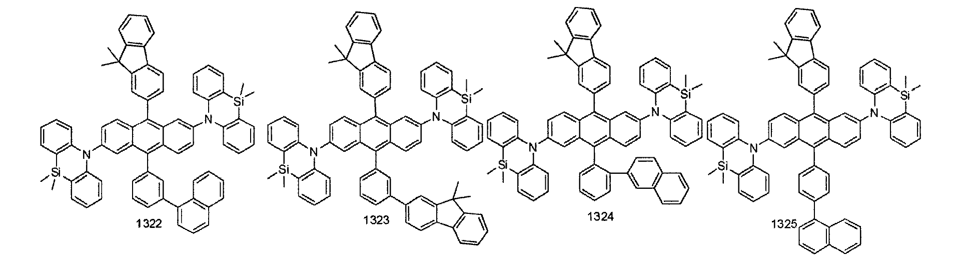

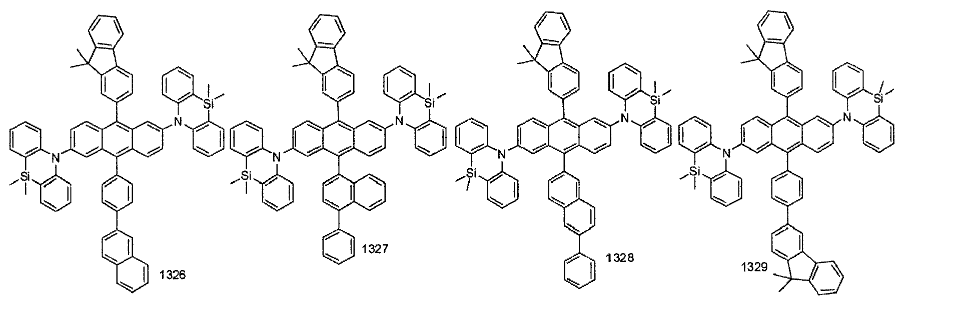

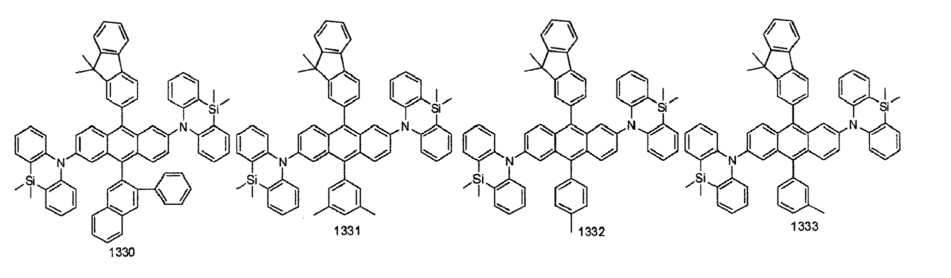

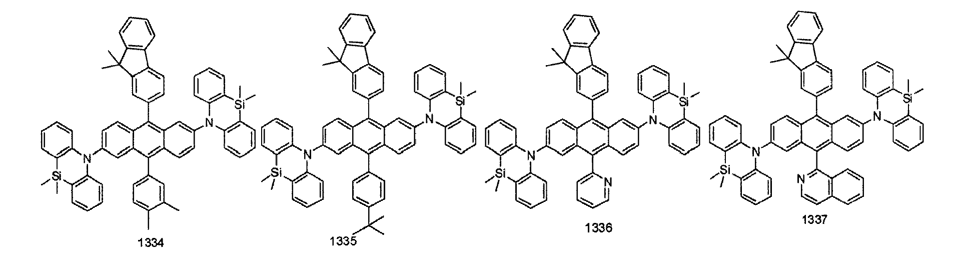

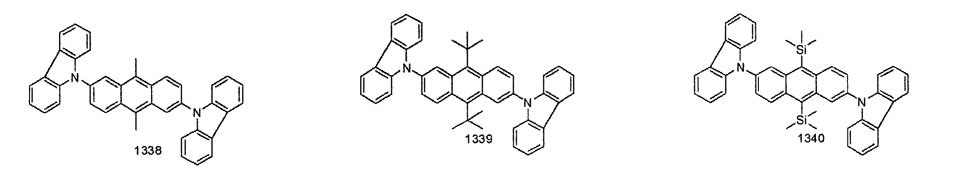

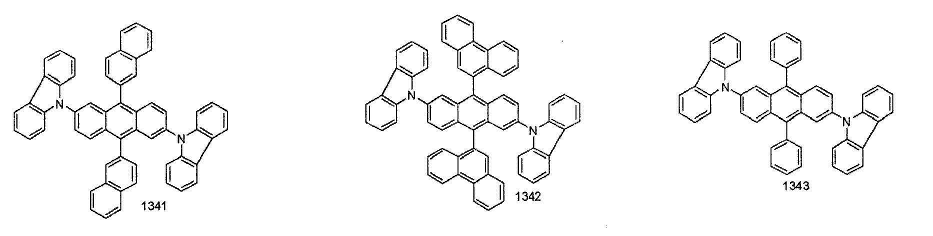

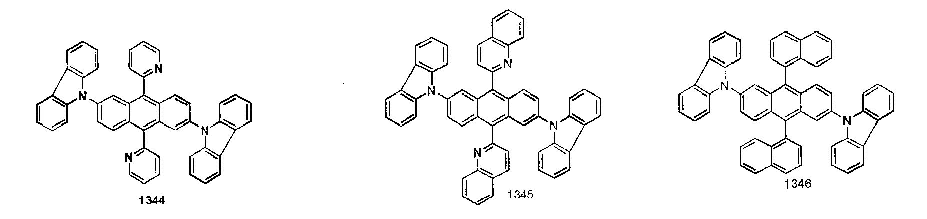

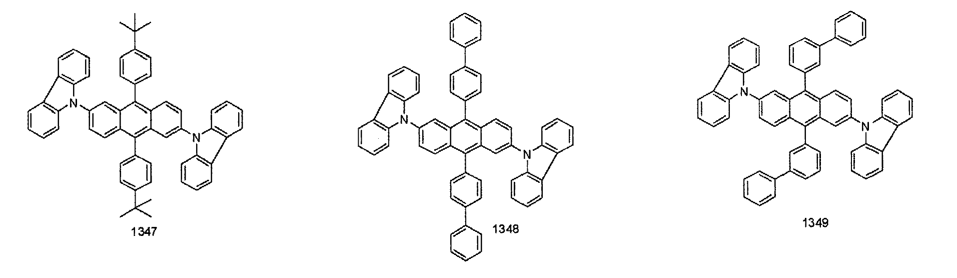

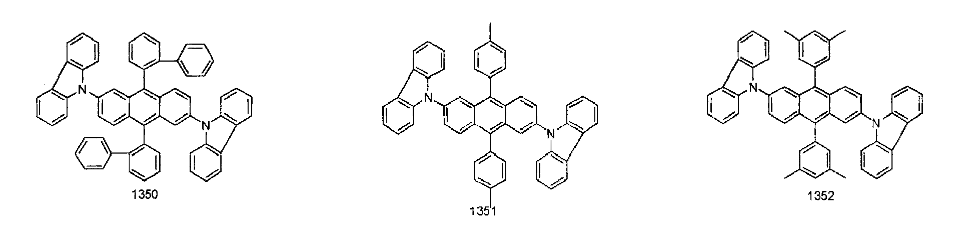

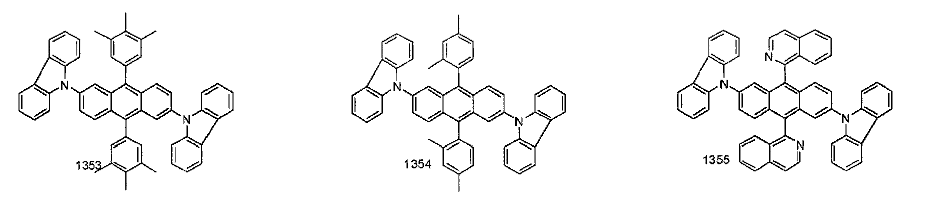

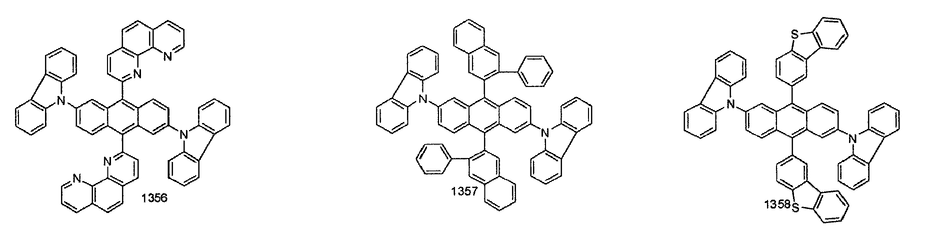

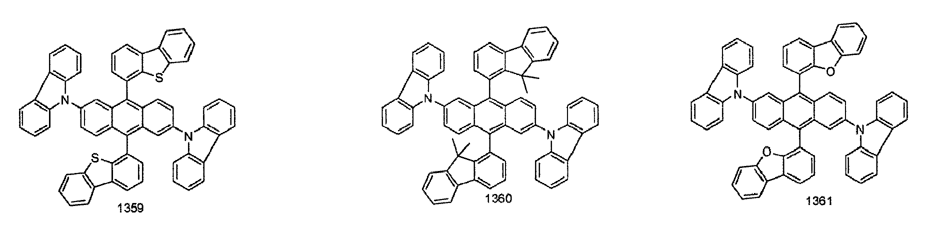

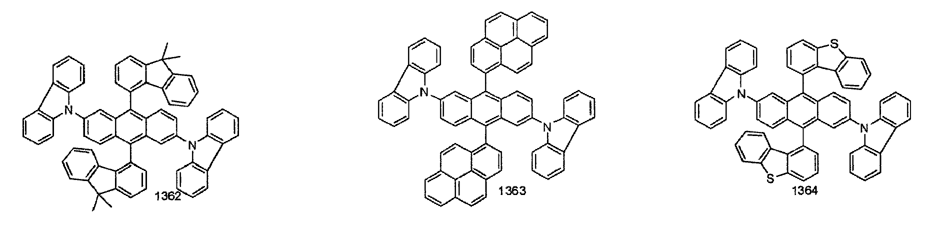

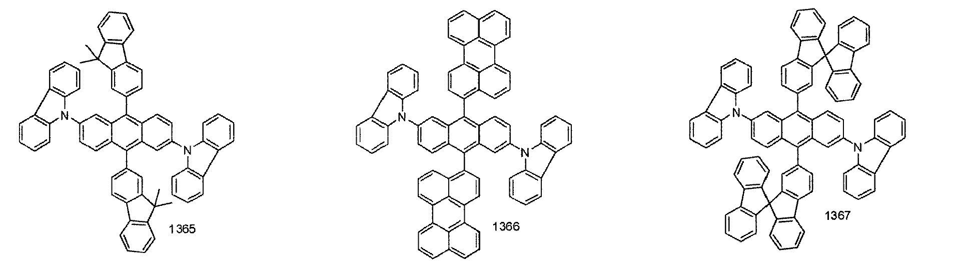

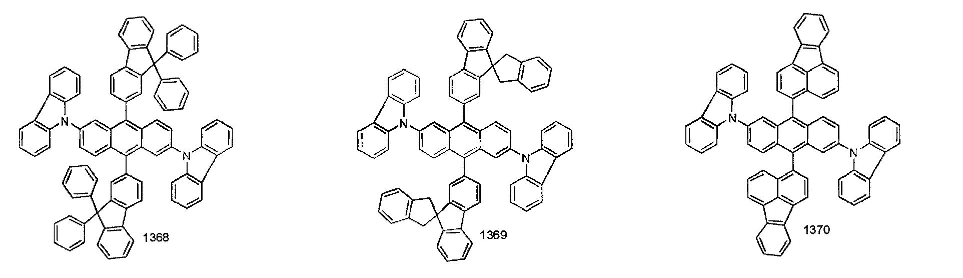

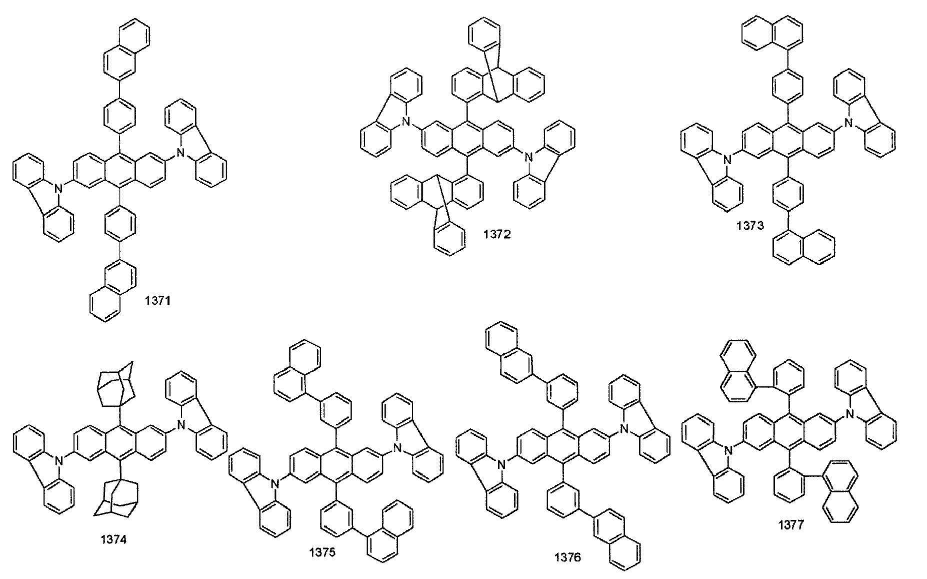

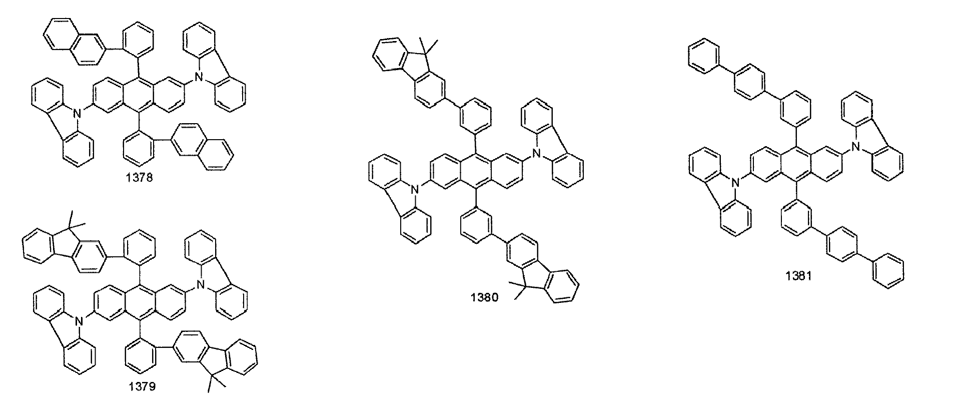

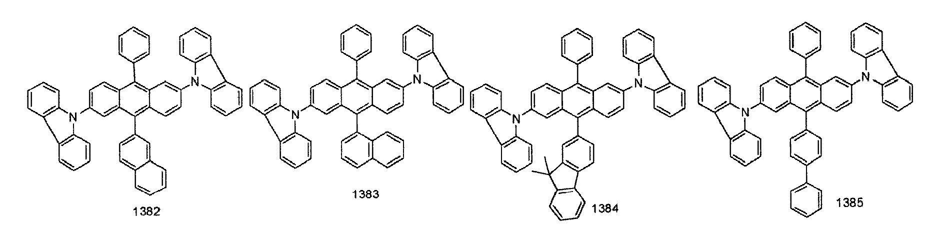

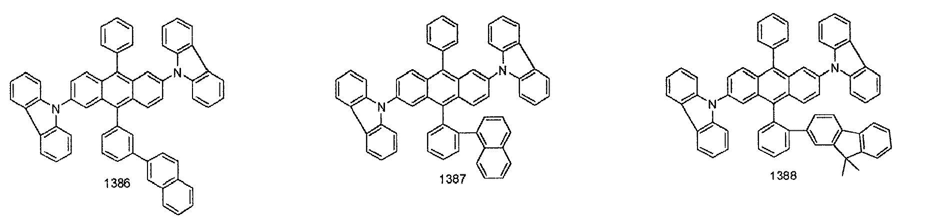

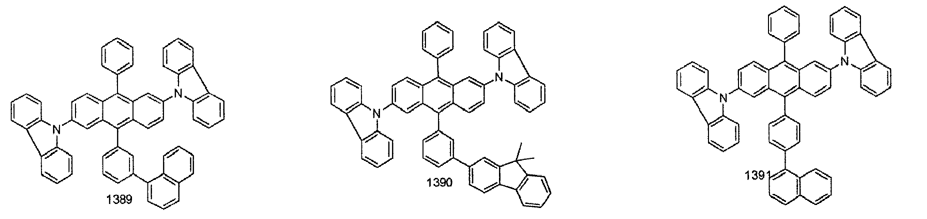

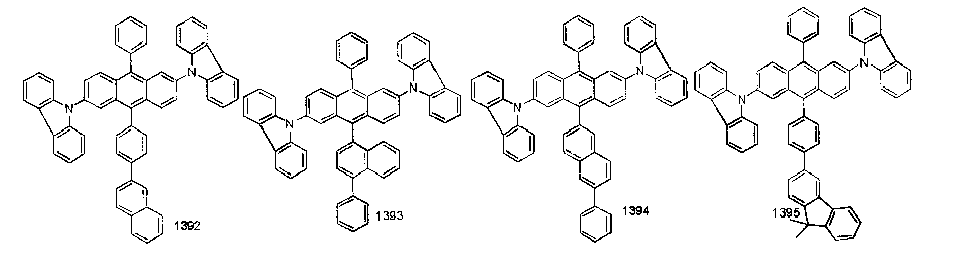

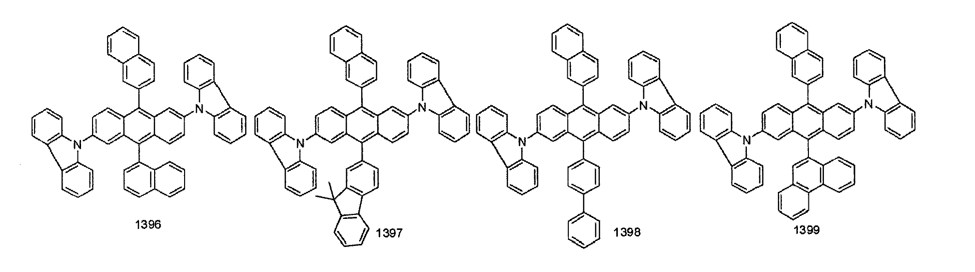

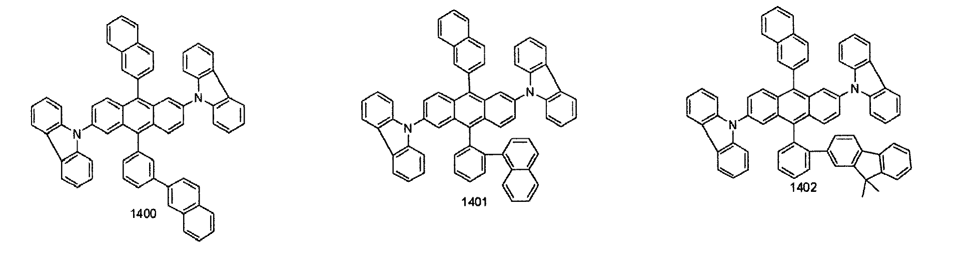

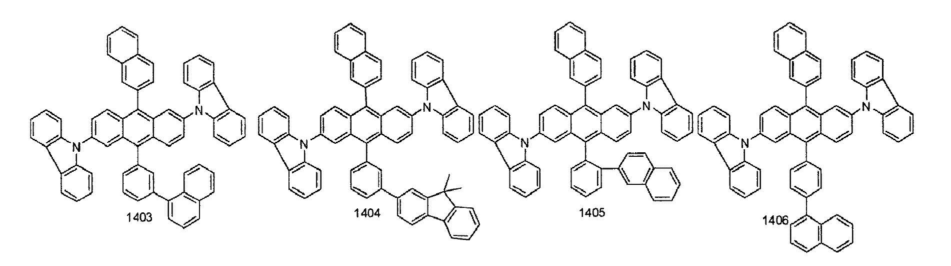

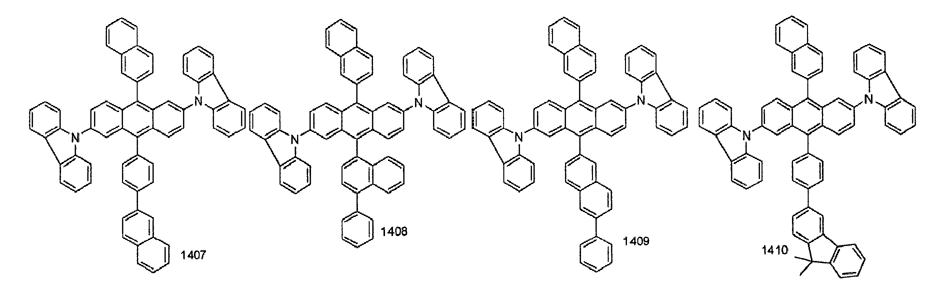

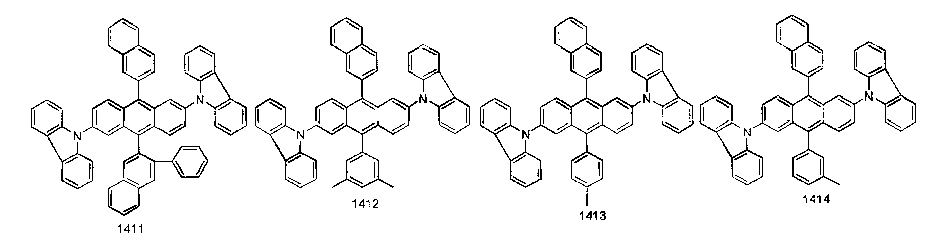

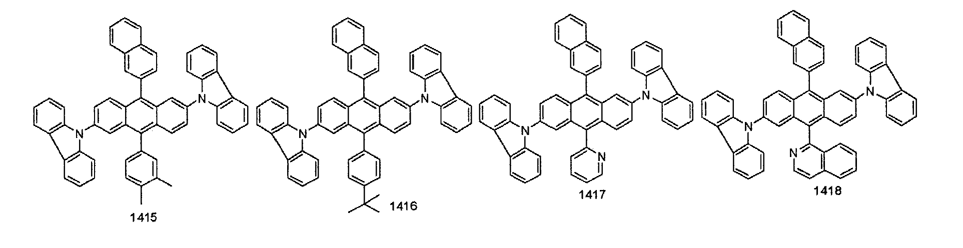

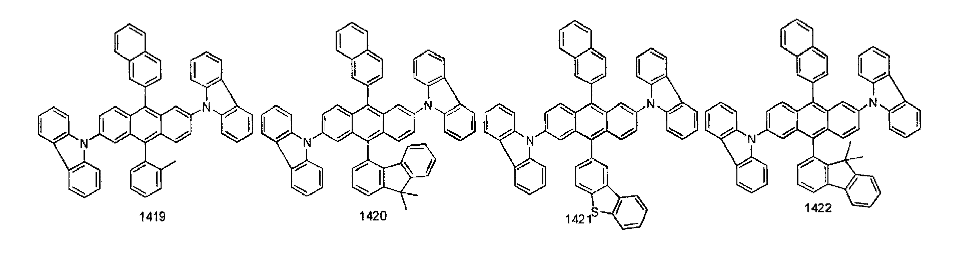

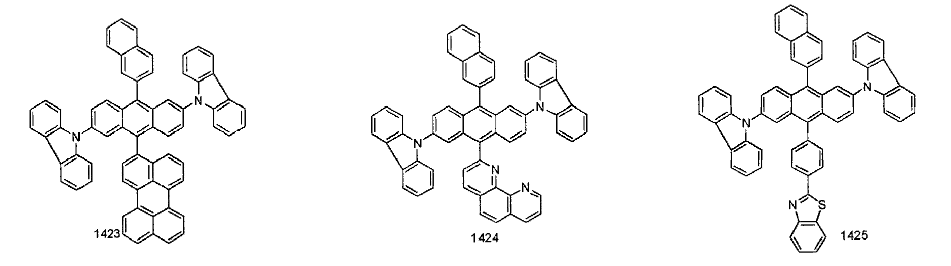

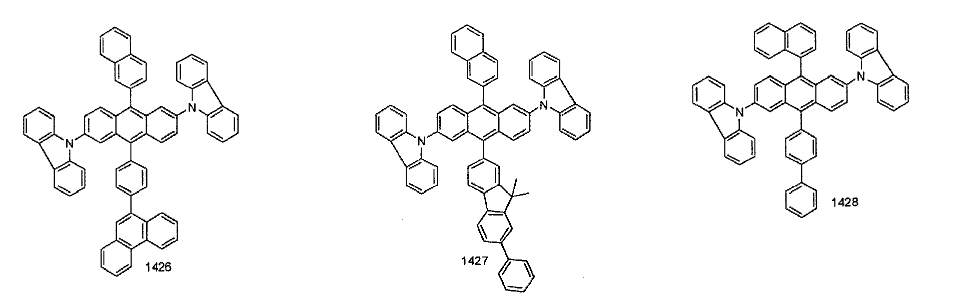

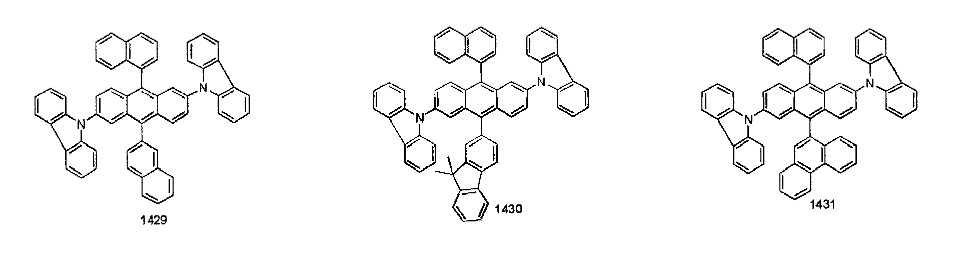

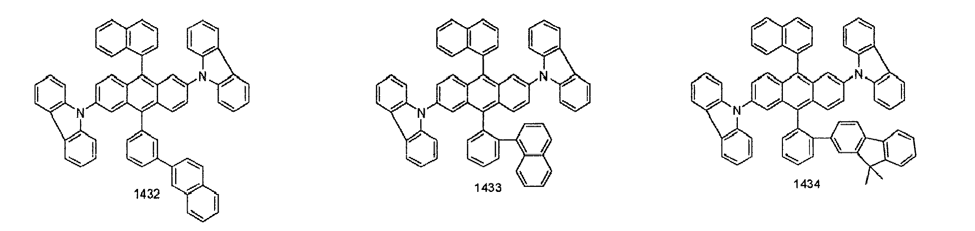

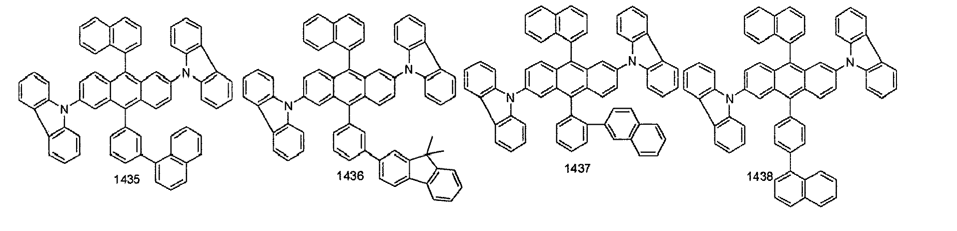

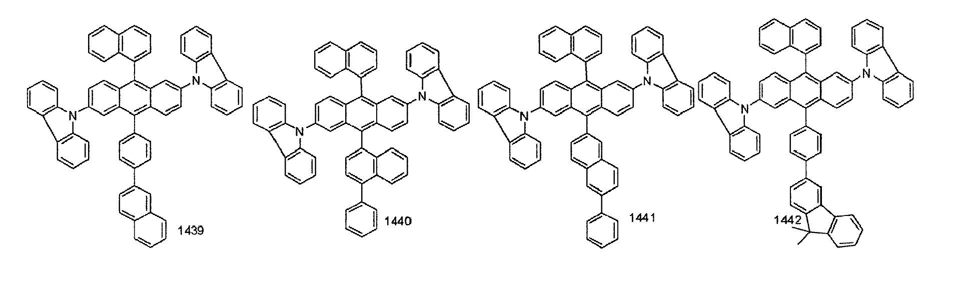

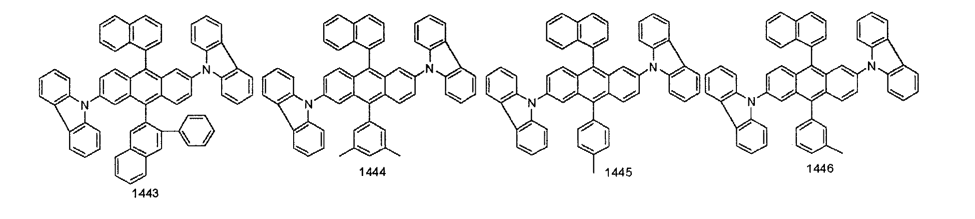

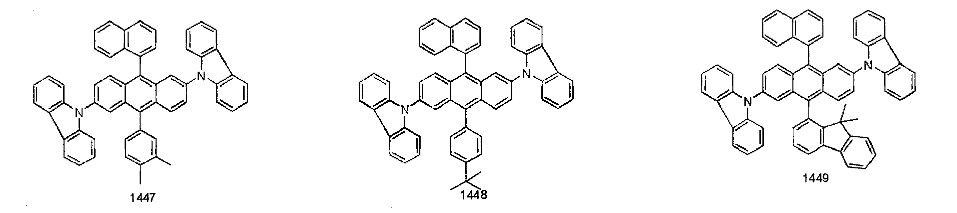

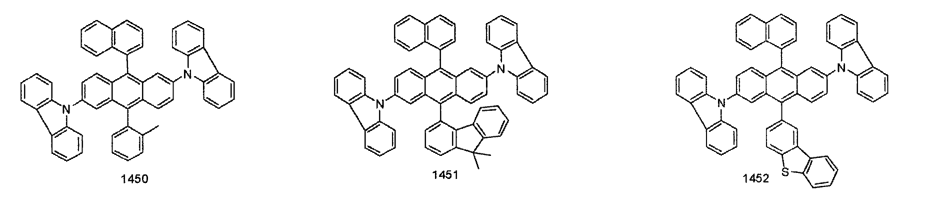

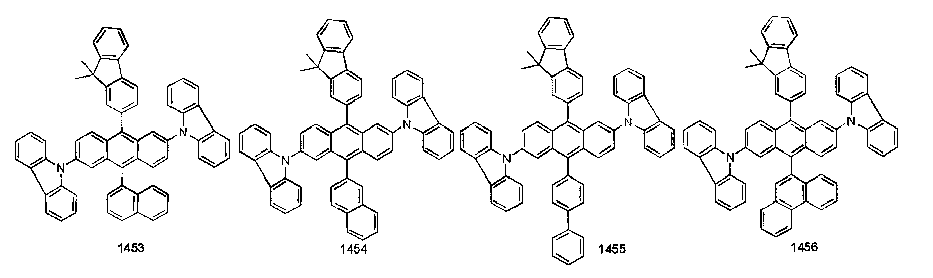

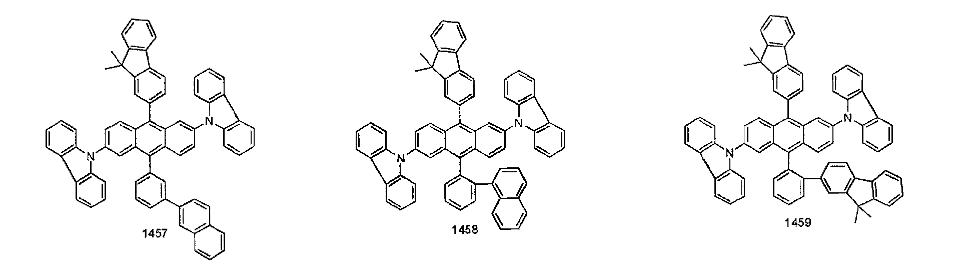

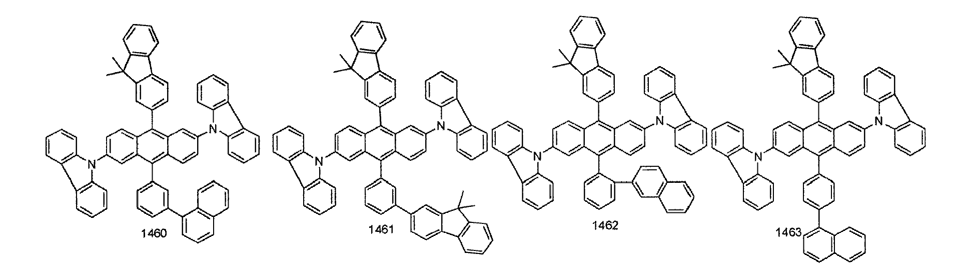

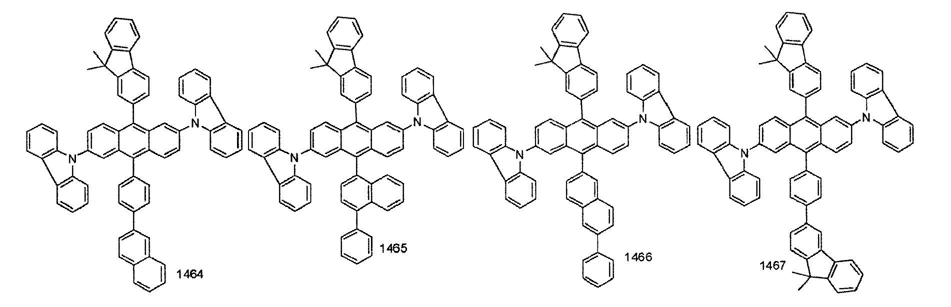

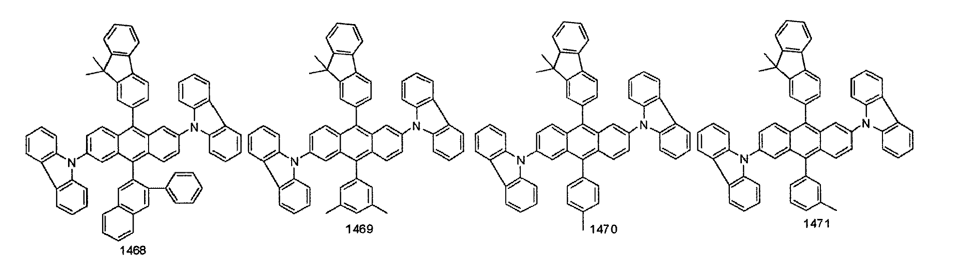

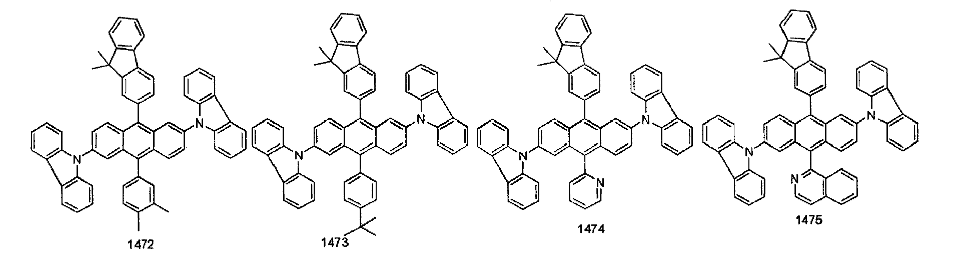

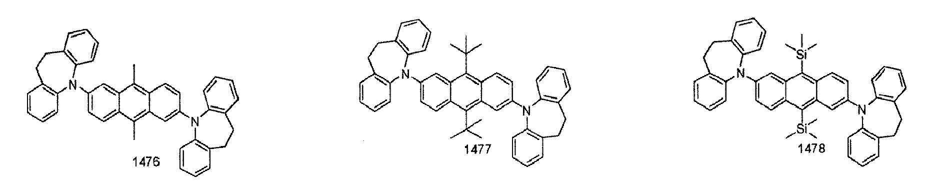

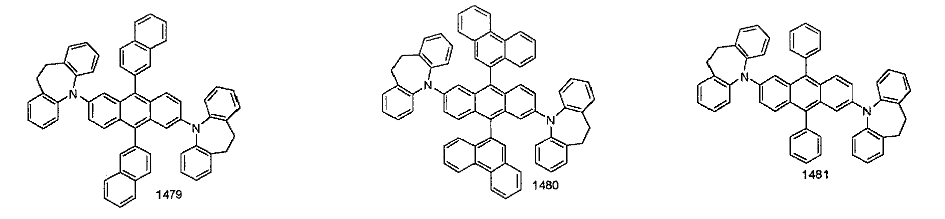

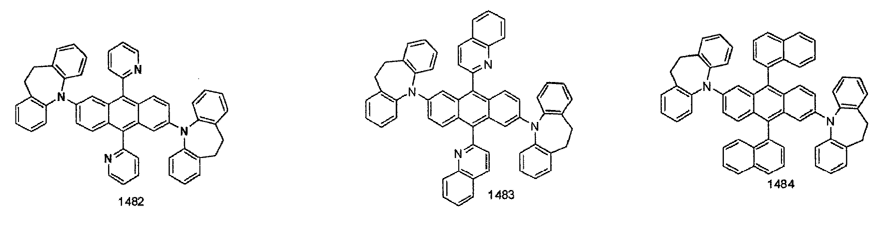

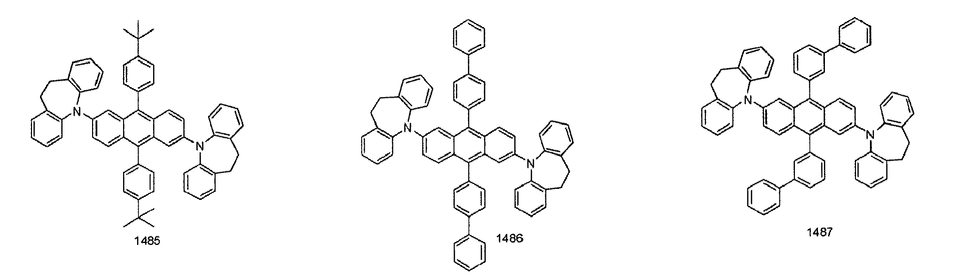

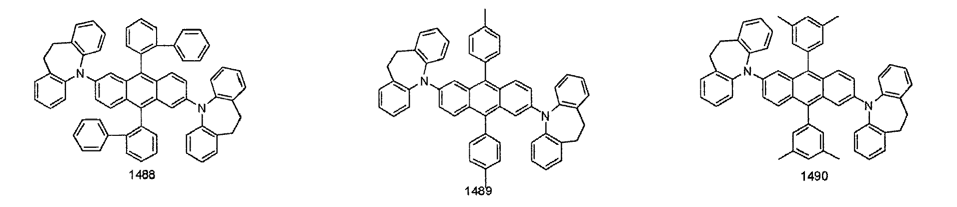

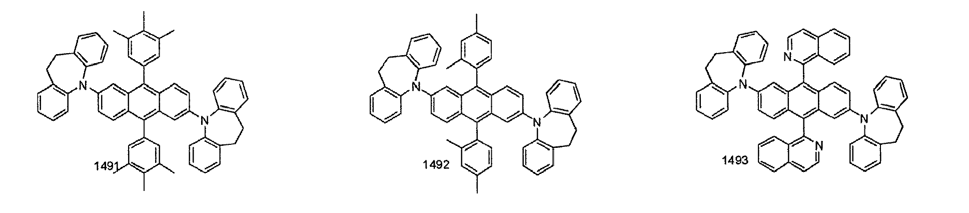

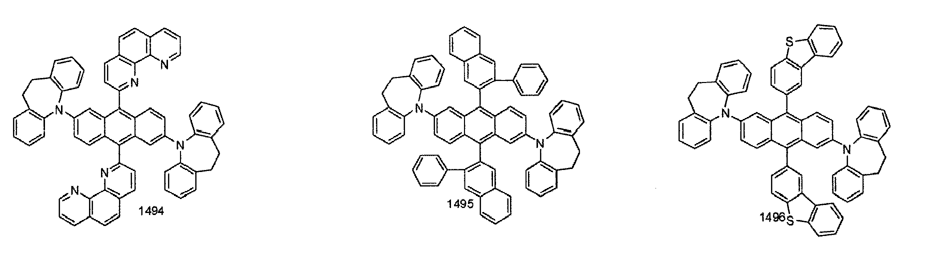

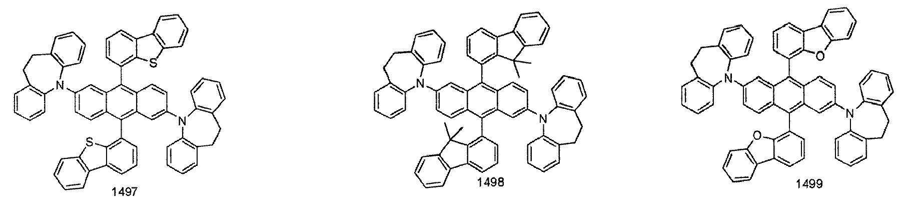

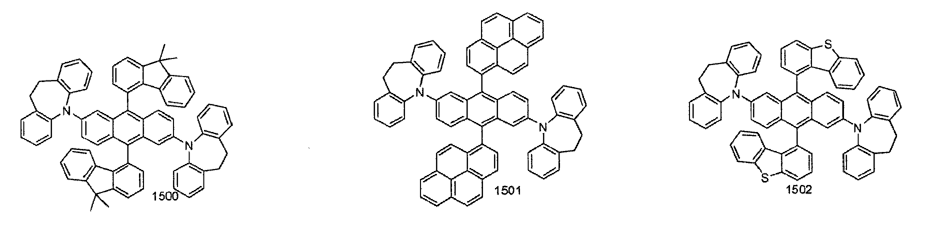

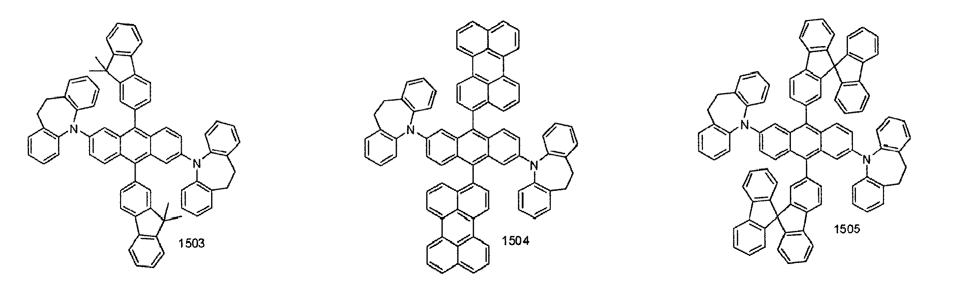

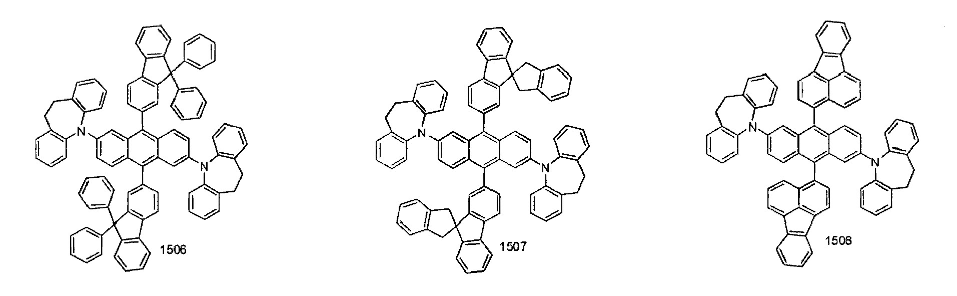

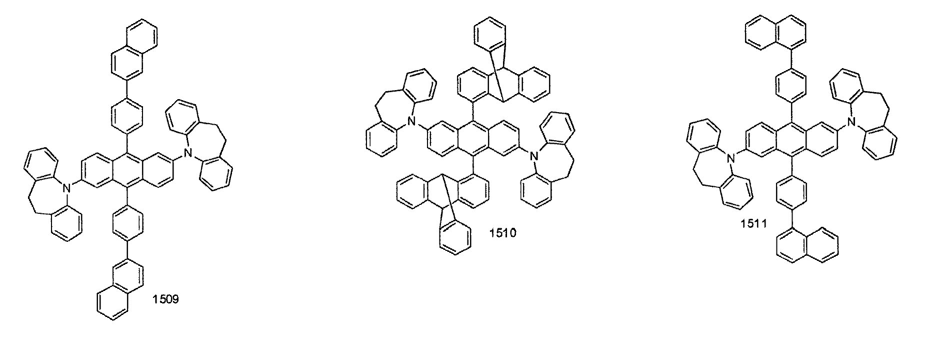

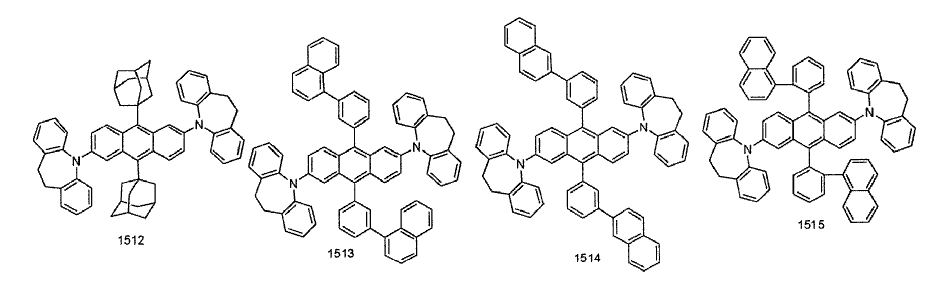

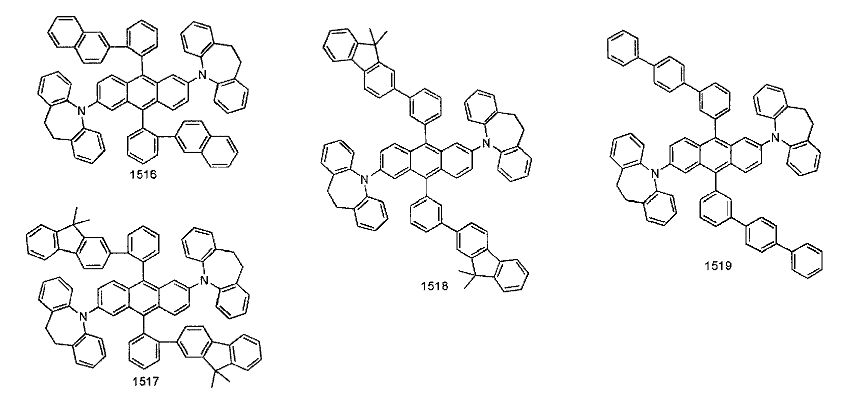

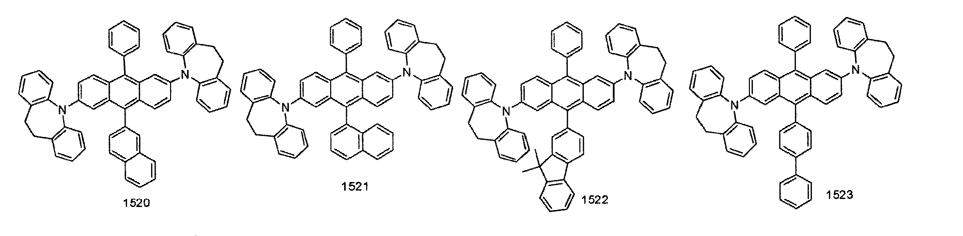

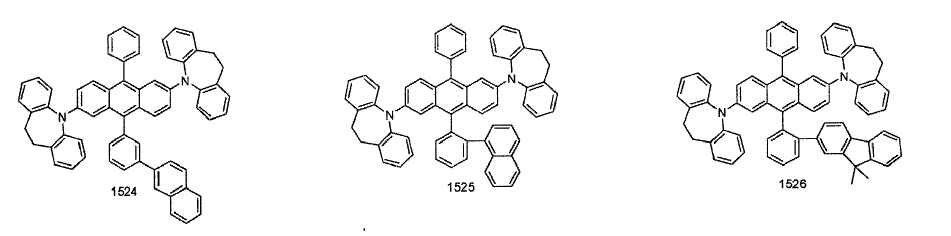

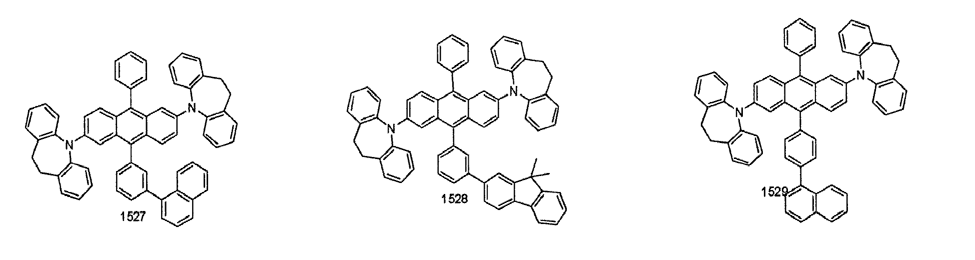

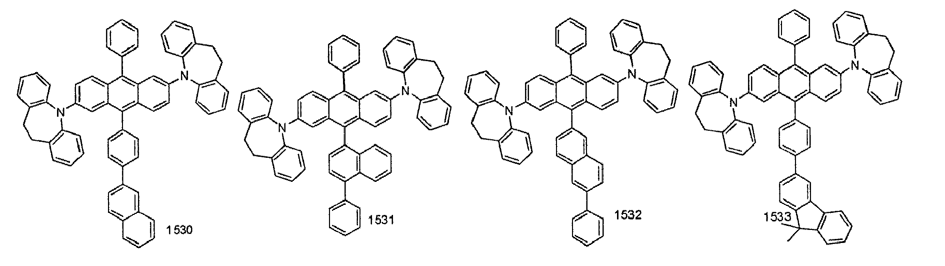

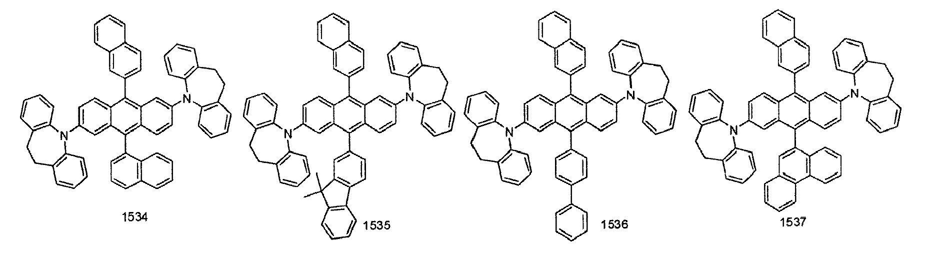

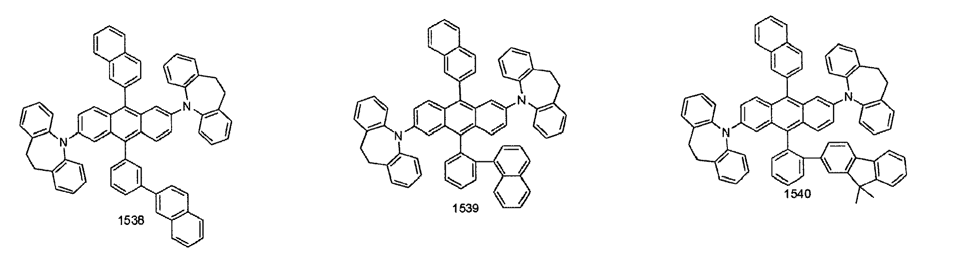

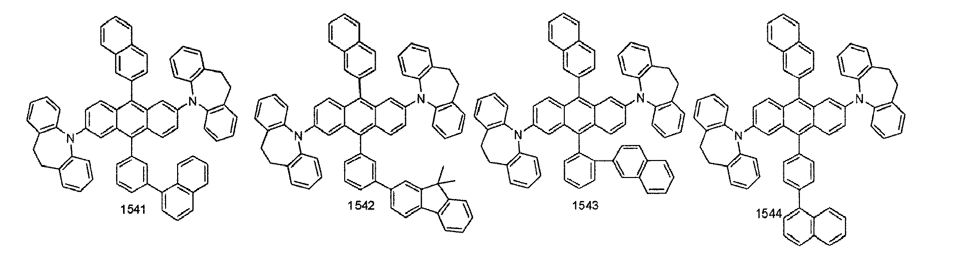

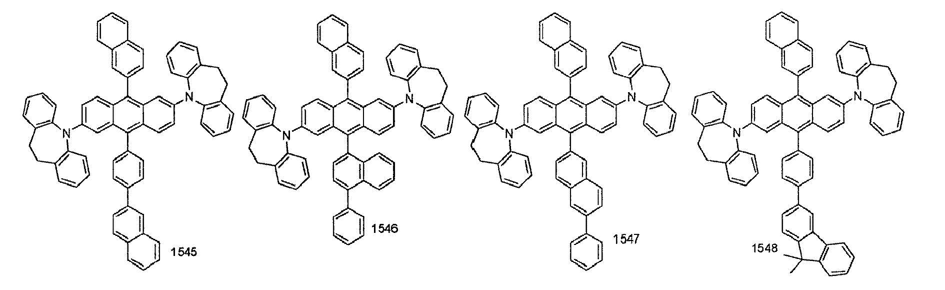

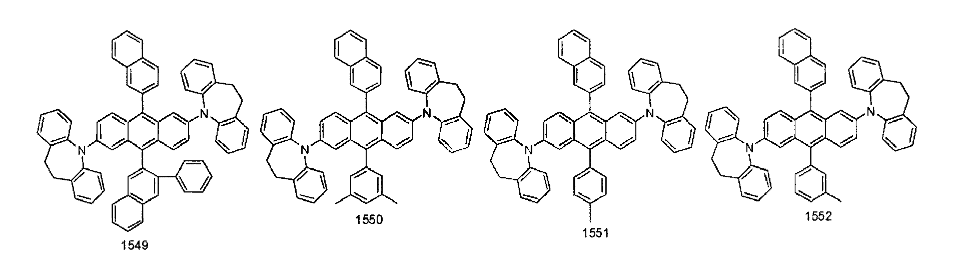

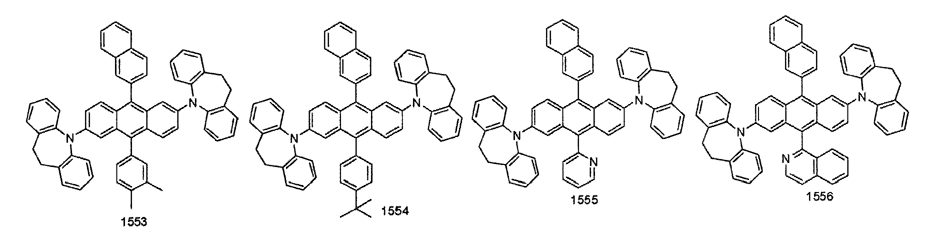

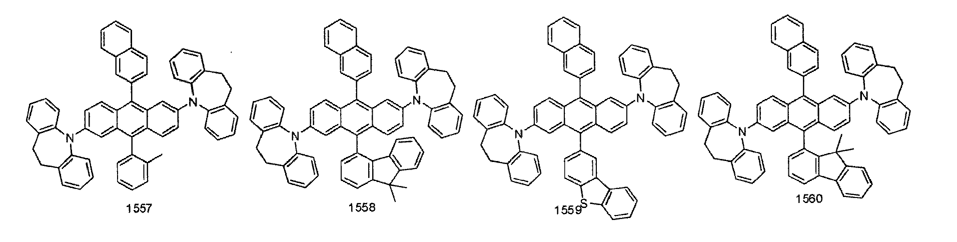

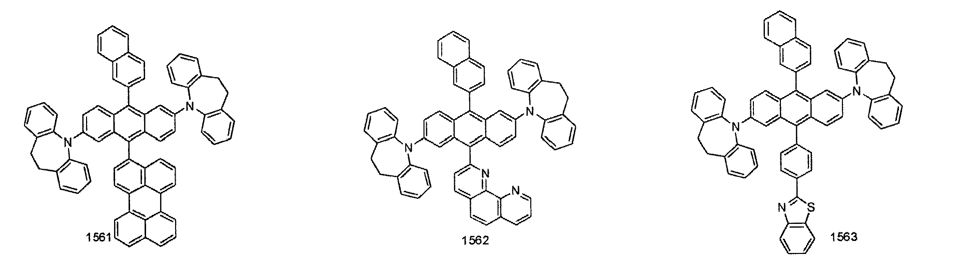

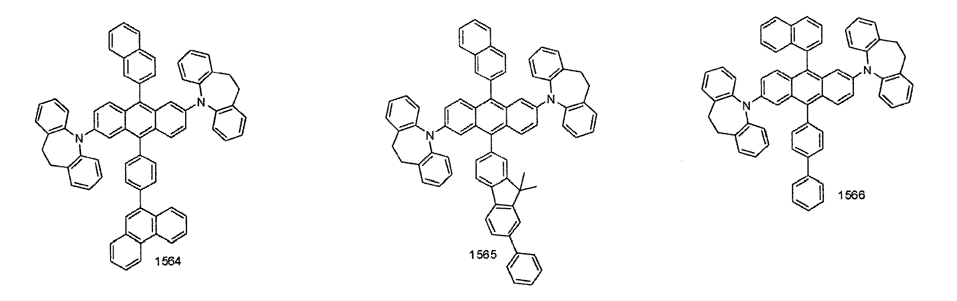

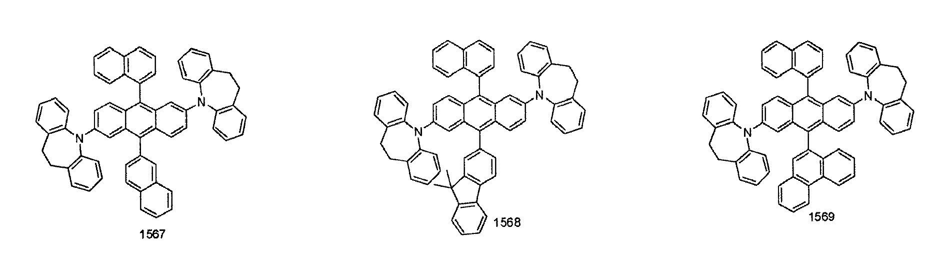

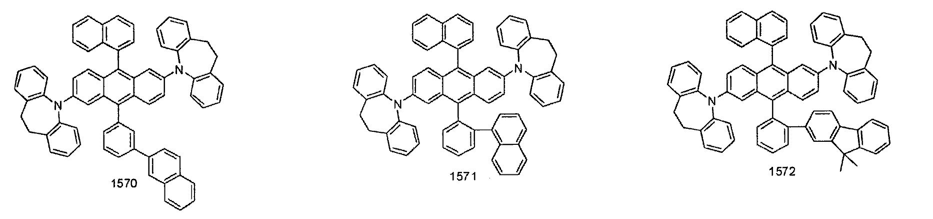

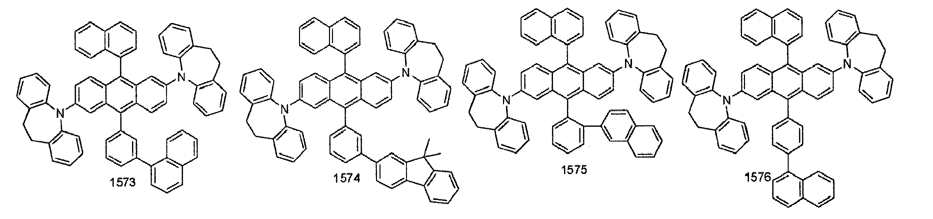

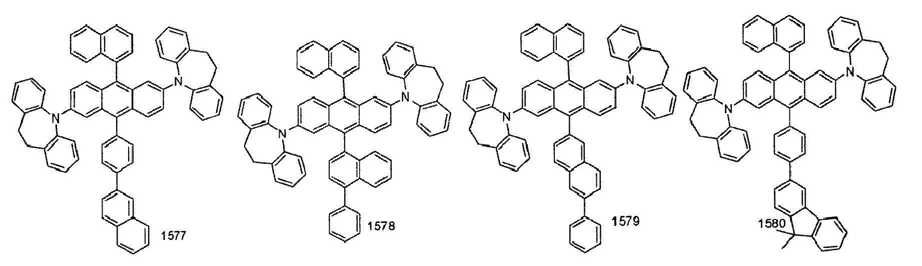

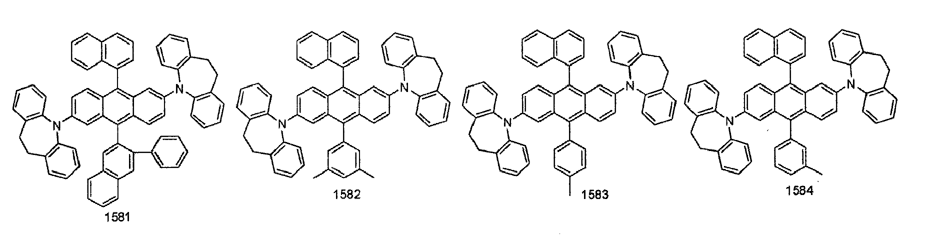

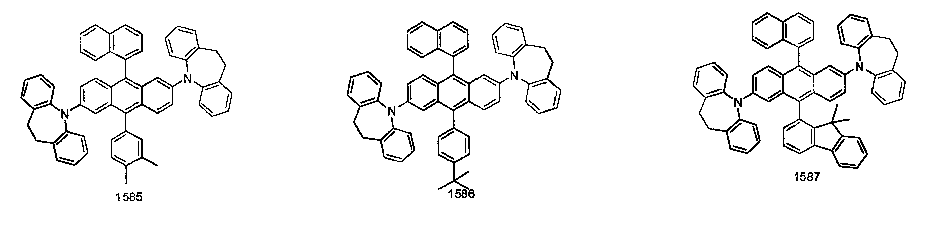

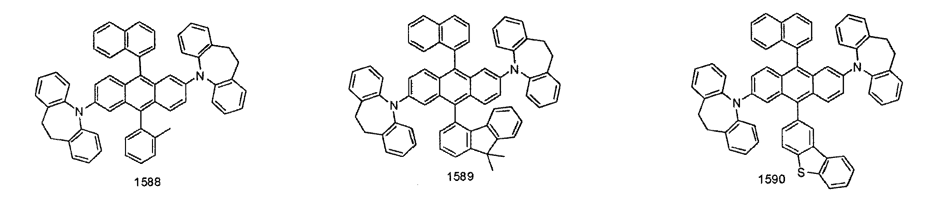

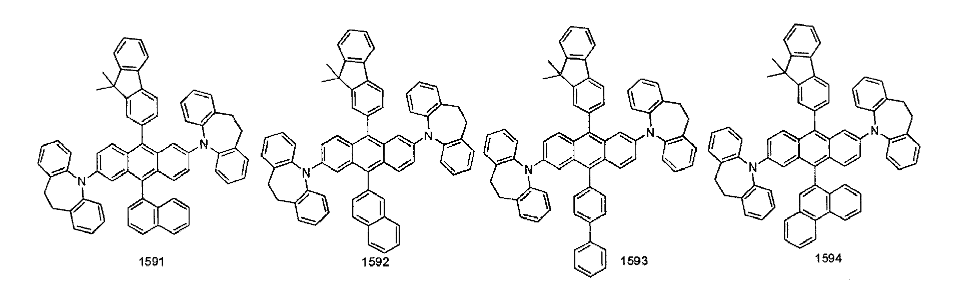

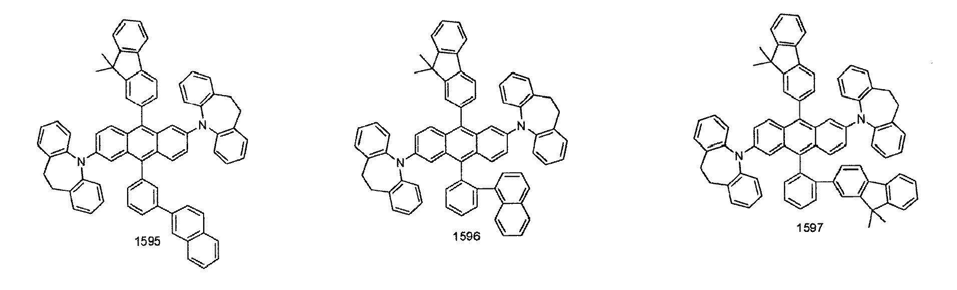

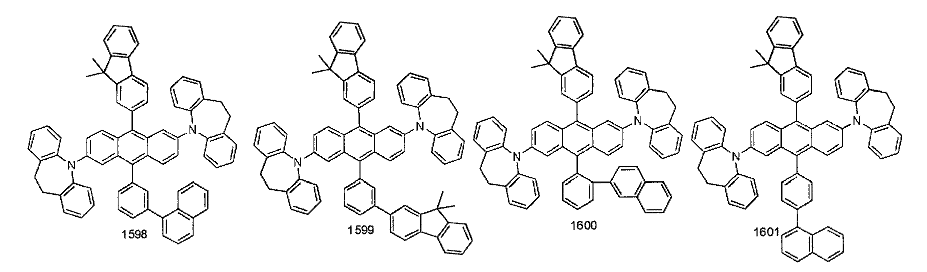

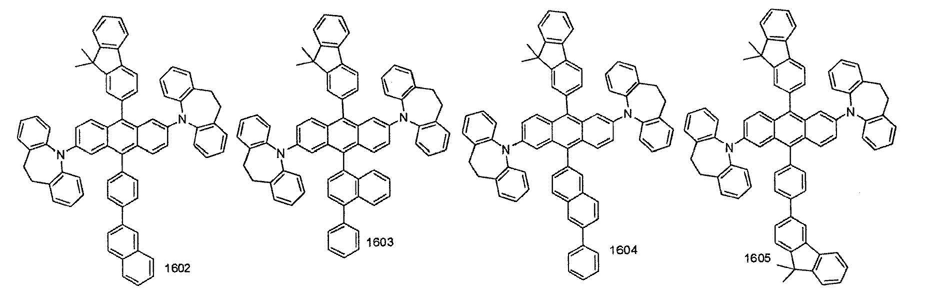

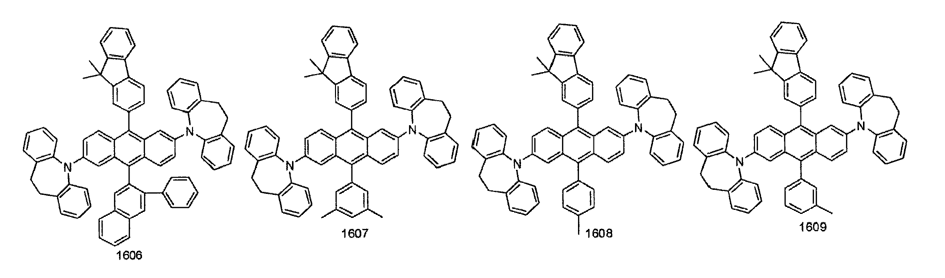

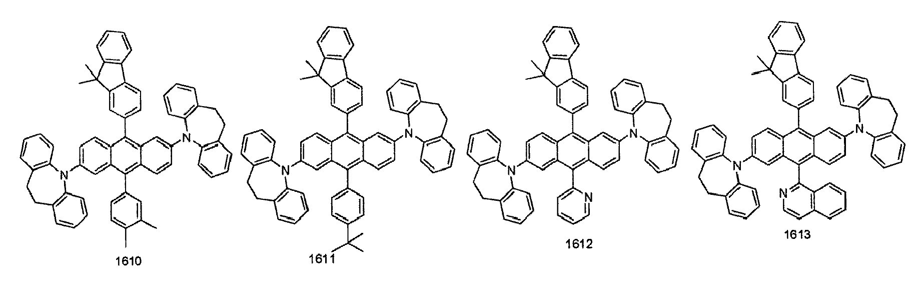

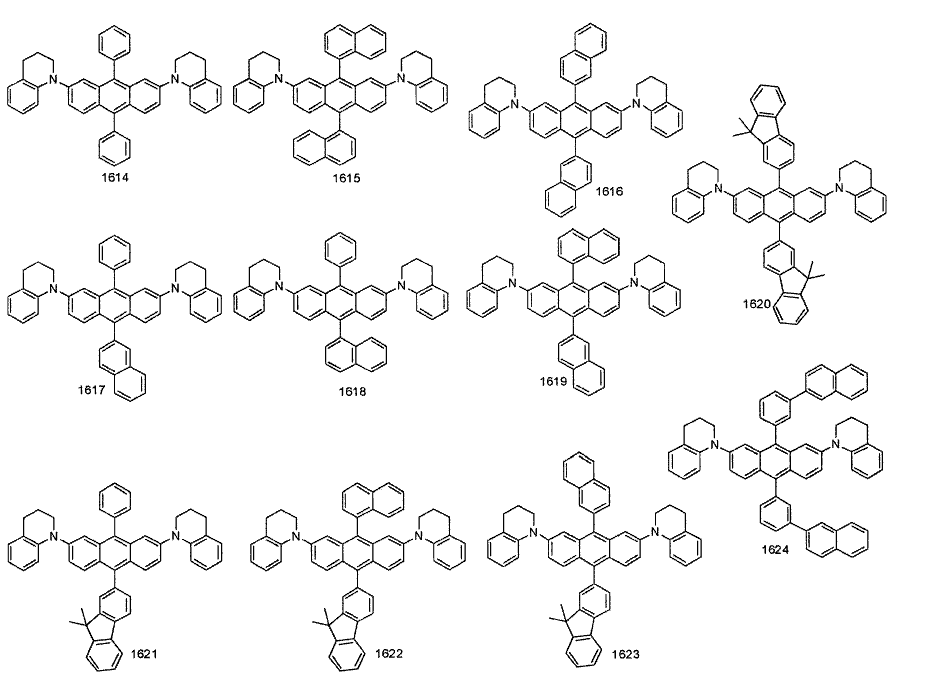

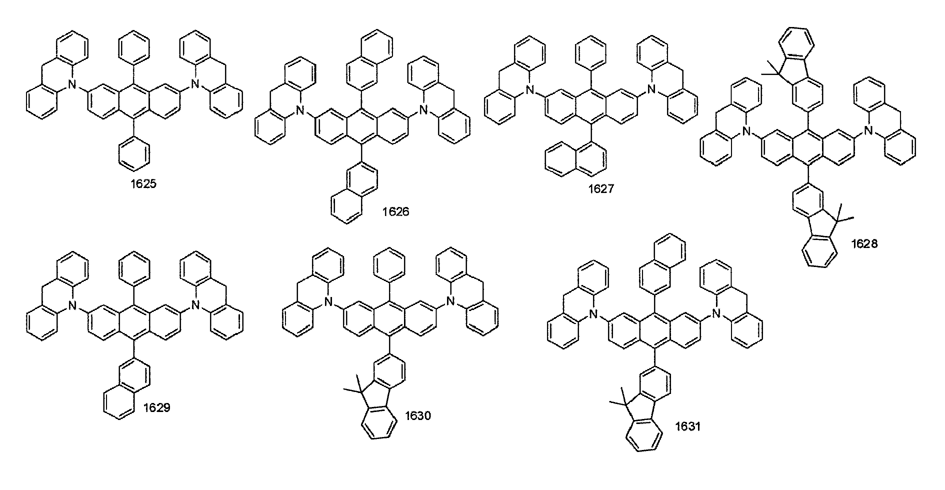

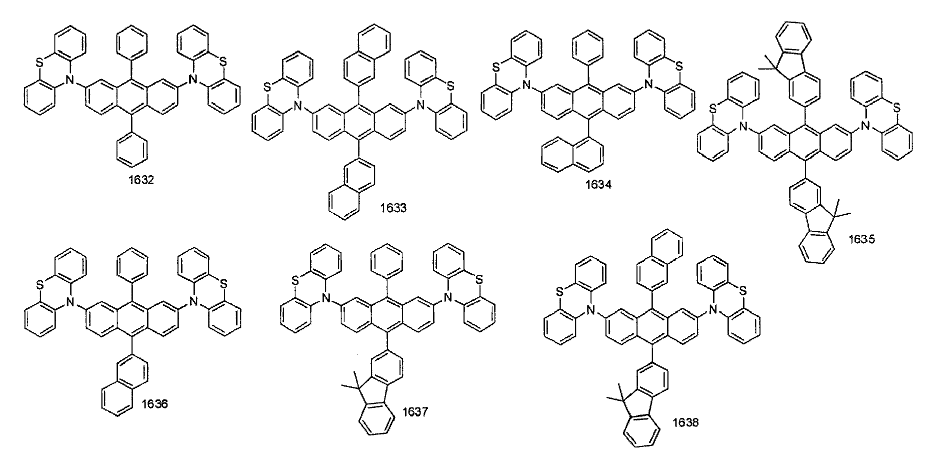

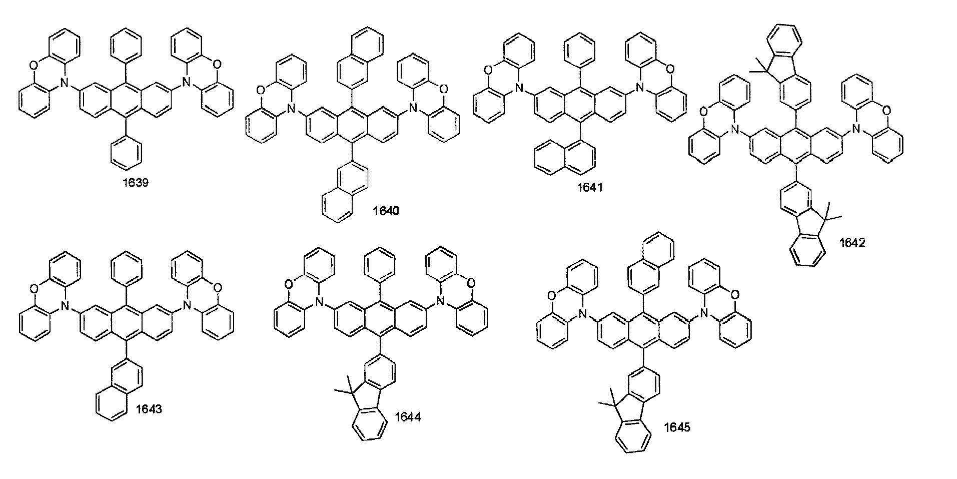

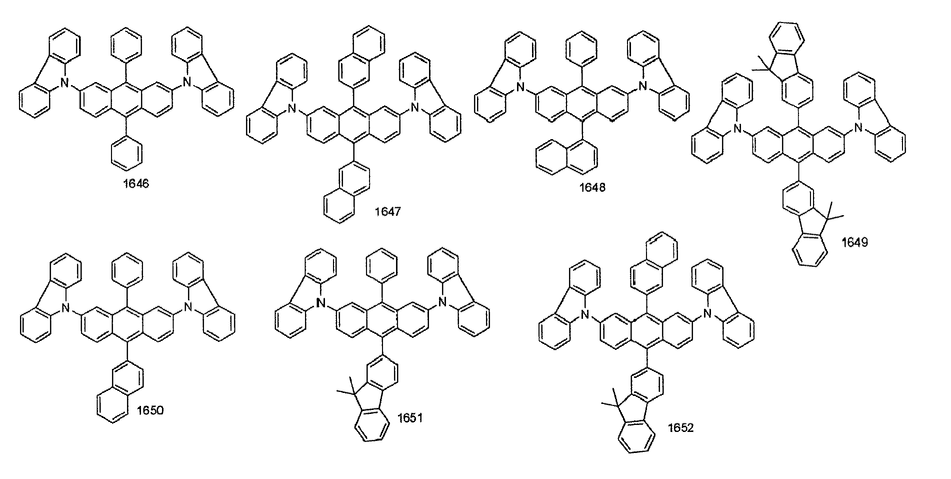

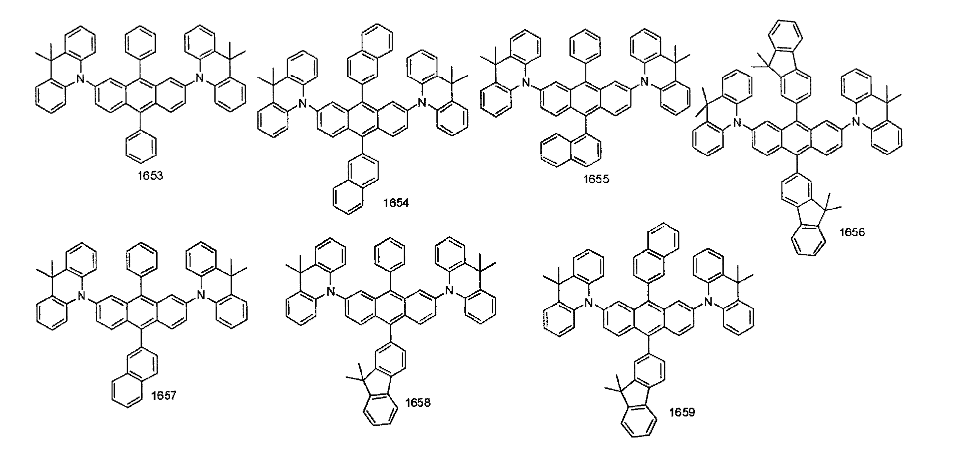

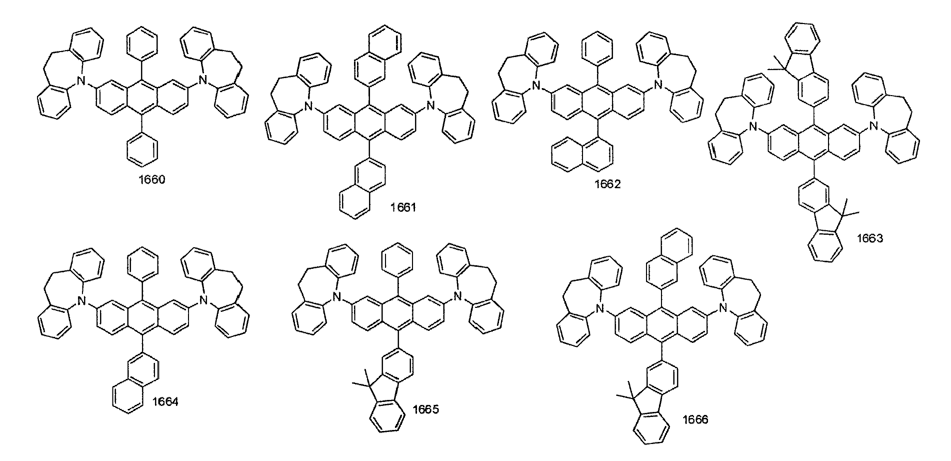

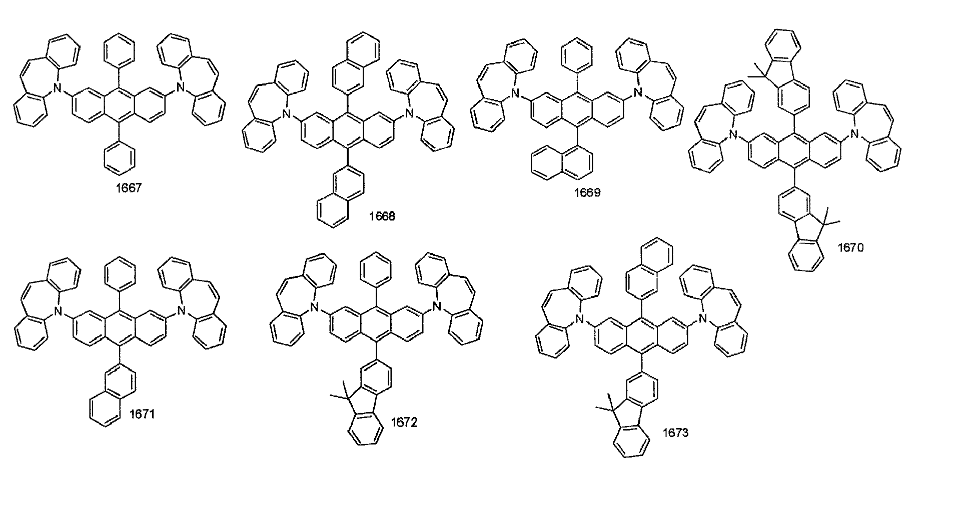

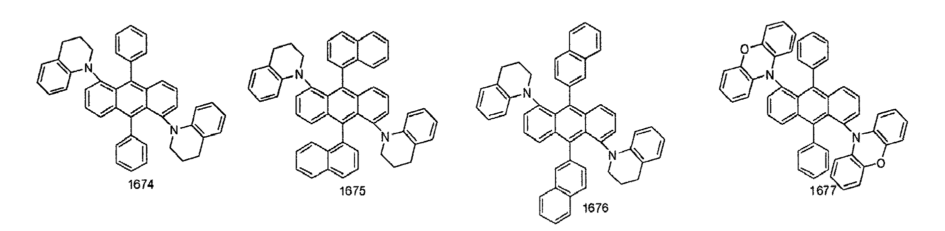

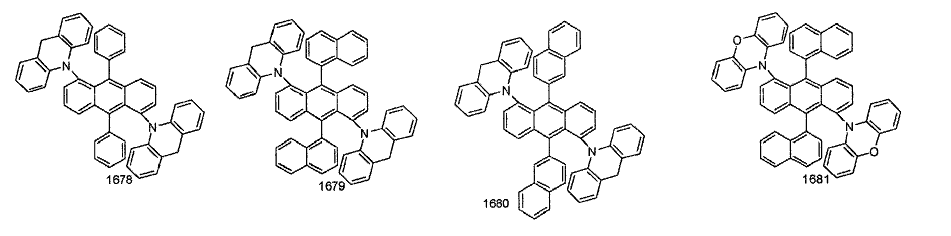

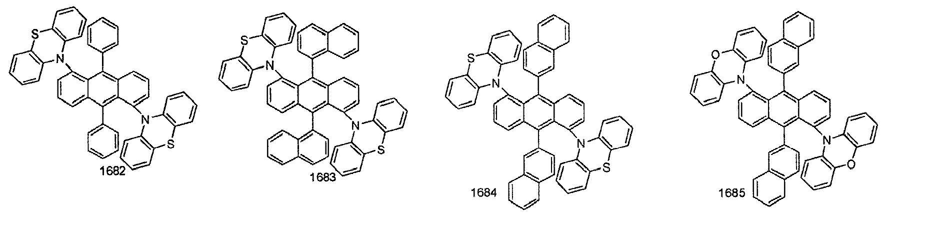

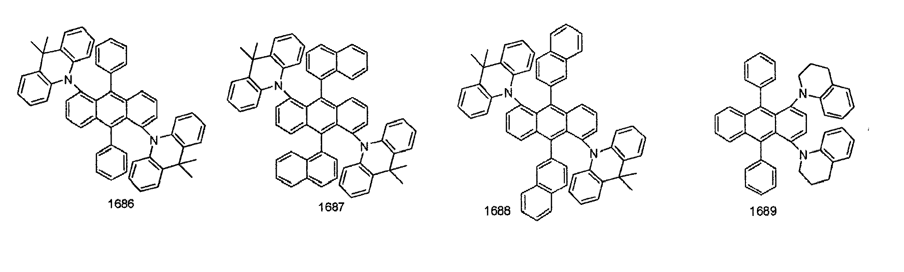

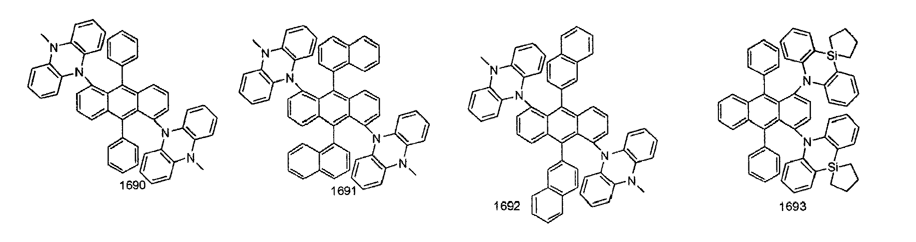

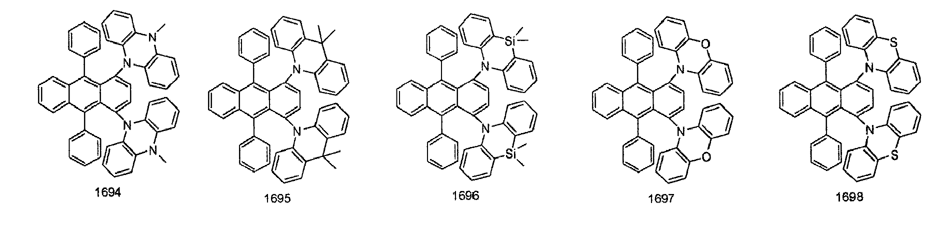

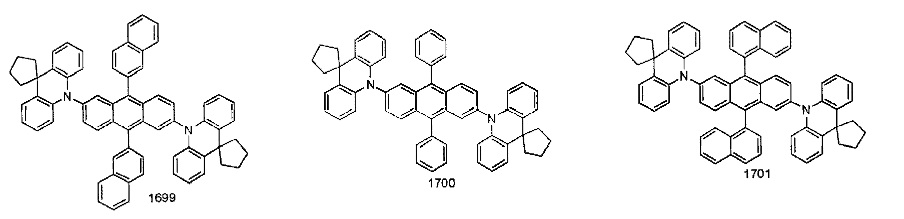

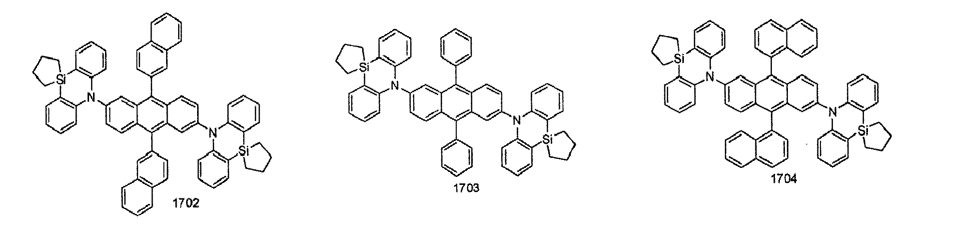

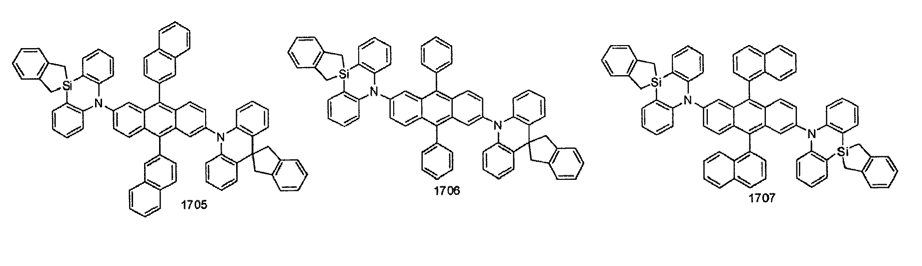

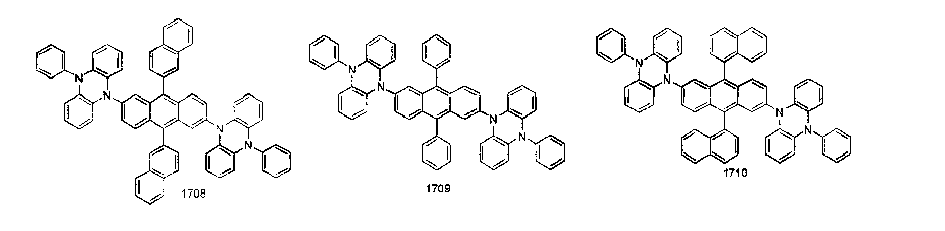

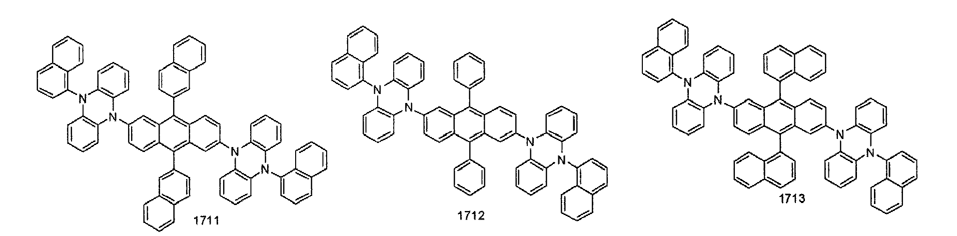

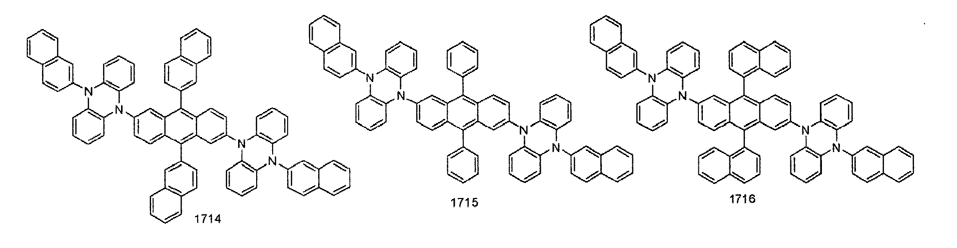

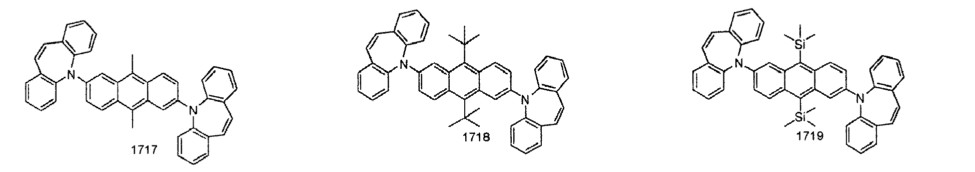

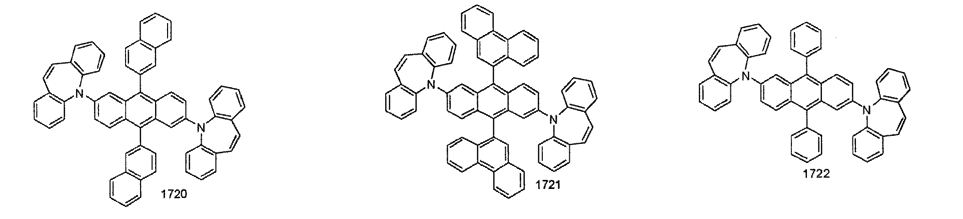

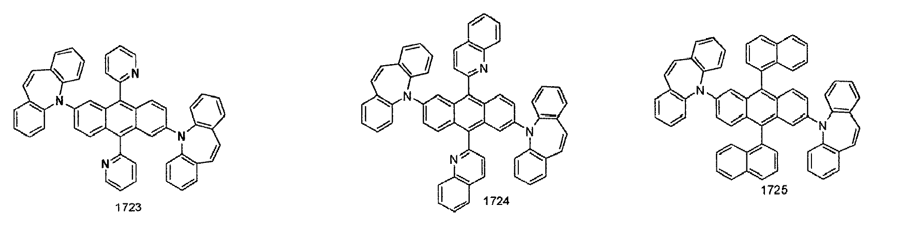

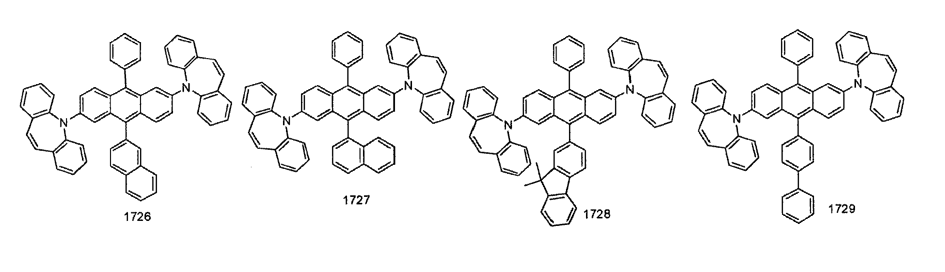

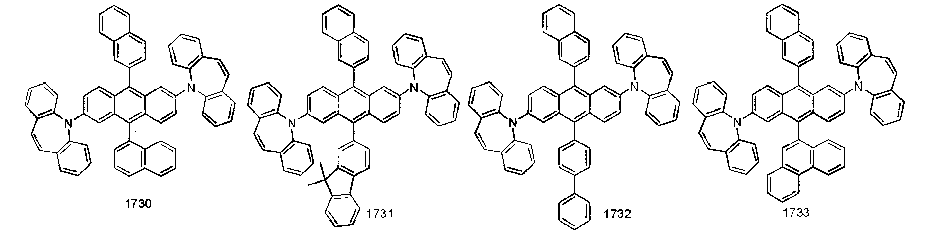

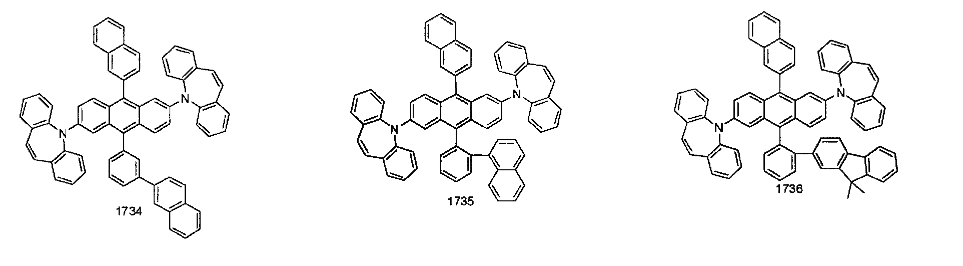

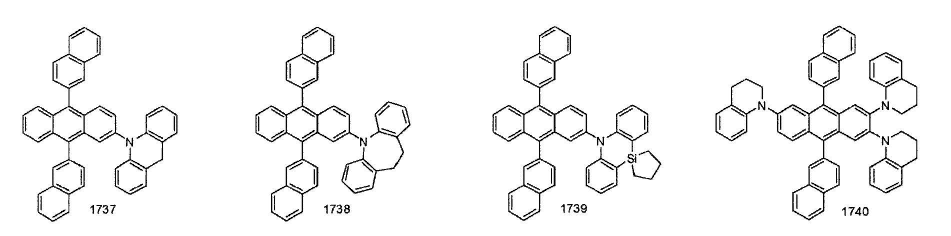

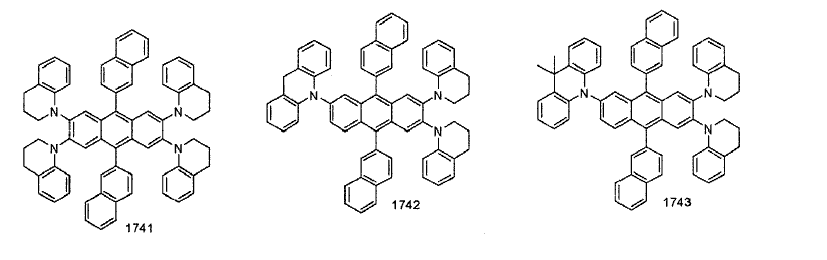

The organic electroluminescent compounds according to the present invention can be prepared according to the procedure illustrated by Reaction Scheme (1): 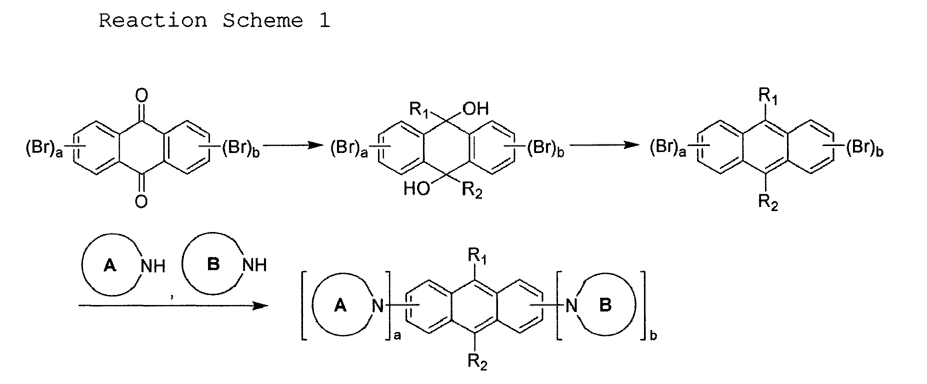 wherein, ring A, ring B, R₁, R₂, a and b are defined as in Chemical Formula (1).

In addition, the present invention provides organic solar cells, which comprise one or more organic electroluminescent compound(s) represented by Chemical Formula (1).

The present invention also provides an organic electroluminescent device which is comprised of a first electrode; a second electrode; and at least one organic layer(s) interposed between the first electrode and the second electrode; wherein the organic layer comprises one or more organic electroluminescent compound(s) represented by Chemical Formula (1).

The organic electroluminescent device according to the present invention is **characterized in that** the organic layer comprises an electroluminescent layer containing one or more organic electroluminescent compound(s) represented by Chemical Formula (1) as electroluminescent dopant, and one or more host (s) .

The host to be applied to an organic electroluminescent device according to the invention is not particularly restricted, but preferably selected from the compounds represented by Chemical Formula (6) or (7):

Chemical Formula 6 (Ar₁)_{c}-L₁₁- (Ar₂)_{d}

Chemical Formula 7 (Ar₃)ₑ-L₁₂-(Ar₄)_{f}

wherein, L₁₁ represents (C6-C60)arylene or (C4-C60)heteroarylene;
L₁₂ represents anthracenylene;
Ar₁ through Ar₄ are independently selected from hydrogen, (C1-C60)alkyl, (C1-C60)alkoxy, halogen, (C4-C60)heteroaryl, (C5-C60)cycloalkyl and (C6-C60)aryl; the cycloalkyl, aryl or heteroaryl of Ar₁ through Ar₄ may be further substituted by one or more substituent(s) selected from a group consisting of (C6-C60)aryl or (C4-C60)heteroaryl with or without one or more substituent(s) selected from a group consisting of (C1-C60)alkyl with or without halogen substituents(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; and
c, d, e and f independently represent an integer from 0 to 4.

The hosts represented by Chemical Formula (6) or (7) can be exemplified by anthracene derivatives or benz[a]anthracene derivatives represented by one of Chemical Formulas (8) to (10): 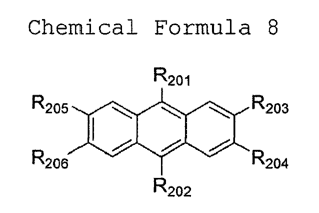 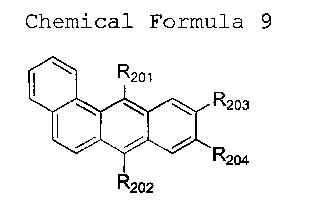 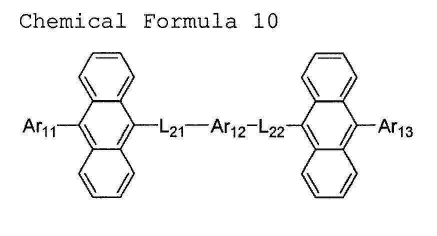 wherein, R₂₀₁ and R₂₀₂ independently represent (C6-C60)aryl, (C4-C60)heteroaryl, 5- or 6-membered heterocycloalkyl containing one or more heteroatom(s) selected from N, O and S, or (C3-C60)cycloalkyl; the aryl or heteroaryl of R₂₀₁ and R₂₀₂ may be further substituted by one or more substituent(s) selected from a group consisting of (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₂₀₃ through R₂₀₆ independently represent hydrogen, (C1-C60)alkyl, (C1-C60)alkoxy, halogen, (C4-C60)heteroaryl, (C5-C60)cycloalkyl or (C6-C60)aryl; the heteroaryl, cycloalkyl or aryl of R₂₀₃ through R₂₀₆ may be further substituted by one or more substituent(s) selected from a group consisting of (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
L₂₁ and L₂₂ independently represent a chemical bond, or (C6-C60)arylene with or without one or more substituent(s) selected from (C1-C60)alkyl, (C1-C60)alkoxy, (C6-C60)aryl, (C4-C60)heteroaryl and halogen;
Ar₁₁ and Ar₁₃ independently represent aryl selected from the following structures, or (C4-C60)heteroaryl; 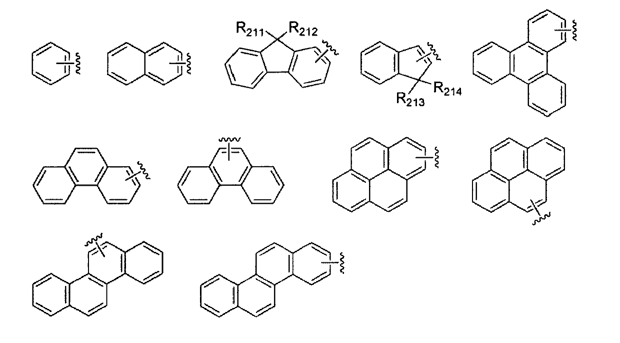 the aryl or heteroaryl of Ar₁₁ and Ar₁₃ may be substituted by one or more substituent(s) selected from (Cl-C60)alkyl, (C1-C60)alkoxy, (C6-C60)aryl and (C4-C60)heteroaryl;
Ar₁₂ represents (C6-C60)arylene, (C4-C60)heteroarylene or a compound having the following structure; 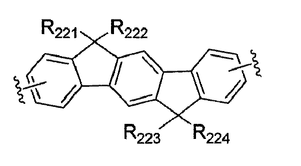 the arylene or heteroarylene of Ar₁₂ may be substituted by one or more substituent(s) selected from (C1-C60)alkyl, (C1-C60)alkoxy, (C6-C60)aryl, (C4-C60)heteroaryl and halogen;

R₂₁₁, R₂₁₂, R₂₁₃ and R₂₁₄ independently represent hydrogen, (C1-C60)alkyl or (C6-C60)aryl, or they may be linked to an adjacent substituent via (C3-C60)alkylene or (C3-C60)alkenylene with or without a fused ring to form an alicyclic ring, or a monocyclic or polycyclic aromatic ring; and
R₂₂₁, R₂₂₂, R₂₂₃ and R₂₂₄ independently represent hydrogen, (C1-C60)alkyl, (C1-C60)alkoxy, (C6-C60)aryl, (C4-C60)heteroaryl or halogen, or they may be linked to an adjacent substituent via (C3-C60)alkylene or (C3-C60)alkenylene with or without a fused ring to form an alicyclic ring, or a monocyclic or polycyclic aromatic ring.

The electroluminescent layer means the layer where electroluminescence occurs, and it may be a single layer or a multi-layer consisting of two or more layers laminated. When a mixture of host-dopant is used according to the constitution of the present invention, noticeable improvement in luminous efficiency due to the inventive electroluminescent host could be confirmed. This can be achieved by the doping concentration of 0.5 to 10% by weight. The host according to the present invention exhibits higher hole and electron conductivity, and excellent stability of material as compared to other conventional host materials, and provides improved device life as well as luminous efficiency.

Thus, it can be described that use of the compound represented by one of Chemical Formulas (8) to (10) as an electroluminescent host significantly supplements electronic drawback of the organic electroluminescent compounds of Chemical Formula (1) according to the present invention.

The host compounds represented by one of Chemical Formulas (8) to (10) can be exemplified by the following compounds, but are not restricted thereto.

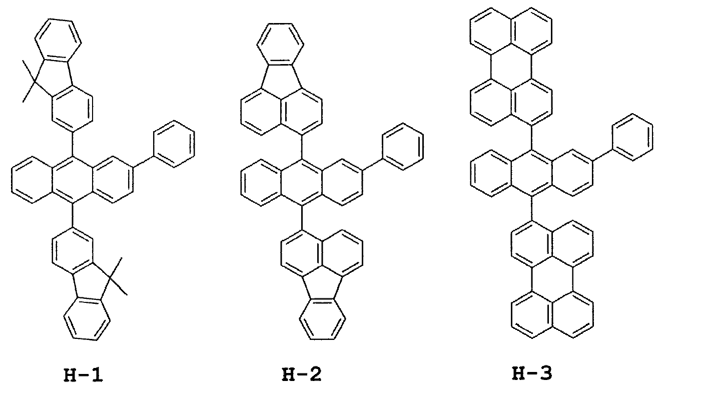

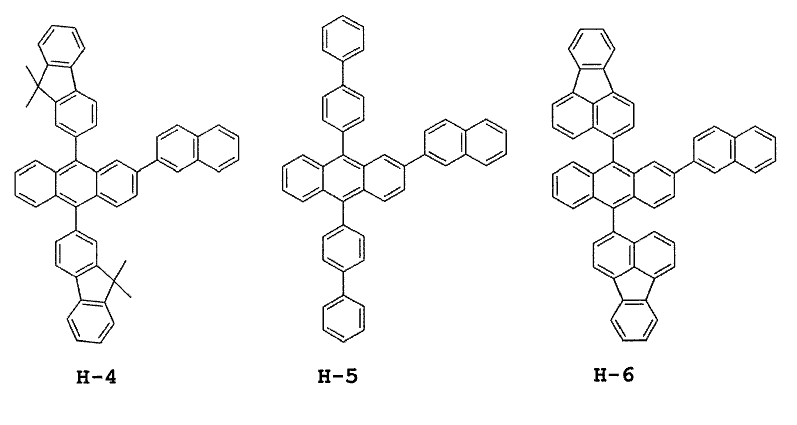

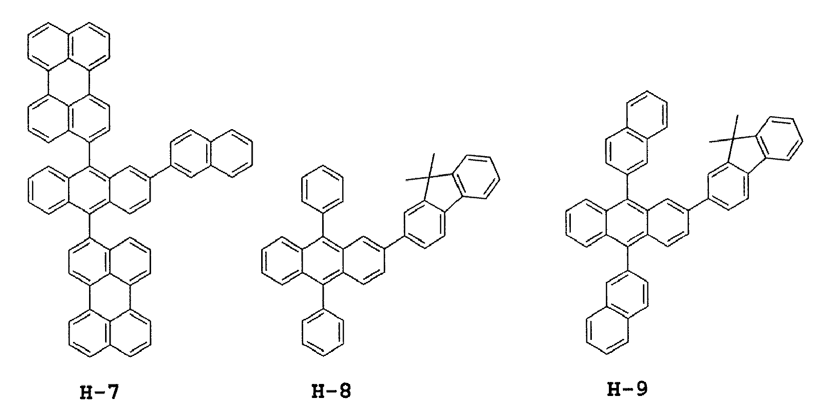

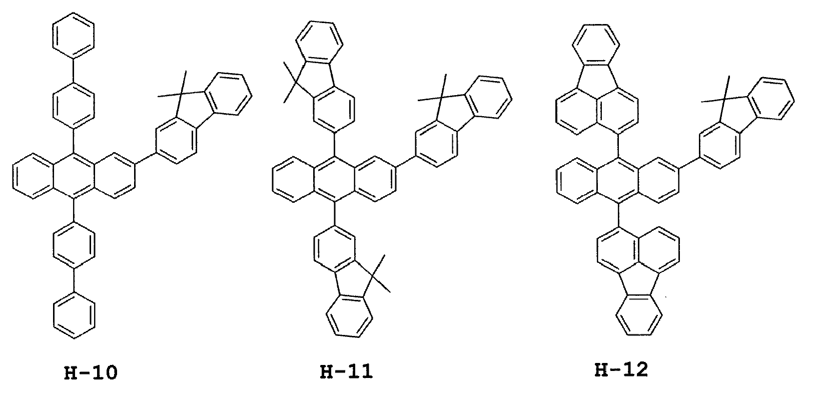

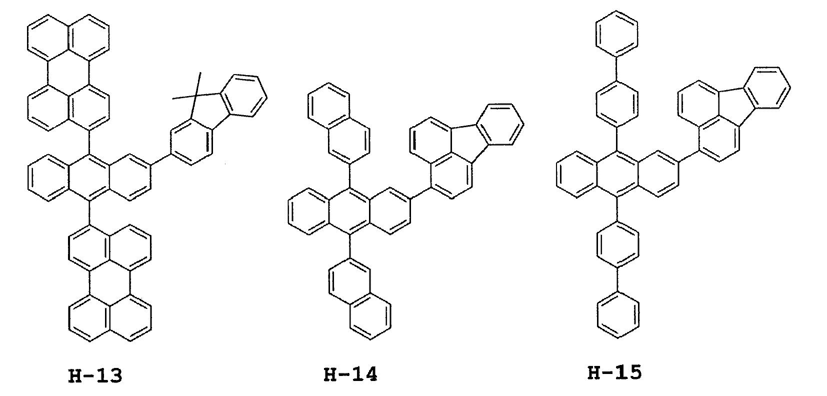

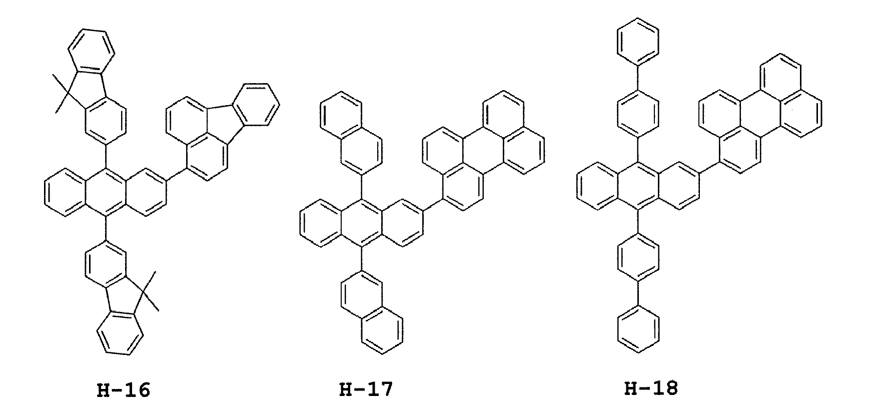

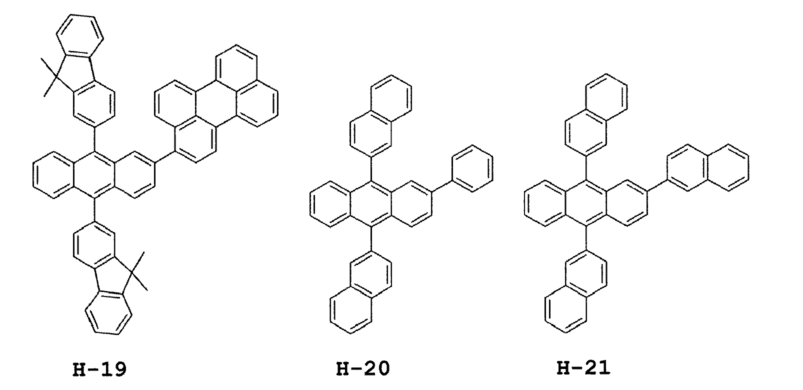

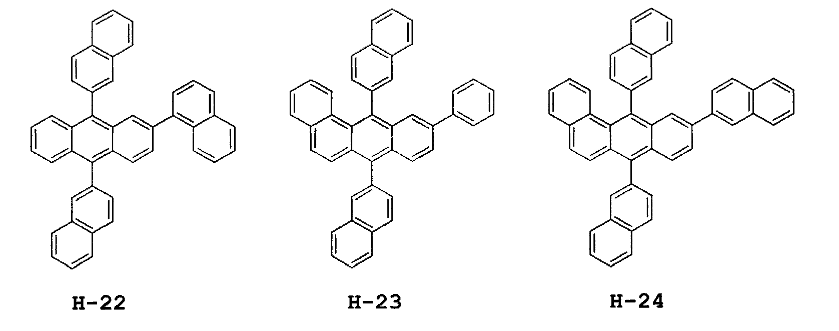

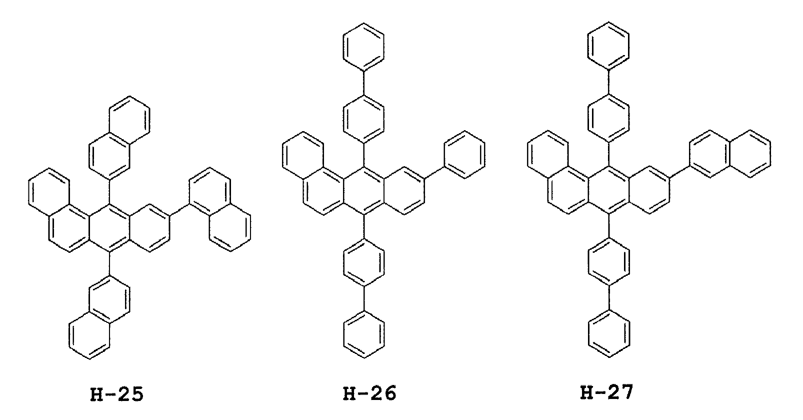

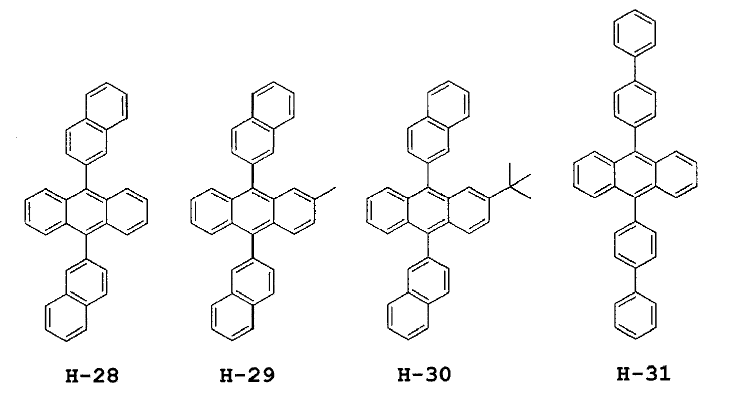

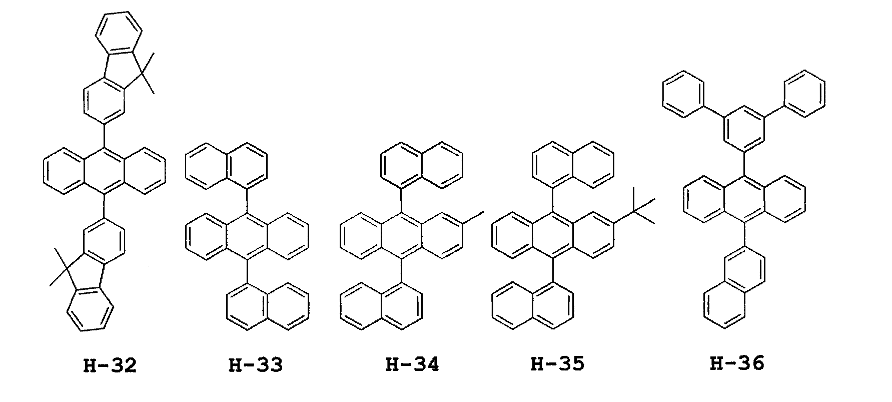

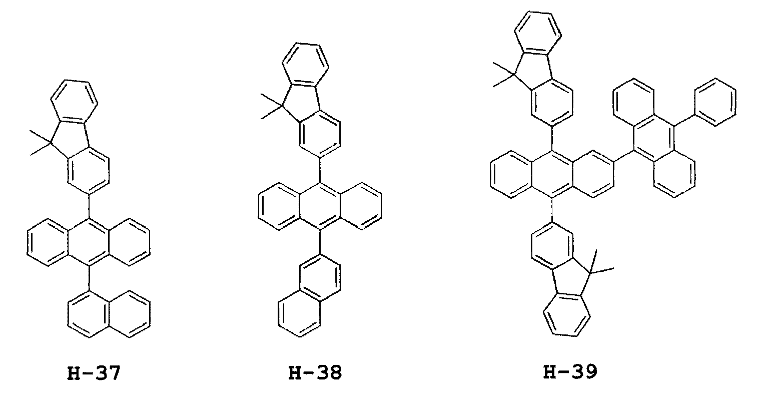

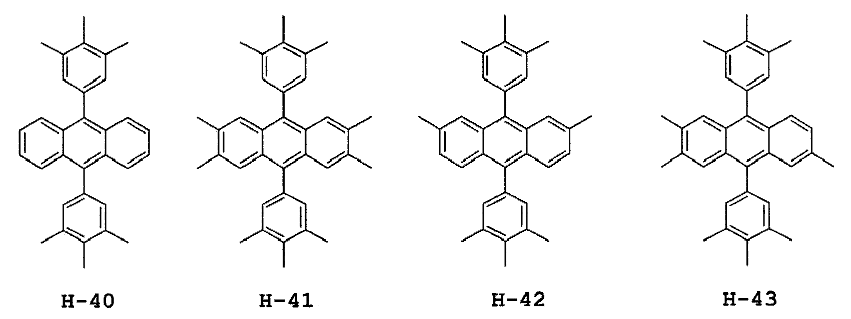

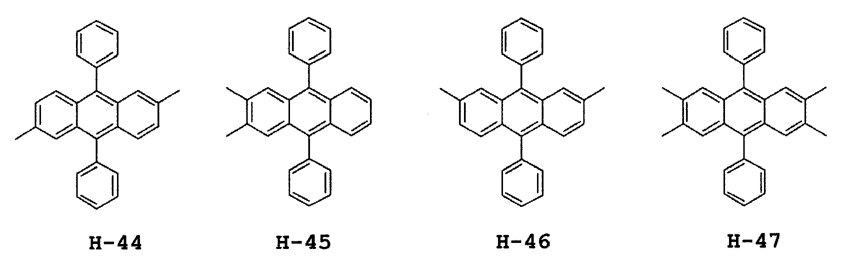

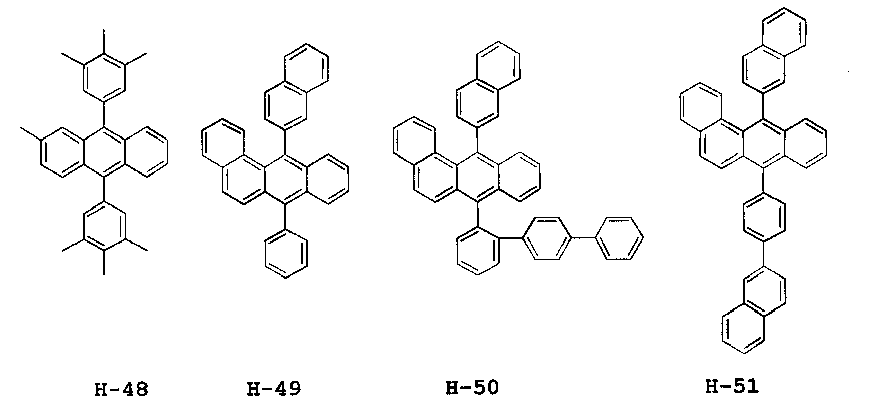

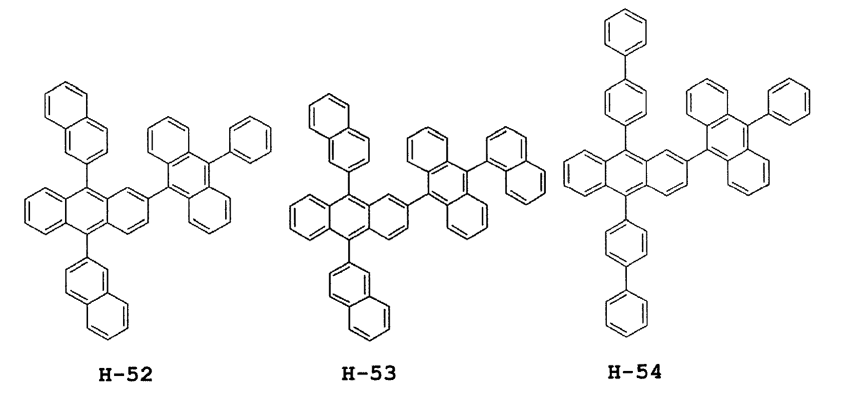

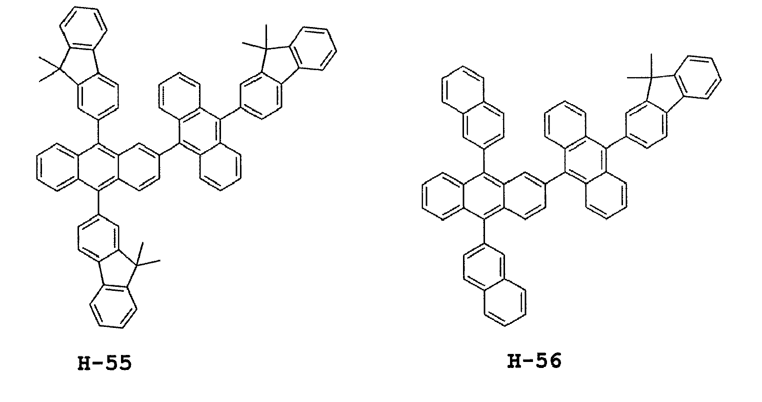

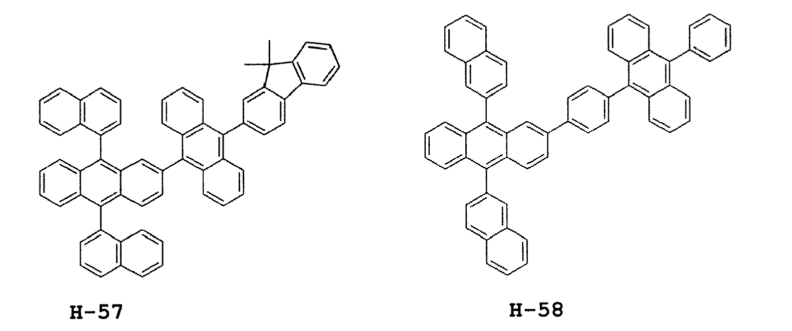

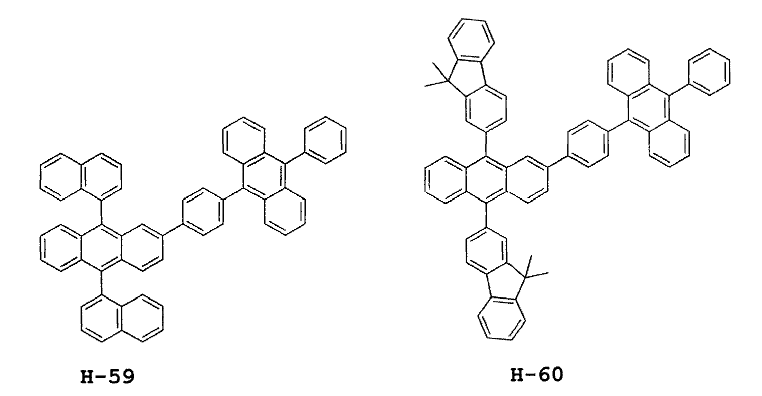

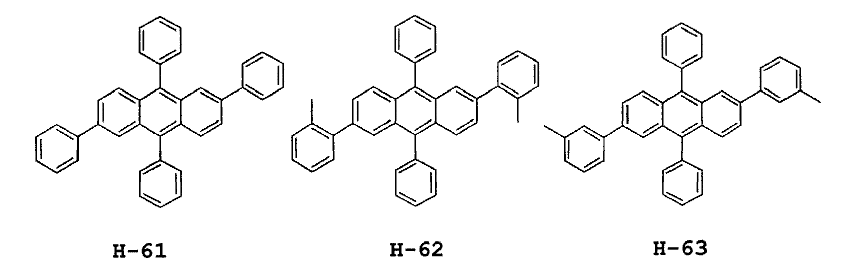

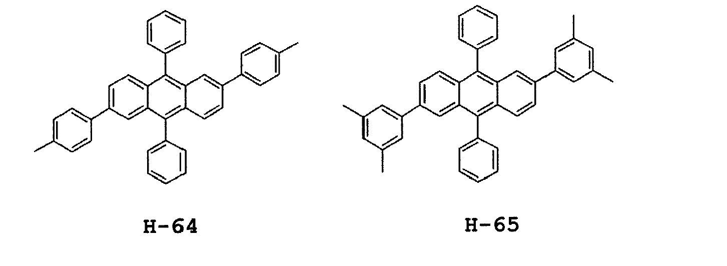

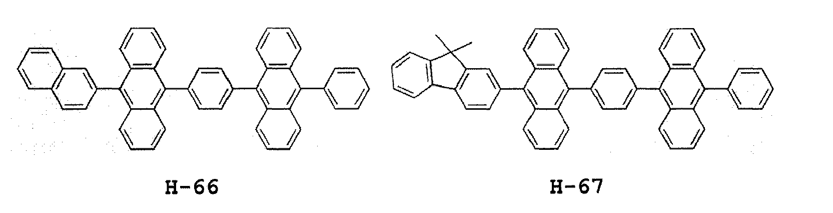

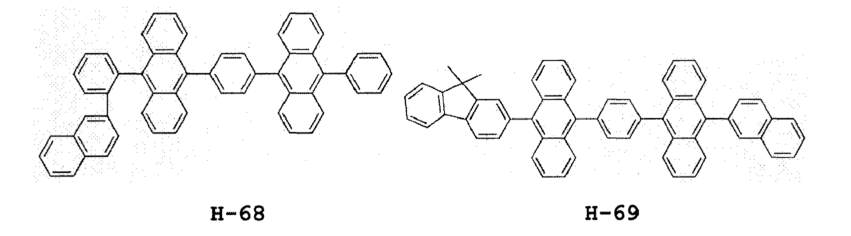

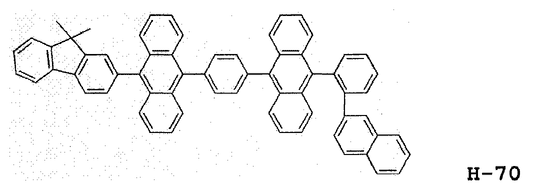

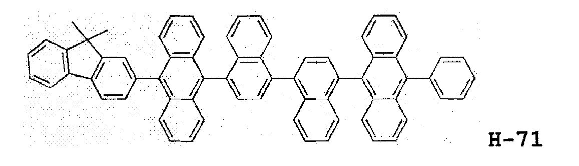

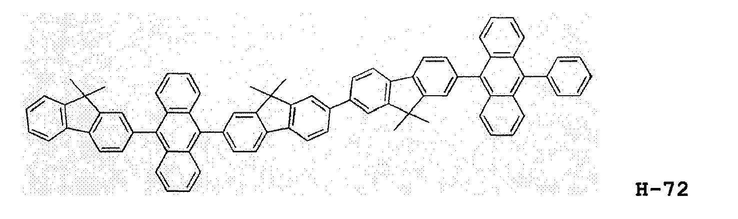

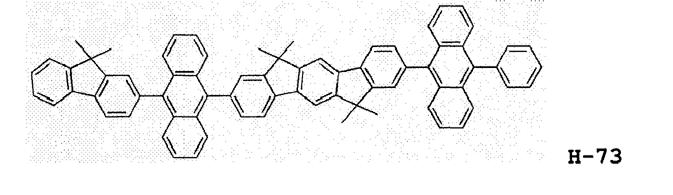

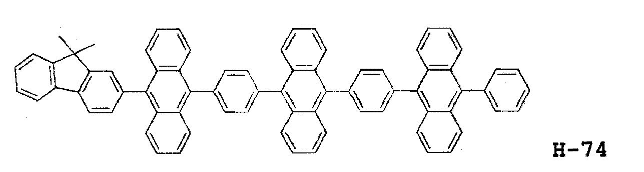

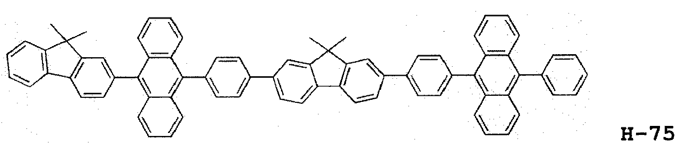

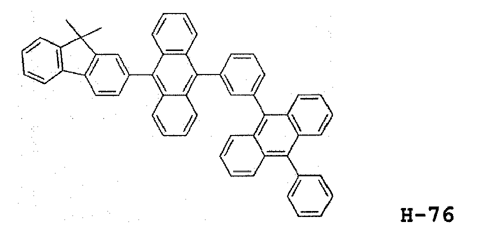

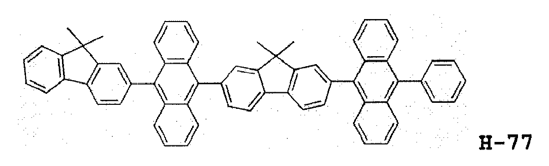

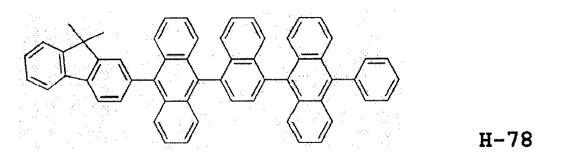

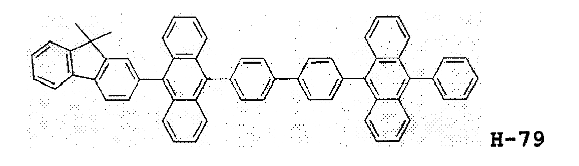

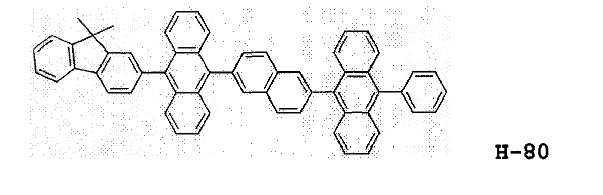

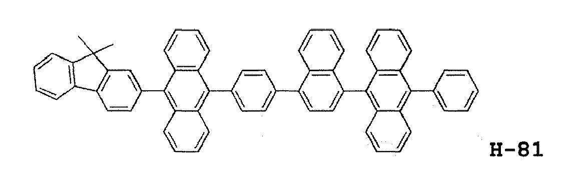

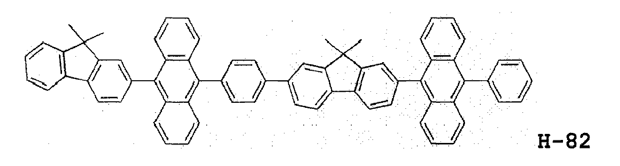

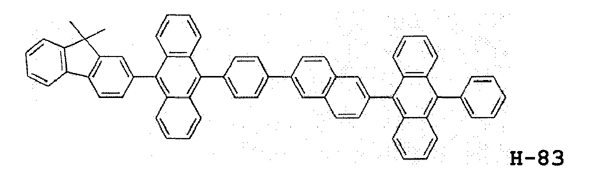

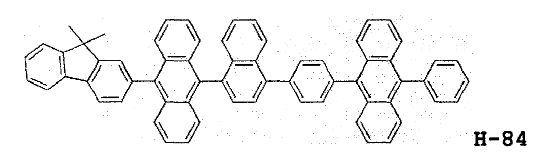

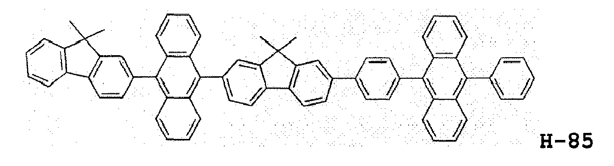

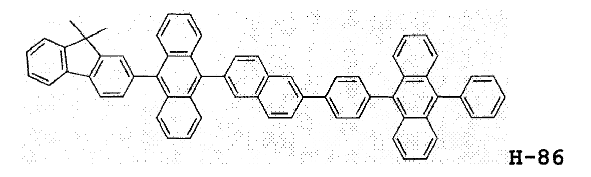

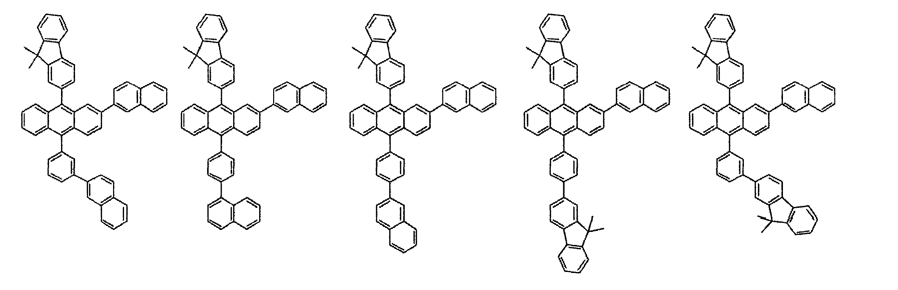

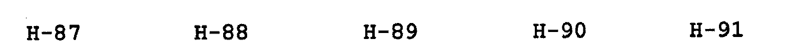

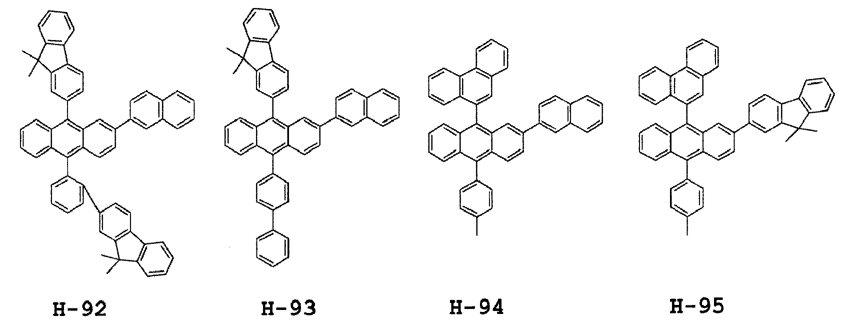

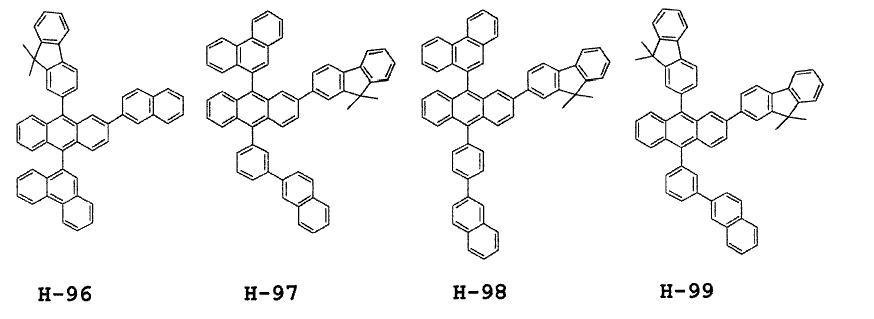

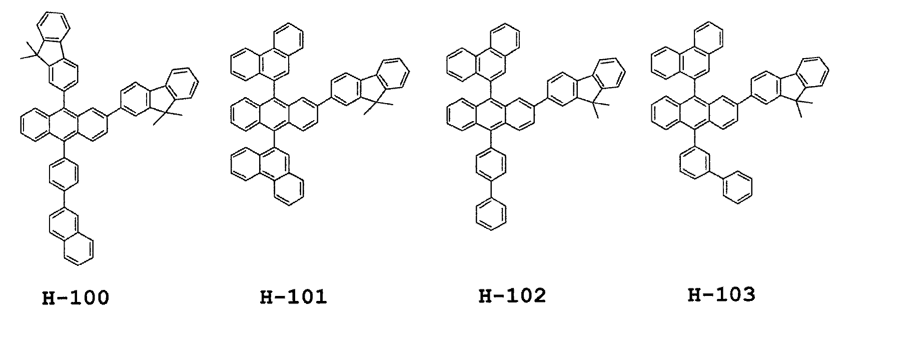

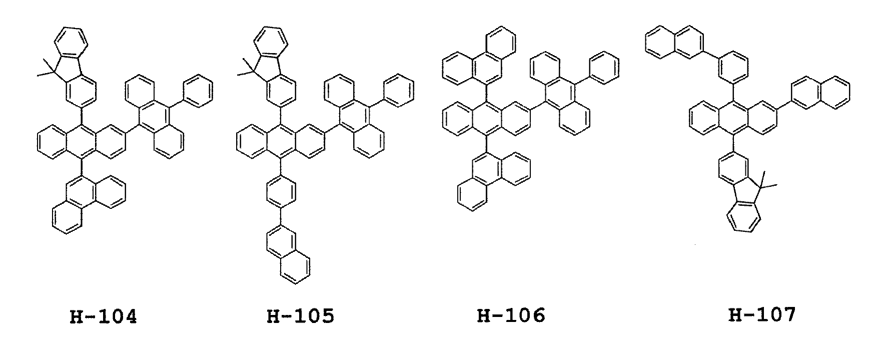

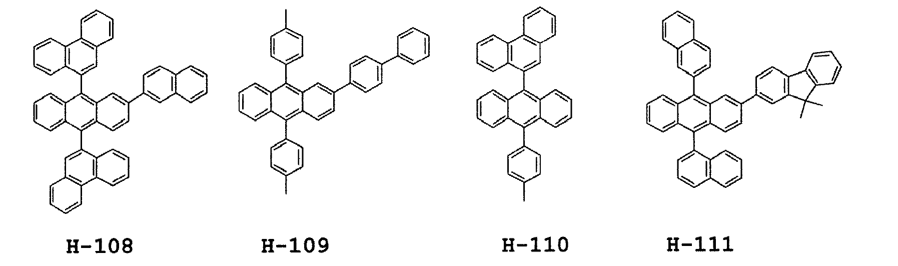

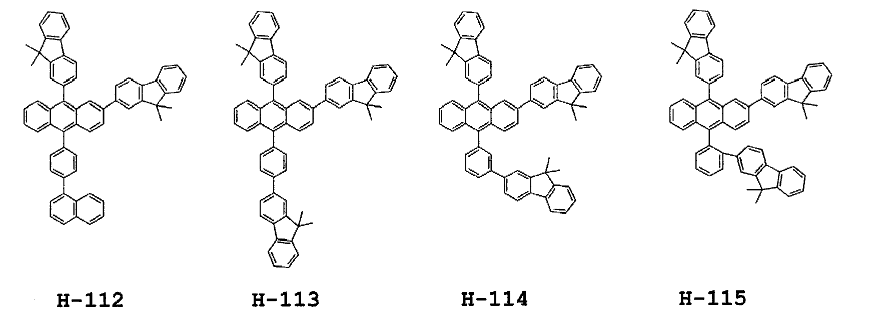

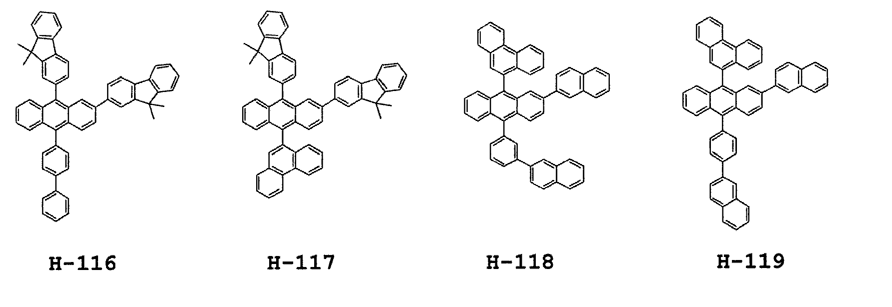

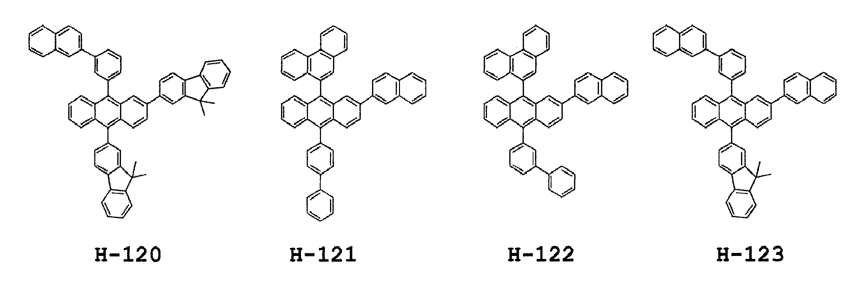

The organic electroluminescent device according to the invention may further comprise one or more compound(s) selected from arylamine compounds and styrylarylamine compounds, as well as the organic electroluminescent compound represented by Chemical Formula (1). Examples of the arylamine or styrylarylamine compounds include the compounds represented by Chemical Formula (11), but they are not restricted thereto: 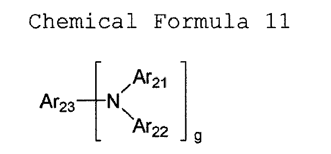 wherein, Ar₂₁ and Ar₂₂ independently represent (C1-C60)alkyl, (C6-C60)aryl, (C4-C60)heteroaryl, (C6-C60)arylamino, (C1-C60)alkylamino, morpholino or thiomorpholino, 5- or 6-membered heterocycloalkyl containing one or more heteroatom(s) selected from N, O and S, or (C3-C60)cycloalkyl, or Ar₂₁ and Ar₂₂ may be linked via (C3-C60)alkylene or (C3-C60)alkenylene with or without a fused ring to form an alicyclic ring, or a monocyclic or polycyclic aromatic ring; the aryl, heteroaryl, arylamino or heterocycloalkyl of Ar₂₁ and Ar₂₂ may be further substituted by one or more substituent(s) selected from halogen, (C1-C60)alkyl, (C2-C60)alkenyl, (C2-C60)alkynyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
Ar₂₃ represents (C6-C60)aryl, (C5-C60)heteroaryl or (C6-C60)arylamino; the aryl, heteroaryl or arylamino of Ar₂₃ may be further substituted by one or more substituent(s) selected from halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C6)arylsilyl, tri(C6-C60)arylsilyl, adamantyl, (C7-C60)bicycloalkyl, (C2-C60)alkenyl, (C2-C60)alkynyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and
g is an integer from 1 to 4.

The arylamine compounds or styrylarylamine compounds may be more specifically exemplified by the following compounds, but they are not restricted thereto.

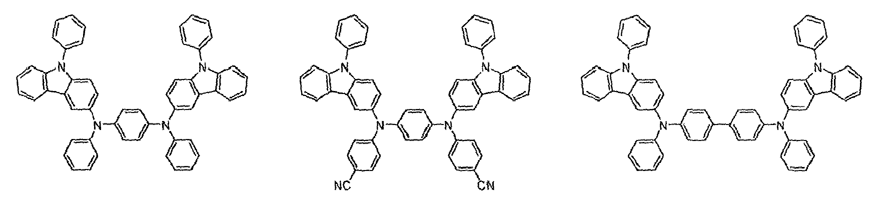

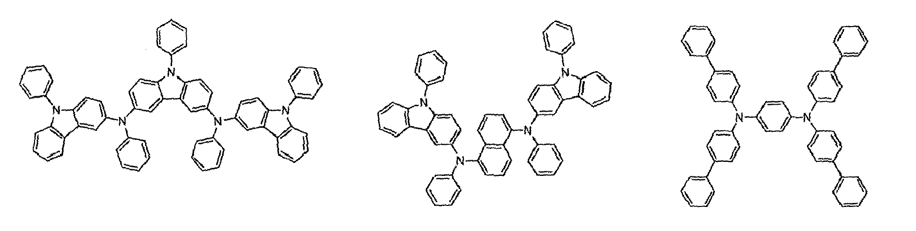

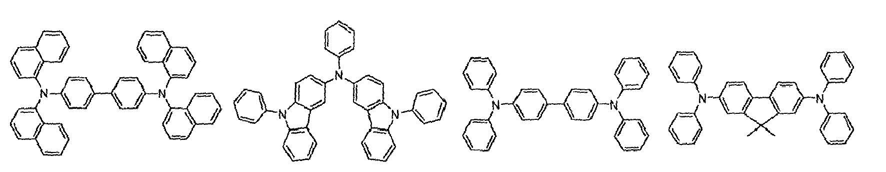

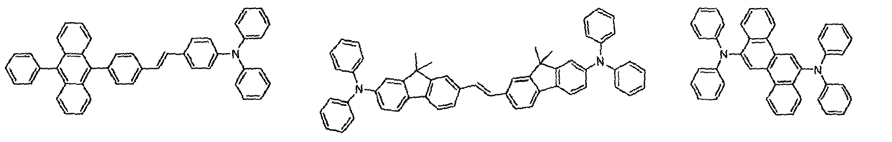

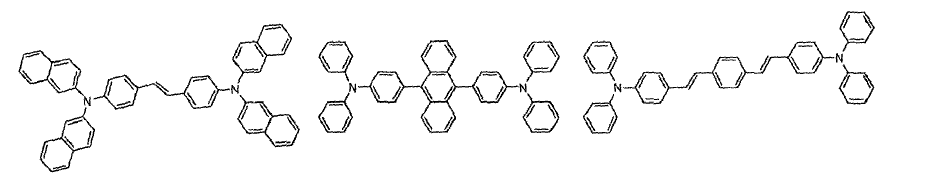

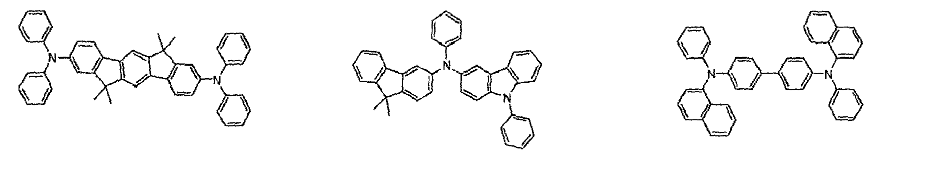

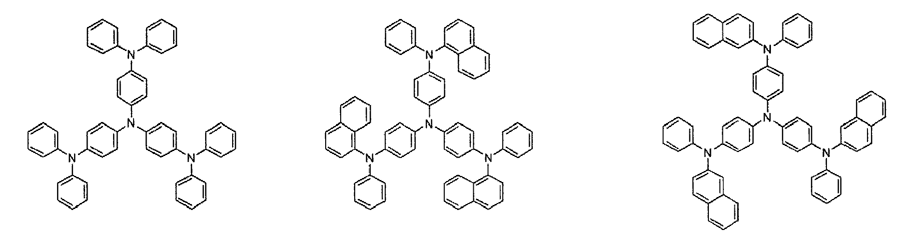

In an organic electroluminescent device according to the present invention, the organic layer may further comprise one or more metal(s) selected from a group consisting of organic metals of Group 1, Group 2, 4^{th} period and 5^{th} period transition metals, lanthanide metals and d-transition elements from the Periodic Table of Elements, as well as the organic electroluminescent compound represented by Chemical Formula (1). The organic layer may comprise a charge generating layer, in addition to an electroluminescent layer.

The present invention can realize an organic electroluminescent device having a pixel structure of independent light-emitting mode, which comprises an organic electroluminescent device containing one of the organic electroluminescent compounds of Chemical Formula (1) as a subpixel, and one or more sub-pixel(s) comprising one or more metallic compound(s) selected from a group consisting of Ir, Pt, Pd, Rh, Re, Os, Tl, Pb, Bi, In, Sn, Sb, Te, Au and Ag, patterned in parallel at the same time.

Further, the organic electroluminescent device is an organic electroluminescent display wherein the organic layer comprises, in addition to the organic electroluminescent compound represented by Chemical Formula (1), one or more compound(s) selected from compounds having the electroluminescent peak of wavelength of not more than 500 nm, or those having the electroluminescent peak of wavelength of not less than 560 nm, at the same time. The compounds having the electroluminescent peak of wavelength of not more than 500 nm, or those having the electroluminescent peak of wavelength of not less than 560 nm may be exemplified by the compounds represented by one of Chemical Formulas (12) to (18), but they are not restricted thereto.

Chemical Formula 12 M¹L³¹L³²L³³

In Chemical Formula (12), M¹ is selected from metals from Group 7, 8, 9, 10, 11, 13, 14, 15 and 16 in the Periodic Table of Elements, and ligands L³¹, L³² and L³³ are independently selected from the following structures: 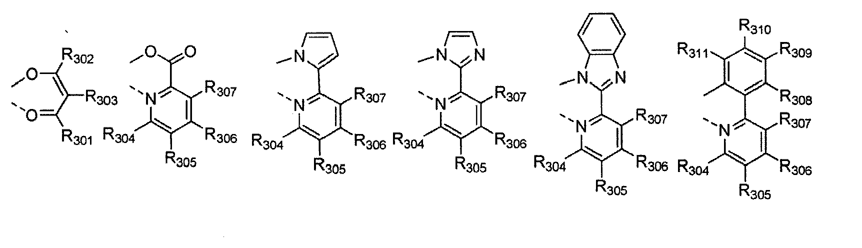 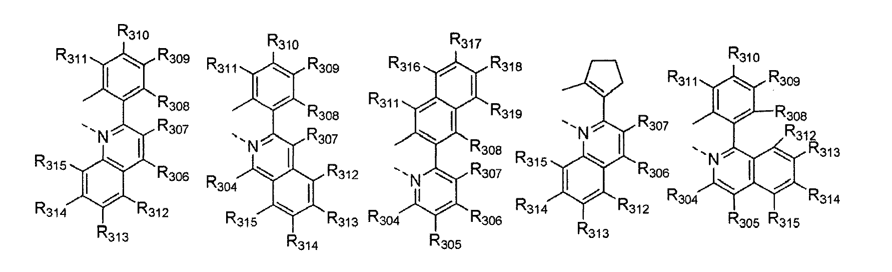 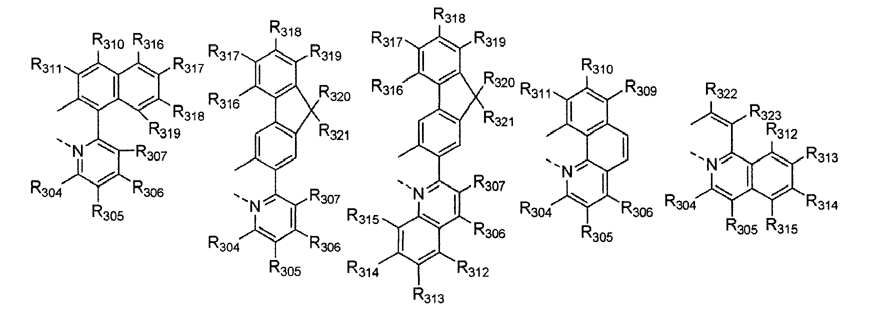 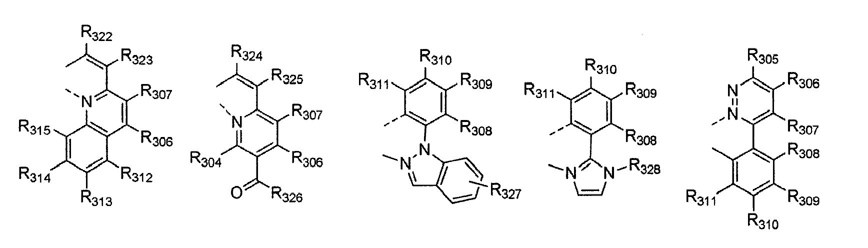 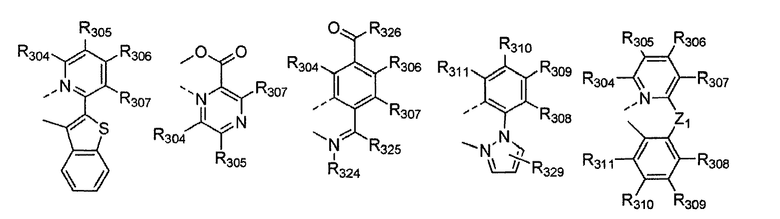 wherein, R₃₀₁ through R₃₀₃ independently represent hydrogen, (C1-C60)alkyl with or without halogen substituent(s), (C6-C60)aryl with or without (C1-C60)alkyl substituent(s), or halogen;
R₃₀₄ through R₃₁₉ independently represent hydrogen, (C1-C60)alkyl, (C1-C30)alkoxy, (C3-C60)cycloalkyl, (C2-C30)alkenyl, (C6-C60)aryl, mono or di(C1-C30)alkylamino, mono or di(C6-30)arylamino, SF₅, tri(C1-C30)alkylsilyl, di(C1-C30)alkyl(C6-C30)arylsilyl, tri(C6-C30)arylsilyl, cyano or halogen; and the alkyl, cycloalkyl, alkenyl or aryl of R₃₀₄ through R₃₁₉ may be further substituted by one or more substituent(s) selected from (C1-C60)alkyl, (C6-C60)aryl and halogen;
R₃₂₀ through R₃₂₃ independently represent hydrogen, (C1-C60)alkyl with or without halogen substituent(s), or (C6-C60)aryl with or without (C1-C60)alkyl substituent(s);
R₃₂₄ and R₃₂₅ independently represent hydrogen, linear or branched (C1-C60)alkyl, (C6-C60)aryl or halogen, or R₃₂₄ and R₃₂₅ may be linked via (C3-C12)alkylene or (C3-C12)alkenylene with or without a fused ring to form an alicyclic ring, or a monocyclic or polycyclic aromatic ring; the alkyl or aryl of R₃₂₄ and R₃₂₅, or the alicyclic ring, or the monocyclic or polycyclic aromatic ring formed therefrom via (C3-C12)alkylene or (C3-C12)alkenylene with or without a fused ring may be further substituted by one or more substituent(s) selected from linear or branched (C1-C60)alkyl with or without halogen substituent(s), (C1-C30)alkoxy, halogen, tri(C1-C30)alkylsilyl, tri(C6-C30)arylsilyl and (C6-C60)aryl;
R₃₂₆ represents (C1-C60)alkyl, (C6-C60)aryl, (C5-C60)heteroaryl or halogen;
R₃₂₇ through R₃₂₉ independently represent hydrogen, (C1-C60)alkyl, (C6-C60)aryl or halogen; the alkyl or aryl of R₃₂₆ through R₃₂₉ may be further substituted by halogen or (C1-C60)alkyl;
Q represents 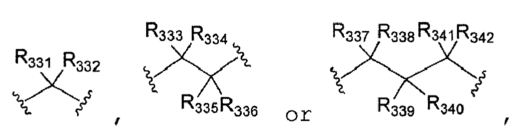 wherein R₃₃₁ through R₃₄₂ independently represent hydrogen, (C1-C60)alkyl with or without halogen substituent(s), (C1-C30)alkoxy, halogen, (C6-C60)aryl, cyano or (C5-C60)cycloalkyl, or each of R₃₃₁ through R₃₄₂ may be linked to an adjacent substituent via alkylene or alkenylene to form a (C5-C7) spiro-ring or a (C5-C9) fused ring, or each of them may be linked to R₃₀₇ or R₃₀₈ via alkylene or alkenylene to form a (C5-C7) fused ring.

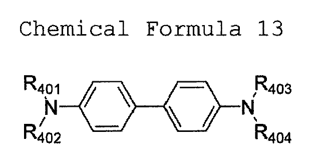

In Chemical Formula (13), R₄₀₁ through R₄₀₄ independently represent (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and the alkyl or aryl of R₄₀₁ through R₄₀₄, or the alicyclic ring, or the monocyclic or polycyclic aromatic ring formed therefrom by linkage via (C3-C60)alkylene or (C3-C60)alkenylene with or without a fused ring may be further substituted by one or more substituent(s) selected from (C1-C60)alkyl with or without halogen substituent(s), (C1-C60)alkoxy, halogen, tri(C1-C60)alkylsilyl, tri(C6-C60)arylsilyl and (C6-C60)aryl.

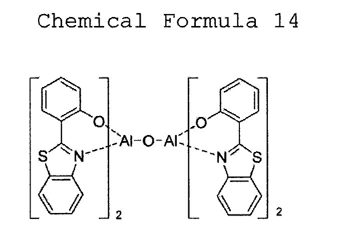

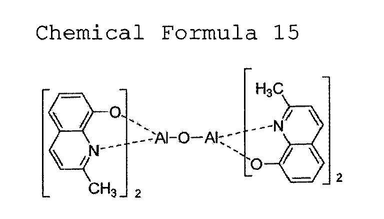

Chemical Formula 16 L³⁴L³⁵M²(T)ₕ

In Chemical Formula (16), the ligands, L³⁴ and L³⁵ are independently selected from the following structures: 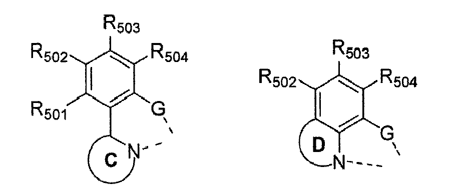 wherein, M² is a bivalent or trivalent metal;
h is 0 when M² is a bivalent metal, while h is 1 when M² is a trivalent metal;
T represents (C6-C60)aryloxy or tri(C6-C60)arylsilyl; the aryloxy and triarylsilyl of T may be further substituted by (C1-C60)alkyl or (C6-C60)aryl;
G represents O, S or Se;
ring C represents oxazole, thiazole, imidazole, oxadiazole, thiadiazole, benzoxazole, benzothiazole, benzimidazole, pyridine or quinoline;
ring D represents pyridine or quinoline, and ring D may be further substituted by (C1-C60)alkyl, or phenyl or naphthyl with or without (C1-C60)alkyl substituent(s);
R₅₀₁ through R₅₀₄ independently represent hydrogen, (C1-C60)alkyl, halogen, tri(C1-C60)alkylsilyl, tri(C6-C60)arylsilyl or (C6-C60)aryl, or each of them may be linked to an adjacent substituent via (C3-C60)alkylene or (C3-C60)alkenylene to form a fused ring, and the pyridine or quinoline may form a chemical bond with R₅₀₁ to form a fused ring;
ring C or the aryl group of R₅₀₁ through R₅₀₄ may be further substitued by (C1-C60)alkyl, halogen, (C1-C60)alkyl with halogen substituent(s), phenyl, naphthyl, tri(C1-C60)alkylsilyl, tri(C6-C60)arylsilyl or amino group.

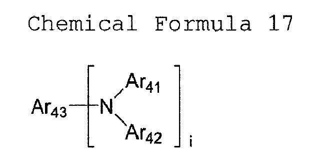

In Chemical Formula (17), Ar₄₁ and Ar₄₂ independently represent (C1-C60)alkyl, (C6-C60)aryl, (C9-C60)heteroaryl, (C6-C60)arylamino, (C1-C60)alkylamino, 5- or 6-membered heterocycloalkyl containing one or more heteroatom(s) selected from N, O and S, or (C3-C60)cycloalkyl, or Ar₄₁ and Ar₄₂ may be linked via (C3-C60)alkylene or (C3-C60)alkenylene with or without a fused ring to form an alicyclic ring, or a monocyclic or polycyclic aromatic ring; the alkyl, aryl, heteroaryl, arylamino, alkylamino, cycloalkyl or heterocycloalkyl of Ar₄₁ and Ar₄₂ may be further substituted by one or more substituent(s) selected from halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; Ar₄₃ represents (C6-C60)arylene, (C4-C60)heteroarylene or arylene represented by one of the following structural formulas: 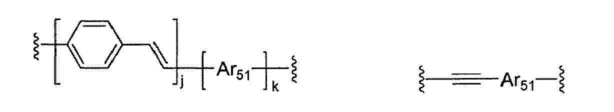 wherein, Ar₅₁ represents (C6-C60)arylene or (C4-C60)heteroarylene,
the arylene or heteroarylene of Ar₄₃ and Ar₅₁ may be further substituted by one or more substituent(s) selected from a group consisting of halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkyloxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
i is an integer from 1 to 4;
j is an integer from 1 to 4; and
k is an integer of 0 or 1.

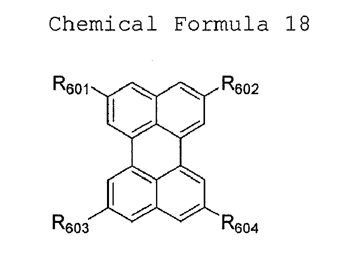

In Chemical Formula (18), R₆₀₁ through R₆₀₄ independently represent hydrogen, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₆₀₁ through R₆₀₄ may be linked to an adjacent substituent via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, arylsilyl, alkylsilyl, alkylamino or arylamino of R₆₀₁ through R₆₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from halogen, (C1-C60)alkyl, (C6-C60)aryl, (C4-C60)heteroaryl, 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

The compounds having electroluminescent peak of wavelength of not more than 500 nm, or those having electroluminescent peak of wavelength of not less than 560 nm, can be exemplified by the following compounds, but they are not restricted thereto.

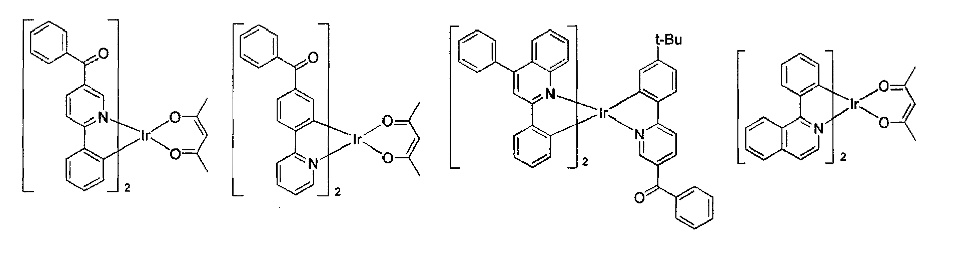

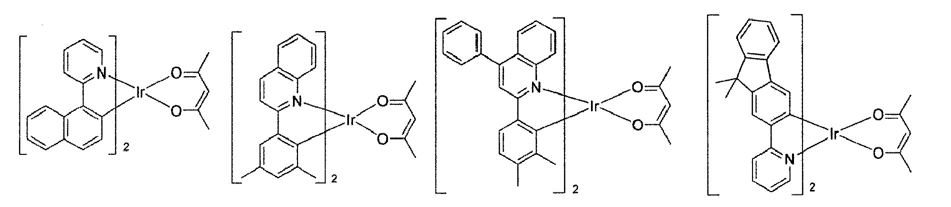

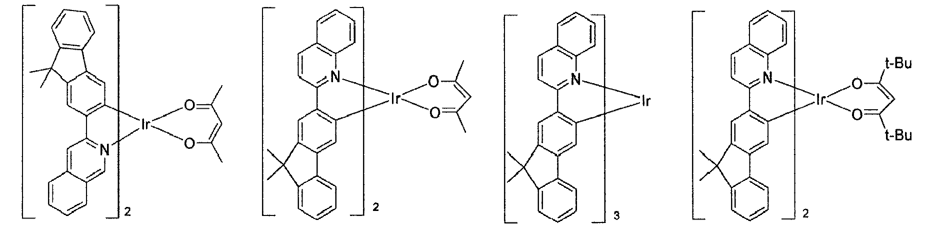

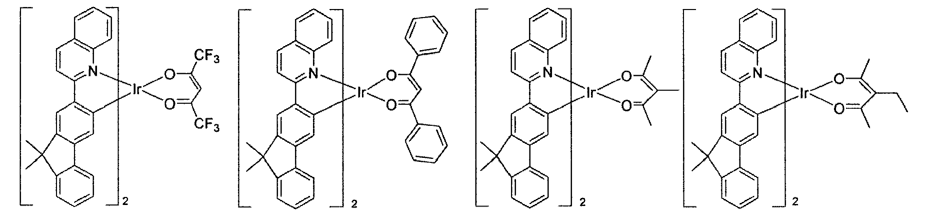

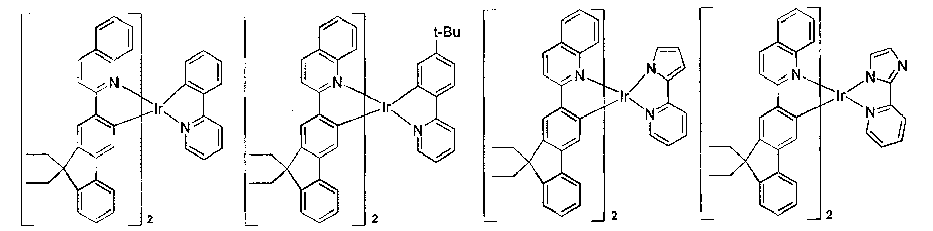

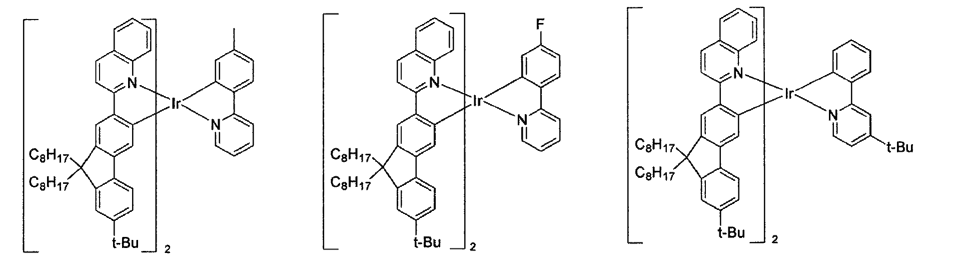

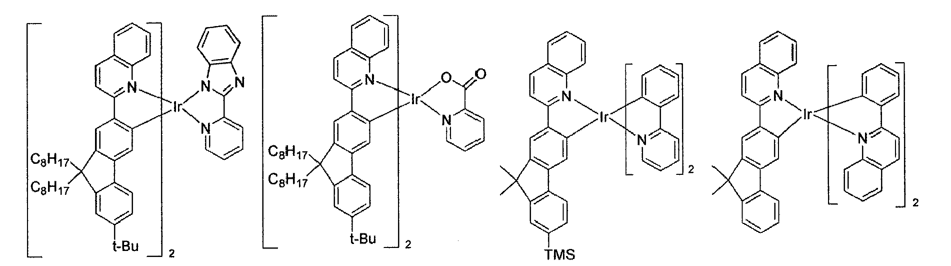

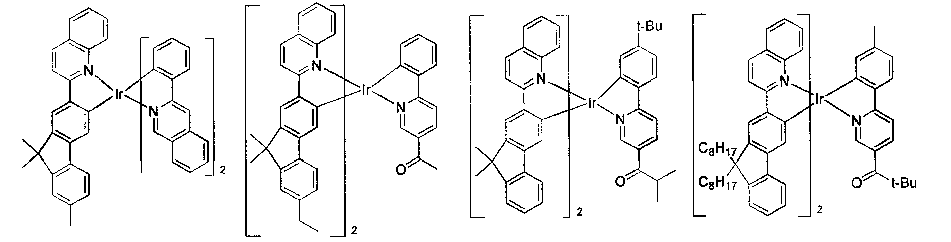

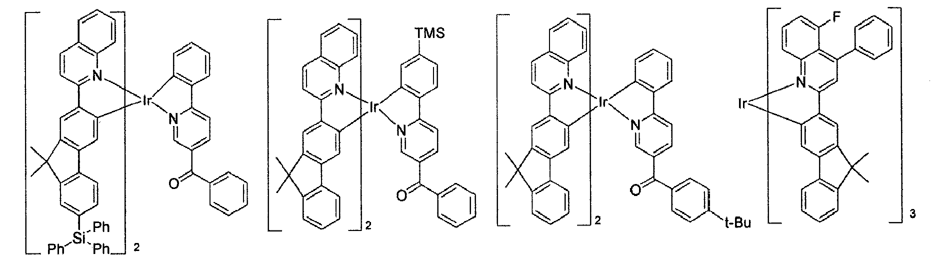

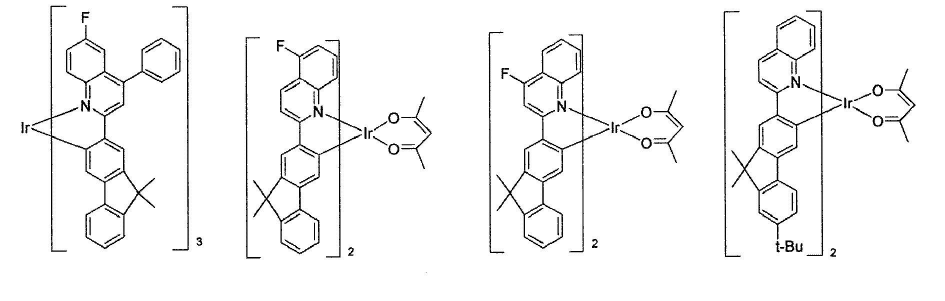

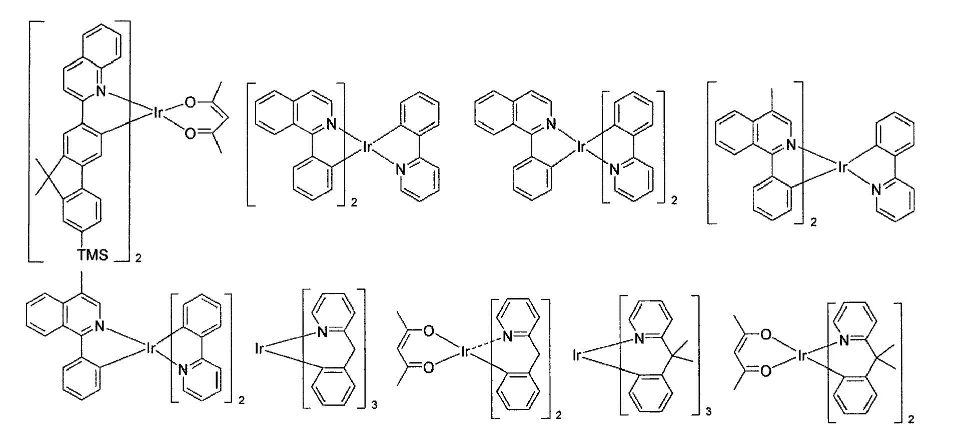

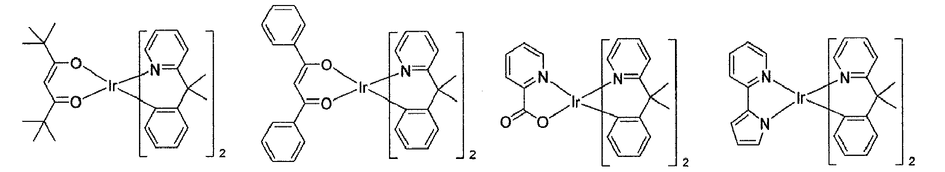

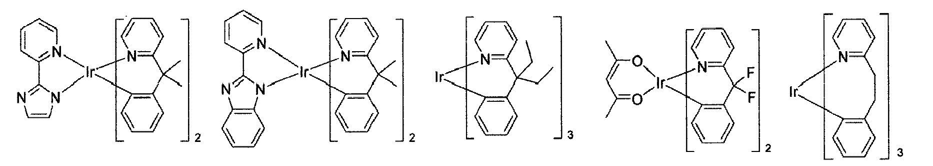

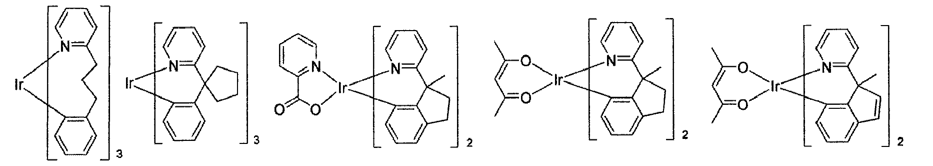

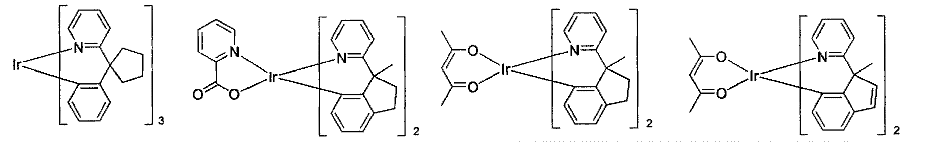

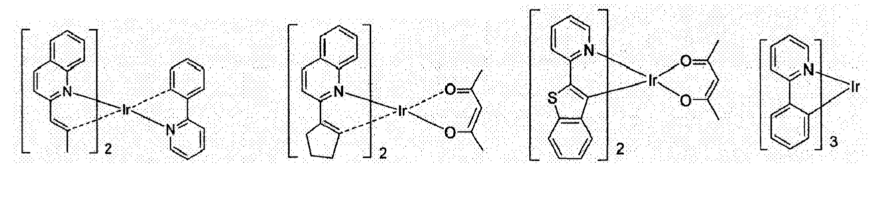

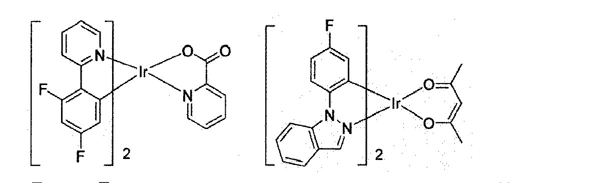

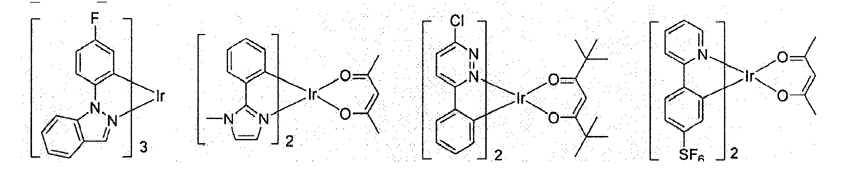

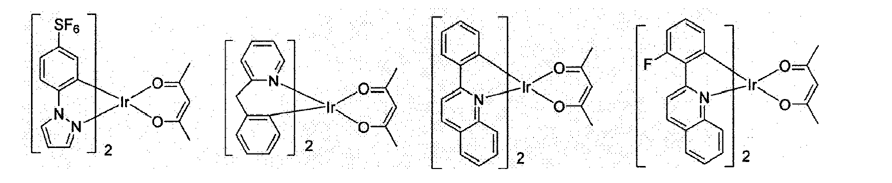

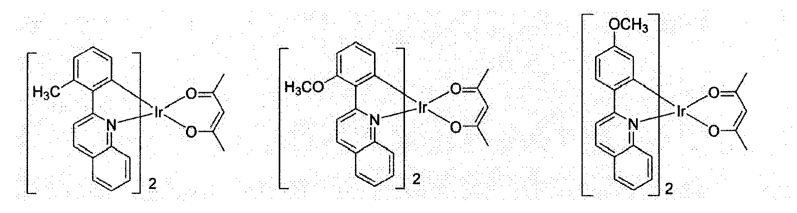

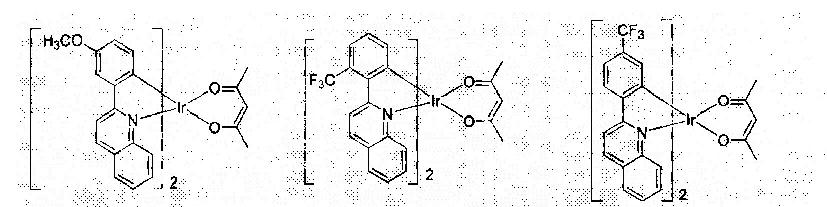

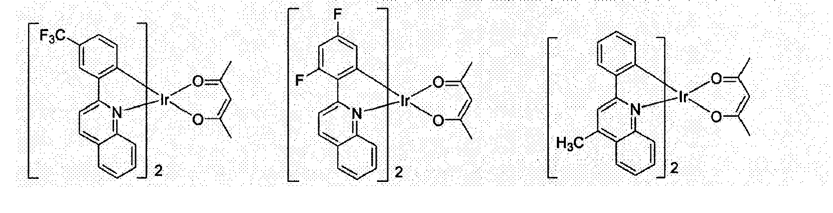

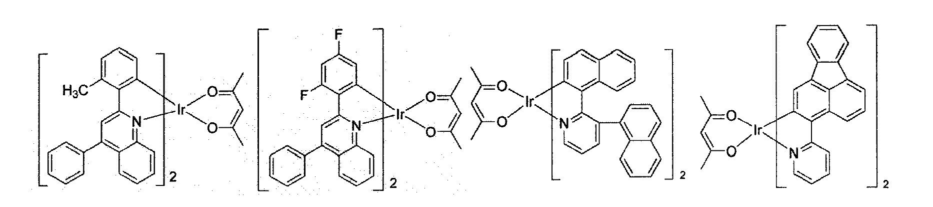

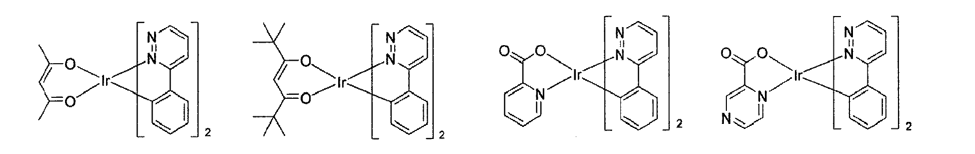

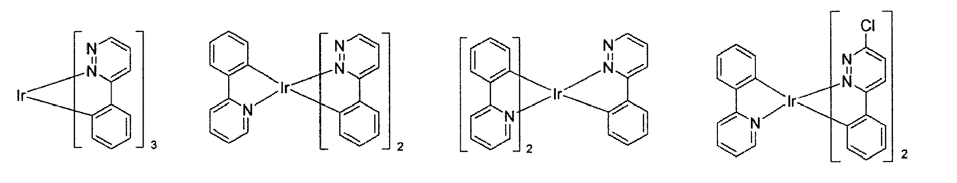

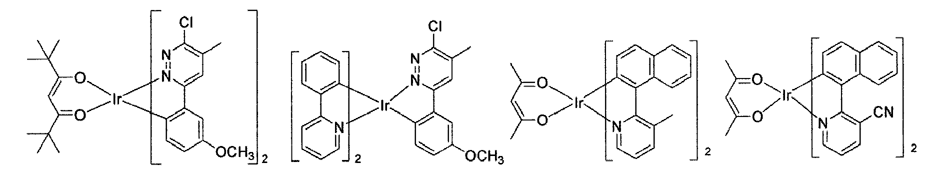

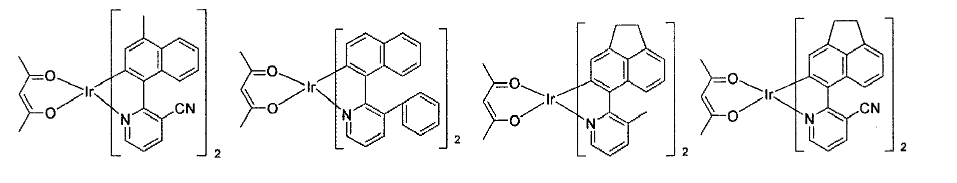

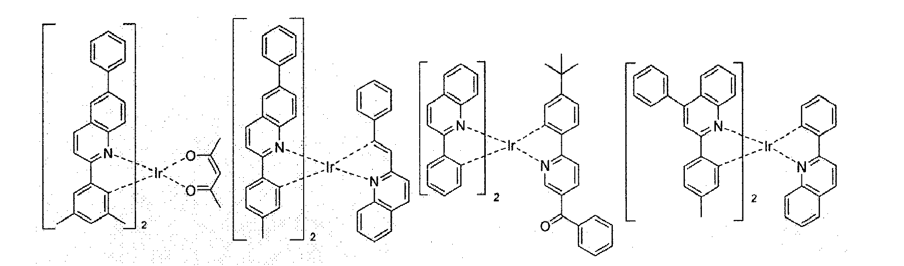

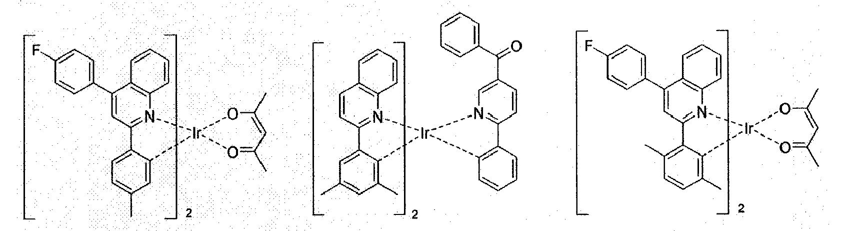

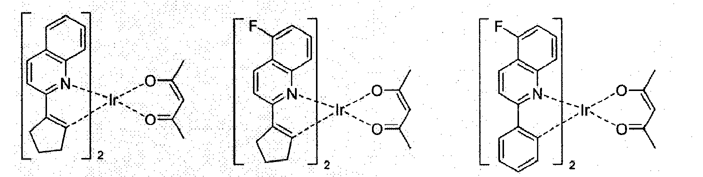

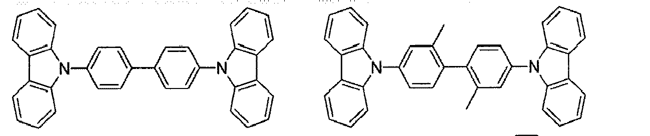

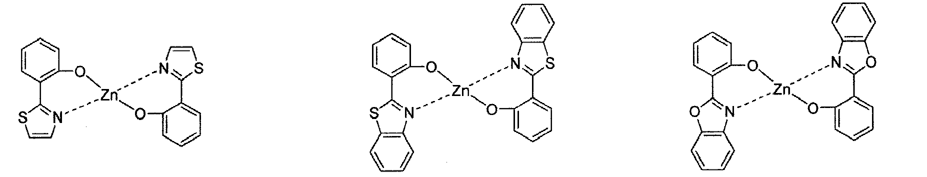

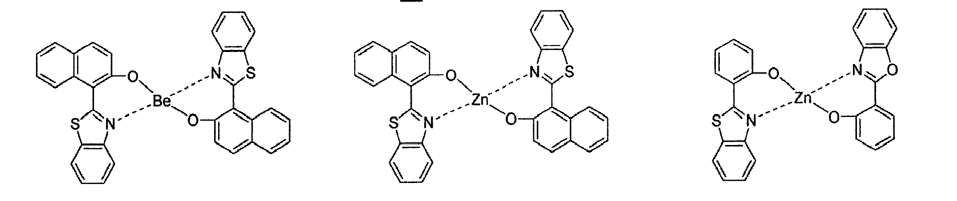

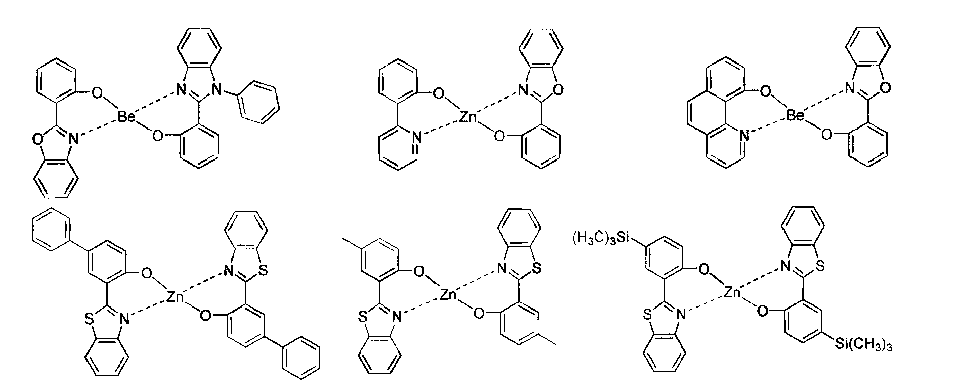

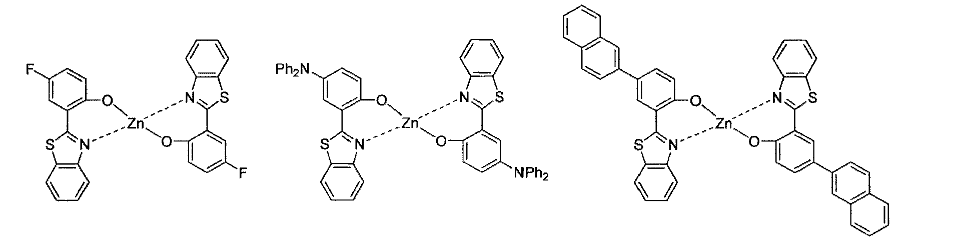

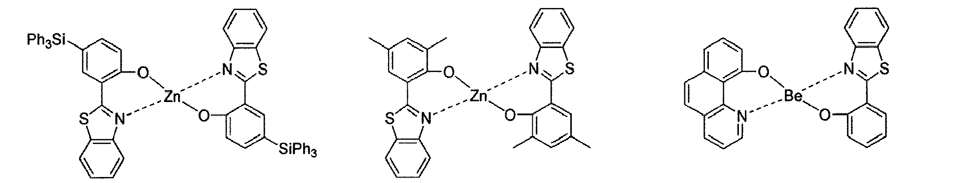

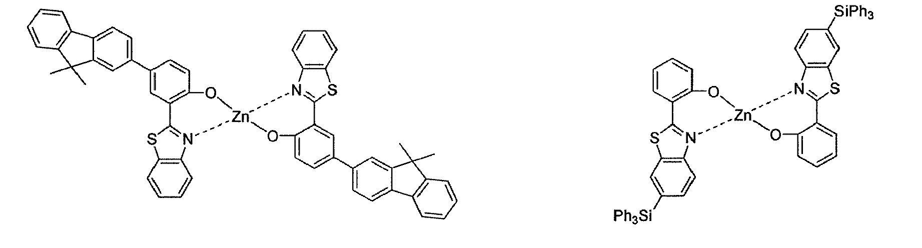

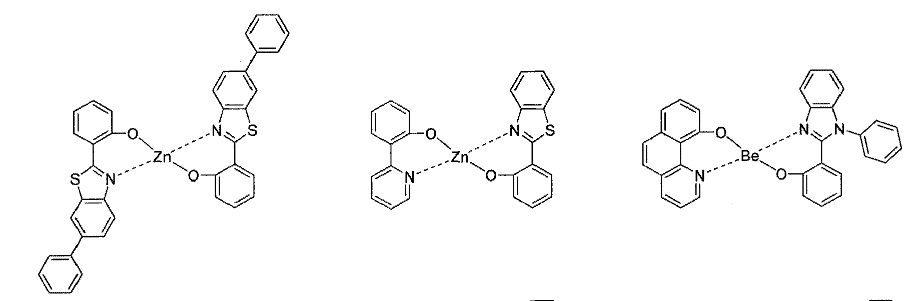

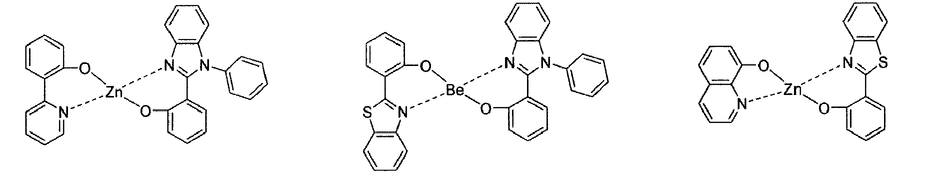

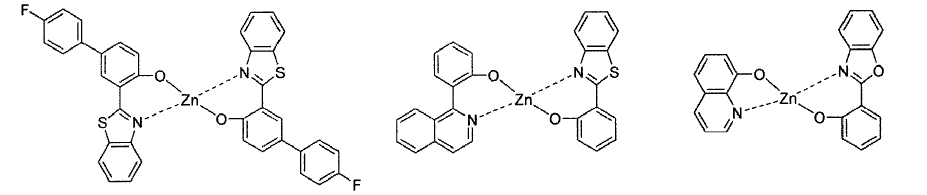

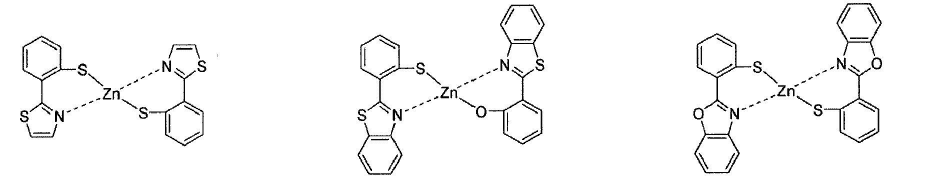

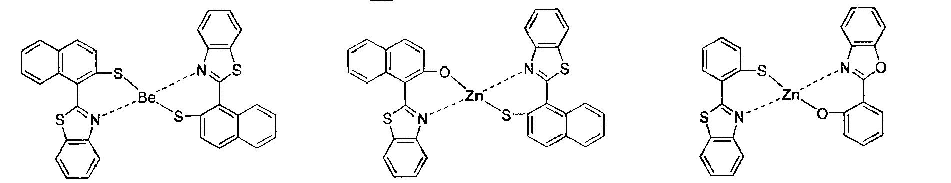

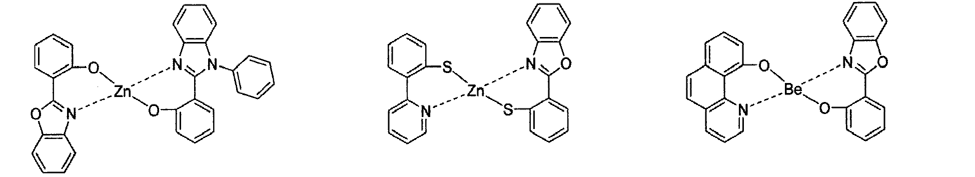

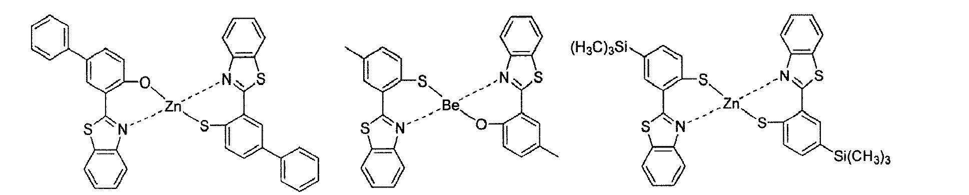

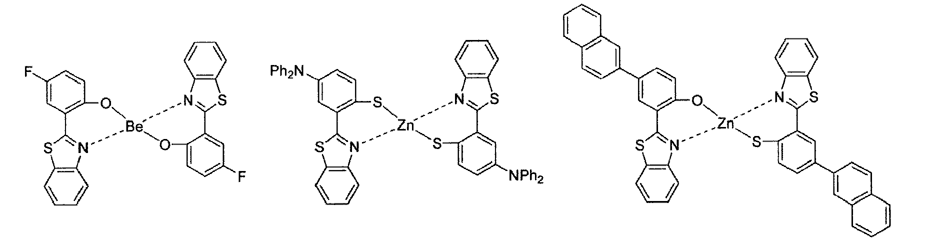

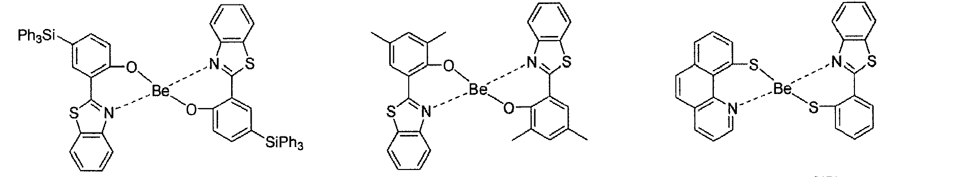

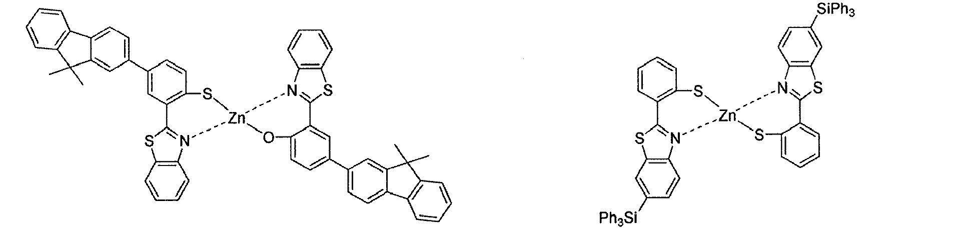

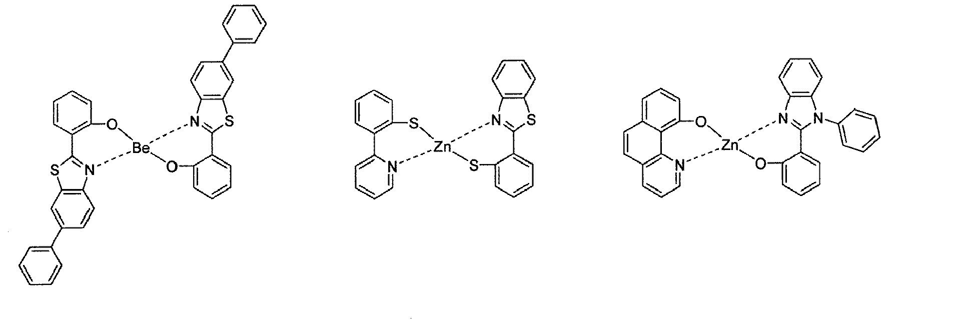

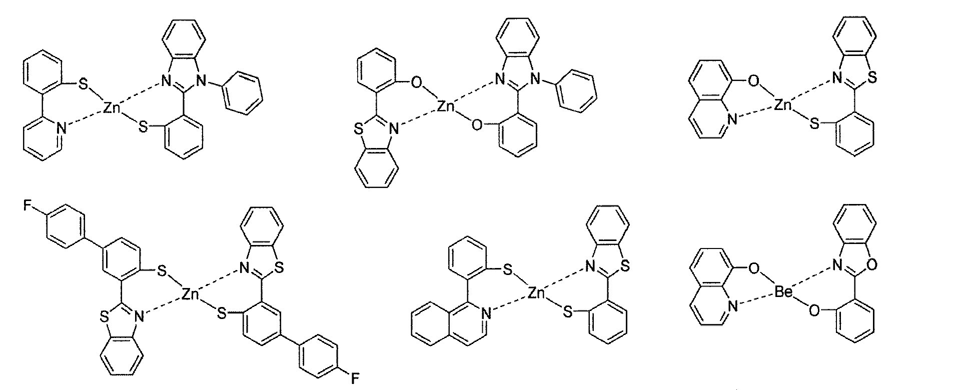

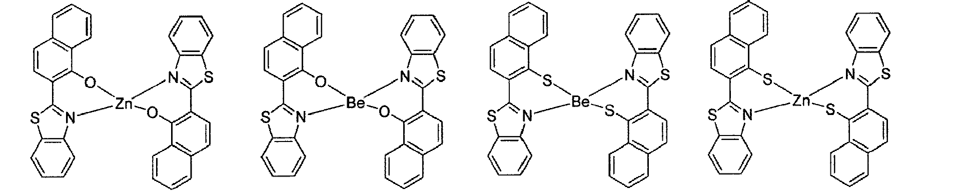

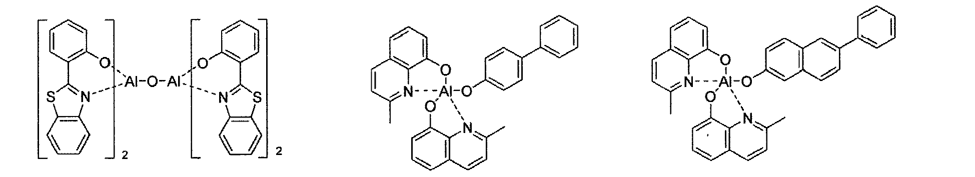

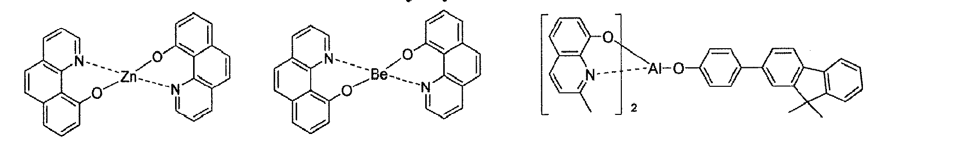

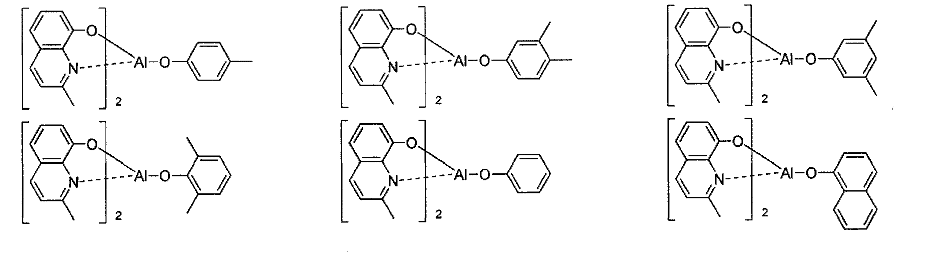

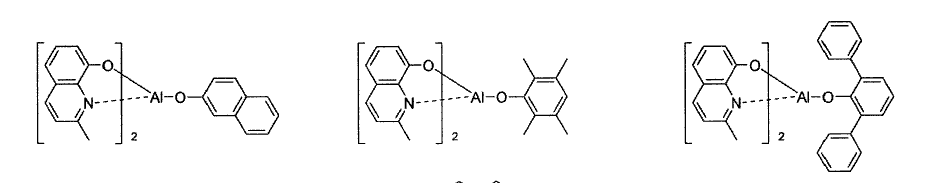

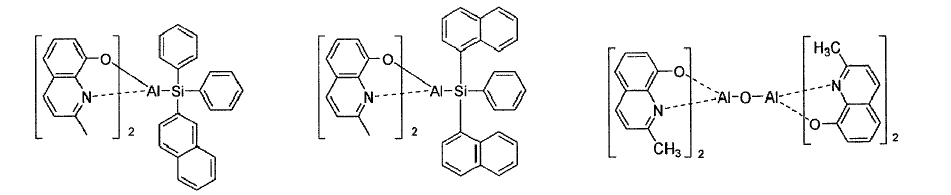

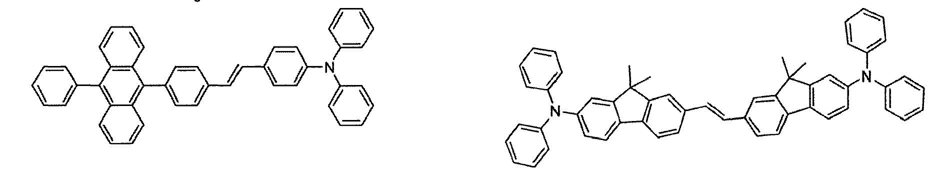

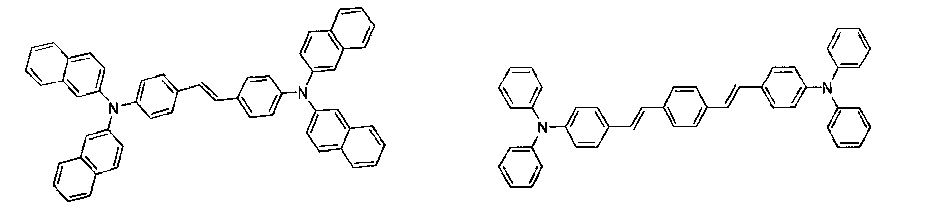

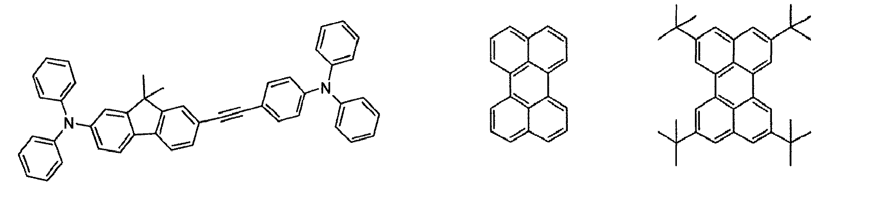

In an organic electroluminescent device according to the present invention, it is preferable to arrange one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer, and a metal halide layer or a metal oxide layer on the cathode surface of the EL medium layer. As the result, stability in operation can be obtained.

Examples of chalcogenides preferably include SiOₓ (1≤X≤2), AlOₓ (1≤X≤1.5), SiON, SiAlON, or the like. Examples of metal halides preferably include LiF, MgF₂, CaF₂, fluorides of rare earth metal or the like. Examples of metal oxides preferably include Cs₂O, Li₂O, MgO, SrO, BaO, CaO, or the like.

In an organic electroluminescent device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

The organic electroluminescent compounds according to the present invention, having high luminous efficiency and excellent life property of material, are advantageous in that they can be employed to manufacture organic light emitting diodes (OLED's) having very good operation life.

### Best Mode

The present invention is further described by referring to representative compounds with regard to the organic electroluminescent compounds according to the invention, preparation thereof and luminescent properties of the devices manufactured therefrom, but those examples are provided for illustration of the embodiments only, not being intended to limit the scope of the invention by any means.

### Preparation Examples

### [Preparation Example 1] Preparation of Compound (1)

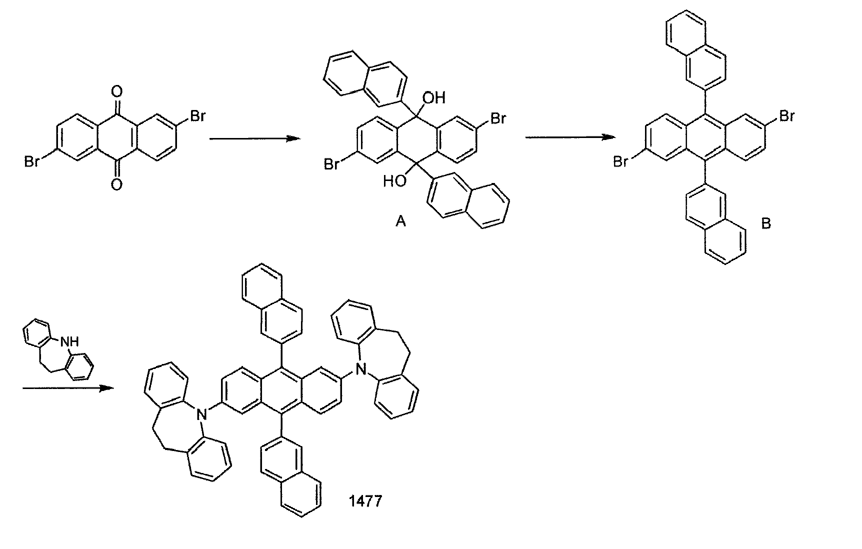

### Preparation of Compound (A)

In dry THF (500 mL), dissolved were 2-bromonaphthalene (44.1 g, 270.7 mmol), and 2.5 M n-butyllithium solution in h-hexane (130.0 mL, 324.9 mmol) was slowly added dropwise thereto at -78C. After stirring for 1 hour, 2,6-dibromoanthracene-9,10-dione (30.0 g, 108.3 mmol) was added thereto, and the resultant mixture was stirred while slowly warming it to room temperature. After 17 hours, water was added, and the mixture was stirred for 30 minutes and then extracted with ethyl acetate (500 mL). The extract was washed with water (500 mL), and dried over magnesium sulfate. Distillation of the organic layer under reduced pressure and drying gave Compound (A) (21.3 g, 47.8 mmol).

### Preparation of Compound (B)

Compound (A) (7.0 g, 14.51 mmol), potassium iodide (KI) (9.64 g, 58.06 mmol) and sodium phosphate monohydrate (NaH₂PO_{2·} H₂O) (9.24 g, 87.12 mmol) were dissolved in acetic acid (150 mL), and the solution was stirred under reflux for 14 hours. Then the reaction mixture was cooled to 25°C, and sodium hydroxide solution (200 mL) was added thereto to make the mixture neutral. The mixture was washed with water (400 mL), and extracted with dichloromethane solvent (300 mL). The extract was dried over magnesium sulfate and filtered through a filter. After removing the solvent under reduced pressure, the obtained residue was purified via methylene chloride/hexane = 1/100 to obtain Compound (B) (4.49 g, 10.0 mmol).

### Preparation of Compound (1477)

In a reaction vessel, Compound (B) (4.0 g, 6.79 mmol), iminodibenzyl (3.3 g, 16.99 mmol), Pd(OAc)₂ (0.07 g, 0.33 mmol), P(t-Bu)₃ (50% in toluene) (0.3 ml, 0.67 mmol), Cs₂CO₃ (6.6 g, 20.38 mmol) and toluene (50 mL) were stirred at 110°C for 5 hours. After adding methanol (50 ml) thereto, the solid produced was filtered under reduced pressure, and washed with distilled water, methanol and hexane. The solid was mixed with ethyl acetate (100 mL), and stirred under reflux for 2 hours. After filtering under reduced pressure, the solid was purified via column chromatography. The solid obtained was dissolved in THF, and the mixture was added to methanol. Filtration under reduced pressure gave Compound (1477) (1.6 g, 1.95 mmol).

The organic electroluminescent compounds (Compounds 1 to 1736) were prepared according to the same procedure as in Preparation Example 1, of which ¹H NMR and MS/FAB data are listed in Table 1.

**Table 1**

| compound | ¹H NMR(CDCl₃, 200 MHz) | MS/FAB | |
|---|---|---|---|
| | | found | calculated |
| 1 | δ = 1.53(8H, m), 1.59(4H, m), 2.64(6H, s), 3.46(8H, m), 7.04(4H, m), 7.81 (2H, m) | 372.55 | 372.26 |
| 2 | δ = 1.48(18H, s), 1.53(8H, m), 1.59(4H, m), 3.46(8H, m), 7.04(4H, m), 7.81(2H, m) 7.04(4H, m), 7.81(2H, m) | 456.71 | 456.35 |
| 5 | δ = 1.53(8H, m), 1.59(4H, m), 3.46(8H, m), 7.01∼7.06(4H, m), 7.58∼7.59(6H, m), 7.73∼7.78(4H. m), 7.92(2H, m), 8(4H, m) | 596.80 | 596.32 |
| 7 | δ = 1.53(8H, m), 1.59(4H, m), 3.46(8H, m), 7.01∼7.06(4H, m), 7.41(2H, m), 7.51∼7.52(8H, m), 7.78(2H, m) | 496.68 | 496.26 |
| 8 | δ = 1.53(8H, m), 1.59(4H, m), 3.46(8H, m), 7.01∼7.06(4H, m), 7.55(4H, m), 7.61(2H, m), 7.78(2H, m), 8.04∼8.08(4H, m), 8.42(2H, m), 8.55(2H, m) | 596.80 | 596.32 |
| 19 | δ = 1.53(8H, m), 1.59(4H, m), 2.34(12H, s), 3.46(8H, m), 7.01∼7.06(4H, m), 7.31(2H, m), 7.6(4H, m), 7.78(2H, m) | 552.79 | 552.35 |
| 26 | δ = 1.53(8H, m), 1.59(4H, m), 3.46(8H, m), 7.01∼7.06(4H, m), 7.41(2H, m), 7.51(4H, m), 7.59(4H, m), 7.78∼7.79(6H, m), 8(4H, m), 8.4(4H, m) | 748.99 | 748.38 |
| 27 | δ = 1.53(8H, m), 1.59(4H, m), 3.46(8H, m). 7.01∼7.06(4H, m), 7.41(2H, m), 7.51∼7.55(8H, m), 7.78∼7.79(6H, m), 8.01(2H, m), 8.08∼8.1 (4H, m), 8.55(2H, m) | 748.99 | 748.38 |
| 28 | δ = 1.53(8H, m), 1.59(4H, m), 3.46(8H, m), 7.01∼7.06(4H, m), 7.5∼7.52(4H, m), 7.78(2H, m), 7.86(2H, m), 7.98∼8(6H, m), 8.45(2H, m) | 708.97 | 708.26 |
| 50 | δ = 1.53(8H, m), 1.59(4H, m), 3.46(8H, m), 7.01∼7.06(4H, m), 7.3(4H, m), 7.39(4H, m), 7.78(2H, m) | 532.67 | 532.27 |
| 92 | δ = 1.53(8H, m), 1.59(4H, m), 3.46(8H, m), 7.01∼7.06(4H, m), 7.36∼7.42(6H, m), 7.48(2H, m), 7.74∼7.84(12H, m), 8.03∼8.12(6H, m) | 861.00 | 860.34 |
| 105 | δ = 1.53(8H, m), 1.59(4H, m), 3.46(8H, m), 7.01∼7.06(4H, m), 7.48(4H, m), 7.55∼7.61(8H, m), 7.7(2H, m), 7.78(2H, m), 8.04∼8.08(4H, m), 8.42(2H, m), 8.55(2H, m) | 748.99 | 748.38 |
| 108 | δ = 1.53(8H, m), 1.59(4H, m), 3.46(8H, m), 7.01∼7.06(4H, m), 7.47(4H, m), 7.58∼7.59(6H. m), 7.73∼7.78(4H, m), 7.85(4H, m), 7.92(2H, m), 8(4H, m) | 748.99 | 748.38 |
| 113 | δ = 1.53(8H, m), 1.59(4H, m), 3.46(8H, m), 7.01∼7.06(4H, m), 7.41(1H, m), 7.51∼7.52(4H, m), 7.58∼7.59(3H, m), 7.73∼7.78(3H, m), 7.92(1H, m), 8(2H, m) | 546.74 | 546.30 |
| 114 | δ = 1.53(8H, m), 1.59(4H, m), 3.46(8H, m), 7.01∼7.06(4H, m), 7.41(1H, m), 7.51∼7.55(6H, m), 7.61(1H, m), 7.78(2H, m), 8.04∼8.08(2H, m), 8.42(1 H, m), 8.55(1 H, m) | 546.74 | 546.30 |
| 115 | δ = 1.53(8H, m), 1.59(4H, m), 1.72(6H, s), 3.46(8H, m), 7.01∼7.06(4H, m), 7.28(1H, m), 7.38∼7.41(2H, m), 7.51∼7.55(5H, m), 7.63(1H, m), 7.77∼7.78(3H, m), 7.87∼7.93(2H, m) | 612.84 | 612.35 |
| 127 | δ = 1.53(8H, m), 1.59(4H, m), 3.46(8H, m), 7.01∼7.06(4H, m), 7.41(2H, m), 7.51∼7.55(8H, m), 7.78∼7.79(4H, m), 8.01(2H, m), 8.55(2H, m) | 622.84 | 622.33 |
| 163 | δ = 1.53(8H, m), 1.59(4H, m), 1.72(6H, s), 3.46(8H, m), 7.01∼7.06(4H, m), 7.41(2H, m), 7.51∼7.52(8H, m), 7.63(2H, m), 7.77∼7.78(4H, m), 7.93(2H, m) | 688.94 | 688.38 |
| 166 | δ = 1.53(8H, m), 1.59(4H, m), 1.72(6H, s), 3.46(8H, m), 7.01∼7.06(4H, m), 7.28(1H, m), 7.38(1H, m), 7.55∼7.63(5H, m), 7.73∼7.78(4H, m), 7.87∼7.93(3H, m), 8(2H, m) | 662.90 | 662.37 |
| 217 | δ = 1.53(8H, m), 1.59(4H, m), 1.72(6H, s), 3.46(8H, m), 7.01∼7.06(4H, m), 7.28(1H, m), 7.38(1H, m), 7.55(3H, m), 7.61∼7.63(2H, m), 7.77∼7.78(3H, m), 7.87∼7.93(2H, m), 8.04∼8.08(2H, m), 8.42(1H, m), 8.55(1H, m) | 662.90 | 662.37 |
| 224 | δ = 1.53(8H, m), 1.59(4H, m), 3.46(8H, m), 7.01∼7.06(4H, m), 7.48(2H, m), 7.55∼7.61 (7H, m), 7.7(1 H, m), 7.78(2H, m), 8.04∼8.08(4H, m), 8.42(2H, m), 8.55(2H, m) | 672.90 | 672.35 |
| 246 | δ = 1.53(8H, m), 1.59(4H, m), 1.72(6H, s), 3.46(8H, m), 7.01∼7.06(4H, m), 7.28(1H, m), 7.38(1H, m), 7.44(1H, m), 7.53∼7.55(4H, m), 7.61(1H, m), 7.78∼7.87(4H, m), 8.04∼8.08(2H, m), 8.42(1H, m), 8.55(1H, m) | 662.90 | 662.37 |
| 267 | δ = 1.53(8H, m), 1.59(4H, m), 1.72(6H, s), 3.46(8H, m), 7.01∼7.06(4H, m), 7.28(1H, m), 7.38(1H, m), 7.55(3H, m), 7.61∼7.63(2H, m), 7.77∼7.78(3H, m), 7.87∼7.93(2H, m), 8.04∼8.08(2H, m), 8.42(1H, m), 8.55(1 H, m) | 662.90 | 662.37 |
| 285 | δ = 1.53(8H, m), 1.59(4H, m), 1.72(6H, s), 2.34(6H, s), 3.46(8H, m), 7.01∼7.06(4H, m), 7.28∼7.31(2H, m), 7.38(1H, 7.55∼7.63(4H, m), 7.77∼7.78(3H, m), 7.87∼7.93(2H, m) | 640.90 | 64.38 |
| 322 | δ = 1.96(4H, m). 2.76(4H, m), 3.06(4H, m), 6.55(2H, m), 6.72(2H, m), 6.83(2H, m), 7.03∼7.07(6H, m), 7.58∼7.59(6H, m), 7.73∼7.75(4H, m), 7.92(2H, m), 8(4H, m) | 692.89 | 692.32 |
| 324 | δ = 1.96(4H, m), 2.76(4H, m), 3.06(4H, m), 6.55(2H, m), 6.72(2H, m), 6.83(2H, m), 7.03∼7.07(6H, m), 7.41(2H, m), 7.51∼7.52(8H, m), 7.75(2H, m) | 592.77 | 592.29 |
| 325 | δ = 1.96(4H, m), 2.76(4H, m), 3.06(4H, m), 6.55(2H, m), 6.72(2H, m), 6.83(2H, m), 7.03∼7.07(6H, m), 7.55(4H, m), 7.61 (2H, m), 7.75(2H, m), 8.04∼8.08(4H, m), 8.42(2H, m), 8.55(2H, m) | 692.89 | 692.32 |
| 327 | δ = 1.96(4H, m), 2.76(4H, m), 3.06(4H, m), 6.55(2H, m), 6.72(2H, m), 6.83(2H, m), 7.03∼7.07(6H, m), 7.35(2H, m), 7.6(2H, m), 7.75∼7.78(4H, m), 7.98(2H, m), 8.06∼8.1(4H, m) | 694.86 | 694.31 |
| 331 | δ = 1.96(4H, m), 2.76(4H, m), 3.06(4H, m), 6.55(2H, m), 6.72(2H, m), 6.83(2H, m), 7.03∼7.07(6H, m), 7.41(4H, m), 7.51∼7.52(16H, m), 7.66(6H, m), 7.75(2H, m) | 897.15 | 896.41 |
| 335 | δ = 1.96(4H, m), 2.34(6H, s), 2.76(4H, m), 3.06(4H, m), 6.55(2H, m), 6.72(2H, m), 6.83(2H, m), 7.03∼7.07(6H, m), 7.29∼7.33(8H, m), 7.75(2H, m) | 620.82 | 620.32 |
| 345 | δ = 1.96(4H, m), 2.76(4H, m), 3.06(4H, m), 6.55(2H, m), 6.72(2H, m), 6.83(2H, m), 7.03∼7.07(6H, m), 7.5∼7.52(4H, m), 7.75(2H, m), 7.86(2H, m), 7.98∼8(6H, m), 8.45(2H, m) | 805.06 | 804.26 |
| 377 | δ = 1.72(6H, s), 1.96(4H, m), 2.76(4H, m), 3.06(4H, m), 6.55(2H, m), 6.72(2H, m), 6.83(2H, m), 7.03∼7.07(6H, m), 7.28(1H, m), 7.38∼7.41(2H, m), 7.51∼7.55(5H, m), 7.63(1H, m), 7.75∼7.77(3H, m), 7.87∼7.93(2H, m) | 708.93 | 708.35 |
| 385 | δ = 1.72(6H, s), 1.96(4H, m), 2.76(4H, m), 3.06(4H, m), 6.55(2H, m), 6.72(2H, m), 6.83(2H, m), 7.03∼7.07(6H, m), 7.28(1H, m), 7.38∼7.41(2H, m), 7.48∼7.63(9H, m), 7.7∼7.77(4H, m), 7.87∼7.93(2H, m) | 785.03 | 784.38 |
| 407 | δ = 1.96(4H, m), 2.76(4H, m), 3.06(4H, m), 6.55(2H, m), 6.72(2H, m), 6.83(2H, m), 7.03∼7.07(6H, m), 7.55∼7.61 (6H, m), 7.73∼7.75(3H, m), 7.92(1H, m), 8∼8.08(4H, m), 8.42(1 H, m), 8.55(1 H, m) | 692.89 | 692.32 |
| 408 | δ = 1.72(6H, s), 1.96(4H, m), 2.76(4H, m), 3.06(4H, m), 6.55(2H, m), 6.72(2H, m), 6.83(2H, m), 7.03∼7.07(6H, m), 7.28(1H, m), 7.38(1H, m), 7.55∼7.63(5H, m), 7.73∼7.77(4H, m), 7.87∼7.93(3H, m), 8(2H, m) | 758.99 | 758.37 |
| 429 | δ = 1.35(9H, s), 1.96(4H, m), 2.76(4H, m), 3.06(4H, m), 6.55(2H, m), 6.72(2H, m), 6.83(2H, m), 7.03∼7.07(6H, m), 7.37∼7.38(4H, m), 7.58∼7.59(3H, m), 7.73∼7.75(3H, m), 7.92(1H, m), 8(2H, m) | 698.94 | 698.37 |
| 448 | δ = 1.96(4H, m), 2.76(4H, m), 3.06(4H, m), 6.55(2H, m), 6.72(2H, m), 6.83(2H, m), 7.03∼7.07(6H, m), 7.55∼7.61(6H, m), 7.73∼7.75(3H, m), 7.92(1H, m), 8∼8.08(4H, m), 8.42(1 H, m), 8.55(1 H, m) | 692.89 | 692.32 |
| 451 | δ = 1.96(4H, m), 2.76(4H, m), 3.06(4H, m), 6.55(2H, m), 6.72(2H, m), 6.83(2H, m), 7.03∼7.07(6H, m). 7.55(2H, m), 7.61(1H, m), 7.75(2H, m), 7.82∼7.93(5H, m), 8.04∼8.12(4H, m), 8.42(1H, m), 8.55(1H, m), 8.93(2H, m) | 742.95 | 742.33 |
| 464 | δ = 1.96(4H, m), 2.76(4H, m), 3.06(4H, m), 6.55(2H, m), 6.72(2H, m), 6.83(2H, m), 7.03∼7.07(6H, m). 7.41(1H, m), 7.51∼7.61(7H, m), 7.75∼7.79(4H, m), 8∼8.08(4H, m), 8.4∼8.42(3H, m), 8.55(1H, m) | 768.98 | 768.35 |
| 479 | δ = 1.72(6H, s), 1.96(4H, m), 2.76(4H, m), 3.06(4H, m), 6.55(2H, m), 6.72(2H, m), 6.83(2H, m), 7.03∼7.07(6H, m), 7.28(1H, m), 7.38(1H, m), 7.55(3H, m), 7.61∼7.63(2H, m), 7.75∼7.77(3H, m), 7.87∼7.93(2H, m), 8.04∼8.08(2H, m), 8.42(1H, m), 8.55(1 H, m) | 758.99 | 758.37 |
| 481 | δ = 1.72(6H, s), 1.96(4H, m), 2.76(4H, m), 3.06(4H, m), 6.55(2H, m), 6.72(2H, m), 6.83(2H, m), 7.03∼7.07(6H, m), 7.25∼7.28(5H, m), 7.38∼7.41(2H, m), 7.51∼7.55(5H, m), 7.63(1 H, m), 7.75∼7.77(3H, m), 7.87∼7.93(2H, m) | 785.03 | 784.38 |
| 501 | δ = 1.35(9H, s), 1.72(6H, s), 1.96(4H, m), 2.76(4H, m), 3.06(4H, m), 6.55(2H, m), 6.72(2H, m), 6.83(2H, m), 7.03∼7.07(6H, m), 7.28(1H, m), 7.37∼7.38(5H, m). 7.55(1H, m), 7.63(1H, m), 7.75∼7.77(3H, m), 7.87∼7.93(2H, m) | 765.04 | 764.41 |
| 511 | δ = 1.48(18H, s), 3,81(4H, s), 6.51(4H, m), 6.69(4H, m), 6.81(2H, m), 6.98∼7.06(10H, m), 7.78(2H, m) | 648.88 | 648.35 |
| 513 | δ = 3.81(4H, s), 6.51(4H, m), 6.69(4H, m), 6.83(2H, m), 6.98∼7.03(10H, m), 7.58∼7.59(6H, m), 7.73∼7.75(4H, m), 7.92(2H, m), 8(4H, m) | 788.97 | 788.32 |
| 519 | δ = 1.35(18H, s), 3.81(4H, s), 6.51(4H, m), 6.69(4H, m), 6.83(2H, m), 6.98∼7.03(10H, m), 7.37∼7.38(8H, m), 7.75(2H, m) | 801.07 | 800.41 |
| 543 | δ = 3.81(4H, s), 6.51(4H, m), 6.69(4H, m), 6.83(2H, m), 6.98∼7.03(10H, m), 7.25(8H, m), 7.58∼7.59(6H, m), 7.73∼7.75(4H, m), 7.92(2H, m), 8(4H, m) | 941.16 | 940.38 |
| 570 | δ = 3.81(4H, s), 6.51(4H, m), 6.69(4H, m), 6.83(2H, m), 6.98∼7.03(10H, m), 7.25(4H, m), 7.41(1H, m), 7.51∼7.52(4H, m), 7.58∼7.59(3H, m), 7.73∼7.75(3H, m), 7.92(1 H, m), 8(2H, m) | 815.01 | 814.33 |
| 584 | δ = 2.34(6H, s), 3.81(4H, s), 6.51(4H, m), 6.69(4H, m), 6.83(2H, m), 6.98∼7.03(10H, m), 7.31(1H, m), 7.58∼7.6(5H, m), 7.73∼7.75(3H, m), 7.92(1 H, m), 8(2H, m) | 766.97 | 766.33 |
| 590 | δ = 3.81(4H, s), 6.51(4H, m), 6.69(4H, m), 6.83(2H, m), 6.98∼7.03(10H, m), 7.1(1H, m), 7.42(1H, m), 7.49(1H, m), 7.58∼7.63(4H, m), 7.73∼7.75(3H, m), 7.92(2H, m), 8(2H, m), 8.42(1H, m) | 789.96 | 789.31 |
| 599 | δ = 1.72(6H, s), 3.81(4H, s), 6.51(4H, m), 6.69(4H, m), 6.83(2H, m), 6.98∼7.03(10H, m), 7.41(1H, m), 7.51∼7.52(4H, m), 7.58∼7.63(5H, m), 7.73∼7.77(5H, m), 7.92∼7.93(3H, m), 8(2H, m) | 931.17 | 930.40 |
| 600 | δ = 3.81 (4H, s), 6.51(4H, m), 6.69(4H, m), 6.83(2H, m), 6.98∼7.03(10H, m), 7.25(4H, m), 7.41(1H, m), 7.51∼7.55(6H, m), 7.61(1H, m), 7.75(2H, m), 8.04∼8.08(2H, m), 8.42(1 H, m), 8.55(1H, m) | 815.01 | 814.33 |
| 602 | δ = 1.72(6H, s), 3.81(4H, s), 6.51(4H, m), 6.69(4H, m), 6.83(2H, m), 6.98∼7.03(10H, m), 7.28(1H, m), 7.38(1H, m), 7.55(3H, m), 7.61∼7.63(2H, m), 7.75∼7.77(3H, m), 7.87∼7.93(2H. m), 8.04∼8.08(2H, m), 8.42(1H, m), 8.55(1H, m) | 855.07 | 854.37 |
| 603 | δ = 3.81(4H, s), 6.51(4H, m), 6.69(4H, m), 6.83(2H, m), 6.98∼7.03(10H, m), 7.55(2H, m), 7.61(1H, m), 7.75(2H, m), 7.82∼7.93(5H, m), 8.04∼8.12(4H, m), 8.42(1H, m), 8.55(1H, m), 8.93(2H, m) | 839.03 | 838.33 |
| 614 | δ = 1.72(6H, s), 3.81(4H, s), 6.51(4H, m), 6.69(4H, m), 6.83(2H, m), 6.98∼7.03(10H, m), 7.25∼7.28(5H, m), 7.38(1H, m), 7.53∼7.55(4H, m), 7.61 (2H, m), 7.75(2H, m), 7.87(1 H, m), 8.04∼8.08(3H, m), 8.42(1H, m), 8.55(1 H, m) | 931.17 | 930.40 |
| 623 | δ = 1.72(6H, s), 3.81 (4H, s), 6.51(4H, m), 6.69(4H, m), 6.83(2H, m), 6.98∼7.03(10H, m), 7.28∼7.38(3H, m), 7.51∼7.55(4H, m), 7.61∼7.63(2H, m), 7.75(2H, m), 7.87(1H, m), 8.04∼8.08(2H, m), 8.42(1H, m), 8.55(1H, m) | 855.07 | 854.37 |
| 624 | δ = 3.81(4H, s), 6.51 (4H, m), 6.69(4H, m), 6.83(2H, m), 6.98∼7.03(10H, m), 7.5∼7.55(4H, m), 7.61(1H, m), 7.75(2H, m), 7.86(1H, m), 7.98∼8.08(5H, m), 8.42∼8.45(2H, m), 8.55(1H, m) | 845.06 | 844.29 |
| 636 | δ = 1.72(6H, s), 3.81(4H, s), 6.51(4H, m), 6.69(4H, m), 6.83(2H, m), 6.98∼7.03(10H, m), 7.25∼7.28(5H, m), 7.38(1H, m), 7.55∼7.63(5H, m), 7.73∼7.77(4H, m), 7.87∼7.93(3H, m), 8(2H, m) | 931.17 | 930.40 |
| 656 | δ = 6.83(2H, m), 6.97(4H, m), 7.03(2H, m), 7.16∼7.21(12H, m), 7.55(4H, m), 7.61(2H, m), 7.75(2H, m), 8.04∼8.08(4H, m), 8.42(2H, m), 8.55(2H, m) | 825.05 | 824.23 |
| 662 | δ = 2.34(12H, s), 6.83(2H, m), 6.97(4H, m), 7.03(2H, m), 7.16∼7.21(12H, m), 7.31(2H, m), 7.6(4H, m), 7.75(2H, m) | 781.04 | 780.26 |
| 679 | δ = 3.49(8H, s), 6.83(2H, m), 6.97(4H, m), 7.03(2H, m), 7.16∼7.21(20H, m), 7.28(2H, m), 7.38(2H, m), 7.55(2H, m), 7.63(2H, m), 7.75∼7.77(4H, m), 7.87∼7.93(4H, m) | 1105.41 | 1104.36 |
| 701 | δ = 6.83(2H, m), 6.97(4H, m), 7.03(2H, m), 7.16∼7.25(16H, m), 7.41(1H, m), 7.51∼7.55(6H, m), 7.61(1H, m), 7.75(2H, m), 8.04∼8.08(2H, m), 8.42(1H, m), 8.55(1H, m) | 851.09 | 850.25 |
| 721 | δ = 6.83(2H, m), 6.97(4H, m), 7.03(2H, m), 7.16∼7.21(12H, m), 7.41(1H, m), 7.51 (2H, m), 7.58∼7.59(5H, m), 7.73∼7.79(5H, m), 7.92(1H, m), 8(4H, m), 8.4(2H, m) | 901.15 | 900.26 |
| 738 | δ = 6.83(2H, m), 6.97(4H, m), 7.03(2H, m), 7.16∼7.25(16H, m), 7.41(1H, m), 7.51∼7.55(6H, m), 7.61(1H, m), 7.75(2H, m), 8.04∼8.08(2H, m), 8.42(1H, m), 8.55(1H, m) | 851.09 | 850.25 |
| 740 | δ = 1.72(6H, s), 6.83(2H, m), 6.97(4H, m), 7.03(2H, m), 7.16∼7.21(12H, m), 7.28(1H, m), 7.38(1H, m), 7.55(3H, m), 7.61∼7.63(2H, m), 7.75∼7.77(3H, m), 7.87∼7.93(2H, m), 8.04∼8.08(2H, m), 8.42(1H, m), 8.55(1H, m) | 891.15 | 890.28 |
| 744 | δ = 1.72(6H, s), 6.83(2H, m), 6.97(4H, m), 7.03(2H, m), 7.16∼7.21(12H, m), 7.28(1H, m), 7.38(1H, m), 7.47(2H, m), 7.55(3H, m), 7.61∼7.63(2H, m), 7.75∼7.77(3H, m), 7.85∼7.93(4H, m), 8.04∼8.08(2H, m), 8.42(1H, m), 8.55(1H, m) | 967.25 | 966.31 |
| 761 | δ = 1.72(6H, s), 6.83(2H, m), 6.97(4H, m), 7.03(2H, m), 7.16∼7.21(12H, m), 7.28∼7.38(3H, m), 7.51∼7.55(4H, m), 7.61∼7.63(2H, 7.75(2H, 7.87(1H, 7.61∼7.63(2H, m), 7.75(2H, m), 7.87(1H, m), 8.04∼8.08(2H, m), 8.42(1 H, m), 8.55(1 H, m) | 891.15 | 890.28 |
| 774 | δ = 1.72(6H, s), 6.83(2H, m), 6.97(4H, m), 7.03(2H, m), 7.16∼7.28(17H, m), 7.38(1H, m), 7.55∼7.63(5H, m), 7.73∼7.77(4H, m), 7.87∼7.93(3H, m), 8(2H, m) | 967.25 | 966.31 |
| 794 | δ = 6.59(4H, m), 6.77(4H, m), 6.83∼6.92(10H, m), 7.03(2H, m), 7.55(4H, m), 7.61(2H, m), 7.75(2H, m), 8.04∼8.08(4H, m), 8.42(2H, m), 8.55(2H, m) | 792.92 | 792.28 |
| 816 | δ = 6.59(4H, m), 6.77(4H, m), 6.83∼6.92(10H, m), 7.03(2H, m), 7.11(8H, m), 7.26∼7.38(16H, m), 7.55(2H, m), 7.63(2H, m), 7.75~7.77(4H, m), 7.87~7.93(4H, m) | 1173.40 | 1172.43 |
| 828 | δ = 1.72(12H, s), 6.59(4H, m), 6.77(4H, m), 6.83∼6.92(10H, m), 7.03(2H, m), 7.28(2H, m), 7.38(2H, m), 7.48(4H, m), 7.55~7.63(6H, m), 7.7~7.77(6H, m), 7.87∼7.93(4H, m) | 1077.31 | 1076.43 |
| 832 | δ = 1.72(6H, s), 6.59(4H, m), 6.77(4H, m), 6.83~6.92(10H, m), 7.03(2H, m), 7.28(1H, m), 7.38∼7.41(2H, m), 7.51∼7.55(5H, m), 7.63(1H, m), 7.75~7.77(3H, m), 7.87~7.93(2H, m) | 808.96 | 808.31 |
| 900 | δ = 6.59(4H, m), 6.77(4H, m), 6.83~6.92(10H, m), 7.03(2H, m), 7.5~7.55(4H, m), 7.61(1H, m), 7.75(2H, m), 7.86(1H, m), 7.98~8.08(5H, m), 8.42~8.45(2H, m), 8.55(1H, m) | 849.01 | 848.25 |
| 938 | δ = 1.72(12H, s), 2.34(12H, s), 6.55(4H, m), 6.73(4H, m), 6.83(2H, m), 7.02~7.05(10H, m), 7.31(2H, m), 7.6(4H, m), 7.75(2H, m) | 801.17 | 800.41 |
| 951 | δ = 1.72(24H, s), 6.55(4H, m), 6.73(4H, m), 6.83(2H, m), 7.02~7.05(10H, m), 7.28(2H, m), 7.38(2H, m), 7.55(2H, m), 7.63(2H, m), 7.75~7.77(4H, m), 7.87∼7.93(4H, m) | 977.28 | 976.48 |
| 986 | δ = 1.72(12H, s), 6.55(4H, m), 6.73(4H, m), 6.83(2H, m), 7.02~7.05(10H, m), 7.48(2H, m), 7.57~7.59(7H, m), 7.7~7.75(5H, m), 7.92(2H, m), 8(4H, m) | 921.18 | 920.41 |
| 998 | δ = 1.72(12H, s), 2.34(6H, s), 6.55(4H, m), 6.73(4H, m), 6.83(2H, m), 7.02~7.05(10H, m), 7.31(1H, m), 7.58~7.6(5H, m), 7.73~7.75(3H, m), 7.92(1 H, m), 8(2H, m) | 823.07 | 822.40 |
| 1007 | δ = 1.72(12H, s), 6.55(4H, m), 6.73(4H, m), 6.83(2H, m), 7.02~7.05(10H, m), 7.5~7.52(2H, m), 7.58~7.59(3H, m), 7.73∼7.75(3H, m), 7.86~7.92(2H, m), 7.98~8(5H, m), 8.45(1H, m) | 901.17 | 900.35 |
| 1016 | δ = 1.72(18H, s), 6.55(4H, m), 6.73(4H, m), 6.83(2H, m), 7.02∼7.05(10H, m), 7.28(1H, m), 7.38(1H, m), 7.55(3H, m), 7.61∼7.63(2H, m), 7.75∼7.77(3H, m), 7.87∼7.93(2H, m), 8.04∼8.08(2H, m), 8.42(1H, m), 8.55(1H, m) | 911.18 | 910.43 |
| 1017 | δ = 1.72(12H, s), 6.55(4H, m), 6.73(4H, m), 6.83(2H, m), 7.02∼7.05(10H, m), 7.55(2H, m), 7.61(1H, m), 7.75(2H, m), 7.82∼7.93(5H, m), 8.04∼8.12(4H, m), 8.42(1H, m), 8.55(1H, m), 8.93(2H, m) | 895.14 | 894.40 |
| 1031 | δ = 1.72(12H, s), 2.34(3H, s), 6.55(4H, m), 6.73(4H, m), 6.83(2H, m), 7.02∼7.05(10H, m), 7.29∼7.33(4H, m), 7.55(2H, m), 7.61(1H, m), 7.75(2H, m), 8.04∼8.08(2H, m), 8.42(1H, m), 8.55(1 H, m) | 809.05 | 808.38 |
| 1038 | δ = 1.72(12H, s), 6.55(4H, m), 6.73(4H, m), 6.83(2H, m), 7.02∼7.05(10H, m), 7.5∼7.55(4H, m), 7.61(1H, m), 7.75(2H, m), 7.86(1H, m), 7.98∼8.08(5H, m), 8.42∼8.45(2H, m), 8.55(1H, m) | 901.17 | 900.35 |
| 1049 | δ = 1.72(18H, s), 6.55(4H, m), 6.73(4H, m), 6.83(2H, m), 7.02∼7.05(10H, m), 7.25∼7.28(5H, m), 7.38(1H, m), 7.55(3H, m), 7.61∼7.63(2H, m), 7.75∼7.77(3H, m), 7.87∼7.93(2H, m), 8.04∼8.08(2H, m), 8.42(1H, m), 8.55(1H, m) | 987.28 | 986.46 |
| 1056 | δ = 1.72(18H, s), 2.34(3H, s), 6.55(4H, m), 6.73(4H, m), 6.83(2H, m), 7.02∼7.05(10H, m), 7.28∼7.38(6H, m), 7.55(1H, m), 7.63(1H, m), 7.75∼7.77(3H, m), 7.87∼7.93(2H, m) | 875.15 | 874.43 |
| 1069 | δ = 3.2(6H, s), 6.35(4H, m), 6.41 (4H, m), 6.52(4H, m), 6.59(4H, m), 6.83(2H, m), 7.03(2H, m), 7.35(2H, m), 7.6(2H, m), 7.75∼7.78(4H, m), 7.98(2H, m), 8.06∼8.1(4H, m) | 820.98 | 820.33 |
| 1071 | δ = 1.35(18H, s), 3.2(6H, s), 6.35(4H, m), 6.41(4H, m), 6.52(4H, m), 6.59(4H, m), 6.83(2H, m), 7.03(2H, m), 7.37∼7.38(8H, m), 7.75(2H, m) | 831.10 | 830.43 |
| 1072 | δ = 3.2(6H, s), 6.35(4H, m), 6.41(4H, m), 6.52(4H, m), 6.59(4H, m), 6.83(2H, m), 7.03(2H, m), 7.25(8H, m), 7.41 (2H, m), 7.51∼7.52(8H, m), 7.75(2H, m) | 871.08 | 870.37 |
| 1105 | δ = 3.2(6H, s), 6.35(4H, m), 6.41 (4H, m), 6.52(4H, m), 6.59(4H, m), 6.83(2H, m), 7.03(2H, m), 7.25(8H, m), 7.41(2H, m), 7.48∼7.57(14H, m), 7.7∼7.75(4H, m) | 1023.27 | 1022.43 |
| 1106 | δ = 3.2(6H, s), 6.35(4H, m), 6.41(4H, m), 6.52(4H, m), 6.59(4H, m), 6.83(2H, m), 7.03(2H, m), 7.41(1H, m), 7.51∼7.52(4H, m), 7.58∼7.59(3H, m), 7.73∼7.75(3H, m), 7.92(1H, m), 8(2H, m) | 768.94 | 768.33 |
| 1123 | δ = 3.2(6H, s), 6.35(4H, m), 6.41(4H, m), 6.52(4H, m), 6.59(4H, m), 6.83(2H, m), 7.03(2H, m), 7.58∼7.59(3H, m), 7.73∼7.75(3H, m), 7.82∼7.93(6H, m), 8(2H, m), 8.12(2H, m), 8.93(2H, m) | 869.06 | 868.36 |
| 1130 | δ = 3.2(6H, s), 6.35(4H, m), 6.41 (4H, m), 6.52(4H, m), 6.59(4H, m), 6.83(2H, m), 7.03(2H, m), 7.25(4H, m), 7.55∼7.61(6H, m), 7.73∼7.75(3H, m), 7.92(1H, m), 8∼8.08(4H, m), 8.42(1H, m), 8.55(1H, m) | 895.10 | 894.37 |
| 1141 | δ = 3.2(6H, s), 6.35(4H, m), 6.41(4H, m), 6.52(4H, m), 6,59(4H, m), 6.83(2H, m), 7∼7.03(3H, m), 7.26(1H, m), 7.51(1H, m), 7.58∼7.59(3H, m), 7.73∼7.75(3H, m), 7.92(1H, m), 8(2H, m), 8.5(1H, m) | 769.93 | 769.32 |
| 1142 | δ = 3.2(6H, s), 6.35(4H, m), 6.41(4H, m), 6.52(4H, m), 6.59(4H, m), 6.83(2H, m), 7.03(2H, m), 7.1(1H, m), 7.42(1H, m), 7.49(1H, m), 7.58~7.63(4H, m), 7.73~7.75(3H, m), 7.92(2H, m), 8(2H, m), 8.42(1H, m) | 819.99 | 819.34 |
| 1148 | δ = 3.2(6H, s), 6.35(4H, m), 6.41(4H, m), 6.52(4H, m), 6.59(4H, m), 6.83(2H, m), 7.03(2H, m), 7.35(1H, m), 7.58~7.59(4H, m), 7.73~7.81(4H, m), 7.92(1H, m), 8(2H, m), 8.06~8.1(2H, m), 8.38(1H, m), 8.83(1H, m) | 871.04 | 870.35 |
| 1155 | δ = 3.2(6H, s), 6.35(4H, m), 6.41(4H, m), 6.52(4H, m), 6.59(4H, m), 6.83(2H, m), 7.03(2H, m), 7.55(2H, m), 7.61(1H, m), 7.75(2H, m), 7.82~7.93(5H, m), 8.04~8.12(4H, m), 8.42(1 H, m), 8.55(1H, m), 8.93(2H, m) | 869.06 | 868.36 |
| 1163 | δ = 3.2(6H, s), 6.35(4H, m), 6.41(4H, m), 6.52(4H, m), 6.59(4H, m), 6.83(2H, m), 7.03(2H, m), 7.25(4H, m), 7.55~7.61(6H, m), 7.73~7.75(3H, m), 7.92(1H, m), 8~8.08(4H, m), 8.42(1H, m), 8.55(1H, m) | 895.10 | 894.37 |
| 1175 | δ = 1.72(6H, s), 3.2(6H, s), 6.35(4H, m), 6.41(4H, m), 6.52(4H, m), 6.59(4H, m), 6.83(2H, m), 7.03(2H, m), 7.28~7.38(3H, m), 7.51~7.55(4H, m), 7.61∼7.63(2H, m), 7.75(2H, m), 7.87(1H, m), 8.04~8.08(2H, m), 8.42(1H, m), 8.55(1H, m) | 885.10 | 884.39 |
| 1183 | δ = 1.72(12H, s), 3.2(6H, s), 6.35(4H, m), 6.41(4H, m), 6.52(4H, m), 6.59(4H, m), 6.83(2H, m), 7.03(2H, m), 7.28(2H, m), 7.38(2H, m), 7.47(2H, m), 7.55(2H, m), 7.63(2H, m), 7.75~7.77(4H, m), 7.85~7.93(6H, m) | 1027.30 | 1026.47 |
| 1191 | δ = 1.72(12H, s), 3.2(6H, s), 6.35(4H, m), 6.41(4H, m), 6.52(4H, m), 6.59(4H, m), 6.83(2H, m), 7.03(2H, m), 7.25~7.28(6H, m), 7.38(2H, m), 7.53~7.55(3H, m), 7.61~7.63(2H, m), 7.75~7.77(3H, m), 7.87~7.93(3H, m), 8.06(1H, m) | 1027.30 | 1026.47 |
| 1203 | δ = 0.66(12H, s), 6.73(8H, m), 6.83(2H, m), 7.03(2H, m), 7.21(4H, m), 7.3(4H, m), 7.58∼7.59(6H, m), 7.73∼7.75(4H, m), 7.92(2H, m), 8(4H, m) | 877.23 | 876.34 |
| 1214 | δ = 0.66(12H, s), 2.34(12H, s), 6.73(8H, m), 6.83(2H, m), 7.03(2H, m), 7.21(4H, m), 7.3∼7.31(6H, m), 7.6(4H, m), 7.75(2H, m) | 833.22 | 832.37 |
| 1233 | δ = 0.66(12H, s), 6.73(8H, m), 6.83(2H, m), 7.03(2H, m), 7.21∼7.3(16H, m), 7.58∼7.59(6H, m), 7.73∼7.75(4H, m), 7.92(2H, m), 8(4H, m) | 1029.42 | 1028.40 |
| 1252 | δ = 0.66(12H, s), 1.72(6H, s), 6.73(8H, m), 6.83(2H, m), 7.03(2H, m), 7.21(4H, m), 7.28∼7.3(5H, m), 7.38∼7.41(2H, m), 7.48∼7.63(9H, m), 7.7∼7.77(4H, m), 7.87∼7.93(2H, m) | 969.37 | 968.40 |
| 1258 | δ = 0.66(12H, s), 6.73(8H, m), 6.83(2H, m), 7.03(2H, m), 7.21 (4H, m), 7.3(4H, m), 7.55∼7.61(6H, m), 7.73∼7.75(3H, m), 7.92(1 H, m), 8∼8.08(4H, m), 8.42(1 H, m), 8.55(1 H, m) | 877.23 | 876.34 |
| 1293 | δ = 0.66(12H, s), 6.73(8H, m), 6.83(2H, m), 7.03(2H, m), 7.21 (4H, m), 7.3(4H, m), 7.55(2H, m), 7.61(1H, m), 7.75(2H, m), 7.82∼7.93(5H, m), 8.04∼8.12(4H, m), 8.42(1H, m), 8.55(1H, m), 8.93(2H, m) | 927.29 | 926.35 |
| 1310 | δ = 0.66(12H, s), 1.35(9H, s), 6.73(8H, m), 6.83(2H, m), 7.03(2H, m), 7.21(4H, m), 7.3(4H, m), 7.37∼7.38(4H, m), 7.55(2H, m), 7.61(1H, m), 7.75(2H, m), 8.04∼8.08(2H, m), 8.42(1H, m), 8.55(1H, m) | 883.28 | 882.38 |
| 1325 | δ = 0.66(12H, s), 1.72(6H, s), 6.73(8H, m), 6.83(2H, m), 7.03(2H, m), 7.21∼7.3(13H, m), 7.38(1H, m), 7.55(3H, m), 7.61∼7.63(2H, m), 7.75∼7.77(3H, m), 7.87∼7.93(2H, m), 8.04∼8.08(2H, m), 8.42(1H, m), 8.55(1H, m) m), 8.55(2H, m) | 1019.43 | 1018.41 |
| 1342 | δ = 7.25∼7.33(6H, m), 7.4(2H, m), 7.5(2H, m), 7.63(2H, m), 7.82∼7.94(16H, m), 8.12(6H, m), 8.55(2H, m), 8.93(4H, m) | 861.04 | 860.32 |
| 1346 | δ = 7.25∼7.33(6H, m), 7.4(2H, m), 7.5∼7.55(6H, m), 7.61∼7.63(4H, m), 7.9∼7.94(6H, m), 8.04∼8.12(6H, m), 8.42(2H, m), 8.55(4H, m) | 760.92 | 760.29 |
| 1393 | δ = 7.25∼7.33(6H, m), 7.4∼7.41(4H, m), 7.5∼7.55(10H, m), 7.63(2H, m), 7.79(2H, m), 7.9∼7.94(6H, m), 8.01(2H, m), 8.12(2H, m), 8.55(4H, m) | 786.96 | 786.30 |
| 1421 | δ = 7.25∼7.33(6H, m), 7.4(2H, m), 7.5∼7.52(4H, m), 7.58∼7.63(5H, m), 7.73(1H, m), 7.86∼8(13H, m), 8.12(2H, m), 8.45(1H, m), 8.55(2H, m) | 817.01 | 816.26 |
| 1448 | δ = 1.35(9H, s), 7.25∼7.4(12H, m), 7.5∼7.55(4H, m), 7.61∼7.63(3H, m), 7.9∼7.94(6H, m), 8.04∼8.12(4H, m), 8.42(1H, m), 8.55(3H, m) | 766.97 | 766.33 |
| 1467 | δ = 1.72(12H, s), 7.25∼7.4(16H, m), 7.5∼7.55(5H, m), 7.61∼7.63(4H, m), 7.77(1H, m), 7.87∼7.94(9H, m), 8.06∼8.12(3H, m), 8.55(2H, m) | 969.22 | 968.41 |
| 1472 | δ = 1.72(6H, s), 2.34(6H, s), 7.14∼7.17(2H, m), 7.25∼7.4(10H, m), 7.5∼7.55(3H, m), 7.63∼7.67(4H, m), 7.77(1H, m), 7.87∼7.94(8H, m), 8.12(2H, m), 8.55(2H, m) | 805.02 | 804.35 |
| 1477 | δ = 1.48(18H, s), 2.88(8H, m), 6.58(4H, m), 6.76∼6.81(6H, m), 7.02∼7.06(10H, m), 7.78(2H, m) | 676.93 | 676.38 |
| 1481 | δ = 2.88(8H, m), 6.58(4H, m), 6.76(4H, m), 6.83(2H, m), 7.02∼7.04(10H, m), 7.41(2H, m), 7.51∼7.52(8H. m), 7.75(2H, m) | 716.91 | 716.32 |
| 1486 | δ = 2.88(8H, m), 6.58(4H, m), 6.76(4H, m), 6.83(2H, m), 7.02∼7.04(10H, m), 7.25(8H, m), 7.41(2H, m), 7.51∼7.52(8H, m), 7.75(2H, m) | 869.10 | 868.38 |
| 1521 | δ = 2.88(8H, m), 6.58(4H, m), 6.76(4H, m), 6.83(2H, m), 7.02∼7.04(10H, m), 7.41(1H, m), 7.51∼7.55(6H, m), 7.61(1H. m), 7.75(2H, m), 8.04∼8.08(2H, m), 8.42(1H, m), 8.55(1H, m) | 766.97 | 766.33 |
| 1528 | δ = 1.72(6H, s), 2.88(8H, m), 6.58(4H, m), 6.76(4H, m), 6.83(2H, m), 7.02∼7.04(10H, m), 7.28(1H, m), 7.38∼7.41(2H, m), 7.48∼7.63(9H, m), 7.7∼7.77(4H, m), 7.87∼7.93(2H, m) | 909.16 | 908.41 |
| 1536 | δ = 2.88(8H, m), 6.58(4H, m), 6.76(4H, m), 6.83(2H, m), 7.02∼7.04(10H, m), 7.25(4H, m), 7.41(1H, m), 7.51∼7.52(4H, m), 7.58∼7.59(3H, m), 7.73∼7.75(3H, m), 7.92(1 H, m), 8(2H, m) | 843.06 | 842.37 |
| 1545 | δ = 2.88(8H, m), 6.58(4H, m), 6.76(4H, m), 6.83(2H, m), 7.02∼7.04(10H, m), 7.25(4H, m), 7.58∼7.59(6H, m), 7.73∼7.75(4H, m), 7.92(2H, m), 8(4H, m) | 893.12 | 892.38 |
| 1566 | δ = 2.88(8H, m), 6.58(4H, m), 6.76(4H, m), 6.83(2H, m), 7.02∼7.04(10H, m), 7.25(4H, m), 7.41(1H, m), 7.51∼7.55(6H, m), 7.61(1H, m), 7.75(2H, m), 8.04∼8.08(2H, m), 8.42(1H, m), 8.55(1H, m) | 843.06 | 842.37 |
| 1573 | δ = 2.88(8H, m), 6.58(4H, m), 6.76(4H, m), 6.83(2H, m), 7.02∼7.04(10H, m), 7.48(2H, m), 7.55∼7.61(7H, m), 7.7∼7.75(3H, m), 8.04∼8.08(4H, m), 8.42(2H, m), 8.55(2H, m) | 893.12 | 892.38 |
| 1586 | δ = 1.35(9H, s), 2.88(8H, m), 6.58(4H, m), 6.76(4H, m), 6.83(2H, m), 7.02∼7.04(10H, m), 7.37∼7.38(4H, m), 7.55(2H, m), 7.61 (1H. m), 7.75(2H, m), 8.04∼8.08(2H, m), 8.42(1H, m), 8.55(1H, m) | 823.07 | 822.40 |
| 1591 | δ = 1.72(6H, s), 2.88(8H, m), 6.58(4H, m), 6.76(4H, m), 6.83(2H, m), 7.02∼7.04(10H, m), 7.28(1H, m), 7.38(1H, m), 7.55(3H, m), 7.61∼7.63(2H, m), 7.75∼7.77(3H, m), 7.87∼7.93(2H, m), 8.04∼8.08(2H, m), 8.42(1H, m), 8.55(1H, m) | 883.13 | 882.40 |
| 1611 | δ = 1.35(9H, s), 1.72(6H, s), 2.88(8H, m), 6.58(4H, m), 6.76(4H, m), 6.83(2H, m), 7.02∼7.04(10H, m), 7.28(1H, m), 7.37∼7.38(5H, m), 7.55(1H, m), 7.63(1H, m), 7.75∼7.77(3H, m), 7.87∼7.93(2H, m) | 889.17 | 888.44 |
| 1615 | δ = 1.96(4H, m), 2.76(4H, m), 3.06(4H, m), 6.55(2H, m), 6.72(2H, m), 6.83(2H, m), 7.03∼7.07(6H, m), 7.55(4H, m), 7.61(2H, 7.75(2H, 8.04∼8.08(4H, 8.42(2H, 7.61 (2H, m), 7.75(2H, m), 8.04∼8.08(4H, m), 8.42(2H, m), 8.55(2H, m) | 692.89 | 692.32 |
| 1616 | δ = 1.96(4H, m), 2.76(4H, m), 3.06(4H, m), 6.55(2H, m), 6.72(2H, m), 6.83(2H, m), 7.03∼7.07(6H, m), 7.58∼7.59(6H, m), 7.73∼7.75(4H, m), 7.92(2H, m), 8(4H, m) | 692.89 | 692.32 |
| 1619 | δ = 1.96(4H, m), 2.76(4H, m), 3.06(4H, m), 6.55(2H, m), 6.72(2H, m), 6.83(2H, m), 7.03∼7.07(6H, m), 7.55∼7.61(6H, m), 7.73∼7.75(3H, m), 7.92(1H, m), 8∼8.08(4H, m), 8.42(1H, m), 8.55(1H, m) | 692.89 | 692.32 |
| 1622 | δ = 1.72(6H, s), 1.96(4H, m), 2.76(4H, m), 3.06(4H, m), 6.55(2H, m), 6.72(2H, m), 6.83(2H, m), 7.03∼7.07(6H, m), 7.28(1H, m), 7.38(1H, m), 7.55(3H, m), 7.61∼7.63(2H, m), 7.75∼7.77(3H, m), 7.87∼7.93(2H, m), 8.04∼8.08(2H, m), 8.42(1H, m), 8.55(1H, m) | 758.99 | 758.37 |
| 1635 | δ = 1.72(12H, s), 6.83(2H, m), 6.97(4H, m), 7.03(2H, m), 7.16∼7.21(12H, m), 7.28(2H, m), 7.38(2H, m), 7.55(2H, m), 7.63(2H, m), 7.75∼7.77(4H, m), 7.87∼7.93(4H, m) | 957.25 | 956.33 |
| 1644 | δ = 1.72(6H, s), 6.59(4H, m), 6.77(4H, m), 6.83∼6.92(10H, m), 7.03(2H, m), 7.28(1H, m), 7.38∼7.41(2H, m), 7.51∼7.55(5H, m), 7.63(1H, m), 7.75∼7.77(3H, m), 7.87∼7.93(2H, m) | 808.96 | 808.31 |
| 1654 | δ = 1.72(12H, s), 6.55(4H, m), 6.73(4H, m), 6.83(2H, m), 7.02∼7.05(10H, m), 7.58∼7.59(6H, m), 7.73∼7.75(4H, m), 7.92(2H, m), 8(4H, m) | 845.08 | 844.38 |
| 1666 | δ = 1.72(6H, s), 2.88(8H, m), 6.58(4H, m), 6.76(4H, m), 6.83(2H, m), 7.02∼7.04(10H, m), 7.28(1H, m), 7.38(1H, m), 7.55∼7.63(5H, m), 7.73∼7.77(4H, m), 7.87∼7.93(3H, m), 8(2H, m) | 883.13 | 882.40 |
| 1674 | δ = 1.96(4H, m), 2.76(4H, m), 3.06(4H, m), 6.55(2H, m), 6.62(2H, m), 6.72(2H, m), 7.05∼7.07(4H, m), 7.22(2H, m), 7.4∼7.41(4H, m), 7.51∼7.52(8H, m) | 592.77 | 592.29 |
| 1678 | δ = 3.81(4H, s), 6.51(4H, m), 6.62(2H, m), 6.69(4H, m), 6.98∼7.01(8H, m), 7.22(2H, m), 7.4∼7.41(4H, m), 7.51∼7.52(8H, m) | 688.86 | 688.29 |
| 1682 | δ = 6.62(2H, m), 6.97(4H, m), 7.16∼7.22(14H, m), 7.4∼7.41(4H, m), 7.51∼7.52(8H, m) | 724.93 | 724.20 |
| 1686 | δ = 1.72(12H, s), 6.55(4H, m), 6.62(2H, m), 6.73(4H, m), 7.02∼7.05(8H, m), 7.22(2H, m), 7.4∼7.41(4H, m), 7.51∼7.52(8H, m) | 744.96 | 744.35 |
| 1690 | δ = 3.2(6H, s), 6.35(4H, m), 6.41(4H, m), 6.52(4H, m), 6.59∼6.62(6H, m), 7.22(2H, m), 7.4∼7.411(4H, m), 7.51∼7.52(8H, m) | 718.88 | 718.31 |
| 1694 | δ = 3.2(6H, s), 6.35(4H, m), 6.41~6.45(6H, m), 6.52(4H, m), 6.59(4H, m), 7.39∼7.41(4H, m), 7.51∼7.52(8H, m), 7.91 (2H, m) | 718.88 | 718.31 |
| 1695 | δ = 1.72(12H, s), 6.45(2H, m), 6.55(4H, m), 6.73(4H, m), 7.02∼7.05(8H, m), 7.39∼7.41(4H, m), 7.51∼7.52(8H, m), 7.91 (2H, m) | 744.96 | 744.35 |
| 1696 | δ = 0.66(12H, s), 6.45(2H, m), 6.73(8H, m), 7.21 (4H, m), 7.3(4H, m), 7.39∼7.41(4H, m), 7.51∼7.52(8H, m), 7.91(2H, m) | 777.11 | 776.30 |
| 1697 | δ = 6.45(2H, m), 6.59(4H, m), 6.77(4H, m), 6.89∼6.92(8H, m), 7.39∼7.41(4H, m), 7.51∼7.52(8H, m), 7.91(2H, m) | 692.80 | 692.25 |
| 1698 | δ = 6.45(2H, m), 6.97(4H, m), 7.16∼7.21(12H, m), 7.39∼7.41(4H, m), 7.51∼7.52(8H, m), 7.91(2H, m) | 724.93 | 724.20 |
| 1699 | δ = 1.51(8H, m), 2.09(8H, m), 6.55(4H, m), 6.73(4H, m), 6.83(2H, m), 7.02∼7.05(10H, m), 7.58∼7.59(6H, m), 7.73∼7.75(4H, m), 7.92(2H, m), 8(4H, m) | 897.15 | 896.46 |
| 1704 | δ = 1.3(8H, m), 1.45(8H, m), 6.73(8H, m), 6.83(2H, m), 7.03(2H, m), 7.21(4H, m), 7.3(4H, m), 7.55(4H, m), 7.61(2H, m), 7.75(2H, m), 8.04∼8.08(4H, m), 8.42(2H, m), 8.55(2H, m) | 929.30 | 928.37 |
| 1708 | δ = 6.38(8H, m), 6.56(8H, m), 6.63(4H, m), 6.81∼6.83(4H, m), 7.03(2H, m), 7.2(4H, m), 7.58∼7.59(6H, m), 7.73∼7.75(4H, m), 7.92(2H, m), 8(4H, m) | 943.14 | 942.37 |
| 1711 | δ = 6.38(8H, m), 6.56(8H, m), 6.83(2H, m), 6.98∼7.03(4H, m), 7.38(2H, m), 7.53∼7.59(12H, m), 7.73∼7.75(4H, m), 7.92(2H, m), 8∼8.07(8H, m) | 1043.26 | 1042.40 |
| 1714 | δ = 6.38(8H, m), 6.56(8H, m), 6.83(2H, m), 7.03(2H, m), 7.36(2H, m), 7.49∼7.5(4H, m), 7.58∼7.59(6H, m), 7.73∼7.77(8H, m), 7.84∼7.92(6H, m), 8(4H, m) | 1043.26 | 1042.40 |
| 1721 | δ = 6.63(4H, m), 6.81∼6.83(6H, m), 6.99∼7.05(10H, m), 7.25(4H, m), 7.75(2H, m), 7.82∼7.93(10H, m), 8.12(4H, m), 8.93(4H, m) | 913.11 | 912.35 |
| 1737 | δ = 3.81(2H, m), 6.51(2H, m), 6.69(2H, m), 6.83(1H, m), 6.98∼7.03(5H, m), 7.39(2H, m), 7.58∼7.59(6H, m), 7.73∼7.75(3H, m), 7.91∼7.92(4H, m), 8(4H, m) | 609.76 | 609.25 |
| 1740 | δ = 1.96(6H, m), 2.76(6H, m), 3.06(6H, m), 6.55(3H, m), 6.72(3H, m), 6.83(1H, m), 6.87(2H, s), 7.03∼7.07(7H, m), 7.58∼7.59(6H, m), 7.73∼7.75(3H, m), 7.92(2H, m), 8(4H, m) | 824.06 | 823.39 |
| 1741 | δ = 1.96(8H, m), 2.76(8H, m), 3.06(8H, m), 6.55(4H, m), 6.72(4H, m), 6.87(4H, s), 7.05∼7.07(8H, m), 7.58∼7.59(6H, m), 7.73(2H, m), 7.92(2H, m), 8(4H, m) | 955.24 | 954.47 |
| 1743 | δ = 1.72(6H, s), 1.96(4H, m), 2.76(4H, m), 3.06(4H, m), 6.55(4H, m), 6.72∼6.73(4H, m), 6.83(1H, m), 6.87(2H, s), 7.02∼7.07(9H, m), 7.58∼7.59(6H, m), 7.73∼7.75(3H, m), 7.92(2H, m), 8(4H, m) | 900.16 | 899.42 |

### [Example 1] Manufacture of OLED's by using the organic electroluminescent compounds of the invention

An OLED device was manufactured by using the electroluminescent material according to the invention.

First, a transparent electrode ITO thin film (15 Ω/□) (2) prepared from glass for OLED (1) (manufactured by Samsung-Corning) was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA) (of which the structure is shown below) was placed in a cell of the vacuum vapor-deposit device, which was then ventilated up to 10⁻⁶ torr of vacuum in the chamber. Electric current was applied to the cell to evaporate 2-TNATA, thereby providing vapor-deposit of a hole injecting layer (3) having 60 nm thickness on the ITO substrate.

Then, to another cell of the vacuum vapor-deposit device, charged was N,N'-bis(α-naphthyl)-N,N'-diphenyl-4,4'-diamine (NPB) (of which the structure is shown below), and electric current was applied to the cell to evaporate NPB, thereby providing vapor-deposit of a hole transport layer (4) having 20 nm thickness on the hole injecting layer.

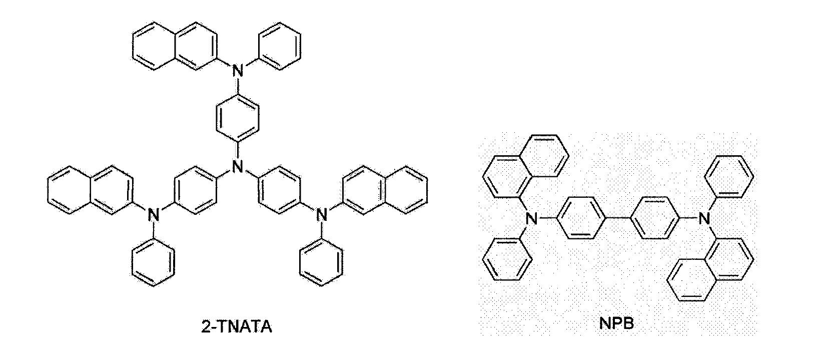

After forming the hole injecting layer and the hole transport layer, an electroluminescent layer was vapor-deposited as follows. To one cell of a vacuum vapor-deposit device, charged was H-78 (of which the structure is shown below) as a host, and a compound according to the invention (Compound 513) was charged to another cell as a dopant. The two substances were evaporated at different rates to give doping at 2 to 5% by weight on the basis of the host, thereby forming a vapor-deposit of an electroluminescent layer (5) with a thickness of 30 nm on the hole transport layer.

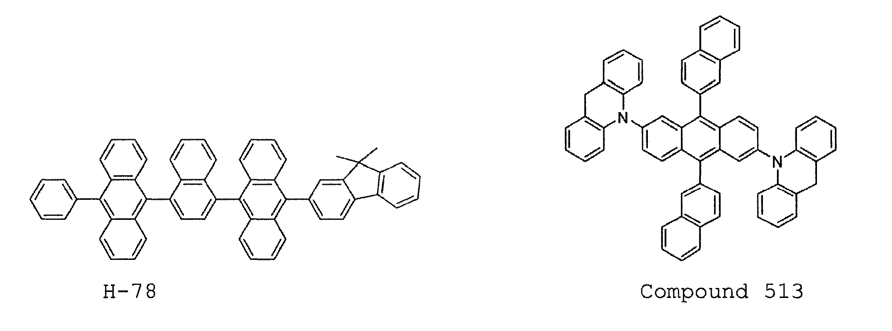

Then, tris(8-hydroxyquinoline)aluminum (III) (Alq) (of which the structure is shown below) was vapor-deposited as an electron transport layer (6) with a thickness of 20 nm, and lithium quinolate (Liq) (of which the structure shown below) was vapor-deposited as an electron injecting layer (7) with a thickness of 1 to 2 nm. Thereafter, an Al cathode (8) was vapor-deposited with a thickness of 150 nm by using another vacuum vapor-deposit device to manufacture an OLED.

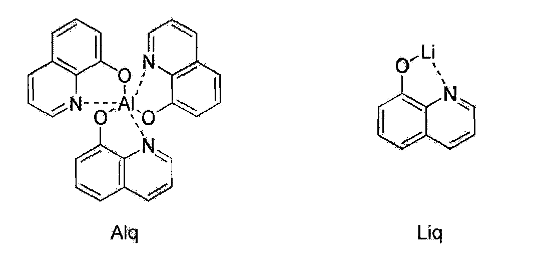

Each material employed for manufacturing an OLED was used as the electroluminescent material after being purified via vacuum sublimation at 10⁻⁶ torr.

### [Comparative Example 1] Manufacture of an OLED by using conventional electroluminescent material

After forming a hole injecting layer and hole transport layer according to the same procedure as described in Example 1, tris(8-hydroxyquinoline)-aluminum (III) (Alq) was charged to another cell of said vacuum vapor-deposit device as electroluminescent host material, while Coumarin 545T (C545T) (of which the structure is shown below) was charged to still another cell. The two substances were evaporated at different rates to carry out doping, thereby vapor-depositing an electroluminescent layer with a thickness of 30 nm on the hole transport layer. The doping concentration preferably is from 1 to 3 mol% on the basis of Alq.

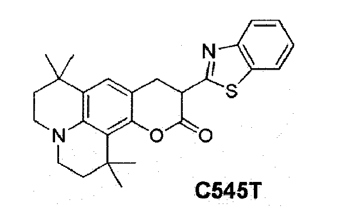

Then, an electron transport layer and an electron injecting layer were vapor-deposited according to the same procedure as in Example 1, and Al cathode was vapor-deposited by using another vacuum vapor-deposit device with a thickness of 150 nm, to manufacture an OLED.

### [Example 2] Electroluminescent properties of OLED's manufactured

The luminous efficiencies of the OLED's comprising the organic electroluminescent compound according to the present invention (Example 1) or conventional EL compounds (Comparative Example 1) were measured at 5,000 cd/m², respectively, and the results are shown in Table 2.

**Table 2**

| | | | | | |
|---|---|---|---|---|---|
| 2 | H-30 | 105 | 3 | 19.5 | Green |
| 3 | H-33 | 367 | 3 | 18.9 | Green |
| 4 | H-78 | 513 | 3 | 19.4 | Green |
| 5 | H-49 | 656 | 3 | 20.3 | Green |
| 6 | H-73 | 889 | 3 | 20.1 | Green |
| 7 | H-79 | 946 | 3 | 19.8 | Green |
| 8 | H-80 | 1066 | 3 | 21.5 | Green |
| 9 | H-81 | 1214 | 3 | 21.3 | Green |
| 10 | H-82 | 1314 | 3 | 19.8 | Green |
| 11 | H-84 | 1342 | 3 | 18.2 | Green |
| 12 | H-86 | 1490 | 3 | 20.9 | Green |
| Comp.1 | Alq | Compound C545T | 1 | 10.3 | Green |

As can be seen from Table 2, the green electroluminescent device wherein the material according the present invention was applied showed more than twice of luminous efficiency as compared to the device employing Alq:C545T as conventional material (Comparative Example 1).

Accordingly, the organic electroluminescent compounds according to the present invention can be used as green electroluminescent material of high efficiency. Moreover, the device, to which the dopant material according to the invention was applied, showed noticeable improvement in view of color purity. The improvement in both color purity and luminous efficiency proves that the materials of the present invention have excellent properties.

## Claims

1. An organic electroluminescent compound represented by Chemical Formula (1): 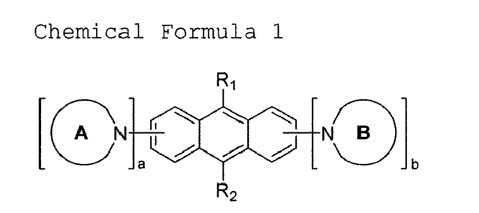 wherein, R₁ and R₂ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or a substituent selected from the following structures: 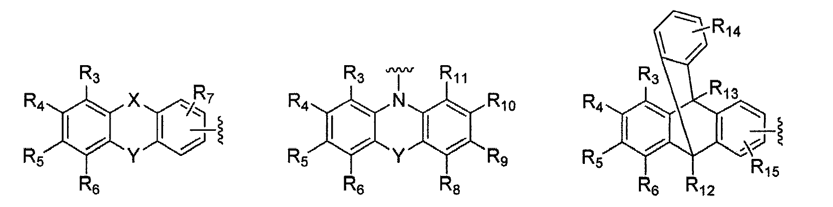 wherein, R₃ through R₁₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₃ through R₁₅ may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, or - (CR₂₁R₂₂)ₙ-, -N(R₂₃)-, -S-, -O-, -Si(R₂₄)(R₂₅)-, -P(R₂₆)-, -C(=O)-, -B(R₂₇)-, -In(R₂₈)-, -Se-, -Ge(R₂₉)(R₃₀)-, -Sn(R₃₁)(R₃₂)-, - Ga(R₃₃)- or -(R₃₄) C=C (R₃₅) -;
R₂₁ through R₃₅ independently represent hydrogen, halogen, (C1-C60)alkyl, (C6-C60)aryl, (C3-C60)heteroaryl, 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₂₁ and R₂₂, R₂₄ and R₂₅, R₂₉ and R₃₀, R₃₁ and R₃₂, or R₃₄ and R₃₅ may be linked via (C3-C60)alkylene or (C3-C60)alkenylene with or without a fused ring to form an alicyclic ring, or a monocyclic or polycyclic aromatic ring;
ring A and ring B independently represent a saturated 5-or 6-membered heterocyclic amino group, which may further contain one or more heteroatom(s) selected from NR₃₆, O and S, or a substituent selected from the following structures: 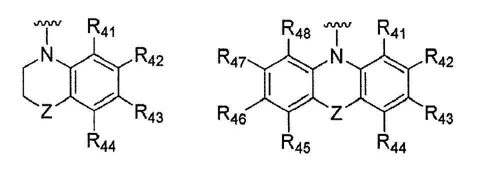 R₃₆ represents hydrogen, halogen, (C1-C60)alkyl, (C6-C60)aryl, (C3-C60)heteroaryl, (C3-C60)cycloalkyl, adamantyl, (C7-C60)bicycloalkyl or (C6-C60)ar(C1-C60)alkyl;
R₄₁ through R₄₈ independently represent hydrogen, halogen, (C1-C60)alkyl, (C6-C60)aryl, (C3-C60)heteroaryl, 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₄₁ through R₄₈ may be linked to an adjacent substituent via (C3-C60)alkylene or (C3-C60)alkenylene with or without a fused ring to form an alicyclic ring, or a monocyclic or polycyclic aromatic ring;
Z represents a chemical bond, or -(CR₅₁R₅₂)ₘ-, -N(R₅₃)-, - S-, -O-, -Si(R₅₄)(R₅₅)-, -P(R₅₆)-, -C(=O)-, -B(R₅₇)-, -In(R₅₈)-, - Se-, -Ge(R₅₉)(R₆₀)-, -Sn(R₆₁)(R₆₂)-, -Ga(R₆₃)- or -(R₆₄)C=C(R₆₅)-;
wherein, R₅₁ through R₆₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₅₁ and R₅₂, R₅₄ and R₅₅, R₅₉ and R₆₀, R₆₁ and R₆₂, or R₆₄ and R₆₅ may be linked via (C3-C60)alkylene or (C3-C60)alkenylene with or without a fused ring to form an alicyclic ring, or a monocyclic or polycyclic aromatic ring;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₁, R₂, R₃ through R₁₅, R₂₁ through R₃₆, R₄₁ through R₄₈, and R₅₁ through R₆₅ may be further substituted by one or more substituent(s) selected from halogen, (C1-C60)alkyl with or without halogen substituent(s), (C6-C60)aryl, (C3-C60)heteroaryl with or without (C6-C60)aryl substituent(s), 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
m and n independently represent an integer from 1 to 4; and
a and b independently represent an integer from 0 to 4, provided that (a + b) is an integer from 1 to 4.

2. The organic electroluminescent compound according to claim 1, wherein ring A and ring B are independently selected from the following structures.

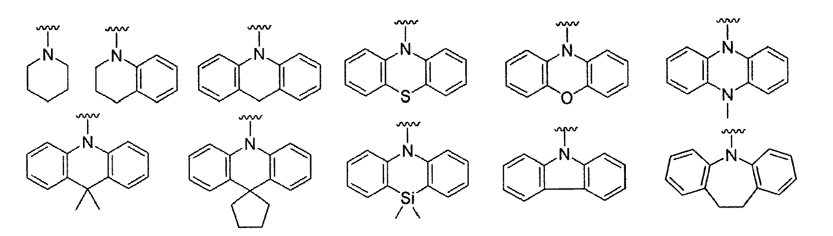

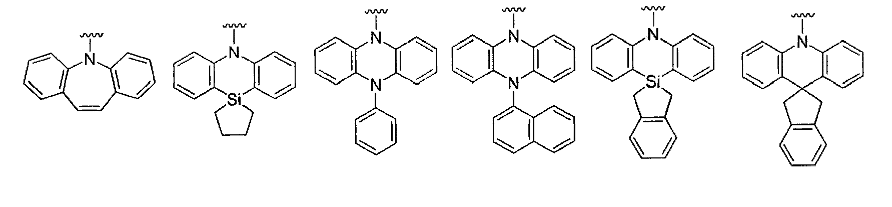

3. An organic electroluminescent device which is comprised of a first electrode; a second electrode; and at least one organic layer(s) interposed between the first electrode and the second electrode; wherein the organic layer comprises an organic electroluminescent compound represented by Chemical Formula (1): 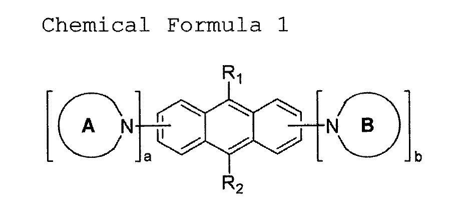 wherein, R₁ and R₂ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or a substituent selected from the following structures: 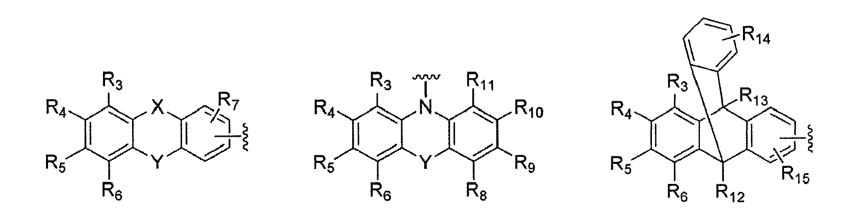 wherein, R₃ through R₁₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₃ through R₁₅ may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, or - (CR₂₁R₂₂)ₙ-, -N(R₂₃)-, -S-, -O-, -Si(R₂₄)(R₂₅)-, -P(R₂₆)**-,** -C(=O)-, -B(R₂₇)-, -In(R₂₈)-, -Se-, -Ge(R₂₉)(R₃₀)-, -Sn(R₃₁)(R₃₂)-, - Ga(R₃₃)- or -(R₃₄) C=C (R₃₅)-;
R₂₁ through R₃₅ independently represent hydrogen, halogen, (C1-C60)alkyl, (C6-C60)aryl, (C3-C60)heteroaryl, 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₂₁ and R₂₂, R₂₄ and R₂₅, R₂₉ and R₃₀, R₃₁ and R₃₂, or R₃₄ and R₃₅ may be linked via (C3-C60)alkylene or (C3-C60)alkenylene with or without a fused ring to form an alicyclic ring, or a monocyclic or polycyclic aromatic ring;
ring A and ring B independently represent a saturated 5-or 6-membered heterocyclic amino group, which may further contain one or more heteroatom(s) selected from NR₃₆, O and S, or a substituent selected from the following structures: 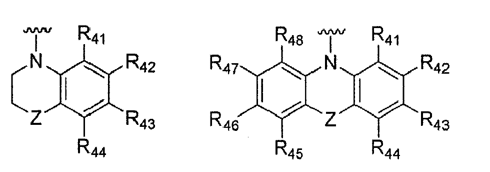 R₃₆ represents hydrogen, halogen, (C1-C60)alkyl, (C6-C60)aryl, (C3-C60)heteroaryl, (C3-C60)cycloalkyl, adamantyl, (C7-C60)bicycloalkyl or (C6-C60)ar(C1-C60)alkyl;
R₄₁ through R₄₈ independently represent hydrogen, halogen, (C1-C60)alkyl, (C6-C60)aryl, (C3-C60)heteroaryl, 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₄₁ through R₄₈ may be linked to an adjacent substituent via (C3-C60)alkylene or (C3-C60)alkenylene with or without a fused ring to form an alicyclic ring, or a monocyclic or polycyclic aromatic ring;
Z represents a chemical bond, or -(CR₅₁R₅₂)ₘ-, -N(R₅₃)-, - S-, -O-, -Si(R₅₄)(R₅₅)-, -P(R₅₆)-, -C(=O)-, -B(R₅₇)-, -In(R₅₈)-, - Se-, -Ge(R₅₉)(R₆₀)-, -Sn(R₆₁)(R₆₂)-, -Ga(R₆₃)- or -(R₆₄)C=C(R₆₅)-;
wherein, R₅₁ through R₆₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60) alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₅₁ and R₅₂, R₅₄ and R₅₅, R₅₉ and R₆₀, R₆₁ and R₆₂, or R₆₄ and R₆₅ may be linked via (C3-C60)alkylene or (C3-C60)alkenylene with or without a fused ring to form an alicyclic ring, or a monocyclic or polycyclic aromatic ring;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₁, R₂, R₃ through R₁₅, R₂₁ through R₃₆, R₄₁ through R₄₈, and R₅₁ through R₆₅ may be further substituted by one or more substituent(s) selected from halogen, (C1-C60)alkyl with or without halogen substituent(s), (C6-C60)aryl, (C3-C60)heteroaryl with or without (C6-C60)aryl substituent(s), 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
m and n independently represent an integer from 1 to 4; and
a and b independently represent an integer from 0 to 4, provided that (a + b) is an integer from 1 to 4 and one or more host(s) selected from the compounds represented by Chemical Formula (6) or (7):
Chemical Formula 6 (Ar₁)_{c}-L₁₁-(Ar₂)_{d}
Chemical Formula 7 (Ar₃)ₑ-L₁₂-(Ar₄)_{f}
wherein, L₁₁ represents (C6-C60)arylene or (C4-C60)heteroarylene;
L₁₂ represents anthracenylene;
Ar₁ through Ar₄ are independently selected from hydrogen, (C1-C60)alkyl, (C1-C60)alkoxy, halogen, (C4-C60)heteroaryl, (C5-C60)cycloalkyl and (C6-C60)aryl; the cycloalkyl, aryl or heteroaryl of Ar₁ through Ar₄ may be further substituted by one or more substituent(s) selected from a group consisting of (C6-C60)aryl or (C4-C60)heteroaryl with or without one or more substituent(s) selected from a group consisting of (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; and
c, d, e and f independently represent an integer from 0 to 4.

4. The organic electroluminescent device according to claim 3, wherein the organic layer comprises one or more compound(s) selected from a group consisting of arylamine compounds and styrylarylamine compounds.

5. The organic electroluminescent device according to claim 3, wherein the organic layer comprises one or more metal(s) selected from a group consisting of organic metals of Group 1, Group 2, 4^{th} period and 5^{th} period transition metals, lanthanide metals and d-transition elements from the Periodic Table of Elements.

6. The organic electroluminescent device according to claim 3, which is an organic electroluminescent display comprising a compound having the electroluminescent peak with wavelength of not more than 500 nm, or a compound having the electroluminescent peak with wavelength of not less than 560 nm.

7. The organic electroluminescent device according to claim 3, wherein the organic layer comprises a charge generating layer as well as the electroluminescent layer.

8. The organic electroluminescent device according to claim 3, wherein a mixed region of reductive dopant and organic substance, or a mixed region of oxidative dopant and organic substance is placed on the inner surface of one or both electrode(s) among the pair of electrodes.

9. An organic solar cell which comprises an organic electroluminescent compound represented by Chemical Formula (1): 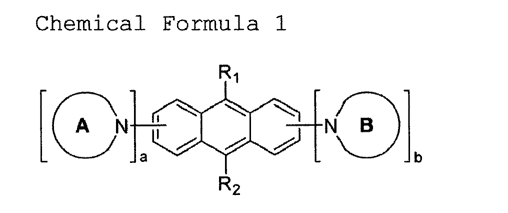 wherein, R₁ and R₂ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or a substituent selected from the following structures: 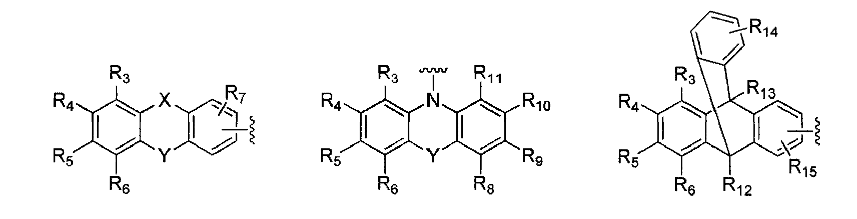 wherein, R₃ through R₁₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₃ through R₁₅ may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, or - (CR₂₁R₂₂)ₙ-, -N(R₂₃)-, -S-, -O-, -Si(R₂₄)(R₂₅)-, -P(R₂₆)-, -C(=O)-, -B(R₂₇)-, -In(R₂₈)-, -Se-, -Ge(R₂₉)(R₃₀)-, -Sn(R₃₁)(R₃₂)-, Ga(R₃₃)- or -(R₃₄)C=C(R₃₅)-;
R₂₁ through R₃₅ independently represent hydrogen, halogen, (C1-C60)alkyl, (C6-C60)aryl, (C3-C60)heteroaryl, 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₂₁ and R₂₂, R₂₄ and R₂₅, R₂₉ and R₃₀, R₃₁ and R₃₂, or R₃₄ and R₃₅ may be linked via (C3-C60)alkylene or (C3-C60)alkenylene with or without a fused ring to form an alicyclic ring, or a monocyclic or polycyclic aromatic ring;
ring A and ring B independently represent a saturated 5-or 6-membered heterocyclic amino group, which may further contain one or more heteroatom(s) selected from NR₃₆, O and S, or a substituent selected from the following structures: 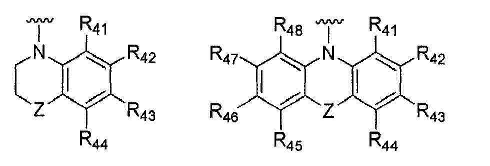 R₃₆ represents hydrogen, halogen, (C1-C60)alkyl, (C6-C60)aryl, (C3-C60)heteroaryl, (C3-C60)cycloalkyl, adamantyl, (C7-C60)bicycloalkyl or (C6-C60)ar(C1-C60)alkyl;
R₄₁ through R₄₈ independently represent hydrogen, halogen, (C1-C60)alkyl, (C6-C60)aryl, (C3-C60)heteroaryl, 5- or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₄₁ through R₄₈ may be linked to an adjacent substituent via (C3-C60)alkylene or (C3-C60)alkenylene with or without a fused ring to form an alicyclic ring, or a monocyclic or polycyclic aromatic ring;
Z represents a chemical bond, or -(CR₅₁R₅₂)ₘ-, -N(R₅₃)-, - S-, -O-, -Si(R₅₄)(R₅₅)-, -P(R₅₆)-, -C(=O)-, -B(R₅₇)-, -In(R₅₈)-, - Se-, -Ge(R₅₉)(R₆₀)-, -Sn(R₆₁)(R₆₂)-, -Ga(R₆₃)- or -(R₆₄)C=C(R₆₅)-;
wherein, R₅₁ through R₆₅ independently represent hydrogen, halogen, (C1-C60)alkyl, (C6-C60)aryl, (C3-C60)heteroaryl, 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or R₅₁ and R₅₂, R₅₄ and R₅₅, R₅₉ and R₆₀, R₆₁ and R₆₂, or R₆₄ and R₆₅ may be linked via (C3-C60)alkylene or (C3-C60)alkenylene with or without a fused ring to form an alicyclic ring, or a monocyclic or polycyclic aromatic ring;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₁, R₂, R₃ through R₁₅, R₂₁ through R₃₆, R₄₁ through R₄₈, and R₅₁ through R₆₅ may be further substituted by one or more substituent(s) selected from halogen, (C1-C60)alkyl with or without halogen substituent(s), (C6-C60)aryl, (C3-C60)heteroaryl with or without (C6-C60)aryl substituent(s), 5-or 6-membered heterocycloalkyl containing one or more heteroatom(s) selected from N, O, S and Si, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, (C1-C60)alkylthio, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
m and n independently represent an integer from 1 to 4; and
a and b independently represent an integer from 0 to 4, provided that (a + b) is an integer from 1 to 4.
